# EUROPEAN PATENT APPLICATION

(11) **EP 4 421 184 A2**
(43) Date of publication of application: **28.08.2024**
(21) Application number: 24173311.2
(22) Date of filing: 19.12.2017
(51) Int. Cl.: C12Q 1/6806

(54) **METHODS AND REAGENTS FOR MOLECULAR BARCODING**

(30) Priority: 23.12.2016 GB 201622219
(62) Divisional of application: 17817856.2
(71) Applicant: CS Genetics Limited, Cambridge, Cambridgeshire CB2 1PH (GB)
(72) Inventor: EDELMAN, Lucas Brandon, Cambridge, CB3 9LW (GB)
(74) Representative: Boult Wade Tennant LLP

(57) **Abstract**

Methods and reagents for preparing nucleic acid samples for sequencing are provided. The samples include formalin-fixed paraffin-embedded (FFPE) samples. The methods comprise contacting a nucleic acid sample with a multimeric barcoding reagent comprising barcode regions linked together and appending barcode sequences to nucleic acid sequences of a target nucleic acid molecule. Methods are also provided that additionally use in-vitro transposition, coupling sequences and/or primer-extension to append barcode sequences to nucleic acid sequences of a target nucleic acid molecule.

## Description

### TECHNICAL FIELD

The present invention relates to molecular barcoding. Provided are multimeric barcoding reagents, libraries of molecular barcoding reagents and kits comprising multimeric barcoding reagents. There are also provided methods relating to the multimeric barcoding reagents and uses of the multimeric barcoding reagents.

### BACKGROUND

'Molecular barcoding' was developed to address problems generated by raw error rates intrinsic to DNA sequence machines (synthetic accuracy), and also problems related to counting individual nucleic acid molecules within a sample (molecular counting).

Molecular barcoding generally involves attaching (for example, by ligation or by primer-extension) a unique nucleic acid label (a 'barcode') to several single target molecules (DNA or RNA) in a solution containing a large number of such molecules. These labelled molecules are then sequenced, which for each reveals both the sequence of the molecular barcode, and at least part of the sequence of the labelled target molecule itself.

This barcoding is typically used towards two different ends. First, it can be used to enable 'redundant sequencing'. For example, imagine a nucleic acid sample containing 1000 copies of a particular gene in a DNA sample; 999 of the copies hold sequences identical to each other, but a single copy has a particular single-nucleotide mutation. Without barcoding, the sequencer will be unable to detect this mutated copy, since the sequencer makes random errors at a higher rate than 1:1000 - i.e. the mutation is so rare in the population of sequenced molecules that it falls below the sequencer's intrinsic background noise threshold.

However, if the 1000 copies have each been labelled with a unique molecular barcode, and each individual labelled molecule is sequenced several times by the sequencing machine (redundant sequencing), you would observe that every time (or, at least 99% of the time, equivalent to the raw accuracy of the sequencer) that the labelled mutated molecule was redundantly sequenced (i.e, every time the target gene sequence was observed to be labelled with that one particular unique barcode that was attached to the mutated starting molecule), that the same apparent mutation would in fact be observed. By contrast, that particular mutation would only be observed approximately 1% of the time (the raw error rate of the sequencer) when the labelled but non-mutated gene copies were redundantly sequenced, as per their respective alternative barcodes.

The barcode thus serves to identify individual input molecules across all their respective multiple copies within the sequencing reaction, allowing a sequence-detection algorithm to specifically focus on their respective reads within a sequencing dataset, and thus avoiding the large amount of stochastic sequence noise (in the form of sequence errors) that is present across the remainder of the dataset. This thus enables 'synthetic accuracy', through redundant sequencing, which is potentially much higher than the raw accuracy of the sequencer itself.

Barcoding can also be used to enable digital 'molecular counting' of input DNA or RNA molecules. In this process, a large number of unique barcodes are attached to input molecules, for example, cDNA copies that have been made from a particular mRNA species. Each input cDNA molecule is labelled (for example, by primer extension) with a single, unique barcode. The molecules are then sequenced, which, as with redundant sequencing, reveals the unique barcode and at least part of each associated labelled input molecule; these molecules are then also each sequenced more than once.

Instead of using this redundant sequencing to reduce sequencing errors, in molecular counting it is used to digitally quantify how many individual molecules of the given target molecule (cDNA in this case) were present in the original sample, by simply counting the total number of unique barcodes that were sequenced and found to be associated with the particular target. Barcode-directed redundant sequencing in this way reduces the chance that any input molecule is stochastically left unsequenced by the sequencing reaction (since each labelled molecule on average is sequenced several times), whilst retaining an accurate measure of input quantity (since redundantly sequenced starting molecules are only counted once, as discriminated by repeated copies of their unique barcode).

Examples of the use of molecular barcodes are provided in US 8728766, US 8685678, US 8722368, Kinde et al., 2011 (PNAS, 108, 23, 9530-9535) and US 20140227705 A1.

A 'synthetic long read' is generated when a long, contiguous sequence of DNA (longer than the readlength attainable on a DNA sequencer) is converted into two or more shorter 'sub-sequences' that are short enough to be read by a DNA sequencer, and which are somehow labelled such that it can be deduced (after sequencing) that the sub-sequences were generated from the same original long DNA sequence. For example, if you want to sequence a particular human gene which is 1000 nucleotides long, but do so with a short-read DNA sequencer with a readlength of 100 nucleotides, you could separate the long sequence into 10 different sub-sequences of 100 nucleotide length, then label each of these 10 sub-sequences with a synthetic, informative 'label' DNA sequence that identifies each of the 10 sub-sequences as coming from the same original 1000 nucleotide DNA molecule, then perform high-throughput DNA sequencing with these 10 resulting DNA molecules, and thus (for each of the 10 resulting DNA molecules) attain both the 100 nucleotide sub-sequence, and the associated identifying DNA label. With this high-throughput DNA data an algorithm can be used which detects these identifying labels and uses them to associate the 10 different 100-nucleotide subsequences with each other as a collective sub-sequence 'grouping', and therewith estimate that the 10 sub-sequences came from a longer, 1000-nucleotide gene, and therewith estimate the total 1000-nucleotide long genetic sequence by 'stitching' the 10 sub-sequences together in silico into a single 1000-nucleotide long gene.

At least two general synthetic long read technologies have been described in the literature: a partitioning-based approach which is described in US 20130079231 A1 and US 2014378345 A1; and a barcode-copying approach which is described in Casbon et al., 2013 (Nucleic Acids Research, 2013, 41, 10, e112), US 8679756 and US 8563274.

'Spatial sequencing' is considered to be the sequencing of nucleic acids with the inclusion of some information about where each sequenced nucleic acid is located within a particular space (for example, within a particular sample, or within a particular cell). However, very few spatial sequencing methods are known. The main known technology is the fluorescent in situ RNA sequencing (FISSEQ) technique. In FISSEQ a sample of cells are cross-linked, and while the cells are still intact, RNA is reverse transcribed into cDNA, and amplified whilst still in the crosslinked cells. Then, each amplified cDNA molecule is sequenced optically whilst still in the cells, with a high-powered and sensitive optical detection system. This method is described in Lee et al., 2014 (Science, 343, 6177, 1360-1363).

The invention addresses two main types of problem in the sequencing field: 1) specific analytic limitations of DNA sequencing machines; and 2) biophysical challenges associated with common types of experimental DNA samples.

Current high-throughput DNA-sequencing machines are powerful platforms used to analyse large amounts of genetic material (from thousands to billions of DNA molecules) and function as systems for both basic research and applied medical applications. However, all current DNA sequencing machines are subject to certain analytic limitations which constrain the scientific and medical applications in which they can be effectively used. The chief such limitations include finite raw readlengths and finite raw accuracy, both of which are described below.

With regard to finite raw readlengths, each DNA sequencing platform is characterised by a typical 'readlength' that it can attain, which is the 'length' in nucleotides of DNA that it can 'read' of each sequenced molecule. For most sequencing machines, this ranges from 100 to ~500 nucleotides.

With regard to finite raw accuracy, each sequencing platform is also characterised by an attainable 'raw accuracy', typically defined as the likelihood that each given nucleotide it sequences has been determined correctly. Typical raw accuracy for the most popular sequencing platforms range between 98 and 99.5%. The related quantity, the 'raw error' rate, is essentially the converse of raw accuracy, and is the per-nucleotide likelihood that the sequencer randomly reports an incorrect nucleotide in a particular sequenced DNA molecule.

In addition, certain common experimental DNA samples pose biophysical challenges for sequencing. These challenges arise from the unique (and troublesome) molecular state of DNA in these samples, which makes it difficult to sequence them or to extract important pieces of genetic information therefrom, irrespective of the sequencing machine employed. For example, Formalin-Fixed Paraffin-Embedded (FFPE) samples are the standard experimental tool for performing molecular pathology from human biopsy specimens. However, the process of creating an FFPE sample - in which the biopsy specimen is fixed (crosslinked and kept physically together and stable at the molecular level) by a harsh chemical, and then embedded in a wax - creates significant damage to the DNA and RNA contained therein. DNA and RNA from FFPE samples is thus heavily fragmented (generally into small fragments between 50 and 200 nucleotides), and also includes sporadic damage to individual nucleotides which makes it essentially impossible to amplify or isolate long, contiguous sequences.

### DESCRIPTION

The invention provides methods of preparing formalin-fixed paraffin-embedded (FFPE) samples for sequencing by appending barcode sequences from multimeric barcoding reagents to fragments of target nucleic acid molecules comprised within FFPE samples. The methods take advantage of the fact that, though the nucleic acids within FFPE samples are fragmented, the fragments of each molecule remain spatially `co-localised' (that is, two fragments of DNA which, for example, originate from the same originally region of chromosomal DNA, will be retained in close physical proximity to each other within the FFPE sample, due to the crosslinking and embedding process). In the methods, a single multimeric barcoding reagent may be used to append barcode sequences to only those fragments that are derived from a single target nucleic acid molecule. The methods may be performed 'in situ' within intact or semi-intact FFPE specimens. Once barcode sequences have been appended using the methods of the invention, sequencing of the resulting barcoded target nucleic acid molecules may be used to provide linked reads/synthetic long reads.

The invention provides a method of preparing a nucleic acid sample for sequencing, wherein the nucleic acid sample comprises a formalin-fixed paraffin-embedded (FFPE) sample, and wherein the method comprises the steps of: (a) contacting the nucleic acid sample with a multimeric barcoding reagent comprising first and second barcode regions linked together, wherein each barcode region comprises a nucleic acid sequence, and (b) appending barcode sequences to each of the nucleic acid sequences of first and second fragments of a target nucleic acid molecule to produce first and second different barcoded target nucleic acid molecules, wherein the first barcoded target nucleic acid molecule comprises the nucleic acid sequence of the first barcode region and the second barcoded target nucleic acid molecule comprises the nucleic acid sequence of the second barcode region.

In the methods, the formalin crosslinks of the FFPE sample may remain fully or partially intact during steps (a) and/or (b).

The first and second fragments of the target nucleic acid molecule may be derived from a single nucleic acid molecule. The first and second fragments of the target nucleic acid molecule may be co-localised in the FFPE sample.

The multimeric barcoding reagent may comprise first and second barcode molecules linked together, and wherein each of the barcode molecules comprises a nucleic acid sequence comprising a barcode region.

The multimeric barcoding reagent may comprise first and second barcoded oligonucleotides, wherein the first barcoded oligonucleotide comprises, optionally in the 5' to 3' direction, a barcode region, and a target region capable of annealing or ligating to the first fragment of the target nucleic acid, and wherein the second barcoded oligonucleotide comprises, optionally in the 5' to 3' direction, a barcode region, and a target region capable of annealing or ligating to the second fragment of the target nucleic acid.

The multimeric barcoding reagent may comprise: (i) first and second barcode molecules linked together, wherein each of the barcode molecules comprises a nucleic acid sequence comprising a barcode region; and (ii) first and second barcoded oligonucleotides, wherein the first barcoded oligonucleotide comprises, optionally in the 5' to 3' direction, a barcode region, and a target region capable of annealing or ligating to the first fragment of the target nucleic acid, and wherein the second barcoded oligonucleotide comprises, optionally in the 5' to 3' direction, a barcode region, and a target region capable of annealing or ligating to the second fragment of the target nucleic acid.

The method may comprise the steps of: (a) contacting the nucleic acid sample with a multimeric barcoding reagent as defined herein; (b) ligating the target region of the first barcoded oligonucleotide to the first fragment of the target nucleic acid molecule to produce a first barcoded target nucleic acid molecule, and ligating the target region of the second barcoded oligonucleotide to the second fragment of the target nucleic acid to produce a second barcoded target nucleic acid molecule, wherein each of the barcoded target nucleic acid molecules comprises at least one nucleotide synthesised from the target nucleic acid as a template.

The method may comprise the steps of: (a) contacting the nucleic acid sample with a multimeric barcoding reagent as defined herein; and (b) annealing the target region of the first barcoded oligonucleotide to the first fragment of the target nucleic acid, and annealing the target region of the second barcoded oligonucleotide to the second fragment of the target nucleic acid, and (c) extending the first and second barcoded oligonucleotides to produce first and second different barcoded target nucleic acid molecules, wherein each of the barcoded target nucleic acid molecules comprises at least one nucleotide synthesised from the target nucleic acid as a template.

The method may comprise the steps of: (a) contacting the sample with a multimeric barcoding reagent comprising first and second barcode molecules linked together, wherein each of the barcode molecules comprises a nucleic acid sequence comprising a barcode region and an adapter region; (b) appending a coupling sequence to first and second fragments of the target nucleic acid molecule; (c) annealing the coupling sequence of the first fragment to the adapter region of the first barcode molecule, and annealing the coupling sequence of the second fragment to the adapter region of the second barcode molecule; and (d) appending barcode sequences to the first and second fragments of the target nucleic acid to produce first and second different barcoded target nucleic acid molecules, wherein the first barcoded target nucleic acid molecule comprises the nucleic acid sequence of the barcode region of the first barcode molecule and the second barcoded target nucleic acid molecule comprises the nucleic acid sequence of the barcode region of the second barcode molecule.

The multimeric barcoding reagent may comprise: (i) first and second barcode molecules linked together, wherein each of the barcode molecules comprises a nucleic acid sequence comprising, optionally in the 5' to 3' direction, an adapter region and a barcode region, and (ii)first and second barcoded oligonucleotides, wherein the first barcoded oligonucleotide comprises a barcode region annealed to the barcode region of the first barcode molecule, and wherein the second barcoded oligonucleotide comprises a barcode region annealed to the barcode region of the second barcode molecule.

The method may comprise the steps of: (a) contacting the nucleic acid sample with a first and second adapter oligonucleotide, wherein the first and second adapter oligonucleotides each comprise, optionally in the 5' to 3' direction, an adaptor region and a target region; (b) annealing the target region of the first adapter oligonucleotide to the first fragment of the target nucleic acid molecule, and annealing the target region of the second adapter oligonucleotide to a second fragment of the target nucleic acid molecule; (c) contacting the nucleic acid sample with a multimeric barcoding reagent as defined herein; (d) annealing the adapter region of the first adapter oligonucleotide to the adapter region of the first barcode molecule, and annealing the adapter region of the second adapter oligonucleotide to the adapter region of the second barcode molecule; and (e) ligating the 3' end of the first barcoded oligonucleotide to the 5' end of the first adapter oligonucleotide to produce a first barcoded-adapter oligonucleotide and ligating the 3' end of the second barcoded oligonucleotide to the 5' end of the second adapter oligonucleotide to produce a second barcoded-adapter oligonucleotide; and wherein either: (i) the first and second barcoded-adapter oligonucleotides are extended to produce first and second different barcoded target nucleic acid molecules each of which comprises at least one nucleotide synthesised from the target nucleic acid molecule as a template, or (ii) the first and second adapter oligonucleotides are extended after step (b) to produce first and second different target nucleic acid molecules each of which comprises at least one nucleotide synthesised from the target nucleic acid molecule as a template, and wherein the first and second barcoded-adapter oligonucleotides produced in step (e) are first and second different barcoded target nucleic acid molecules.

The methods may further comprise a step of removing or depleting (all or a fraction of) paraffin from the nucleic acid sample. This step may be performed prior to or during the step of contacting the nucleic acid sample with the multimeric barcoding reagent (and/or adapter oligonucleotides). Alternatively, this step may be performed prior to or during the step of appending barcode sequences to each of the nucleic acid sequences of first and second fragments of a target nucleic acid molecule. The step may be performed by one or more wash steps. Optionally, a wash step may comprise exposure to a xylene solvent solution. Optionally, a wash step may comprise exposure to a solution comprising a different solvent solution.

The methods may further comprise a step of (partially or fully) removing crosslinks from the nucleic acid sample. This step may be performed prior to or during the step of contacting the nucleic acid sample with the multimeric barcoding reagent (and/or adapter oligonucleotides). Alternatively, this step may be performed prior to or during the step of appending barcode sequences to each of the nucleic acid sequences of first and second fragments of the target nucleic acid molecule.

The step of partially or fully removing/reversing crosslinks may be performed by a thermal incubation step, wherein said thermal incubation step takes place at a temperature of at least 30 degrees Celsius, at least 37 degrees Celsius, at least 45 degrees Celsius, at least 50 degrees Celsius, at least 55 degrees Celsius, at least 60 degrees Celsius, at least 65 degrees Celsius, at least 70 degrees Celsius, at least 80 degrees Celsius, or at least 90 degrees Celsius. Preferably, said thermal incubation step takes place at a temperature of approximately 65 degrees Celsius.

The step of partially or fully removing/reversing crosslinks may be performed for at least 30 seconds, at least 60 seconds, at least 5 minutes, at least 10 minutes, at least 30 minutes, at least 60 minutes, at least 2 hours, at least 4 hours, at least 8 hours, at least 12 hours, or at least 16 hours. Preferably, said step of partially or fully reversing crosslinks is performed for between 30 minutes and 90 minutes.

The methods may further comprise a step of proteinase digestion of the nucleic acid sample. This step may be partial or full proteinase digestion of the nucleic acid sample. This step may be performed prior to or during the step of contacting the nucleic acid sample with the multimeric barcoding reagent (and/or adapter oligonucleotides). Alternatively, this step may be performed prior to or during the step of appending barcode sequences to each of the nucleic acid sequences of first and second fragments of the target nucleic acid molecule. Optionally, said proteinase digestion step may be performed using Proteinase K (from Tritirachium album).

The step of proteinase digestion may be performed during a thermal incubation step, wherein said thermal incubation step takes place at a temperature of at least 30 degrees Celsius, at least 37 degrees Celsius, at least 45 degrees Celsius, at least 50 degrees Celsius, at least 55 degrees Celsius, at least 60 degrees Celsius, at least 65 degrees Celsius, at least 70 degrees Celsius, or at least 80 degrees Celsius. Preferably, said thermal incubation step takes place at a temperature of approximately 60 degrees Celsius.

The crosslink reversal step and the proteinase digestion step may both be performed. Optionally, said crosslink reversal step and a proteinase digestion step may be performed simultaneously within a single thermal incubation step. Optionally, said crosslink reversal step and a proteinase digestion step may be performed in sequence within two sequential thermal incubation steps.

The paraffin may be depleted from the nucleic acid sample by one or more wash steps, and then a partial or full crosslink reversal step and a proteinase digestion step are performed simultaneously within a single thermal incubation, and then barcode sequences from a multimeric barcoding reagent are appended to at least two nucleic acid molecules within the resulting sample.

In the methods, the step of contacting the nucleic acid sample with a a multimeric barcoding reagent may be performed during or before a step or steps of crosslink reversal step and/or proteinase digestion. Optionally, the multimeric barcoding reagent may be retained within the sample solution during the step(s) of crosslink reversal and/or proteinase digestion, and/or a thermal incubation step therein.

In the methods, one or more centrifugation-purification steps may be performed, wherein a nucleic acid sample solution is centrifuged to pellet the sample or semi-processed sample, and wherein the resulting supernatant is aspirated, and wherein the sample is re-suspended in a second reaction solution or reaction buffer. Optionally, such a step may be performed to change buffers and/or reaction solutions. Optionally, such a step may be performed to remove enzymes and/or other reaction components.

One or more heat-inactivation steps may be performed, to inactivate an enzyme within the sample solution.

In the methods, a ribonuclease inhibitor may be included within a reaction solution or buffer.

Optionally, a primer extension step may be performed after a step of fully reversing the crosslinks and/or purifying nucleic acids from the sample. Optionally, a primer extension step may be performed with a reverse transcriptase in a reverse-transcription step.

In the methods, the nucleic acid molecules within a nucleic acid sample may have their ends converted into blunt double-stranded ends in a blunting reaction, and then have their ends converted into a form with single 3' adenosine overhangs. In such methods, the barcoded oligonucleotides may comprise a double-stranded end with a single 3' thymine overhang capable of annealing to the single 3' adenosine overhangs within the nucleic acid molecules, and wherein the barcoded oligonucleotides are ligated to nucleic acid molecules in a double-stranded A/T ligation reaction.

In the methods, coupling sequences and/or adapter oligonucleotides may be appended to fragments of nucleic acid molecules within a nucleic acid sample prior to appending barcode sequences.

In the methods, after the step of contacting the nucleic acid sample with the multimeric barcoding reagent (and/or adapter oligonucleotides) and/or after the step of appending barcode sequences to each of the nucleic acid sequences of first and second fragments of a target nucleic acid molecule, a full crosslink-reversal step may be performed. Optionally, said full crosslink reversal step may be performed during a thermal incubation step, wherein said thermal incubation step takes place at a temperature of at least 30 degrees Celsius, at least 37 degrees Celsius, at least 45 degrees Celsius, at least 50 degrees Celsius, at least 55 degrees Celsius, at least 60 degrees Celsius, or at least 65 degrees Celsius. Optionally, following said full crosslink-reversal step, the resulting nucleic acids may be purified with a cleanup, purification, or size-selection step.

In the methods, after the step of contacting the nucleic acid sample with the multimeric barcoding reagent (and/or adapter oligonucleotides) and/or after the step of appending barcode sequences to each of the nucleic acid sequences of first and second fragments of a target nucleic acid molecule, a full proteinase digestion step may be performed. Optionally, said full proteinase digestion step may be performed during a thermal incubation step, wherein said thermal incubation step takes place at a temperature of at least 30 degrees Celsius, at least 37 degrees Celsius, at least 45 degrees Celsius, at least 50 degrees Celsius, at least 55 degrees Celsius, at least 60 degrees Celsius, or at least 65 degrees Celsius. Optionally, said proteinase digestion step may be performed by Proteinase K (from Tritirachium album). Optionally, following said full crosslink-reversal step, the resulting nucleic acids may be purified with a cleanup, purification, or size-selection step.

In the methods, after the step of contacting the nucleic acid sample with the multimeric barcoding reagent (and/or adapter oligonucleotides) and/or after the step of appending barcode sequences to each of the nucleic acid sequences of first and second fragments of a target nucleic acid molecule, a full crosslink-reversal step and a full proteinase digestion step may both be performed. Optionally, said full crosslink reversal step and full proteinase digestion step may be performed simultaneously within a single thermal incubation step. Optionally, following said simultaneous steps, the resulting nucleic acids may be purified with a cleanup, purification, or size-selection step.

The nucleic acid sample may comprise immune cells e.g. T cells and/or B cells.

The target nucleic acid molecule may be genomic DNA or mRNA. For example, the mRNA molecules may comprise alpha and/or beta chains of a T cell receptor, and/or heavy and/or light chains of an immunoglobulin.

The methods may comprise the steps of: (i) contacting the nucleic acid sample with a library of multimeric barcoding reagents comprising a multimeric barcoding reagent for each of two or more target nucleic acid molecules, wherein each multimeric barcoding reagent is as defined herein, and (ii) producing at least two barcoded target nucleic acid molecules from each of the at least two target nucleic acid molecules, and wherein the at least two barcoded target nucleic acid molecules produced from a single target nucleic acid molecule each comprise the nucleic acid sequence of a barcode region from the same multimeric barcoding reagent.

The methods may comprise the steps of: (a) contacting the nucleic acid sample with a library of multimeric barcoding reagents, wherein the library comprises first and second multimeric barcoding reagents, wherein each multimeric barcoding reagent comprises first and second different barcode regions linked together, wherein each barcode region comprises a nucleic acid sequence, and wherein the first and second barcode regions of the first multimeric barcoding reagent are different to the first and second barcode regions of the second multimeric barcoding reagent, and (b) appending barcode sequences from the first multimeric barcoding reagent to each of the nucleic acid sequences of first and second fragments of a first target nucleic acid molecule to produce first and second different barcoded target nucleic acid molecules, wherein the first barcoded target nucleic acid molecule comprises the nucleic acid sequence of the first barcode region of the first multimeric barcoding reagent and the second barcoded target nucleic acid molecule comprises the nucleic acid sequence of the second barcode region of the first multimeric barcoding reagent, and appending barcode sequences from the second multimeric barcoding reagent to each of the nucleic acid sequences of first and second fragments of a second target nucleic acid molecule to produce third and fourth different barcoded target nucleic acid molecules, wherein the third barcoded target nucleic acid molecule comprises the nucleic acid sequence of the first barcode region of the second multimeric barcoding reagent and the fourth barcoded target nucleic acid molecule comprises the nucleic acid sequence of the second barcode region of the second multimeric barcoding reagent. In the methods, prior to and/or during step (a) and/or prior to and/or during step (b), the method further comprises the step of removing or depleting all or a fraction of the paraffin from the nucleic acid sample.

The first and second target nucleic acid molecules may be genomic DNA. The first and second target nucleic acid molecules may be mRNA.

The first and second multimeric barcoding reagents may each comprise first and second barcode molecules linked together, and each of the barcode molecules may comprise a nucleic acid sequence comprising a barcode region.

The first and second multimeric barcoding reagents may each comprise first and second barcoded oligonucleotides. The first barcoded oligonucleotide may comprise, optionally in the 5' to 3' direction, a barcode region, and a target region capable of annealing or ligating to the first fragment of the target nucleic acid. The second barcoded oligonucleotide may comprise, optionally in the 5' to 3' direction, a barcode region, and a target region capable of annealing or ligating to the second fragment of the target nucleic acid.

The first and second multimeric barcoding reagents may each comprise: (i) first and second barcode molecules linked together, wherein each of the barcode molecules comprises a nucleic acid sequence comprising a barcode region; and (ii) first and second barcoded oligonucleotides, wherein the first barcoded oligonucleotide comprises, optionally in the 5' to 3' direction, a barcode region, and a target region capable of annealing or ligating to the first fragment of the target nucleic acid, and wherein the second barcoded oligonucleotide comprises, optionally in the 5' to 3' direction, a barcode region, and a target region capable of annealing or ligating to the second fragment of the target nucleic acid.

The method may comprise (a) contacting the nucleic acid sample with a library of multimeric barcoding reagents as defined herein; (b) for each of the first and second multimeric barcoding reagents in the library, ligating the target region of the first barcoded oligonucleotide to the first fragment of a target nucleic acid molecule to produce a first barcoded target nucleic acid molecule, and ligating the target region of the second barcoded oligonucleotide to the second fragment of the target nucleic acid molecule to produce a second barcoded target nucleic acid molecule, wherein each of the barcoded target nucleic acid molecules comprises at least one nucleotide synthesised from the target nucleic acid as a template.

The method may comprise: (a) contacting the nucleic acid sample with a library of multimeric barcoding reagent as defined herein; (b) for each of the first and second multimeric barcoding reagents in the library, annealing the target region of the first barcoded oligonucleotide to the first fragment of a target nucleic acid, and annealing the target region of the second barcoded oligonucleotide to the second fragment of the target nucleic acid; and (c) for each of the first and second multimeric barcoding reagents in the library, extending the first and second barcoded oligonucleotides to produce first and second different barcoded target nucleic acid molecules, wherein each of the barcoded target nucleic acid molecules comprises at least one nucleotide synthesised from the target nucleic acid as a template.

The method may comprise: (a) appending a coupling sequence to each of first and second fragments of at least first and second target nucleic acid molecules; (b) contacting the sample with a library of multimeric barcoding reagents, wherein the library comprises first and second multimeric barcoding reagents, wherein each multimeric barcoding reagent comprises first and second barcode molecules linked together, wherein each of the barcode molecules comprises a nucleic acid sequence comprising a barcode region and an adapter region, wherein each multimeric barcoding reagent comprises first and second different barcode regions linked together, and wherein the first and second barcode regions of the first multimeric barcoding reagent are different to the first and second barcode regions of the second multimeric barcoding reagent; (c) (i) for the first target nucleic acid molecule, annealing the coupling sequence of the first fragment to the adapter region of the first barcode molecule of the first multimeric barcoding reagent, and annealing the coupling sequence of the second fragment to the adapter region of the second barcode molecule of the first multimeric barcoding reagent, and (ii) for the second target nucleic acid molecule, annealing the coupling sequence of the first fragment to the adapter region of the first barcode molecule of the second multimeric barcoding reagent, and annealing the coupling sequence of the second fragment to the adapter region of the second barcode molecule of the second multimeric barcoding reagent; and (d) (i) appending barcode sequences to the first and second fragments of the first target nucleic acid to produce first and second different barcoded target nucleic acid molecules, wherein the first barcoded target nucleic acid molecule comprises the nucleic acid sequence of the barcode region of the first barcode molecule of the first multimeric barcoding reagent and the second barcoded target nucleic acid molecule comprises the nucleic acid sequence of the barcode region of the second barcode molecule of the first multimeric barcoding reagent, and (ii) appending barcode sequences to the first and second fragments of the second target nucleic acid to produce third and fourth different barcoded target nucleic acid molecules, wherein the third barcoded target nucleic acid molecule comprises the nucleic acid sequence of the barcode region of the first barcode molecule of the second multimeric barcoding reagent and the fourth barcoded target nucleic acid molecule comprises the nucleic acid sequence of the barcode region of the second barcode molecule of the second multimeric barcoding reagent.

The first and second multimeric barcoding reagents may each comprise: (i) first and second barcode molecules linked together, wherein each of the barcode molecules comprises a nucleic acid sequence comprising, optionally in the 5' to 3' direction, an adapter region and a barcode region, and (ii) first and second barcoded oligonucleotides, wherein the first barcoded oligonucleotide comprises a barcode region annealed to the barcode region of the first barcode molecule, and wherein the second barcoded oligonucleotide comprises a barcode region annealed to the barcode region of the second barcode molecule.

The method may comprise: (a) contacting the nucleic acid sample with a first and second adapter oligonucleotides for each of first and second target nucleic acid molecules, wherein each first and second adapter oligonucleotide comprises, optionally in the 5' to 3' direction, an adaptor region and a target region; (b) for each of first and second target nucleic acid molecules, annealing the target region of a first adapter oligonucleotide to a first fragment of the target nucleic acid molecule, and annealing the target region of a second adapter oligonucleotide to a second fragment of the target nucleic acid molecule; (c) contacting the nucleic acid sample with a library of multimeric barcoding reagents as defined herein; (d) (i) annealing the adapter region of the first adapter oligonucleotide for the first target nucleic acid molecule to the adapter region of the first barcode molecule of the first multimeric barcoding reagent, and annealing the adapter region of the second adapter oligonucleotide for the first target nucleic acid molecule to the adapter region of the second barcode molecule of the first multimeric barcoding reagent, and (ii) annealing the adapter region of the first adapter oligonucleotide for the second target nucleic acid molecule to the adapter region of the first barcode molecule of the second multimeric barcoding reagent, and annealing the adapter region of the second adapter oligonucleotide for the second target nucleic acid molecule to the adapter region of the second barcode molecule of the second multimeric barcoding reagent; and (e)(i) ligating the 3' end of the first barcoded oligonucleotide of the first multimeric barcoding reagent to the 5' end of the first adapter oligonucleotide for the first target nucleic acid molecule to produce a first barcoded-adapter oligonucleotide and ligating the 3' end of the second barcoded oligonucleotide of the first multimeric barcoding reagent to the 5' end of the second adapter oligonucleotide for the first target nucleic acid molecule to produce a second barcoded-adapter oligonucleotide, and wherein either (1) the first and second barcoded-adapter oligonucleotides are extended to produce first and second different barcoded target nucleic acid molecules each of which comprises at least one nucleotide synthesised from the first target nucleic acid molecule as a template, or (2) the first and second adapter oligonucleotides for the first target nucleic acid molecule are extended after step (b) to produce first and second different target nucleic acid molecules each of which comprises at least one nucleotide synthesised from the first target nucleic acid molecule as a template, and wherein the first and second barcoded-adapter oligonucleotides produced in step (e) are first and second different barcoded target nucleic acid molecules, and (e)(ii) ligating the 3' end of the first barcoded oligonucleotide of the second multimeric barcoding reagent to the 5' end of the first adapter oligonucleotide for the second target nucleic acid molecule to produce a third barcoded-adapter oligonucleotide and ligating the 3' end of the second barcoded oligonucleotide of the second multimeric barcoding reagent to the 5' end of the second adapter oligonucleotide for the second target nucleic acid molecule to produce a fourth barcoded-adapter oligonucleotide, and wherein either (1) the third and fourth barcoded-adapter oligonucleotides are extended to produce third and fourth different barcoded target nucleic acid molecules each of which comprises at least one nucleotide synthesised from the second target nucleic acid molecule as a template, or (2) the first and second adapter oligonucleotides for the second target nucleic acid molecule are extended after step (b) to produce third and fourth different target nucleic acid molecules each of which comprises at least one nucleotide synthesised from the second target nucleic acid molecule as a template, and wherein the third and fourth barcoded-adapter oligonucleotides produced in step (e) are third and fourth different barcoded target nucleic acid molecules.

The method may further comprise the step of partially or fully removing crosslinks from the nucleic acid sample.

The method may further comprise the step of proteinase digestion of the nucleic acid sample.

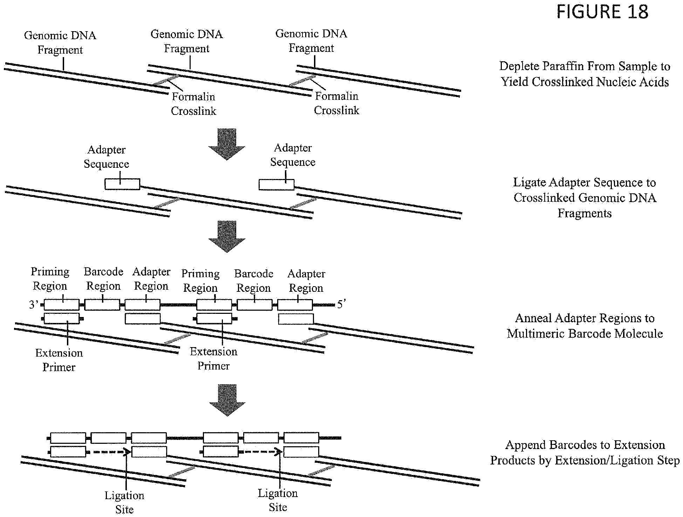

Figure 18 illustrates an example of a method of appending barcode sequences to fragments of a target nucleic acid molecule within an FFPE sample using coupling sequences. In the method, nucleic acid sequences consisting of coupling sequences are appended to fragments of a target nucleic acid molecule within an FFPE sample, and then these coupling sequences are used in a step of appending barcode sequences to the fragments of the target nucleic acid molecule. In this specific embodiment, fragmented genomic DNA within a formalin-fixed, paraffin-embedded sample (e.g. as may comprise a biopsy specimen from a tumour tissue) is barcoded prior to a sequencing reaction.

In a first step, the paraffin wax within the FFPE specimen is removed (for example, by treatment with a solvent such as xylene). Optionally (but not shown here), a step of partial reversal of the formalin crosslinks, and/or a step of limited proteinase digestion of proteins within the sample, may also be performed.

Coupling sequences are then appended to the ends of fragments of genomic DNA. This coupling sequence is complementary to an adapter region comprised within a barcode molecule and/or within a multimeric barcoding reagent. The 5' ends of each coupling sequence may comprise a phosphate group, capable of being ligated. In this specific embodiment, single-stranded coupling sequences are appended to fragments of genomic DNA with a single-stranded ligation reaction (e.g., coupling sequences may be appended to overhanging 5' ends of fragmented genomic DNA, with a single stranded ligase such as T4 RNA Ligase I). Alternatively (but not shown here), double-stranded ligation reactions (for example, with blunted genomic DNA fragments), or primer-extension reactions may instead be used to append coupling sequences.

Following this step of appending coupling sequences, barcode sequences from multimeric barcoding reagents are appended to fragments of genomic DNA. In this embodiment, the coupling sequences that have been appended to the fragments of genomic DNA are annealed to adapter regions of a multimeric barcode molecule. Extension primers are then also annealed along each barcode molecule, upstream of each barcode sequence. An extension-ligation reaction is then performed, wherein the extension primers are extended across the barcode sequence, and then ligated to the annealed coupling sequence appended to each fragment of genomic DNA, thus appending each barcode sequence and creating barcoded fragments of genomic DNA (i.e. barcoded target nucleic acid molecules)..

The invention further provides methods of barcoding using in-vitro transposition. A method of preparing a nucleic acid sample for sequencing, wherein the method comprises the steps of: (a) contacting the nucleic acid sample with a transposome complex comprising a transposon and a transposase; (b) fragmenting a target nucleic acid molecule in the nucleic acid sample and inserting first and second transferred strands at different positions in the target nucleic acid molecule while maintaining the contiguity of the target nucleic acid molecule, wherein the first and second transferred strands are from the same transposon or from different transposons; (c) contacting the nucleic acid sample with a multimeric barcoding reagent comprising first and second barcode regions linked together, wherein each barcode region comprises a nucleic acid sequence, and (d) appending a barcode sequence to each of first and second nucleic acid sequences to produce first and second different barcoded target nucleic acid molecules, wherein the first barcoded target nucleic acid molecule comprises the nucleic acid sequence of the first barcode region, the nucleic acid sequence of all or part of the first transferred strand and a nucleic acid sequence of the region that is adjacent to the first transferred strand in the target nucleic molecule, and wherein the second barcoded target nucleic acid molecule comprises the nucleic acid sequence of the second barcode region, the nucleic acid sequence of all or part of the second transferred strand and a nucleic acid sequence of the region that is adjacent to the second transferred strand in the target nucleic molecule.

In the methods, the transposase may comprise a Tn5 transposase, or a modified and/or hyperactive derivative thereof. The contiguity of the target nucleic acid molecule may be maintained by using such a Tn5 transposome (e.g. as described in WO2016061517 and Amini et al, Nature Genetics, 2014 Dec; 46(12):1343-9).

In the methods, after step (c) or step (d), the transposase(s) may be inactivated and/or removed from the nucleic acids within the nucleic acid sample e.g. by treatment with a detergent, such as treatment with a solution comprising sodium dodecyl sulfate The transposase(s) may inactivated and/or removed from the nucleic acids within the nucleic acid samples by treatment with a high temperature incubation step, such as an incubation at a temperature of at least 45 degrees Celsius, at least 50 degrees Celsius, at least 60 degrees Celsius, at least 70 degrees Celsius, or at least 80 degrees Celsius.

In the methods, during the fragmentation step (b), the average distance between any two adjacent fragmentation events may be 100-10,000 nucleotides, 200-5000 nucleotides, 300-1000 nucleotides or 400-500 nucleotides. Preferably, the average distance between any two adjacent fragmentation events is 200-2000 nucleotides. The average distance between any two adjacent fragmentation events may be approximately 100 nucleotides, approximately 200 nucleotides, approximately 300 nucleotides, approximately 400 nucleotides, approximately 500 nucleotides, approximately 1000 nucleotides, approximately 2000 nucleotides, approximately 5000 nucleotides, or approximately 10,000 nucleotides.

In the methods, steps (b)-(d) may be performed sequentially or simultaneously.

In the methods, the multimeric barcoding reagent may comprise first and second barcode molecules linked together, and wherein each of the barcode molecules comprises a nucleic acid sequence comprising a barcode region. Optionally, barcode molecules may each further comprise an adapter region. Optionally, the first transferred strand comprises an adapter region capable of annealing to the adapter region of the first barcode molecule and the second transferred strand comprises an adapter region capable of annealing to the adapter region of the second barcode molecule. The adapter region of each transferred strand may be at its 5' end.

In the method, step (d) may comprise the step of annealing the adapter region of the first transferred strand to the adapter region of the first barcode molecule, and annealing the adapter region of the second transferred strand to the adapter region of the second barcode molecule.

In the methods, the multimeric barcoding reagent may comprise first and second barcoded oligonucleotides, wherein the first barcoded oligonucleotide comprises in the 5' to 3' direction a barcode region and a target region capable of annealing to the first transferred strand in the target nucleic acid molecule, and wherein the second barcoded oligonucleotide comprises in the 5' to 3' direction a barcode region and a target region capable of annealing to the second transferred strand in the target nucleic acid molecule.

In the methods, the multimeric barcoding reagent may comprise: (i) first and second barcode molecules linked together, wherein each of the barcode molecules comprises a nucleic acid sequence comprising a barcode region; and (ii) first and second barcoded oligonucleotides, wherein the first barcoded oligonucleotide comprises in the 5' to 3' direction a barcode region annealed to the barcode region of the first barcode molecule and a target region capable of annealing to the first transferred strand in the target nucleic acid molecule, and wherein the second barcoded oligonucleotide comprises in the 5' to 3' direction a barcode region annealed to the barcode region of the second barcode molecule and a target region capable of annealing to the second transferred strand in the target nucleic acid molecule.

In the methods, at least one of the transposons of the transposome complex may comprise a coupling sequence, such that the first and second transferred strands each comprise a coupling sequence.

The method may comprise: (i) contacting the sample with a multimeric barcoding reagent comprising first and second barcode molecules linked together, wherein each of the barcode molecules comprises a nucleic acid sequence comprising a barcode region and an adapter region; (ii) appending a coupling sequence to first and second transferred strands in the target nucleic acid molecule; (iii) annealing the coupling sequence of the first transferred strand to the adapter region of the first barcode molecule, and annealing the coupling sequence of the second transferred strand to the adapter region of the second barcode molecule; and (iv) appending a barcode sequence to each of first and second nucleic acid sequences to produce first and second different barcoded target nucleic acid molecules, wherein the first barcoded target nucleic acid molecule comprises the nucleic acid sequences of the first barcode region, the coupling sequence of the first transferred strand, all or part of the first transferred strand and a region that is adjacent to the first transferred strand in the target nucleic molecule, and wherein the second barcoded target nucleic acid molecule comprises the nucleic acid sequences of the second barcode region, the coupling sequence of the second transferred strand, all or part of the second transferred strand and a region that is adjacent to the second transferred strand in the target nucleic molecule.

In the method, each of the barcode molecules may comprise a nucleic acid sequence comprising, in the 5' to 3' direction, an adapter region and a barcode region, and step (iv) may comprises: annealing and extending a first extension primer using the barcode region of the first barcode molecule as a template to produce a first barcoded oligonucleotide, and annealing and extending a second extension primer using the barcode region of the second barcode molecule as a template to produce a second barcoded oligonucleotide, wherein the first barcoded oligonucleotide comprises a sequence complementary to the barcode region of the first barcode molecule and the second barcoded oligonucleotide comprises a sequence complementary to the barcode region of the second barcode molecule, (ii) ligating the 3' end of the first barcoded oligonucleotide to the 5' end of the coupling sequence of the first transferred strand to produce a first barcoded target nucleic acid molecule and ligating the 3' end of the second barcoded oligonucleotide to the 5' end of the coupling sequence of the second transferred strand to produce a second barcoded target nucleic acid molecule.

In the method, the multimeric barcoding reagent may comprise: (i) first and second barcode molecules linked together, wherein each of the barcode molecules comprises a nucleic acid sequence comprising, optionally in the 5' to 3' direction, an adapter region and a barcode region, and (ii) first and second barcoded oligonucleotides, wherein the first barcoded oligonucleotide comprises a barcode region annealed to the barcode region of the first barcode molecule, and wherein the second barcoded oligonucleotide comprises a barcode region annealed to the barcode region of the second barcode molecule; and wherein step (iv) of the method may comprise ligating the first barcoded oligonucleotide to the coupling sequence of the first transferred strand to produce a first barcoded target nucleic acid molecule and ligating the second barcoded oligonucleotide to the coupling sequence of the second transferred strand to produce a second barcoded target nucleic acid molecule.

The method may comprise: (i) contacting the sample with a multimeric barcoding reagent comprising first and second barcode molecules linked together, wherein each of the barcode molecules comprises a nucleic acid sequence comprising a barcode region and an adapter region, and wherein the first transferred strand comprises an adapter region capable of annealing to the adapter region of the first barcode molecule and the second transferred strand comprises an adapter region capable of annealing to the adapter region of the second barcode molecule; (ii) annealing the adapter region of the first transferred strand to the adapter region of the first barcode molecule, and annealing the adapter region of the second transferred strand to the adapter region of the second barcode molecule; and (iv) appending a barcode sequence to each of first and second nucleic acid sequences to produce first and second different barcoded target nucleic acid molecules, wherein the first barcoded target nucleic acid molecule comprises the nucleic acid sequences of the first barcode region, the adapter region of the first transferred strand, all or part of the first transferred strand and a region that is adjacent to the first transferred strand in the target nucleic molecule, and wherein the second barcoded target nucleic acid molecule comprises the nucleic acid sequences of the second barcode region, the adapter region of the second transferred strand, all or part of the second transferred strand and a region that is adjacent to the second transferred strand in the target nucleic molecule.

In the method, each of the barcode molecules may comprise a nucleic acid sequence comprising, in the 5' to 3' direction, an adapter region and a barcode region, and step (iv) may comprise: annealing and extending a first extension primer using the barcode region of the first barcode molecule as a template to produce a first barcoded oligonucleotide, and annealing and extending a second extension primer using the barcode region of the second barcode molecule as a template to produce a second barcoded oligonucleotide, wherein the first barcoded oligonucleotide comprises a sequence complementary to the barcode region of the first barcode molecule and the second barcoded oligonucleotide comprises a sequence complementary to the barcode region of the second barcode molecule, (ii) ligating the 3' end of the first barcoded oligonucleotide to the 5' end of the adapter region of the first transferred strand to produce a first barcoded target nucleic acid molecule and ligating the 3' end of the second barcoded oligonucleotide to the 5' end of the adapter region of the second transferred strand to produce a second barcoded target nucleic acid molecule.

In the method, the multimeric barcoding reagent may comprise: (i) first and second barcode molecules linked together, wherein each of the barcode molecules comprises a nucleic acid sequence comprising, optionally in the 5' to 3' direction, an adapter region and a barcode region, and (ii) first and second barcoded oligonucleotides, wherein the first barcoded oligonucleotide comprises a barcode region annealed to the barcode region of the first barcode molecule, and wherein the second barcoded oligonucleotide comprises a barcode region annealed to the barcode region of the second barcode molecule; and wherein step (iv) of the method may comprise ligating the first barcoded oligonucleotide to the adapter region of the first transferred strand to produce a first barcoded target nucleic acid molecule and ligating the second barcoded oligonucleotide to the adapter region of the second transferred strand to produce a second barcoded target nucleic acid molecule.

In the methods, the multimeric barcoding reagent may comprise: (i) first and second barcode molecules linked together, wherein each of the barcode molecules comprises a nucleic acid sequence comprising, optionally in the 5' to 3' direction, an adapter region and a barcode region, and (ii) first and second barcoded oligonucleotides, wherein the first barcoded oligonucleotide comprises a barcode region annealed to the barcode region of the first barcode molecule, and wherein the second barcoded oligonucleotide comprises a barcode region annealed to the barcode region of the second barcode molecule.

In the methods, the first and second transferred strands may each comprise an adapter region capable of hybridizing to the multimeric barcoding reagent.

In the methods, the 5' end of the first and second transferred strands may each comprise a terminal 5' phosphate group capable of being ligated to a 3' end of a nucleic acid strand.

The method may comprise the steps of: (i) performing steps of contacting (step (a)) and fragmenting (step (b)) for each of at least two target nucleic acid molecules; (ii) contacting the nucleic acid sample with a library of multimeric barcoding reagents comprising a multimeric barcoding reagent for each of two or more target nucleic acid molecules (step (c)), wherein each multimeric barcoding reagent is as defined herein, and (iii) performing the step of appending (step (d)) wherein at least two barcoded target nucleic acid molecules are produced from each of the at least two target nucleic acid molecules, and wherein the at least two barcoded target nucleic acid molecules produced from a single target nucleic acid molecule each comprise the nucleic acid sequence of a barcode region from the same multimeric barcoding reagent.

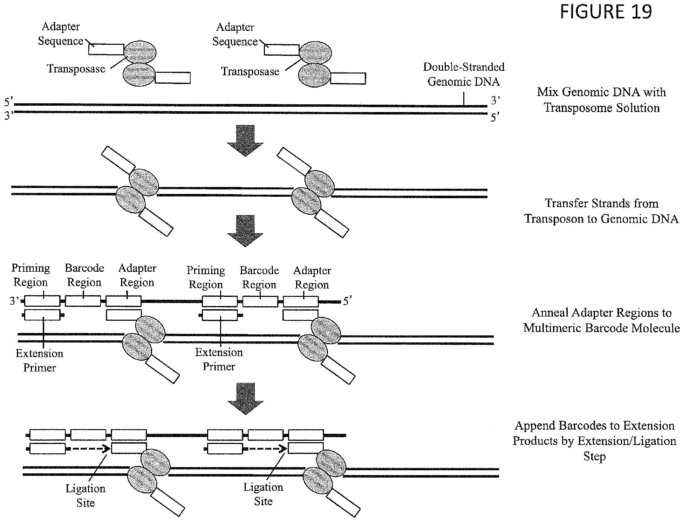

Figure 19 illustrates an example of a method of appending barcode sequences using a contiguity-preserving in vitro transposition process. In this method, genomic DNA is contacted by a solution comprising synthetic transposomes (wherein each transposome comprises, at least, a transposase enzyme and a synthetic transposon sequence). In this embodiment, each transposase is complexed with a transferred strand and a non-transferred strand, wherein the transferred strand comprises, at its 5' end, an adapter region which is complementary to an adapter region comprised within a barcode molecule and/or multimeric barcoding reagent.

The transferred strand within a transposon is then transferred by its associated transposase into the genomic DNA molecule in a step which simultaneously fragments the genomic DNA molecule, such that the transferred strand is appended to the 5' end of a newly-created fragment of genomic DNA. The conditions of this in vitro transposition process allow two or more such in vitro transposition events to take place along a single genomic DNA molecule. Importantly, this in vitro transposition process is conducted in a contiguity-preserving manner, such that the transposase enzymes remain bound to the sites of in vitro transposition, and the fragments of genomic DNA created during said in vitro transposition process remain bound to each other thereat. The 5' ends of each transferred strand may comprise a phosphate group, capable of being ligated.

Following this in vitro transposition step, barcode sequences from multimeric barcoding reagents are appended to the fragments of genomic DNA. In this embodiment, the adapter regions of the transferred strands within the synthetic transposons are annealed to adapter regions of a multimeric barcode molecule. Extension primers are then also annealed along each barcode molecule, upstream of each barcode sequence. An extension-ligation reaction is then performed, wherein the extension primers are extended across the barcode sequence, and then ligated to the annealed adapter region of each transferred strand, thus appending each barcode sequence and creating barcoded in vitro transposition products (i.e. barcoded target nucleic acid molecules).

The invention further provides a method of preparing a nucleic acid sample for sequencing, wherein the method comprises the steps of: (a) appending a nucleic acid sequence consisting of a first coupling sequence to a first sub-sequence of a target nucleic acid and appending a nucleic acid sequence consisting of a second coupling sequence to a second subsequence of the target nucleic acid, wherein the first coupling sequence consists of a nucleic acid sequence capable of annealing to the adapter region of a first hybridization molecule, and wherein the second coupling sequence consists of a nucleic acid sequence capable of annealing to the adapter region of a second hybridization molecule; (b) contacting the nucleic acid sample with a multimeric barcoding reagent comprising first and second hybridization molecules linked together, wherein each of the hybridization molecules comprises a nucleic acid sequence comprising a hybridization region and an adapter region, wherein the multimeric barcoding reagent further comprises first and second barcoded oligonucleotides, and wherein the first barcoded oligonucleotide is annealed to the hybridization region of the first hybridization molecule and wherein the second barcoded oligonucleotide is annealed to the hybridization region of the second hybridization molecule; (c) annealing the first coupling sequence (appended to the first sub-sequence of the target nucleic acid) to the adapter region of the first hybridization molecule, and annealing the second coupling sequence (appended to the second sub-sequence of the target nucleic acid) to the adapter region of the second hybridization molecule; and (d) appending the first barcoded oligonucleotide to the first coupling sequence to produce a first barcoded target nucleic acid molecule and appending the second barcoded oligonucleotide to the second coupling sequence to produce a second barcoded target nucleic acid molecule, wherein the first and second barcoded target nucleic acid molecules are different.

Step (a) may comprise ligating a first adapter oligonucleotide to the first sub-sequence of a target nucleic acid, wherein the first adapter oligonucleotide consists of the first coupling sequence, and ligating a second adapter oligonucleotide to the second subsequence of the target nucleic acid, wherein the second adaptor oligonucleotide consists of the second coupling sequence.

The first and second adapter oligonucleotides may be single-stranded or double-stranded, or single-stranded in one or more regions and double-stranded in one or more regions. They may be ligated to the first and second sub-sequences of the target nucleic acid by a single-stranded ligation reaction or a double-stranded ligation reaction. The adapter oligonucleotides may comprise a blunt, recessed, or overhanging 5' or 3' region capable of ligating to sub-sequences of the target nucleic acid. The coupling sequences may be appended to sub-sequences of the target nucleic acid by a double-stranded ligation reaction. Adapter oligonucleotides that are at least partially double-stranded may comprise a single-stranded region complementary to and capable of annealing to the adapter region of a hybridisation molecule (or barcode molecule) of a multimeric barcoding reagent.

Optionally, the ends of the sub-sequences of the target nucleic acid may be converted into blunt double-stranded ends in a blunting reaction, and wherein the adapter oligonucleotides comprise a blunt double-stranded end, and wherein the adapter oligonucleotides are ligated to nucleic acid molecules in a blunt-end ligation reaction

Optionally, the sub-sequences of the target nucleic acid have their ends converted into blunt double-stranded ends in a blunting reaction, and then have their ends converted into a form with single 3' adenosine overhangs, and wherein the adapter oligonucleotides comprise a double-stranded end with a single 3' thymine overhang capable of annealing to the single 3' adenosine overhangs within the nucleic acid molecules, and wherein the adapter oligonucleotides are ligated to nucleic acid molecules in a double-stranded A/T ligation reaction.

Optionally, the sub-sequences of the target nucleic acid are contacted with a restriction enzyme, wherein the restriction enzyme digests the nucleic acid molecules at restriction sites to create ligation junctions at these restriction sites, and wherein the adapter oligonucleotides comprise an end compatible with these ligation junctions, and wherein the adapter oligonucleotides are then ligated to nucleic acid molecules at said ligation junctions in a double-stranded ligation reaction.

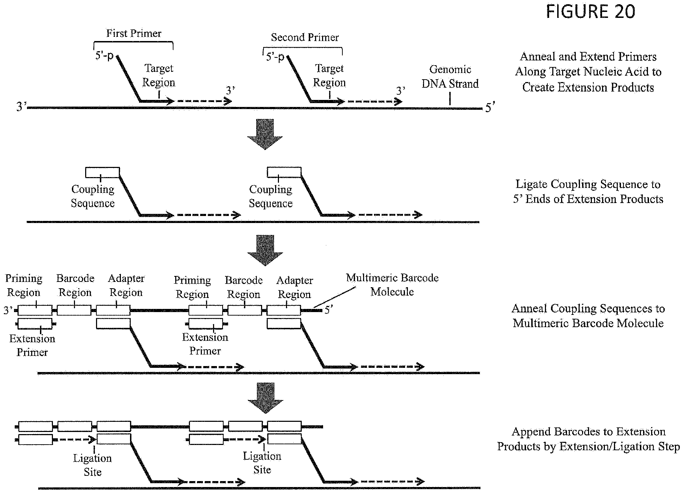

Figure 20 illustrates an example of a method of appending barcode sequences using coupling sequences. In the figure, nucleic acid sequences consisting of coupling sequences are appended to target nucleic acid sequences, and then these coupling sequences are used in a step of appending barcode sequences to the target nucleic acid sequences. In this specific embodiment, primers are annealed to and extended along a genomic DNA strand. These primers may comprise random sequences within their 3' ends (thus capable of performing random priming); alternatively the primers may comprise one or more target-specific sequences within their 3' ends. In this specific embodiment, these primers comprise a single-stranded 5' region that is not annealed to the genomic DNA strand, as well as a 5' phosphate group.

Following a step of extending the annealed primers along the genomic DNA strand, a coupling sequence is appended to the 5' end of each primer in a single-stranded ligation reaction. This coupling sequence is complementary to an adapter region comprised within a barcode molecule and/or within a multimeric barcoding reagent. The 5' ends of each coupling sequence may comprise a phosphate group, capable of being ligated.

Following this step of appending coupling sequences, barcode sequences from multimeric barcoding reagents are appended to the primer-extension products. In this embodiment, the coupling sequences that have been appended to the primer-extension products are annealed to adapter regions of a multimeric barcode molecule. Extension primers are then also annealed along each barcode molecule, upstream of each barcode sequence. An extension-ligation reaction is then performed, wherein the extension primers are extended across the barcode sequence, and then ligated to the annealed coupling sequence appended to each primer-extension primer, thus appending each barcode sequence and creating barcoded primer-extension products (i.e. barcoded target nucleic acid molecules).

The invention further provides a method of preparing a nucleic acid sample for sequencing, wherein the method comprises the steps of: (a) appending a first coupling sequence to a first sub-sequence of a target nucleic acid and appending a second coupling sequence to a second subsequence of the target nucleic acid, wherein the coupling sequences are appended to the sub-sequences of the target nucleic acid by a terminal transferase enzyme; (b) contacting the nucleic acid sample with a multimeric barcoding reagent comprising first and second hybridization molecules linked together, wherein each of the hybridization molecules comprises a nucleic acid sequence comprising a hybridization region and an adapter region, and wherein the multimeric barcoding reagent further comprises first and second barcoded oligonucleotides, wherein the first barcoded oligonucleotide is annealed to the hybridization region of the first hybridization molecule and wherein the second barcoded oligonucleotide is annealed to the hybridization region of the second hybridization molecule; (c) annealing the first coupling sequence (appended to the first sub-sequence of the target nucleic acid) to the adapter region of the first hybridization molecule, and annealing the second coupling sequence (appended to the second sub-sequence of the target nucleic acid) to the adapter region of the second hybridization molecule; and (d) appending the first barcoded oligonucleotide to the first coupling sequence to produce a first barcoded target nucleic acid molecule and appending the second barcoded oligonucleotide to the second coupling sequence to produce a second barcoded target nucleic acid molecule, wherein the first and second barcoded target nucleic acid molecules are different.

In the methods, the multimeric barcoding reagent may comprise first and second barcode molecules linked together, wherein each of the barcode molecules comprises a nucleic acid sequence comprising a barcode region and an adapter region, wherein the multimeric barcoding reagent further comprises first and second barcoded oligonucleotides, and wherein the first barcoded oligonucleotide is annealed to the barcode region of the first barcode molecule and wherein the second barcoded oligonucleotide is annealed to the barcode region of the second barcode molecule.

In the methods, step (d) may comprise ligating the first barcoded oligonucleotide to the first coupling sequence to produce a first barcoded target nucleic acid molecule and ligating the second barcoded oligonucleotide to the second coupling sequence to produce a second barcoded target nucleic acid molecule.

In the methods, step (d) may comprise ligating the 3' end of the first barcoded oligonucleotide to the 5' end of the first coupling sequence to produce a first barcoded target nucleic acid molecule and ligating the 3' end of the second barcoded oligonucleotide to the 5' end of the second coupling sequence to produce a second barcoded target nucleic acid molecule.

The invention provides a method of preparing a nucleic acid sample for sequencing, wherein the method comprises the steps of: (a) appending a first coupling sequence to a first sub-sequence of a target nucleic acid and appending a second coupling sequence to a second subsequence of the target nucleic acid, wherein the coupling sequences are appended to the sub-sequences of the target nucleic acid by a terminal transferase enzyme; (b) contacting the nucleic acid sample with a multimeric barcoding reagent comprising first and second barcode molecules linked together, wherein each of the barcode molecules comprises a nucleic acid sequence comprising, a barcode region and an adapter region; (c) annealing the first coupling sequence to the adapter region of the first barcode molecule, and annealing the second coupling sequence to the adapter region of the second barcode molecule; and (d) appending barcode sequences to each of the at least two sub-sequences of the target nucleic acid to produce first and second different barcoded target nucleic acid molecules, wherein the first barcoded target nucleic acid molecule comprises the nucleic acid sequence of the barcode region of the first barcode molecule and the second barcoded target nucleic acid molecule comprises the nucleic acid sequence of the barcode region of the second barcode molecule.

In the methods, each of the barcode molecules may comprise a nucleic acid sequence comprising, in the 5' to 3' direction, a barcode region and an adapter region, and step (d) may comprise extending the first coupling sequence using the barcode region of the first barcode molecule as a template to produce a first barcoded target nucleic acid molecule, and extending the second coupling sequence using the barcode region of the second barcode molecule as a template to produce a second barcoded target nucleic acid molecule, wherein the first barcoded target nucleic acid molecule comprises a sequence complementary to the barcode region of the first barcode molecule and the second barcoded target nucleic acid molecule comprises a sequence complementary to the barcode region of the second barcode molecule.

In the methods, each of the barcode molecules may comprise a nucleic acid sequence comprising, in the 5' to 3' direction, an adapter region and a barcode region, and step (d) may comprise (i) annealing and extending a first extension primer using the barcode region of the first barcode molecule as a template to produce a first barcoded oligonucleotide, and annealing and extending a second extension primer using the barcode region of the second barcode molecule as a template to produce a second barcoded oligonucleotide, wherein the first barcoded oligonucleotide comprises a sequence complementary to the barcode region of the first barcode molecule and the second barcoded oligonucleotide comprises a sequence complementary to the barcode region of the second barcode molecule, (ii) ligating the 3' end of the first barcoded oligonucleotide to the 5' end of the first coupling sequence to produce a first barcoded target nucleic acid molecule and ligating the 3' end of the second barcoded oligonucleotide to the 5' end of the second coupling sequence to produce a second barcoded target nucleic acid molecule.

In the methods, the first coupling sequence may comprise at least one nucleotide complementary to the adapter region of the first hybridization molecule or the first barcode molecule, and the second coupling sequence may comprise at least one nucleotide complementary to the adapter region of the second hybridization molecule or the second barcode molecule. The complementary sequence may be at least 5, at least 10, at least 15, at least 20, at least 25 or at least 50 contiguous nucleotides. Preferably, the complementary sequence is at least 8 contiguous nucleotides.

The first coupling sequence may consist of a nucleic acid sequence that is complementary to all or portion of the adapter region of the first hybridization molecule or the first barcode molecule, and the second coupling sequence may consist of a nucleic acid sequence that is complementary to all or a portion of the adapter region of the second hybridization molecule or the second barcode molecule.

In the methods, the terminal transferase enzyme may be a terminal deoxynucleotidyl transferase enzyme. Alternatively, the terminal transferase enzyme may be any enzyme with terminal transferase behaviour, such as polymerase enzymes which exhibit terminal transferase behaviour, poly(A) polymerase or poly(U) polymerase enzymes. The coupling sequence may comprise at least two contiguous nucleotides of a homopolymeric sequence

In the methods, the coupling sequence may be appended to a synthetic nucleic acid sequence, such as a primer or a primer extension product, a transposon or transposon strand, a second coupling sequence, or any other synthetic sequence.

In the methods, a coupling sequence may be appended to a primer that is annealed across at least part of its sequence to a sub-sequence of the target nucleic acid. Optionally said coupling sequence may be appended before or after the primer has been extended. Optionally said primer may comprise a target-specific primers. Optionally the primer may comprise one or more random or degenerate bases.

In any of the methods, the target nucleic acid may be genomic DNA or mRNA.

The method may comprise: (i) appending first and second coupling sequences to two or more target nucleic acids in the nucleic acid sample, wherein the coupling sequences are appended according to any of the methods described herein; (ii) contacting the nucleic acid sample with a library of multimeric barcoding reagents comprising a multimeric barcoding reagent for each of two or more target nucleic acid molecules, wherein each multimeric barcoding reagent is as defined herein; and (iii) performing step (d) wherein at least two barcoded target nucleic acid molecules are produced from each of the at least two target nucleic acids, and wherein the at least two barcoded target nucleic acid molecules produced from a single target nucleic acid each comprise the nucleic acid sequence of a barcode region from the same multimeric barcoding reagent.

The invention further provides a method of preparing a nucleic acid sample for sequencing, wherein the method comprises the steps of: (a) contacting the nucleic acid sample with first and second primers, wherein each primer comprises a target region; (b) annealing the target region of the first primer to a first sub-sequence of a target nucleic acid molecule, and annealing the target region of the second primer to a second sub-sequence of the same target nucleic acid molecule; (c) extending the first primer and the second primer to produce a first primer-extension product and a second primer-extension product, wherein the first and second primer-extension products each comprise at least one nucleotide synthesised from the target nucleic acid molecule as a template; (d) contacting the nucleic acid sample with a multimeric barcoding reagent comprising first and second barcode regions linked together, wherein each barcode region comprises a nucleic acid sequence, and (e) appending a barcode sequence to each of the first and second primers to produce first and second different barcoded primers, wherein the first barcoded primer comprises the barcode sequence of the first barcode region and the second barcoded primer comprise the barcode sequence of the second barcode region, wherein either (i) the first and second barcoded primers are extended to produce first and second barcoded target nucleic acid molecules, or (ii) the first and second extension primers are extended after step (b) to produce first and second primer-extension products, and wherein the first and second barcoded primers produced in step (e) are first and second different barcoded target nucleic acid molecules; and wherein the first and second primers remain hybridized to the target nucleic acid molecule until the first and second different barcoded target nucleic acid molecules have been produced.

In the methods, the primers may each comprise an adapter region capable of annealing to the multimeric barcoding reagent and step (d) further comprises annealing the first and second primers to the multimeric barcoding reagent. The adapter region may be at the 5' end of each primer. Optionally, the adapter region may be partially or fully complementary to an adapter region of a multimeric barcoding reagent (e.g. an adapter region of a multimeric hybridization molecule or multimeric barcode molecule). Optionally, the adapter region may be or comprise a coupling sequence.

In the methods, following a primer-extension reaction (step (c)) but before appending barcode sequences (step (e)), a coupling sequence may be appended to the primer extension products. Optionally, the coupling sequence may be appended by a single-stranded or double-stranded ligation process. Optionally, the coupling sequence may comprise an adapter region that is partially or fully complementary to an adapter region of a multimeric barcoding reagent. Optionally, the coupling sequence may be annealed to an adapter region of the multimeric barcoding reagent during step (e).

In the methods, the multimeric barcoding reagent may comprise: (i) first and second barcode molecules linked together, wherein each of the barcode molecules comprises a nucleic acid sequence comprising, optionally in the 5' to 3' direction, an adapter region and a barcode region; and (ii) first and second barcoded oligonucleotides, wherein the first barcoded oligonucleotide comprises a barcode region annealed to the barcode region of the first barcode molecule, and wherein the second barcoded oligonucleotide comprises a barcode region annealed to the barcode region of the second barcode molecule.

The method may comprise: (a) contacting the nucleic acid sample with first and second adapter oligonucleotides (as the first and second primers described herein), wherein the first adapter oligonucleotide comprises in the 5' to 3' direction an adapter region capable of annealing to the adapter region of the first barcode molecule and a target region capable of annealing to the first sub-sequence of the target nucleic acid molecule, and wherein the second adapter oligonucleotide comprises in the 5' to 3' direction an adapter region capable of annealing to the adapter region of the second barcode molecule and a target region capable of annealing to the second sub-sequence of the target nucleic acid molecule; (b) annealing the target region of the first adapter oligonucleotide to the first sub-sequence of the target nucleic acid molecule, and annealing the target region of the second adapter oligonucleotide to the second sub-sequence of the same target nucleic acid nucleic acid molecule; (c) extending the first adapter oligonucleotide and the second adapter oligonucleotide to produce a first primer-extension product and a second primer-extension product; (d) contacting the nucleic acid sample with a multimeric barcoding reagent as defined herein and annealing the adapter region of the first adapter oligonucleotide to the adapter region of the first barcode molecule, and annealing the adapter region of the second adapter oligonucleotide to the adapter region of the second barcode molecule, and (e) ligating the 3' end of the first barcoded oligonucleotide to the 5' end of the first adapter oligonucleotide to produce a first barcoded-adapter oligonucleotide and ligating the 3' end of the second barcoded oligonucleotide to the 5' end of the second adapter oligonucleotide to produce a second barcoded-adapter oligonucleotide; and wherein either: the first and second barcoded-adapter oligonucleotides are extended to produce first and second different barcoded target nucleic acid molecules each of which comprises at least one nucleotide synthesised from the target nucleic acid molecule as a template, or the first and second adapter oligonucleotides are extended after step (b) to produce first and second different target nucleic acid molecules each of which comprises at least one nucleotide synthesised from the target nucleic acid molecule as a template, and wherein the first and second barcoded-adapter oligonucleotides produced in step (e) are first and second different barcoded target nucleic acid molecules.

The target nucleic acid molecule may be a single (intact) target nucleic acid molecule.

Each target region of a primer (e.g. an adapter oligonucleotide) may comprise a sequence capable of annealing to only a single sub-sequence of a target nucleic acid within a sample of nucleic acids (i.e. a target specific sequence). Each target region may be capable of random priming. Each target region may comprise one or more random, or one or more degenerate, sequences to enable the target region to anneal to more than one sub-sequence of a target nucleic acid. Each target region may comprise at least 5, at least 10, at least 15, at least 20, at least 25, at least 50 or at least 100 nucleotides. Preferably, each target region comprises at least 5 nucleotides. Each target region may comprise 5 to 100 nucleotides, 5 to 10 nucleotides, 10 to 20 nucleotides, 20 to 30 nucleotides, 30 to 50 nucleotides, 50 to 100 nucleotides, 10 to 90 nucleotides, 20 to 80 nucleotides, 30 to 70 nucleotides or 50 to 60 nucleotides. Preferably, each target region comprises 30 to 70 nucleotides. Preferably each target region comprises deoxyribonucleotides, optionally all of the nucleotides in a target region are deoxyribonucleotides. One or more of the deoxyribonucleotides may be a modified deoxyribonucleotide (e.g. a deoxyribonucleotide modified with a biotin moiety or a deoxyuracil nucleotide). Each target region may comprise one or more universal bases (e.g. inosine), one or modified nucleotides and/or one or more nucleotide analogues.

The primers may comprise a mixture of one or more target-specific primers and one or more primers capable of random priming.

In the methods, prior to the step of contacting the nucleic acid sample with primers (e.g. adapter oligonucleotides), the method may comprise appending first and second coupling sequences to the target nucleic acid, wherein the first and second coupling sequences are the sub-sequences of the target nucleic acid to which the primers anneal in step (b). A target region of a primer may be partially or fully complementary to a coupling sequence (e.g. a sequence not found within the genomic DNA and/or messenger RNA target nucleic acid).

The method may be performed with at least 2, at least 5, at least 10, at least 100, at least 1000, at least 10,000, at least 100,000, at least 1,000,000, at least 10,000,000, at least 100,000,000, or at least at least 1,000,000,000 different primers (in place of the first and second primers). Preferably, the method is performed with at least 5 different primers.

The multimeric barcoding reagent used in the methods may comprise at least 5, at least 10, at least 20, at least 25, at least 50, at least 75, at least 100, at least 200, at least 500, at least 1000, at least 5000, or at least 10,000 different barcode regions. Preferably, the multimeric barcoding reagent comprises at least 5 barcode regions.

In any of the methods, the target nucleic acid may be genomic DNA or mRNA.

In the methods, the step of extending the primers (step (c)) may be performed using a strand displacing polymerase. Alternatively, the step may be performed using a non-strand displacing polymerase.

Optionally, step (c) may be performed using a polymerase either (i) without significant strand-displacement activity or (ii) with strand-displacement activity. Optionally, the primer-extension reaction is performed by a mixture of two or more polymerases, including at least one polymerase without significant strand-displacement behaviour and at least one polymerase with significant strand-displacement behaviour.

In the methods, the primer-extension reaction may be performed using a polymerase without significant 5'-to-3' exonuclease behaviour.

In the methods, a first primer extension product may at least partially displace a second primer-extension product located on the same molecule during a process of primer extension by a polymerase with strand displacement behaviour.

The primer-extension reaction may be performed by a phi29 DNA polymerase or derivatives or variants thereof. The primer-extension reaction may be performed by a Bst or Bsm DNA polymerase or derivatives or variants thereof

The primer-extension step may be performed for less than 5 seconds, less than 10 seconds, less than 30 seconds, less than 60 seconds, less than 90 seconds, less than 2 minutes, less than 3 minutes, less than 5 minutes, less than 10 minutes, less than 15 minutes, less than 30 minutes, less than 60 minutes, less than 2 hours, or less than 4 hours.

The primer-extension step may be performed in a solution where at least one deoxynucleotide triphosphate (such as dATP, dTTP, dCTP, or dGTP) or at least one corresponding modified deoxynucleotide triphosphate is present at a concentration of less than 200 micromolar, less than 100 micromolar, less than 50 micromolar, less than 25 micromolar, less than 10 micromolar, less than 5 micromolar, less than 1 micromolar, less than 500 nanomolar, less than 250 nanomolar, less than 100 nanomolar, or less than 50 nanomolar. Optionally, the cumulative net concentration of all deoxynucleotide triphosphate in solution may be less than 200 micromolar, less than 100 micromolar, less than 50 micromolar, less than 25 micromolar, less than 10 micromolar, less than 5 micromolar, less than 1 micromolar, less than 500 nanomolar, less than 250 nanomolar, less than 100 nanomolar, or less than 50 nanomolar.

The primer-extension step may be performed in a solution comprising one or more chain-terminating molecules, such as a dideoxynucleotide triphosphate molecules (e.g. 2',3' dideoxynucleotide triphosphate molecules such as ddATP, ddCTP, ddGTP, or ddTTP), or any other chain-terminating molecules. Optionally, such chain-terminating molecules may reduce the degree of strand displacement in a sequencing reaction as above wherein a polymerase with strand displacement behaviour has been used.

The primer-extension step may be performed in a solution comprising one or more modified deoxynucleotide triphosphate molecules, such as a biotin-conjugated deoxynucleotide triphosphate, a polyethylene glycol-conjugated deoxynucleotide triphosphate, or a deoxynucleotide triphosphate conjugated to a spacer or a linker molecule, such as a multi-carbon spacer such as a 3-carbon spacer, a 6-carbon spacer, a 12-carbon spacer, or an 18-carbon spacer. Optionally, such modified deoxynucleotide triphosphate molecules may slow or reduce the primer extension in a sequencing reaction as above.

The primer-extension step may be performed at a temperature less than 70 degrees Celsius, less than 65 degrees Celsius, less than 60 degrees Celsius, less than 50 degrees Celsius, less than 40 degrees Celsius, less than 30 degrees Celsius, less than 20 degrees Celsius, less than 15 degrees Celsius, or less than 10 degrees Celsius.

The primer-extension step may be terminated by a heat-inactivation step. Optionally, this heat-inactivation step may be performed at an incubation temperature of at least 45 degrees Celsius, at least 50 degrees Celsius, at least 60 degrees Celsius, at least 70 degrees Celsius, or at least 80 degrees Celsius.

The primer-extension step may be terminated by contacting the primer-extension solution with a detergent solution. The primer-extension step may be terminated by contacting the primer-extension solution with a chelator solution

In the methods, before contacting the sample with a multimeric barcoding reagent, a cleanup step may be performed wherein primers not extended along a nucleic acid template are preferentially removed or depleted from the sample. Optionally, this step may be performed by a gel-based size selection step. Optionally, this size selection step may be performed with a solid-phase reversible immobilisation process, such as a size selection step involving magnetic or superparamagnetic beads. Optionally, this size selection step may be performed with a column-based nucleic acid purification or size-selection step. Optionally, this size selection step may preferentially remove nucleic acid molecules less than 50 nucleotides in length, less than 100 nucleotides in length, less than 150 nucleotides in length, less than 200 nucleotides in length, less than 300 nucleotides in length, less than 400 nucleotides in length, less than 500 nucleotides in length, or less than 1000 nucleotides in length.

Optionally, before contacting the sample with a multimeric barcoding reagent, non-extended primers within the solution are digested or partially digested with an exonuclease-digestion step. Optionally, this exonuclease-digestion step may be performed by e. coli Exonuclease I, or e. coli Lambda exonuclease.

In the methods, the primer extension reaction (step (c)) may be performed after the step of appending barcode sequences from a multimeric barcoding reagent (step (e)).

Ìn the methods, a phi29 polymerase or a derivative thereof may be used to perform the primer-extension process, and said primer-extension process may be terminated within a time period less than three hours in length.

In the methods, a phi29 polymerase or a derivative thereof may be used to perform the primer-extension process, and the primers employed in said primer-extension process may comprise at least one degenerate base.

The method may comprise the steps of: (i) contacting the nucleic acid sample with first and second primers (e.g. first and second adapter oligonucleotides) for each of at least two target nucleic acid molecules, wherein each primer comprises a target region; (ii) performing steps (b) and (c) for each target nucleic acid molecule; (iii) contacting the nucleic acid sample with a library of multimeric barcoding reagents comprising a multimeric barcoding reagent for each target nucleic acid molecule, wherein each multimeric barcoding reagent is as defined herein; and (iv) performing step (e) wherein at least two barcoded target nucleic acid molecules are produced from each of the at least two target nucleic acid molecules, and wherein the barcoded target nucleic acid molecules produced from a single target nucleic acid molecule each comprise the nucleic acid sequence of a barcode region from the same multimeric barcoding reagent.

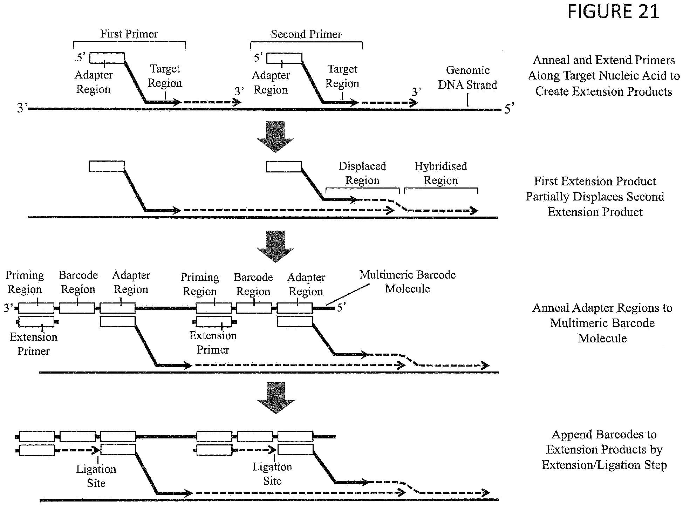

Figure 21 illustrates an example of a method of preparing a nucleic acid sample for sequencing to enable synthetic long read sequencing. The method is a primer-extension method that uses a strand-displacing polymerase. In the figure, two (or any larger number of) primers are annealed to a strand of genomic DNA, and then extended with a polymerase that exhibits strand displacement behaviour. These primers may comprise random sequences within their 3' ends (thus capable of performing random priming); alternatively the primers may comprise one or more target-specific sequences within their 3' ends. These primers comprise an adapter region within their 5' regions, which are capable of annealing to an adapter region within a barcode molecule.

In the primer-extension reaction, the 3' end of each annealed primer is extended by a polymerase. During the process of primer extension, a first primer-extension product, located 3' along the genomic DNA template relative to a second primer-extension product, will eventually be extended until a point at which it reaches the annealed primer of a second primer extension product. The 5' ends of each primer may comprise a phosphate group, capable of being ligated.

Due to the strand-displacing behaviour of the polymerase, a polymerase synthesising this first primer-extension product will, upon encountering the second primer-extension molecule, begin to displace the said second primer-extension product. In this method, the primer-extension reaction is terminated before the entire second primer-extension product is displaced by the first primer-extension product. That is, the primer-extension step is terminated whilst at least a first and second primer-extension product each are hybridised to the genomic DNA strand by at least some span (i.e., the first and second primer extension products each have at least one nucleotide annealed to the strand of genomic DNA).

Following this primer-extension step, barcode sequences from multimeric barcoding reagents are appended to the primer-extension products. In this embodiment, the adapter regions of the primers used for primer-extension are annealed to adapter regions of a multimeric barcode molecule. Extension primers are then also annealed along each barcode molecule, upstream of each barcode sequence. An extension-ligation reaction is then performed, wherein the extension primers are extended across the barcode sequence, and then ligated to the annealed adapter region of each primer-extension primer, thus appending each barcode sequence and creating barcoded primer-extension products (i.e. barcoded target nucleic acid molecules).

The methods of the invention may be performed with any of the multimeric barcoding reagents, kits comprising multimeric barcoding reagents or libraries of multimeric barcoding reagents described herein. Further details of the methods of the invention are provided below.

### 1. GENERAL PROPERTIES OF MULTIMERIC BARCODING REAGENTS

The invention provides multimeric barcoding reagents for labelling one or more target nucleic acids. A multimeric barcoding reagent comprises two or more barcode regions are linked together (directly or indirectly).

Each barcode region comprises a nucleic acid sequence. The nucleic acid sequence may be single-stranded DNA, double-stranded DNA, or single stranded DNA with one or more double-stranded regions.

Each barcode region may comprise a sequence that identifies the multimeric barcoding reagent. For example, this sequence may be a constant region shared by all barcode regions of a single multimeric barcoding reagent. Each barcode region may contain a unique sequence which is not present in other regions, and may thus serve to uniquely identify each barcode region. Each barcode region may comprise at least 5, at least 10, at least 15, at least 20, at least 25, at least 50 or at least 100 nucleotides. Preferably, each barcode region comprises at least 5 nucleotides. Preferably each barcode region comprises deoxyribonucleotides, optionally all of the nucleotides in a barcode region are deoxyribonucleotides. One or more of the deoxyribonucleotides may be a modified deoxyribonucleotide (e.g. a deoxyribonucleotide modified with a biotin moiety or a deoxyuracil nucleotide). The barcode regions may comprise one or more degenerate nucleotides or sequences. The barcode regions may not comprise any degenerate nucleotides or sequences.

The multimeric barcoding reagent may comprise at least 5, at least 10, at least 20, at least 25, at least 50, at least 75, at least 100, at least 200, at least 500, at least 1000, at least 5000, or at least 10,000 barcode regions. Preferably, the multimeric barcoding reagent comprises at least 5 barcode regions.

The multimeric barcoding reagent may comprise at least 2, at least 3, at least 4, at least 5, at least 10, at least 20, at least 25, at least 50, at least 75, at least 100, at least 200, at least 500, at least 1000, at least 5000, at least 10⁴, at least 10⁵, or at least 10⁶ unique or different barcode regions. Preferably, the multimeric barcoding reagent comprises at least 5 unique or different barcode regions.

A multimeric barcoding reagent may comprise: first and second barcode molecules linked together (i.e. a multimeric barcode molecule), wherein each of the barcode molecules comprises a nucleic acid sequence comprising a barcode region.

The barcode molecules of a multimeric barcode molecule may be linked on a nucleic acid molecule. The barcode molecules of a multimeric barcode molecule may be comprised within a (single) nucleic acid molecule. A multimeric barcode molecule may comprise a single, contiguous nucleic acid sequence comprising two or more barcode molecules. A multimeric barcode molecule may be a single-stranded nucleic acid molecule (e.g. single-stranded DNA), a double-stranded-stranded nucleic acid molecule or a single stranded molecule comprising one or more double-stranded regions. A multimeric barcode molecule may comprise one or more phosphorylated 5' ends capable of ligating to 3' ends of other nucleic acid molecules. Optionally, in a double-stranded region or between two different double-stranded regions, a multimeric barcode molecule may comprise one or more nicks, or one or more gaps, where the multimeric barcode molecule itself has been divided or separated. Any said gap may be at least one, at least 2, at least 5, at least 10, at least 20, at least 50, or at least 100 nucleotides in length. Said nicks and/or gaps may serve the purpose of increasing the molecular flexibility of the multimeric barcode molecule and/or multimeric barcoding reagent, for example to increase the accessibility of the molecule or reagent to interact with target nucleic acid molecules. Said nicks and/or gaps may also enable more efficient purification or removal of said molecules or reagents. A molecule and/or reagent comprising said nick(s) and/or gap(s) may retain links between different barcode molecules by having a complementary DNA strand which is jointly hybridised to regions of two or more divided parts of a multimeric barcode molecule.

A multimeric barcode molecule (and/or libraries of multimeric barcode molecules) may be synthesised by any chemical and/or enzymatic method of oligonucleotide synthesis. A multimeric barcode molecule (and/or libraries of multimeric barcode molecules) may be synthesised by phosphoramidite oligonucleotide synthesis. A multimeric barcode molecule (and/or libraries of multimeric barcode molecules) may be synthesised by microarray-based oligonucleotide synthesis. A multimeric barcode molecule (and/or libraries of multimeric barcode molecules) may be synthesised by a combined chemical and enzymatic process; for example, a multimeric barcode molecule (and/or libraries of multimeric barcode molecules) may be synthesised by phosphoramidite oligonucleotide synthesis, and then an enzymatic step may be employed to further process and/or synthesise said multimeric barcode molecule(s); for example, said enzymatic step may comprise a ligation step, wherein a further sequence is appended to the chemically-synthesised multimeric barcode molecule(s) by a ligation process.

A multimeric barcode molecule (and/or libraries of multimeric barcode molecules) may be processed and/or purified and/or size-selected by any method. Optionally, a multimeric barcode molecule (and/or libraries of multimeric barcode molecules) may be purified by a gel electrophoresis process, such as agarose gel electrophoresis, or polyacrylamide electrophoresis; optionally any gel electrophoresis process may be employed to isolate multimeric barcode molecules of one or more different length and/or size ranges. Optionally, a multimeric barcode molecule (and/or libraries of multimeric barcode molecules) may be purified by size-exclusion chromatography.

Sequences (e.g. barcode sequences, and/or adapter sequences) from two or more multimeric barcode molecules may be appended to each other by any means to create an appended multimeric barcode molecule. Two or more multimeric barcode molecules may be appended to each other in a double-stranded or single-stranded ligation reaction (e.g. wherein the 5' end of a first multimeric barcode molecule is ligated to the 3' end of a second multimeric barcode molecule).

Two or more multimeric barcode molecules may be appended to each other in a double-stranded or single-stranded ligation reaction, wherein the first and second multimeric barcode molecules each comprise at least one double-stranded region, and wherein the first and second multimeric barcode molecules are each digested with a restriction enzyme capable of digesting a sequence comprised within said double-stranded regions, and wherein sequences from the first and second multimeric barcode molecules are appended to each other by a double-stranded ligation reaction, wherein the sites of digestion of each multimeric barcode molecule produced from the restriction-enzyme digestion step are ligated to each other (e.g. in a double-stranded ligation reaction).

Two or more multimeric barcode molecules may be appended to each other in an overlap-extension reaction, wherein the 3' end of a first multimeric barcode molecule is at least partially complementary to a region of a second multimeric barcode molecule, and wherein said 3' end is annealed to said second multimeric barcode molecule and then subject to a primer-extension step using sequence from the second multimeric barcode molecule as a template, wherein the resulting extension product produced from the 3' end of the first multimeric barcode molecule comprises at least one barcode sequence complementary to a barcode sequence within the second multimeric barcode molecule. Optionally, the second multimeric barcode molecule may comprise a blocked 3' end, such that a polymerase is not able to extend the 3' end of the second multimeric barcode molecule. Optionally, any such first and/or second multimeric barcode molecules may comprise at least 2, at least 3, at least 4, at least 5, at least 10, at least 50, at least 100, at least 500, at least 1000, or at least 10,000 barcodes, and/or barcode regions, and/or barcode molecules. Optionally, any such overlap-extension process may be repeated for at least 2, at least 5, at least 10, at least 50, at least 100, at least 500, or at least 1000 times and/or cycles.

Optionally, any such process of appending sequences from two or more multimeric barcode molecules to each other may be performed using and/or performed upon a library of multimeric barcode molecules, wherein the library comprises at least 2, at least 3, at least 4, at least 5, at least 10, at least 20, at least 25, at least 50, at least 75, at least 100, at least 200, at least 500, at least 1000, at least 5000, at least 10⁴, at least 10⁵, at least 10⁶, at least 10⁷, at least 10⁸, or at least 10⁹ multimeric barcode molecules.

The barcode molecules may be linked by a support e.g. a macromolecule, solid support or semi-solid support. The sequences of the barcode molecules linked to each support may be known. The barcode molecules may be linked to the support directly or indirectly (e.g. via a linker molecule). The barcode molecules may be linked by being bound to the support and/or by being bound or annealed to linker molecules that are bound to the support. The barcode molecules may be bound to the support (or to the linker molecules) by covalent linkage, non-covalent linkage (e.g. a protein-protein interaction or a streptavidin-biotin bond) or nucleic acid hybridization. The linker molecule may be a biopolymer (e.g. a nucleic acid molecule) or a synthetic polymer. The linker molecule may comprise one or more units of ethylene glycol and/or poly(ethylene) glycol (e.g. hexa-ethylene glycol or penta-ethylene glycol). The linker molecule may comprise one or more ethyl groups, such as a C3 (three-carbon) spacer, C6 spacer, C12 spacer, or C18 spacer.

The barcode molecules may be linked by a macromolecule by being bound to the macromolecule and/or by being annealed to the macromolecule.

The barcode molecules may be linked to the macromolecule directly or indirectly (e.g. via a linker molecule). The barcode molecules may be linked by being bound to the macromolecule and/or by being bound or annealed to linker molecules that are bound to the macromolecule. The barcode molecules may be bound to the macromolecule (or to the linker molecules) by covalent linkage, non-covalent linkage (e.g. a protein-protein interaction or a streptavidin-biotin bond) or nucleic acid hybridization. The linker molecule may be a biopolymer (e.g. a nucleic acid molecule) or a synthetic polymer. The linker molecule may comprise one or more units of ethylene glycol and/or poly(ethylene) glycol (e.g. hexa-ethylene glycol or penta-ethylene glycol). The linker molecule may comprise one or more ethyl groups, such as a C3 (three-carbon) spacer, C6 spacer, C12 spacer, or C18 spacer.

The macromolecule may be a synthetic polymer (e.g. a dendrimer) or a biopolymer such as a nucleic acid (e.g. a single-stranded nucleic acid such as single-stranded DNA), a peptide, a polypeptide or a protein (e.g. a multimeric protein).

The dendrimer may comprise at least 2, at least 3, at least 5, or at least 10 generations.

The macromolecule may be a nucleic acid comprising two or more nucleotides each capable of binding to a barcode molecule. Additionally or alternatively, the nucleic acid may comprise two or more regions each capable of hybridizing to a barcode molecule.

The nucleic acid may comprise a first modified nucleotide and a second modified nucleotide, wherein each modified nucleotide comprises a binding moiety (e.g. a biotin moiety, or an alkyne moiety which may be used for a click-chemical reaction) capable of binding to a barcode molecule. Optionally, the first and second modified nucleotides may be separated by an intervening nucleic acid sequence of at least one, at least two, at least 5 or at least 10 nucleotides.

The nucleic acid may comprise a first hybridisation region and a second hybridisation region, wherein each hybridisation region comprises a sequence complementary to and capable of hybridizing to a sequence of at least one nucleotide within a barcode molecule. The complementary sequence may be at least 5, at least 10, at least 15, at least 20, at least 25 or at least 50 contiguous nucleotides. Preferably, the complementary sequence is at least 8 contiguous nucleotides. Optionally, the first and second hybridisation regions may be separated by an intervening nucleic acid sequence of at least one, at least two, at least 5 or at least 10 nucleotides.

The macromolecule may be a protein such as a multimeric protein e.g. a homomeric protein or a heteromeric protein. For example, the protein may comprise streptavidin e.g. tetrameric streptavidin.

The support may be a solid support or a semi-solid support. The support may comprise a planar surface. The support may be a slide e.g. a glass slide. The slide may be a flow cell for sequencing. If the support is a slide, the first and second barcode molecules may be immobilized in a discrete region on the slide. Optionally, the barcode molecules of each multimeric barcoding reagent in a library are immobilized in a different discrete region on the slide to the barcode molecules of the other multimeric barcoding reagents in the library. The support may be a plate comprising wells, optionally wherein the first and second barcode molecules are immobilized in the same well. Optionally, the barcode molecules of each multimeric barcoding reagent in library are immobilized in a different well of the plate to the barcode molecules of the other multimeric barcoding reagents in the library.

Preferably, the support is a bead (e.g. a gel bead). The bead may be an agarose bead, a silica bead, a styrofoam bead, a gel bead (such as those available from 10x Genomics^{®}), an antibody conjugated bead, an oligo-dT conjugated bead, a streptavidin bead or a magnetic bead (e.g. a superparamagnetic bead). The bead may be of any size and/or molecular structure. For example, the bead may be 10 nanometres to 100 microns in diameter, 100 nanometres to 10 microns in diameter, or 1 micron to 5 microns in diameter. Optionally, the bead is approximately 10 nanometres in diameter, approximately 100 nanometres in diameter, approximately 1 micron in diameter, approximately 10 microns in diameter or approximately 100 microns in diameter. The bead may be solid, or alternatively the bead may be hollow or partially hollow or porous. Beads of certain sizes may be most preferable for certain barcoding methods. For example, beads less than 5.0 microns, or less than 1.0 micron, may be most useful for barcoding nucleic acid targets within individual cells. Preferably, the barcode molecules of each multimeric barcoding reagent in a library are linked together on a different bead to the barcode molecules of the other multimeric barcoding reagents in the library.

The support may be functionalised to enable attachment of two or more barcode molecules. This functionalisation may be enabled through the addition of chemical moieties (e.g. carboxylated groups, alkynes, azides, acrylate groups, amino groups, sulphate groups, or succinimide groups), and/or protein-based moieties (e.g. streptavidin, avidin, or protein G) to the support. The barcode molecules may be attached to the moieties directly or indirectly (e.g. via a linker molecule).

Functionalised supports (e.g. beads) may be brought into contact with a solution of barcode molecules under conditions which promote the attachment of two or more barcode molecules to each bead in the solution (generating multimeric barcoding reagents).

In a library of multimeric barcoding reagents, the barcode molecules of each multimeric barcoding reagent in a library may be linked together on a different support to the barcode molecules of the other multimeric barcoding reagents in the library.

The multimeric barcoding reagent may comprise: at least 2, at least 3, at least 4, at least 5, at least 10, at least 20, at least 25, at least 50, at least 75, at least 100, at least 200, at least 500, at least 1000, at least 5000, at least 10⁴, at least 10⁵, or at least 10⁶ barcode molecules linked together, wherein each barcode molecule is as defined herein; and a barcoded oligonucleotide annealed to each barcode molecule, wherein each barcoded oligonucleotide is as defined herein. Preferably, the multimeric barcoding reagent comprises at least 5 barcode molecules linked together, wherein each barcode molecule is as defined herein; and a barcoded oligonucleotide annealed to each barcode molecule, wherein each barcoded oligonucleotide is as defined herein.

The multimeric barcoding reagent may comprise: at least 2, at least 3, at least 4, at least 5, at least 10, at least 20, at least 25, at least 50, at least 75, at least 100, at least 200, at least 500, at least 1000, at least 5000, at least 10⁴, at least 10⁵, or at least 10⁶ unique or different barcode molecules linked together, wherein each barcode molecule is as defined herein; and a barcoded oligonucleotide annealed to each barcode molecule, wherein each barcoded oligonucleotide is as defined herein. Preferably, the multimeric barcoding reagent comprises at least 5 unique or different barcode molecules linked together, wherein each barcode molecule is as defined herein; and a barcoded oligonucleotide annealed to each barcode molecule, wherein each barcoded oligonucleotide is as defined herein.

A multimeric barcoding reagent may comprise two or more barcoded oligonucleotides as defined herein, wherein the barcoded oligonucleotides each comprise a barcode region. A multimeric barcoding reagent may comprise: at least 2, at least 3, at least 4, at least 5, at least 10, at least 20, at least 25, at least 50, at least 75, at least 100, at least 200, at least 500, at least 1000, at least 5000, at least 10,000, at least 100,000, or at least 1,000,000 unique or different barcoded oligonucleotides. Preferably, the multimeric barcoding reagent comprises at least 5 unique or different barcoded oligonucleotides.

The barcoded oligonucleotides of a multimeric barcoding reagent are linked together (directly or indirectly). The barcoded oligonucleotides of a multimeric barcoding reagent are linked together by a support e.g. a macromolecule, solid support or semi-solid support, as described herein. The multimeric barcoding reagent may comprise one or more polymers to which the barcoded oligonucleotides are annealed or attached. For example, the barcoded oligonucleotides of a multimeric barcoding reagent may be annealed to a multimeric hybridization molecule e.g. a multimeric barcode molecule. Alternatively, the barcoded oligonucleotides of a multimeric barcoding reagent may be linked together by a macromolecule (such as a synthetic polymer e.g. a dendrimer, or a biopolymer e.g. a protein) or a support (such as a solid support or a semi-solid support e.g. a gel bead). Additionally or alternatively, the barcoded oligonucleotides of a (single) multimeric barcoding reagent may linked together by being comprised within a (single) lipid carrier (e.g. a liposome or a micelle).

A multimeric barcoding reagent may comprise: first and second hybridization molecules linked together (i.e. a multimeric hybridization molecule), wherein each of the hybridization molecules comprises a nucleic acid sequence comprising a hybridization region; and first and second barcoded oligonucleotides, wherein the first barcoded oligonucleotide is annealed to the hybridization region of the first hybridization molecule and wherein the second barcoded oligonucleotide is annealed to the hybridization region of the second hybridization molecule.

The hybridization molecules comprise or consist of deoxyribonucleotides. One or more of the deoxyribonucleotides may be a modified deoxyribonucleotide (e.g. a deoxyribonucleotide modified with a biotin moiety or a deoxyuracil nucleotide). The hybridization molecules may comprise one or more degenerate nucleotides or sequences. The hybridization molecules may not comprise any degenerate nucleotides or sequences.

The hybridization molecules of a multimeric hybridization molecule may be linked on a nucleic acid molecule. Such a nucleic acid molecule may provide the backbone to which single-stranded barcoded oligonucleotides may be annealed. The hybridization molecules of a multimeric hybridization molecule may be comprised within a (single) nucleic acid molecule. A multimeric hyrbidization molecule may comprise a single, contiguous nucleic acid sequence comprising two or more hybridization molecules. A multimeric hybridization molecule may be a single-stranded nucleic acid molecule (e.g. single-stranded DNA) comprising two or more hybridization molecules. A multimeric hybridization molecule may comprise one or more double-stranded regions. Optionally, in a double-stranded region or between two different double-stranded regions, a multimeric hybridization molecule may comprise one or more nicks, or one or more gaps, where the multimeric hybridization molecule itself has been divided or separated. Any said gap may be at least one, at least 2, at least 5, at least 10, at least 20, at least 50, or at least 100 nucleotides in length. Said nicks and/or gaps may serve the purpose of increasing the molecular flexibility of the multimeric hybridization molecule and/or multimeric barcoding reagent, for example to increase the accessibility of the molecule or reagent to interact with target nucleic acid molecules. Said nicks and/or gaps may also enable more efficient purification or removal of said molecules or reagents. A molecule and/or reagent comprising said nick(s) and/or gap(s) may retain links between different hybridization molecules by having a complementary DNA strand which is jointly hybridised to regions of two or more divided parts of a multimeric hybridization molecule.

The hybridization molecules may be linked by a macromolecule by being bound to the macromolecule and/or by being annealed to the macromolecule.

The hybridization molecules may be linked to the macromolecule directly or indirectly (e.g. via a linker molecule). The hybridization molecules may be linked by being bound to the macromolecule and/or by being bound or annealed to linker molecules that are bound to the macromolecule. The hybridization molecules may be bound to the macromolecule (or to the linker molecules) by covalent linkage, non-covalent linkage (e.g. a protein-protein interaction or a streptavidin-biotin bond) or nucleic acid hybridization. The linker molecule may be a biopolymer (e.g. a nucleic acid molecule) or a synthetic polymer. The linker molecule may comprise one or more units of ethylene glycol and/or poly(ethylene) glycol (e.g. hexa-ethylene glycol or penta-ethylene glycol). The linker molecule may comprise one or more ethyl groups, such as a C3 (three-carbon) spacer, C6 spacer, C12 spacer, or C18 spacer.

The macromolecule may be a synthetic polymer (e.g. a dendrimer) or a biopolymer such as a nucleic acid (e.g. a single-stranded nucleic acid such as single-stranded DNA), a peptide, a polypeptide or a protein (e.g. a multimeric protein).

The dendrimer may comprise at least 2, at least 3, at least 5, or at least 10 generations.

The macromolecule may be a nucleic acid comprising two or more nucleotides each capable of binding to a hybridization molecule. Additionally or alternatively, the nucleic acid may comprise two or more regions each capable of hybridizing to a hybridization molecule.

The nucleic acid may comprise a first modified nucleotide and a second modified nucleotide, wherein each modified nucleotide comprises a binding moiety (e.g. a biotin moiety, or an alkyne moiety which may be used for a click-chemical reaction) capable of binding to a hybridization molecule. Optionally, the first and second modified nucleotides may be separated by an intervening nucleic acid sequence of at least one, at least two, at least 5 or at least 10 nucleotides.

The nucleic acid may comprise a first hybridisation region and a second hybridisation region, wherein each hybridisation region comprises a sequence complementary to and capable of hybridizing to a sequence of at least one nucleotide within a hybridization molecule. The complementary sequence may be at least 5, at least 10, at least 15, at least 20, at least 25 or at least 50 contiguous nucleotides. Optionally, the first and second hybridisation regions may be separated by an intervening nucleic acid sequence of at least one, at least two, at least 5 or at least 10 nucleotides.

The macromolecule may be a protein such as a multimeric protein e.g. a homomeric protein or a heteromeric protein. For example, the protein may comprise streptavidin e.g. tetrameric streptavidin.

The hybridization molecules may be linked by a support. The hybridization molecules may be linked to the support directly or indirectly (e.g. via a linker molecule). The hybridization molecules may be linked by being bound to the support and/or by being bound or annealed to linker molecules that are bound to the support. The hybridization molecules may be bound to the support (or to the linker molecules) by covalent linkage, non-covalent linkage (e.g. a protein-protein interaction or a streptavidin-biotin bond) or nucleic acid hybridization. The linker molecule may be a biopolymer (e.g. a nucleic acid molecule) or a synthetic polymer. The linker molecule may comprise one or more units of ethylene glycol and/or poly(ethylene) glycol (e.g. hexa-ethylene glycol or penta-ethylene glycol). The linker molecule may comprise one or more ethyl groups, such as a C3 (three-carbon) spacer, C6 spacer, C12 spacer, or C18 spacer.

The support may be a solid support or a semi-solid support. The support may comprise a planar surface. The support may be a slide e.g. a glass slide. The slide may be a flow cell for sequencing. If the support is a slide, the first and second hybridization molecules may be immobilized in a discrete region on the slide. Optionally, the hybridization molecules of each multimeric barcoding reagent in a library are immobilized in a different discrete region on the slide to the hybridization molecules of the other multimeric barcoding reagents in the library. The support may be a plate comprising wells, optionally wherein the first and second hybridization molecules are immobilized in the same well. Optionally, the hybridization molecules of each multimeric barcoding reagent in library are immobilized in a different well of the plate to the hybridization molecules of the other multimeric barcoding reagents in the library.

Preferably, the support is a bead (e.g. a gel bead). The bead may be an agarose bead, a silica bead, a styrofoam bead, a gel bead (such as those available from 10x Genomics^{®}), an antibody conjugated bead, an oligo-dT conjugated bead, a streptavidin bead or a magnetic bead (e.g. a superparamagnetic bead). The bead may be of any size and/or molecular structure. For example, the bead may be 10 nanometres to 100 microns in diameter, 100 nanometres to 10 microns in diameter, or 1 micron to 5 microns in diameter. Optionally, the bead is approximately 10 nanometres in diameter, approximately 100 nanometres in diameter, approximately 1 micron in diameter, approximately 10 microns in diameter or approximately 100 microns in diameter. The bead may be solid, or alternatively the bead may be hollow or partially hollow or porous. Beads of certain sizes may be most preferable for certain barcoding methods. For example, beads less than 5.0 microns, or less than 1.0 micron, may be most useful for barcoding nucleic acid targets within individual cells. Preferably, the hybridization molecules of each multimeric barcoding reagent in a library are linked together on a different bead to hybridization molecules of the other multimeric barcoding reagents in the library.

The support may be functionalised to enable attachment of two or more hybridization molecules. This functionalisation may be enabled through the addition of chemical moieties (e.g. carboxylated groups, alkynes, azides, acrylate groups, amino groups, sulphate groups, or succinimide groups), and/or protein-based moieties (e.g. streptavidin, avidin, or protein G) to the support. The hybridization molecules may be attached to the moieties directly or indirectly (e.g. via a linker molecule).

Functionalised supports (e.g. beads) may be brought into contact with a solution of hybridization molecules under conditions which promote the attachment of two or more hybridization molecules to each bead in the solution (generating multimeric barcoding reagents).

In a library of multimeric barcoding reagents, the hybridization molecules of each multimeric barcoding reagent in a library may be linked together on a different support to the hybridization molecules of the other multimeric barcoding reagents in the library.

Optionally, the hybridization molecules are attached to the beads by covalent linkage, non-covalent linkage (e.g. a streptavidin-biotin bond) or nucleic acid hybridization.

The multimeric barcoding reagent may comprise: at least 2, at least 3, at least 4, at least 5, at least 10, at least 20, at least 25, at least 50, at least 75, at least 100, at least 200, at least 500, at least 1000, at least 5000, or at least 10,000 hybridization molecules linked together, wherein each hybridization molecule is as defined herein; and a barcoded oligonucleotide annealed to each hybridization molecule, wherein each barcoded oligonucleotide is as defined herein. Preferably, the multimeric barcoding reagent comprises at least 5 hybridization molecules linked together, wherein each hybridization molecule is as defined herein; and a barcoded oligonucleotide annealed to each hybridization molecule, wherein each barcoded oligonucleotide is as defined herein.

The multimeric barcoding reagent may comprise: at least 2, at least 3, at least 4, at least 5, at least 10, at least 20, at least 25, at least 50, at least 75, at least 100, at least 200, at least 500, at least 1000, at least 5000, or at least 10,000 unique or different hybridization molecules linked together, wherein each hybridization molecule is as defined herein; and a barcoded oligonucleotide annealed to each hybridization molecule, wherein each barcoded oligonucleotide is as defined herein. Preferably, the multimeric barcoding reagent comprises at least 5 unique or different hybridization molecules linked together, wherein each hybridization molecule is as defined herein; and a barcoded oligonucleotide annealed to each hybridization molecule, wherein each barcoded oligonucleotide is as defined herein.

The multimeric hybridization molecule may be a multimeric barcode molecule, wherein the first hybridization molecule is a first barcode molecule and the second hybridization molecule is a second barcode molecule. A multimeric barcoding reagent may comprise: first and second barcode molecules linked together (i.e. a multimeric barcode molecule), wherein each of the barcode molecules comprises a nucleic acid sequence comprising a barcode region; and first and second barcoded oligonucleotides, wherein the first barcoded oligonucleotide is annealed to the barcode region of the first barcode molecule, and wherein the second barcoded oligonucleotide is annealed to the barcode region of the second barcode molecule.

The barcoded oligonucleotides of a multimeric barcoding reagent may comprise: a first barcoded oligonucleotide comprising, optionally in the 5' to 3' direction, a barcode region, and a target region capable of annealing or ligating to a first sub-sequence of the target nucleic acid; and a second barcoded oligonucleotide comprising, optionally in the 5' to 3' direction, a barcode region, and a target region capable of annealing or ligating to a second sub-sequence of the target nucleic acid.

The barcoded oligonucleotides of a multimeric barcoding reagent may comprise: a first barcoded oligonucleotide comprising a barcode region, and a target region capable of ligating to a first sub-sequence of the target nucleic acid; and a second barcoded oligonucleotide comprising a barcode region, and a target region capable of ligating to a second sub-sequence of the target nucleic acid.

The barcoded oligonucleotides of a multimeric barcoding reagent may comprise: a first barcoded oligonucleotide comprising, in the 5' to 3' direction, a barcode region, and a target region capable of annealing to a first sub-sequence of the target nucleic acid; and a second barcoded oligonucleotide comprising, in the 5' to 3' direction, a barcode region, and a target region capable of annealing to a second sub-sequence of the target nucleic acid.

### 2. GENERAL PROPERTIES OF BARCODED OLIGONUCLEOTIDES

A barcoded oligonucleotide comprises a barcode region. The barcoded oligonucleotides may comprise, optionally in the 5' to 3' direction, a barcode region and a target region. The target region is capable of annealing or ligating to a sub-sequence of the target nucleic acid.

Alternatively, a barcoded oligonucleotide may consist essentially of or consist of a barcode region.

The 5' end of a barcoded oligonucleotide may be phosphorylated. This may enable the 5' end of the barcoded oligonucleotide to be ligated to the 3' end of a target nucleic acid. Alternatively, the 5' end of a barcoded oligonucleotide may not be phosphorylated.

A barcoded oligonucleotide may be a single-stranded nucleic acid molecule (e.g. single-stranded DNA). A barcoded oligonucleotide may comprise one or more double-stranded regions. A barcoded oligonucleotide may be a double-stranded nucleic acid molecule (e.g. double-stranded DNA).

The barcoded oligonucleotides may comprise or consist of deoxyribonucleotides. One or more of the deoxyribonucleotides may be a modified deoxyribonucleotide (e.g. a deoxyribonucleotide modified with a biotin moiety or a deoxyuracil nucleotide). The barcoded oligonucleodides may comprise one or more degenerate nucleotides or sequences. The barcoded oligonucleotides may not comprise any degenerate nucleotides or sequences.

The barcode regions of each barcoded oligonucleotide may comprise different sequences. Each barcode region may comprise a sequence that identifies the multimeric barcoding reagent. For example, this sequence may be a constant region shared by all barcode regions of a single multimeric barcoding reagent. The barcode region of each barcoded oligonucleotide may contain a unique sequence which is not present in other barcoded oligonucleotides, and may thus serve to uniquely identify each barcoded oligonucleotide. Each barcode region may comprise at least 5, at least 10, at least 15, at least 20, at least 25, at least 50 or at least 100 nucleotides. Preferably, each barcode region comprises at least 5 nucleotides. Preferably each barcode region comprises deoxyribonucleotides, optionally all of the nucleotides in a barcode region are deoxyribonucleotides. One or more of the deoxyribonucleotides may be a modified deoxyribonucleotide (e.g. a deoxyribonucleotide modified with a biotin moiety or a deoxyuracil nucleotide). The barcode regions may comprise one or more degenerate nucleotides or sequences. The barcode regions may not comprise any degenerate nucleotides or sequences.

The target regions of each barcoded oligonucleotide may comprise different sequences. Each target region may comprise a sequence capable of annealing to only a single sub-sequence of a target nucleic acid within a sample of nucleic acids (i.e. a target specific sequence). Each target region may comprise one or more random, or one or more degenerate, sequences to enable the target region to anneal to more than one sub-sequence of a target nucleic acid. Each target region may comprise at least 5, at least 10, at least 15, at least 20, at least 25, at least 50 or at least 100 nucleotides. Preferably, each target region comprises at least 5 nucleotides. Each target region may comprise 5 to 100 nucleotides, 5 to 10 nucleotides, 10 to 20 nucleotides, 20 to 30 nucleotides, 30 to 50 nucleotides, 50 to 100 nucleotides, 10 to 90 nucleotides, 20 to 80 nucleotides, 30 to 70 nucleotides or 50 to 60 nucleotides. Preferably, each target region comprises 30 to 70 nucleotides. Preferably each target region comprises deoxyribonucleotides, optionally all of the nucleotides in a target region are deoxyribonucleotides. One or more of the deoxyribonucleotides may be a modified deoxyribonucleotide (e.g. a deoxyribonucleotide modified with a biotin moiety or a deoxyuracil nucleotide). Each target region may comprise one or more universal bases (e.g. inosine), one or modified nucleotides and/or one or more nucleotide analogues.

The target regions may be used to anneal the barcoded oligonucleotides to sub-sequences of target nucleic acids, and then may be used as primers for a primer-extension reaction or an amplification reaction e.g. a polymerase chain reaction. Alternatively, the target regions may be used to ligate the barcoded oligonucleotides to sub-sequences of target nucleic acids. The target region may be at the 5' end of a barcoded oligonucleotide. Such a target region may be phosphorylated. This may enable the 5' end of the target region to be ligated to the 3' end of a sub-sequence of a target nucleic acid.

The barcoded oligonucleotides may further comprise one or more adapter region(s). An adapter region may be between the barcode region and the target region. A barcoded oligonucleotide may, for example, comprise an adapter region 5' of a barcode region (a 5' adapter region) and/or an adapter region 3' of the barcode region (a 3' adapter region). Optionally, the barcoded oligonucleotides comprise, in the 5' to 3' direction, a barcode region, an adapter region and a target region.

The adapter region(s) of the barcoded oligonucleotides may comprise a sequence complementary to an adapter region of a multimeric barcode molecule or a sequence complementary to a hybridization region of a multimeric hybridization molecule. The adapter region(s) of the barcoded oligonucleotides may enable the barcoded oligonucleotides to be linked to a macromolecule or support (e.g. a bead). The adapter region(s) may be used for manipulating, purifying, retrieving, amplifying, or detecting barcoded oligonucleotides and/or target nucleic acids to which they may anneal or ligate.

The adapter region of each barcoded oligonucleotide may comprise a constant region. Optionally, all adapter regions of barcoded oligonucleotides of each multimeric barcoding reagent are substantially identical. The adapter region may comprise at least 1, at least 2, at least 3, at least 4, at least 5, at least 6, at least 8, at least 10, at least 15, at least 20, at least 25, at least 50, at least 100, or at least 250 nucleotides. Preferably, the adapter region comprises at least 4 nucleotides. Preferably each adapter region comprises deoxyribonucleotides, optionally all of the nucleotides in an adapter region are deoxyribonucleotides. One or more of the deoxyribonucleotides may be a modified deoxyribonucleotide (e.g. a deoxyribonucleotide modified with a biotin moiety or a deoxyuracil nucleotide). Each adapter region may comprise one or more universal bases (e.g. inosine), one or modified nucleotides and/or one or more nucleotide analogues.

The barcoded oligonucleotides may be synthesized by a chemical oligonucleotide synthesis process. The barcoded oligonucleotides synthesis process may include one or more step of an enzymatic production process, an enzymatic amplification process, or an enzymatic modification procedure, such as an in vitro transcription process, a reverse transcription process, a primer-extension process, or a polymerase chain reaction process.

These general properties of barcoded oligonucleotides are applicable to any of the multimeric barcoding reagents described herein.

### 3. GENERAL PROPERTIES OF LIBRARIES OF MULTIMERIC BARCODING REAGENTS

The invention provides a library of multimeric barcoding reagents comprising first and second multimeric barcoding reagents as defined herein, wherein the barcode regions of the first multimeric barcoding reagent are different to the barcode regions of the second multimeric barcoding reagent.

The library of multimeric barcoding reagents may comprise at least 5, at least 10, at least 20, at least 25, at least 50, at least 75, at least 100, at least 250, at least 500, at least 10³, at least 10⁴, at least 10⁵, at least 10⁶, at least 10⁷, at least 10⁸ or at least 10⁹ multimeric barcoding reagents as defined herein. Preferably, the library comprises at least 10 multimeric barcoding reagents as defined herein. Preferably, the first and second barcode regions of each multimeric barcoding reagent are different to the barcode regions of at least 9 other multimeric barcoding reagents in the library.

The first and second barcode regions of each multimeric barcoding reagent may be different to the barcode regions of at least 4, at least 9, at least 19, at least 24, at least 49, at least 74, at least 99, at least 249, at least 499, at least 999 (i.e. 10³-1), at least 10⁴-1, at least 10⁵-1, at least 10⁶-1, at least 10⁷-1, at least 10⁸-1 or at least 10⁹-1 other multimeric barcoding reagents in the library. The first and second barcode regions of each multimeric barcoding reagent may be different to the barcode regions of all of the other multimeric barcoding reagents in the library. Preferably, the first and second barcode regions of each multimeric barcoding reagent are different to the barcode regions of at least 9 other multimeric barcoding reagents in the library.

The barcode regions of each multimeric barcoding reagent may be different to the barcode regions of at least 4, at least 9, at least 19, at least 24, at least 49, at least 74, at least 99, at least 249, at least 499, at least 999 (i.e. 10³-1), at least 10⁴-1, at least 10⁵-1, at least 10⁶-1, at least 10⁷-1, at least 10⁸-1 or at least 10⁹-1 other multimeric barcoding reagents in the library. The barcode regions of each multimeric barcoding reagent may be different to the barcode regions of all of the other multimeric barcoding reagents in the library. Preferably, the barcode regions of each multimeric barcoding reagent are different to the barcode regions of at least 9 other multimeric barcoding reagents in the library.

The invention provides a library of multimeric barcoding reagents comprising first and second multimeric barcoding reagents as defined herein, wherein the barcode regions of the barcoded oligonucleotides of the first multimeric barcoding reagent are different to the barcode regions of the barcoded oligonucleotides of the second multimeric barcoding reagent.

Different multimeric barcoding reagents within a library of multimeric barcoding reagents may comprise different numbers of barcoded oligonucleotides.

The library of multimeric barcoding reagents may comprise at least 5, at least 10, at least 20, at least 25, at least 50, at least 75, at least 100, at least 250, at least 500, at least 10³, at least 10⁴, at least 10⁵, at least 10⁶, at least 10⁷, at least 10⁸ or at least 10⁹ multimeric barcoding reagents as defined herein. Preferably, the library comprises at least 10 multimeric barcoding reagents as defined herein. Preferably, the barcode regions of the first and second barcoded oligonucleotides of each multimeric barcoding reagent are different to the barcode regions of the barcoded oligonucleotides of at least 9 other multimeric barcoding reagents in the library.

The barcode regions of the first and second barcoded oligonucleotides of each multimeric barcoding reagent may be different to the barcode regions of the barcoded oligonucleotides of at least 4, at least 9, at least 19, at least 24, at least 49, at least 74, at least 99, at least 249, at least 499, at least 999 (i.e. 10³-1), at least 10⁴-1, at least 10⁵-1, at least 10⁶-1, at least 10⁷-1, at least 10⁸-1 or at least 10⁹-1 other multimeric barcoding reagents in the library. The barcode regions of the first and second barcoded oligonucleotides of each multimeric barcoding reagent may be different to the barcode regions of the barcoded oligonucleotides of all of the other multimeric barcoding reagents in the library. Preferably, the barcode regions of the first and second barcoded oligonucleotides of each multimeric barcoding reagent are different to the barcode regions of the barcoded oligonucleotides of at least 9 other multimeric barcoding reagents in the library.

The barcode regions of the barcoded oligonucleotides of each multimeric barcoding reagent may be different to the barcode regions of the barcoded oligonucleotides of at least 4, at least 9, at least 19, at least 24, at least 49, at least 74, at least 99, at least 249, at least 499, at least 999 (i.e. 10³-1), at least 10⁴-1, at least 10⁵-1, at least 10⁶-1, at least 10⁷-1, at least 10⁸-1 or at least 10⁹-1 other multimeric barcoding reagents in the library. The barcode regions of the barcoded oligonucleotides of each multimeric barcoding reagent may be different to the barcode regions of the barcoded oligonucleotides of all of the other multimeric barcoding reagents in the library. Preferably, the barcode regions of the barcoded oligonucleotides of each multimeric barcoding reagent are different to the barcode regions of the barcoded oligonucleotides of at least 9 other multimeric barcoding reagents in the library.

These general properties of libraries of multimeric barcoding reagents are applicable to any of the multimeric barcoding reagents described herein.

### 4. MULTIMERIC BARCODING REAGENTS COMPRISING BARCODED OLIGONUCLEOTIDES ANNEALED TO A MULTIMERIC BARCODE MOLECULE

The invention provides a multimeric barcoding reagent for labelling a target nucleic acid, wherein the reagent comprises: first and second barcode molecules linked together (i.e. a multimeric barcode molecule), wherein each of the barcode molecules comprises a nucleic acid sequence comprising a barcode region; and first and second barcoded oligonucleotides, wherein the first barcoded oligonucleotide comprises, optionally in the 5' to 3' direction, a barcode region annealed to the barcode region of the first barcode molecule and a target region capable of annealing or ligating to a first sub-sequence of the target nucleic acid, and wherein the second barcoded oligonucleotide comprises, optionally in the 5' to 3' direction, a barcode region annealed to the barcode region of the second barcode molecule and a target region capable of annealing or ligating to a second sub-sequence of the target nucleic acid.

The invention provides a multimeric barcoding reagent for labelling a target nucleic acid, wherein the reagent comprises: first and second barcode molecules linked together (i.e. a multimeric barcode molecule), wherein each of the barcode molecules comprises a nucleic acid sequence comprising a barcode region; and first and second barcoded oligonucleotides, wherein the first barcoded oligonucleotide comprises a barcode region annealed to the barcode region of the first barcode molecule and a target region capable of ligating to a first sub-sequence of the target nucleic acid, and wherein the second barcoded oligonucleotide comprises a barcode region annealed to the barcode region of the second barcode molecule and a target region capable of ligating to a second sub-sequence of the target nucleic acid.

The invention provides a multimeric barcoding reagent for labelling a target nucleic acid, wherein the reagent comprises: first and second barcode molecules linked together (i.e. a multimeric barcode molecule), wherein each of the barcode molecules comprises a nucleic acid sequence comprising a barcode region; and first and second barcoded oligonucleotides, wherein the first barcoded oligonucleotide comprises in the 5' to 3' direction a barcode region annealed to the barcode region of the first barcode molecule and a target region capable of annealing to a first sub-sequence of the target nucleic acid, and wherein the second barcoded oligonucleotide comprises in the 5' to 3' direction a barcode region annealed to the barcode region of the second barcode molecule and a target region capable of annealing to a second sub-sequence of the target nucleic acid.

The invention provides a multimeric barcoding reagent for labelling a target nucleic acid, wherein the reagent comprises: first and second barcode molecules linked together (i.e. a multimeric barcode molecule), wherein each of the barcode molecules comprises a nucleic acid sequence comprising a barcode region; and first and second barcoded oligonucleotides, wherein the first barcoded oligonucleotide comprises a barcode region annealed to the barcode region of the first barcode molecule and capable of ligating to a first sub-sequence of the target nucleic acid, and wherein the second barcoded oligonucleotide comprises a barcode region annealed to the barcode region of the second barcode molecule and capable of ligating to a second sub-sequence of the target nucleic acid.

Each barcoded oligonucleotide may consist essentially of or consist of a barcode region.

Preferably, the barcode molecules comprise or consist of deoxyribonucleotides. One or more of the deoxyribonucleotides may be a modified deoxyribonucleotide (e.g. a deoxyribonucleotide modified with a biotin moiety or a deoxyuracil nucleotide). The barcode molecules may comprise one or more degenerate nucleotides or sequences. The barcode molecules may not comprise any degenerate nucleotides or sequences.

The barcode regions may uniquely identify each of the barcode molecules. Each barcode region may comprise a sequence that identifies the multimeric barcoding reagent. For example, this sequence may be a constant region shared by all barcode regions of a single multimeric barcoding reagent. Each barcode region may comprise at least 5, at least 10, at least 15, at least 20, at least 25, at least 50 or at least 100 nucleotides. Preferably, each barcode region comprises at least 5 nucleotides. Preferably each barcode region comprises deoxyribonucleotides, optionally all of the nucleotides in a barcode region are deoxyribonucleotides. One or more of the deoxyribonucleotides may be a modified deoxyribonucleotide (e.g. a deoxyribonucleotide modified with a biotin moiety or a deoxyuracil nucleotide). The barcode regions may comprise one or more degenerate nucleotides or sequences. The barcode regions may not comprise any degenerate nucleotides or sequences.

Preferably, the barcode region of the first barcoded oligonucleotide comprises a sequence that is complementary and annealed to the barcode region of the first barcode molecule and the barcode region of the second barcoded oligonucleotide comprises a sequence that is complementary and annealed to the barcode region of the second barcode molecule. The complementary sequence of each barcoded oligonucleotide may be at least 5, at least 10, at least 15, at least 20, at least 25, at least 50 or at least 100 contiguous nucleotides.

The target regions of the barcoded oligonucleotides (which are not annealed to the multimeric barcode molecule(s)) may be non-complementary to the multimeric barcode molecule(s).

The barcoded oligonucleotides may comprise a linker region between the barcode region and the target region. The linker region may comprise one or more contiguous nucleotides that are not annealed to the multimeric barcode molecule and are non-complementary to the subsequences of the target nucleic acid. The linker may comprise 1 to 100, 5 to 75, 10 to 50, 15 to 30 or 20 to 25 non-complementary nucleotides. Preferably, the linker comprises 15 to 30 non-complementary nucleotides. The use of such a linker region enhances the efficiency of the barcoding reactions performed using the multimeric barcoding reagents.

Barcode molecules may further comprise one or more nucleic acid sequences that are not complementary to barcode regions of barcoded oligonucleotides. For example, barcode molecules may comprise one or more adapter regions. A barcode molecule, may, for example, comprise an adapter region 5' of a barcode region (a 5' adapter region) and/or an adapter region 3' of the barcode region (a 3' adapter region). The adapter region(s) (and/or one or more portions of an adapter region) may be complementary to and anneal to oligonucleotides e.g. the adapter regions of barcoded oligonucleotides. Alternatively, the adapter region(s) (and/or one or more portions of an adapter region) of barcode molecule may not be complementary to sequences of barcoded oligonucleotides. The adapter region(s) may be used for manipulating, purifying, retrieving, amplifying, and/or detecting barcode molecules.

The multimeric barcoding reagent may be configured such that: each of the barcode molecules comprises a nucleic acid sequence comprising in the 5' to 3' direction an adapter region and a barcode region; the first barcoded oligonucleotide comprises, optionally in the 5' to 3' direction, a barcode region annealed to the barcode region of the first barcode molecule, an adapter region annealed to the adapter region of the first barcode molecule and a target region capable of annealing to a first sub-sequence of the target nucleic acid; and the second barcoded oligonucleotide comprises, optionally in the 5' to 3' direction, a barcode region annealed to the barcode region of the second barcode molecule, an adapter region annealed to the adapter region of the second barcode molecule and a target region capable of annealing to a second sub-sequence of the target nucleic acid.

The adapter region of each barcode molecule may comprise a constant region. Optionally, all adapter regions of a multimeric barcoding reagent are substantially identical. The adapter region may comprise at least 1, at least 2, at least 3, at least 4, at least 5, at least 6, at least 8, at least 10, at least 15, at least 20, at least 25, at least 50, at least 100, or at least 250 nucleotides. Preferably, the adapter region comprises at least 4 nucleotides. Preferably each adapter region comprises deoxyribonucleotides, optionally all of the nucleotides in an adapter region are deoxyribonucleotides. One or more of the deoxyribonucleotides may be a modified deoxyribonucleotide (e.g. a deoxyribonucleotide modified with a biotin moiety or a deoxyuracil nucleotide). Each adapter region may comprise one or more universal bases (e.g. inosine), one or modified nucleotides and/or one or more nucleotide analogues.

The barcoded oligonucleotides may comprise a linker region between the adapter region and the target region. The linker region may comprise one or more contiguous nucleotides that are not annealed to the multimeric barcode molecule and are non-complementary to the subsequences of the target nucleic acid. The linker may comprise 1 to 100, 5 to 75, 10 to 50, 15 to 30 or 20 to 25 non-complementary nucleotides. Preferably, the linker comprises 15 to 30 non-complementary nucleotides. The use of such a linker region enhances the efficiency of the barcoding reactions performed using the multimeric barcoding reagents.

The barcode molecules of a multimeric barcode molecule may be linked on a nucleic acid molecule. Such a nucleic acid molecule may provide the backbone to which single-stranded barcoded oligonucleotides may be annealed. Alternatively, the barcode molecules of a multimeric barcode molecule may be linked together by any of the other means described herein.

The multimeric barcoding reagent may comprise: at least 2, at least 3, at least 4, at least 5, at least 10, at least 20, at least 25, at least 50, at least 75, at least 100, at least 200, at least 500, at least 1000, at least 5000, or at least 10,000 barcode molecules linked together, wherein each barcode molecule is as defined herein; and a barcoded oligonucleotide annealed to each barcode molecule, wherein each barcoded oligonucleotide is as defined herein. Preferably, the multimeric barcoding reagent comprises at least 5 barcode molecules linked together, wherein each barcode molecule is as defined herein; and a barcoded oligonucleotide annealed to each barcode molecule, wherein each barcoded oligonucleotide is as defined herein.

The multimeric barcoding reagent may comprise: at least 2, at least 3, at least 4, at least 5, at least 10, at least 20, at least 25, at least 50, at least 75, at least 100, at least 200, at least 500, at least 1000, at least 5000, at least 10⁴, at least 10⁵, or at least 10⁶ unique or different barcode molecules linked together, wherein each barcode molecule is as defined herein; and a barcoded oligonucleotide annealed to each barcode molecule, wherein each barcoded oligonucleotide is as defined herein. Preferably, the multimeric barcoding reagent comprises at least 5 unique or different barcode molecules linked together, wherein each barcode molecule is as defined herein; and a barcoded oligonucleotide annealed to each barcode molecule, wherein each barcoded oligonucleotide is as defined herein.

The multimeric barcoding reagent may comprise: at least 5, at least 10, at least 20, at least 25, at least 50, at least 75, at least 100, at least 200, at least 500, at least 1000, at least 5000, or at least 10,000 barcode regions, wherein each barcode region is as defined herein; and a barcoded oligonucleotide annealed to each barcode region, wherein each barcoded oligonucleotide is as defined herein. Preferably, the multimeric barcoding reagent comprises at least 5 barcode regions, wherein each barcode region is as defined herein; and a barcoded oligonucleotide annealed to each barcode region, wherein each barcoded oligonucleotide is as defined herein.

The multimeric barcoding reagent may comprise: at least 2, at least 3, at least 4, at least 5, at least 10, at least 20, at least 25, at least 50, at least 75, at least 100, at least 200, at least 500, at least 1000, at least 5000, at least 10⁴, at least 10⁵, or at least 10⁶ unique or different barcode regions, wherein each barcode region is as defined herein; and a barcoded oligonucleotide annealed to each barcode region, wherein each barcoded oligonucleotide is as defined herein. Preferably, the multimeric barcoding reagent comprises at least 5 unique or different barcode regions, wherein each barcode region is as defined herein; and a barcoded oligonucleotide annealed to each barcode region, wherein each barcoded oligonucleotide is as defined herein.

Figure 1 shows a multimeric barcoding reagent, including first (D1, E1, and F1) and second (D2, E2, and F2) barcode molecules, which each include a nucleic acid sequence comprising a barcode region (E1 and E2). These first and second barcode molecules are linked together, for example by a connecting nucleic acid sequence (S). The multimeric barcoding reagent also comprises first (A1, B1, C1, and G1) and second (A2, B2, C2, and G2) barcoded oligonucleotides. These barcoded oligonucleotides each comprise a barcode region (B1 and B2) and a target region (G1 and G2).

The barcode regions within the barcoded oligonucleotides may each contain a unique sequence which is not present in other barcoded oligonucleotides, and may thus serve to uniquely identify each such barcode molecule. The target regions may be used to anneal the barcoded oligonucleotides to sub-sequences of target nucleic acids, and then may be used as primers for a primer-extension reaction or an amplification reaction e.g. a polymerase chain reaction.

Each barcode molecule may optionally also include a 5' adapter region (F1 and F2). The barcoded oligonucleotides may then also include a 3' adapter region (C1 and C2) that is complementary to the 5' adapter region of the barcode molecules.

Each barcode molecule may optionally also include a 3' region (D1 and D2), which may be comprised of identical sequences within each barcode molecule. The barcoded oligonucleotides may then also include a 5' region (A1 and A2) which is complementary to the 3' region of the barcode molecules. These 3' regions may be useful for manipulation or amplification of nucleic acid sequences, for example sequences that are generated by labeling a nucleic acid target with a barcoded oligonucleotide. The 3' region may comprise at least 4, at least 5, at least 6, at least 8, at least 10, at least 15, at least 20, at least 25, at least 50, at least 100, or at least 250 nucleotides. Preferably, the 3' region comprises at least 4 nucleotides. Preferably each 3' region comprises deoxyribonucleotides, optionally all of the nucleotides in an 3' region are deoxyribonucleotides. One or more of the deoxyribonucleotides may be a modified deoxyribonucleotide (e.g. a deoxyribonucleotide modified with a biotin moiety or a deoxyuracil nucleotide). Each 3' region may comprise one or more universal bases (e.g. inosine), one or modified nucleotides and/or one or more nucleotide analogues.

The invention provides a library of multimeric barcoding reagents comprising at least 10 multimeric barcoding reagents for labelling a target nucleic acid for sequencing, wherein each multimeric barcoding reagent comprises: first and second barcode molecules comprised within a (single) nucleic acid molecule, wherein each of the barcode molecules comprises a nucleic acid sequence comprising a barcode region; and first and second barcoded oligonucleotides, wherein the first barcoded oligonucleotide comprises, optionally in the 5' to 3' direction, a barcode region complementary and annealed to the barcode region of the first barcode molecule and a target region capable of annealing or ligating to a first sub-sequence of the target nucleic acid, and wherein the second barcoded oligonucleotide comprises, optionally in the 5' to 3' direction, a barcode region complementary and annealed to the barcode region of the second barcode molecule and a target region capable of annealing or ligating to a second sub-sequence of the target nucleic acid. Preferably, the barcode regions of the first and second barcoded oligonucleotides of each multimeric barcoding reagent are different to the barcode regions of the barcoded oligonucleotides of at least 9 other multimeric barcoding reagents in the library.

### 5. MULTIMERIC BARCODING REAGENTS COMPRISING BARCODED OLIGONUCLEOTIDES ANNEALED TO A MULTIMERIC HYBRIDIZATION MOLECULE

The invention provides a multimeric barcoding reagent for labelling a target nucleic acid, wherein the reagent comprises: first and second hybridization molecules linked together (i.e. a multimeric hybridization molecule), wherein each of the hybridization molecules comprises a nucleic acid sequence comprising a hybridization region; and first and second barcoded oligonucleotides, wherein the first barcoded oligonucleotide comprises, optionally in the 5' to 3' direction, an adapter region annealed to the hybridization region of the first hybridization molecule, a barcode region, and a target region capable of annealing or ligating to a first sub-sequence of the target nucleic acid, and wherein the second barcoded oligonucleotide comprises, optionally in the 5' to 3' direction, an adapter region annealed to the hybridization region of the second hybridization molecule, a barcode region, and a target region capable of annealing or ligating to a second sub-sequence of the target nucleic acid.

Optionally, the first and second barcoded oligonucleotides each comprise an adapter region and a target region in a single contiguous sequence that is complementary and annealed to a hybridization region of a hybridization molecule, and also capable of annealing or ligating to a sub-sequence of a target nucleic acid.

The invention provides a multimeric barcoding reagent for labelling a target nucleic acid, wherein the reagent comprises: first and second hybridization molecules linked together (i.e. a multimeric hybridization molecule), wherein each of the hybridization molecules comprises a nucleic acid sequence comprising a hybridization region; and first and second barcoded oligonucleotides, wherein the first barcoded oligonucleotide comprises, optionally in the 5' to 3' direction, a barcode region, an adapter region annealed to the hybridization region of the first hybridization molecule and a target region capable of annealing or ligating to a first sub-sequence of the target nucleic acid, and wherein the second barcoded oligonucleotide comprises, optionally in the 5' to 3' direction, a barcode region, an adapter region annealed to the hybridization region of the second hybridization molecule and a target region capable of annealing or ligating to a second sub-sequence of the target nucleic acid.

Optionally, the first and second barcoded oligonucleotides each comprise an adapter region and a target region in a single contiguous sequence that is complementary and annealed to a hybridization region of a hybridization molecule, and also capable of annealing or ligating to a sub-sequence of a target nucleic acid.

The invention provides a multimeric barcoding reagent for labelling a target nucleic acid, wherein the reagent comprises: first and second hybridization molecules linked together (i.e. a multimeric hybridization molecule), wherein each of the hybridization molecules comprises a nucleic acid sequence comprising a hybridization region; and first and second barcoded oligonucleotides, wherein the first barcoded oligonucleotide comprises (in the 5'-3' or 3'-5' direction) an adapter region annealed to the hybridization region of the first hybridization molecule, a barcode region and a target region capable of ligating to a first sub-sequence of the target nucleic acid, and wherein the second barcoded oligonucleotide comprises (in the 5'-3' or 3'-5' direction) an adapter region annealed to the hybridization region of the second hybridization molecule, a barcode region and a target region capable of ligating to a second sub-sequence of the target nucleic acid.

Optionally, the first and second barcoded oligonucleotides each comprise an adapter region and a target region in a single contiguous sequence that is complementary and annealed to a hybridization region of a hybridization molecule, and also capable of ligating to a sub-sequence of a target nucleic acid.

The invention provides a multimeric barcoding reagent for labelling a target nucleic acid, wherein the reagent comprises: first and second hybridization molecules linked together (i.e. a multimeric hybridization molecule), wherein each of the hybridization molecules comprises a nucleic acid sequence comprising a hybridization region; and first and second barcoded oligonucleotides, wherein the first barcoded oligonucleotide comprises (in the 5'-3' or 3'-5' direction) a barcode region, an adapter region annealed to the hybridization region of the first hybridization molecule and a target region capable of ligating to a first sub-sequence of the target nucleic acid, and wherein the second barcoded oligonucleotide comprises (in the 5'-3' or 3'-5' direction) a barcode region, an adapter region annealed to the hybridization region of the second hybridization molecule and a target region capable of ligating to a second sub-sequence of the target nucleic acid.

Optionally, the first and second barcoded oligonucleotides each comprise an adapter region and a target region in a single contiguous sequence that is complementary and annealed to a hybridization region of a hybridization molecule, and also capable of ligating to a sub-sequence of a target nucleic acid.

The invention provides a multimeric barcoding reagent for labelling a target nucleic acid, wherein the reagent comprises: first and second hybridization molecules linked together (i.e. a multimeric hybridization molecule), wherein each of the hybridization molecules comprises a nucleic acid sequence comprising a barcode region; and first and second barcoded oligonucleotides, wherein the first barcoded oligonucleotide comprises in the 5' to 3' direction an adapter region annealed to the hybridization region of the first hybridization molecule, a barcode region and a target region capable of annealing to a first sub-sequence of the target nucleic acid, and wherein the second barcoded oligonucleotide comprises in the 5' to 3' direction an adapter region annealed to the hybridization region of the second hybridization molecule, a barcode region and a target region capable of annealing to a second sub-sequence of the target nucleic acid.

The invention provides a multimeric barcoding reagent for labelling a target nucleic acid, wherein the reagent comprises: first and second hybridization molecules linked together (i.e. a multimeric hybridization molecule), wherein each of the hybridization molecules comprises a nucleic acid sequence comprising a barcode region; and first and second barcoded oligonucleotides, wherein the first barcoded oligonucleotide comprises in the 5' to 3' direction a barcode region, an adapter region annealed to the hybridization region of the first hybridization molecule and a target region capable of annealing to a first sub-sequence of the target nucleic acid, and wherein the second barcoded oligonucleotide comprises in the 5' to 3' direction a barcode region, an adapter region annealed to the hybridization region of the second hybridization molecule and a target region capable of annealing to a second sub-sequence of the target nucleic acid.

Optionally, the first and second barcoded oligonucleotides each comprise an adapter region and a target region in a single contiguous sequence that is complementary and annealed to a hybridization region of a hybridization molecule, and also capable of annealing to a sub-sequence of a target nucleic acid.

Preferably, the adapter region of the first barcoded oligonucleotide comprises a sequence that is complementary and annealed to the hybridization region of the first hybridization molecule and the adapter region of the second barcoded oligonucleotide comprises a sequence that is complementary and annealed to the hybridization region of the second hybridization molecule. The complementary sequence of each barcoded oligonucleotide may be at least 5, at least 10, at least 15, at least 20, at least 25, at least 50 or at least 100 contiguous nucleotides.

The hybridization region of each hybridization molecule may comprise a constant region. Preferably, all hybridization regions of a multimeric barcoding reagent are substantially identical. Optionally, all hybridization regions of a library of multimeric barcoding reagents are substantially identical. The hybridization region may comprise at least 1, at least 2, at least 3, at least 4, at least 5, at least 6, at least 8, at least 10, at least 15, at least 20, at least 25, at least 50, at least 100, or at least 250 nucleotides. Preferably, the hybridization region comprises at least 4 nucleotides. Preferably each hybridization region comprises deoxyribonucleotides, optionally all of the nucleotides in a hybridization region are deoxyribonucleotides. One or more of the deoxyribonucleotides may be a modified deoxyribonucleotide (e.g. a deoxyribonucleotide modified with a biotin moiety or a deoxyuracil nucleotide). Each hybridization region may comprise one or more universal bases (e.g. inosine), one or modified nucleotides and/or one or more nucleotide analogues.

The target regions of the barcoded oligonucleotides may not be annealed to the multimeric hybridization molecule(s). The target regions of the barcoded oligonucleotides may be non-complementary to the multimeric hybridization molecule(s).

The barcoded oligonucleotides may comprise a linker region between the adapter region and the target region. The linker region may comprise one or more contiguous nucleotides that are not annealed to the multimeric hybridization molecule and are non-complementary to the subsequences of the target nucleic acid. The linker may comprise 1 to 100, 5 to 75, 10 to 50, 15 to 30 or 20 to 25 non-complementary nucleotides. Preferably, the linker comprises 15 to 30 non-complementary nucleotides. The use of such a linker region enhances the efficiency of the barcoding reactions performed using the multimeric barcoding reagents.

Hybridization molecules may further comprise one or more nucleic acid sequences that are not complementary to barcoded oligonucleotides. For example, hybridization molecules may comprise one or more adapter regions. A hybridization molecule, may, for example, comprise an adapter region 5' of a hybridization region (a 5' adapter region) and/or an adapter region 3' of the hybridization region (a 3' adapter region). The adapter region(s) may be used for manipulating, purifying, retrieving, amplifying, and/or detecting hybridization molecules.

The adapter region of each hybridization molecule may comprise a constant region. Optionally, all adapter regions of a multimeric hybridization reagent are substantially identical. The adapter region may comprise at least 1, at least 2, at least 3, at least 4, at least 5, at least 6, at least 8, at least 10, at least 15, at least 20, at least 25, at least 50, at least 100, or at least 250 nucleotides. Preferably, the adapter region comprises at least 4 nucleotides. Preferably each adapter region comprises deoxyribonucleotides, optionally all of the nucleotides in an adapter region are deoxyribonucleotides. One or more of the deoxyribonucleotides may be a modified deoxyribonucleotide (e.g. a deoxyribonucleotide modified with a biotin moiety or a deoxyuracil nucleotide). Each adapter region may comprise one or more universal bases (e.g. inosine), one or modified nucleotides and/or one or more nucleotide analogues.

The barcoded oligonucleotides may comprise a linker region between the adapter region and the target region. The linker region may comprise one or more contiguous nucleotides that are not annealed to the multimeric hybridization molecule and are non-complementary to the subsequences of the target nucleic acid. The linker may comprise 1 to 100, 5 to 75, 10 to 50, 15 to 30 or 20 to 25 non-complementary nucleotides. Preferably, the linker comprises 15 to 30 non-complementary nucleotides. The use of such a linker region enhances the efficiency of the barcoding reactions performed using the multimeric barcoding reagents.

The invention provides a library of multimeric barcoding reagents comprising at least 10 multimeric barcoding reagents for labelling a target nucleic acid for sequencing, wherein each multimeric barcoding reagent comprises: first and second hybridization molecules comprised within a (single) nucleic acid molecule, wherein each of the hybridization molecules comprises a nucleic acid sequence comprising a hybridization region; and first and second barcoded oligonucleotides, wherein the first barcoded oligonucleotide comprises, optionally in the 5' to 3' direction, an adapter region complementary and annealed to the hybridization region of the first hybridization molecule, a barcode region and a target region capable of annealing or ligating to a first sub-sequence of the target nucleic acid, and wherein the second barcoded oligonucleotide comprises, optionally in the 5' to 3' direction, an adapter region complementary and annealed to the hybridization region of the second hybridization molecule, a barcode region and a target region capable of annealing or ligating to a second sub-sequence of the target nucleic acid.

Preferably, the barcode regions of the first and second barcoded oligonucleotides of each multimeric barcoding reagent are different to the barcode regions of the barcoded oligonucleotides of at least 9 other multimeric barcoding reagents in the library.

The invention provides a library of multimeric barcoding reagents comprising at least 10 multimeric barcoding reagents for labelling a target nucleic acid for sequencing, wherein each multimeric barcoding reagent comprises: first and second hybridization molecules comprised within a (single) nucleic acid molecule, wherein each of the hybridization molecules comprises a nucleic acid sequence comprising a hybridization region; and first and second barcoded oligonucleotides, wherein the first barcoded oligonucleotide comprises, optionally in the 5' to 3' direction, a barcode region, an adapter region complementary and annealed to the hybridization region of the first hybridization molecule and a target region capable of annealing or ligating to a first sub-sequence of the target nucleic acid, and wherein the second barcoded oligonucleotide comprises, optionally in the 5' to 3' direction, a barcode region, an adapter region complementary and annealed to the hybridization region of the second hybridization molecule and a target region capable of annealing or ligating to a second sub-sequence of the target nucleic acid. Preferably, the barcode regions of the first and second barcoded oligonucleotides of each multimeric barcoding reagent are different to the barcode regions of the barcoded oligonucleotides of at least 9 other multimeric barcoding reagents in the library.

### 6. MULTIMERIC BARCODING REAGENTS COMPRISING BARCODED OLIGONUCLEOTIDES LINKED BY A MACROMOLECULE

The invention provides a multimeric barcoding reagent for labelling a target nucleic acid, wherein the reagent comprises first and second barcoded oligonucleotides linked together by a macromolecule, and wherein the barcoded oligonucleotides each comprise a barcode region.

The first barcoded oligonucleotide may further comprise a target region capable of annealing or ligating to a first sub-sequence of the target nucleic acid, and the second barcoded oligonucleotide may further comprise a target region capable of annealing or ligating to a second sub-sequence of the target nucleic acid.

The first barcoded oligonucleotide may comprise in the 5'-3' direction a barcode region and a target region capable of annealing to a first sub-sequence of the target nucleic acid, and the second barcoded oligonucleotide may comprise in the 5'-3' direction a barcode region and a target region capable of annealing to a second sub-sequence of the target nucleic acid.

The barcoded oligonucleotides may further comprise any of the features described herein.

The barcoded oligonucleotides may be linked by a macromolecule by being bound to the macromolecule and/or by being annealed to the macromolecule.

The barcoded oligonucleotides may be linked to the macromolecule directly or indirectly (e.g. via a linker molecule). The barcoded oligonucleotides may be linked by being bound to the macromolecule and/or by being bound or annealed to linker molecules that are bound to the macromolecule. The barcoded oligonucleotides may be bound to the macromolecule (or to the linker molecules) by covalent linkage, non-covalent linkage (e.g. a protein-protein interaction or a streptavidin-biotin bond) or nucleic acid hybridization. The linker molecule may be a biopolymer (e.g. a nucleic acid molecule) or a synthetic polymer. The linker molecule may comprise one or more units of ethylene glycol and/or poly(ethylene) glycol (e.g. hexa-ethylene glycol or penta-ethylene glycol). The linker molecule may comprise one or more ethyl groups, such as a C3 (three-carbon) spacer, C6 spacer, C12 spacer, or C18 spacer.

The macromolecule may be a synthetic polymer (e.g. a dendrimer) or a biopolymer such as a nucleic acid (e.g. a single-stranded nucleic acid such as single-stranded DNA), a peptide, a polypeptide or a protein (e.g. a multimeric protein).

The dendrimer may comprise at least 2, at least 3, at least 5, or at least 10 generations.

The macromolecule may be a nucleic acid comprising two or more nucleotides each capable of binding to a barcoded oligonucleotide. Additionally or alternatively, the nucleic acid may comprise two or more regions each capable of hybridizing to a barcoded oligonucleotide.

The nucleic acid may comprise a first modified nucleotide and a second modified nucleotide, wherein each modified nucleotide comprises a binding moiety (e.g. a biotin moiety, or an alkyne moiety which may be used for a click-chemical reaction) capable of binding to a barcoded oligonucleotide. Optionally, the first and second modified nucleotides may be separated by an intervening nucleic acid sequence of at least one, at least two, at least 5 or at least 10 nucleotides.

The nucleic acid may comprise a first hybridisation region and a second hybridisation region, wherein each hybridisation region comprises a sequence complementary to and capable of hybridizing to a sequence of at least one nucleotide within a barcoded oligonucleotide. The complementary sequence may be at least 5, at least 10, at least 15, at least 20, at least 25 or at least 50 contiguous nucleotides. Optionally, the first and second hybridisation regions may be separated by an intervening nucleic acid sequence of at least one, at least two, at least 5 or at least 10 nucleotides.

The macromolecule may be a protein such as a multimeric protein e.g. a homomeric protein or a heteromeric protein. For example, the protein may comprise streptavidin e.g. tetrameric streptavidin.

Libraries of multimeric barcoding reagents comprising barcoded oligonucleotides linked by a macromolecule are also provided. Such libraries may be based on the general properties of libraries of multimeric barcoding reagents described herein. In the libraries, each multimeric barcoding reagent may comprise a different macromolecule.

### 7. MULTIMERIC BARCODING REAGENTS COMPRISING BARCODED OLIGONUCLEOTIDES LINKED BY A SOLID SUPPORT OR A SEMI-SOLID SUPPORT

The invention provides a multimeric barcoding reagent for labelling a target nucleic acid, wherein the reagent comprises first and second barcoded oligonucleotides linked together by a solid support or a semi-solid support, and wherein the barcoded oligonucleotides each comprise a barcode region.

The first barcoded oligonucleotide may further comprise a target region capable of annealing or ligating to a first sub-sequence of the target nucleic acid, and the second barcoded oligonucleotide may further comprise a target region capable of annealing or ligating to a second sub-sequence of the target nucleic acid.

The first barcoded oligonucleotide may comprise in the 5'-3' direction a barcode region and a target region capable of annealing to a first sub-sequence of the target nucleic acid, and the second barcoded oligonucleotide may comprise in the 5'-3' direction a barcode region and a target region capable of annealing to a second sub-sequence of the target nucleic acid.

The barcoded oligonucleotides may further comprise any of the features described herein.

The barcoded oligonucleotides may be linked by a solid support or a semi-solid support. The barcoded oligonucleotides may be linked to the support directly or indirectly (e.g. via a linker molecule). The barcoded oligonucleotides may be linked by being bound to the support and/or by being bound or annealed to linker molecules that are bound to the support. The barcoded oligonucleotides may be bound to the support (or to the linker molecules) by covalent linkage, non-covalent linkage (e.g. a protein-protein interaction or a streptavidin-biotin bond) or nucleic acid hybridization. The linker molecule may be a biopolymer (e.g. a nucleic acid molecule) or a synthetic polymer. The linker molecule may comprise one or more units of ethylene glycol and/or poly(ethylene) glycol (e.g. hexa-ethylene glycol or penta-ethylene glycol). The linker molecule may comprise one or more ethyl groups, such as a C3 (three-carbon) spacer, C6 spacer, C12 spacer, or C18 spacer.

The support may comprise a planar surface. The support may be a slide e.g. a glass slide. The slide may be a flow cell for sequencing. If the support is a slide, the first and second barcoded oligonucleotides may be immobilized in a discrete region on the slide. Optionally, the barcoded oligonucleotides of each multimeric barcoding reagent in a library are immobilized in a different discrete region on the slide to the barcoded oligonucleotides of the other multimeric barcoding reagents in the library. The support may be a plate comprising wells, optionally wherein the first and second barcoded oligonucleotides are immobilized in the same well. Optionally, the barcoded oligonucleotides of each multimeric barcoding reagent in library are immobilized in a different well of the plate to the barcoded oligonucleotides of the other multimeric barcoding reagents in the library.

Preferably, the support is a bead (e.g. a gel bead). The bead may be an agarose bead, a silica bead, a styrofoam bead, a gel bead (such as those available from 10x Genomics^{®}), an antibody conjugated bead, an oligo-dT conjugated bead, a streptavidin bead or a magnetic bead (e.g. a superparamagnetic bead). The bead may be of any size and/or molecular structure. For example, the bead may be 10 nanometres to 100 microns in diameter, 100 nanometres to 10 microns in diameter, or 1 micron to 5 microns in diameter. Optionally, the bead is approximately 10 nanometres in diameter, approximately 100 nanometres in diameter, approximately 1 micron in diameter, approximately 10 microns in diameter or approximately 100 microns in diameter. The bead may be solid, or alternatively the bead may be hollow or partially hollow or porous. Beads of certain sizes may be most preferable for certain barcoding methods. For example, beads less than 5.0 microns, or less than 1.0 micron, may be most useful for barcoding nucleic acid targets within individual cells. Preferably, the barcoded oligonucleotides of each multimeric barcoding reagent in a library are linked together on a different bead to the barcoded oligonucleotides of the other multimeric barcoding reagents in the library.

The support may be functionalised to enable attachment of two or more barcoded oligonucleotides. This functionalisation may be enabled through the addition of chemical moieties (e.g. carboxylated groups, alkynes, azides, acrylate groups, amino groups, sulphate groups, or succinimide groups), and/or protein-based moieties (e.g. streptavidin, avidin, or protein G) to the support. The barcoded oligonucleotides may be attached to the moieties directly or indirectly (e.g. via a linker molecule).

Functionalised supports (e.g. beads) may be brought into contact with a solution of barcoded oligonucleotides under conditions which promote the attachment of two or more barcoded oligonucleotides to each bead in the solution (generating multimeric barcoding reagents).

Libraries of multimeric barcoding reagents comprising barcoded oligonucleotides linked by a support are also provided. Such libraries may be based on the general properties of libraries of multimeric barcoding reagents described herein. In the libraries, each multimeric barcoding reagent may comprise a different support (e.g. a differently labelled bead). In a library of multimeric barcoding reagents, the barcoded oligonucleotides of each multimeric barcoding reagent in a library may be linked together on a different support to the barcoded oligonucleotides of the other multimeric barcoding reagents in the library.

### 8. MULTIMERIC BARCODING REAGENTS COMPRISING BARCODED OLIGONUCLEOTIDES LINKED TOGETHER BY BEING COMPRISED WITHIN A LIPID CARRIER

The invention provides a multimeric barcoding reagent for labelling a target nucleic acid, wherein the reagent comprises first and second barcoded oligonucleotides and a lipid carrier, wherein the first and second barcoded oligonucleotides are linked together by being comprised within the lipid carrier, and wherein the barcoded oligonucleotides each comprise a barcode region.

The first barcoded oligonucleotide may further comprise a target region capable of annealing or ligating to a first sub-sequence of the target nucleic acid, and the second barcoded oligonucleotide may further comprise a target region capable of annealing or ligating to a second sub-sequence of the target nucleic acid.

The first barcoded oligonucleotide may comprise in the 5'-3' direction a barcode region and a target region capable of annealing to a first sub-sequence of the target nucleic acid, and the second barcoded oligonucleotide may comprise in the 5'-3' direction a barcode region and a target region capable of annealing to a second sub-sequence of the target nucleic acid.

The barcoded oligonucleotides may further comprise any of the features described herein.

The invention provides a library of multimeric barcoding reagents comprising first and second multimeric barcoding reagents as defined herein, wherein the barcoded oligonucleotides of the first multimeric barcoding reagent are comprised within a first lipid carrier, and wherein the barcoded oligonucleotides of the second multmeric barcoding reagent are comprised with a second lipid carrier, and wherein the barcode regions of the barcoded oligonucleotides of the first multimeric barcoding reagent are different to the barcode regions of the barcoded oligonucleotides of the second multimeric barcoding reagent.

The library of multimeric barcoding reagents may comprise at least 5, at least 10, at least 20, at least 25, at least 50, at least 75, at least 100, at least 250, at least 500, at least 10³, at least 10⁴, at least 10⁵, at least 10⁶, at least 10⁷, at least 10⁸ or at least 10⁹ multimeric barcoding reagents as defined herein. Preferably, the library comprises at least 10 multimeric barcoding reagents as defined herein. Preferably, the barcode regions of the first and second barcoded oligonucleotides of each multimeric barcoding reagent are different to the barcode regions of the barcoded oligonucleotides of at least 9 other multimeric barcoding reagents in the library.

The barcoded oligonucleodides of each multimeric barcoding reagent are comprised within a different lipid carrier.

The lipid carrier may be a liposome or a micelle. The lipid carrier may be a phospholipid carrier. The lipid carrier may comprise one or more amphiphilic molecules. The lipid carrier may comprise one or more phospholipids. The phospholipid may be phosphatidylcholine. The lipid carrier may comprise one or more of the following constituents: phophatidylethanolamine, phosphatidylserine, cholesterol, cardiolipin, dicetylphosphate, stearylamine, phosphatidylglycerol, dipalmitoylphosphatidylcholine, distearylphosphatidylcholine, and/or any related and/or derivative molecules thereof. Optionally, the lipid carrier may comprise any combination of two or more constituents described above, with or without further constituents.

The lipid carrier (e.g. a liposome or a micelle) may be unilamellar or multilamellar. A library of multimeric barcoding reagents may comprise both unilamellar and multilamellar lipid carriers. The lipid carrier may comprise a copolymer e.g. a block copolymer.

The lipid carrier may comprise at least 2, at least 3, at least 5, at least 10, at least 50, at least 100, at least 500, at least 1000, at least 10,000, or at least 100,000 barcoded oligonucleotides, or any greater number of barcoded oligonucleotides.

Any lipid carrier (e.g. liposome or micelle, and/or liposomal or micellar reagent) may on average be complexed with 1, or less than 1, or greater than 1 multimeric barcoding reagent(s) to form a library of such multimeric barcoding reagent(s).

The invention provides a library of multimeric barcoding reagents comprising at least 10 multimeric barcoding reagents as defined herein, wherein each multimeric barcoding reagent comprises first and second barcoded oliognucleotides comprised within a different lipid carrier, and wherein the barcode regions of the first and second barcoded oligonucleotides of each multimeric barcoding reagent are different to the barcode regions of the barcoded oligonucleotides of at least 9 other multimeric barcoding reagents in the library.

A method for preparing multimeric barcoding reagents comprises loading barcoded oligonucleotides and/or multimeric barcoding reagent(s) into lipid carriers (e.g. liposomes or micelles). The method may comprise a step of passive, active, and/or remote loading. Preformed lipid carriers (e.g. liposomes and/or micelles) may be loaded by contacting them with a solution of barcoded oligonucleotides and/or multimeric barcoding reagent(s). Lipid carriers (e.g. liposomes and/or micelles) may be loaded by contacting them with a solution of barcoded oligonucleotides and/or multimeric barcoding reagent(s) prior to and/or during the formation or synthesis of the lipid carriers. The method may comprise passive encapsulation and/or trapping of barcoded oligonucleotides and/or multimeric barcoding reagent(s) in lipid carriers.

Lipid carriers (e.g. liposomes and/or micelles) may be prepared by a method based on sonication, a French press-based method, a reverse phase method, a solvent evaporation method, an extrusion-based method, a mechanical mixing-based method, a freeze/thaw-based method, a dehydrate/rehydrate-based method, and/or any combination hereof.

Lipid carriers (e.g. liposomes and/or micelles) may be stabilized and/or stored prior to use using known methods.

Any of the multimeric barcoding reagents or kits described herein may be comprised with a lipid carrier.

### 9. KITS COMPRISING MULTIMERIC BARCODING REAGENTS AND ADAPTER OLIGONUCLEOTIDES

The invention further provides kits comprising one or more of the components defined herein. The invention also provides kits specifically adapted for performing any of the methods defined herein.

The invention further provides a kit for labelling a target nucleic acid, wherein the kit comprises: (a) a multimeric barcoding reagent comprising (i) first and second barcode molecules linked together (i.e. a multimeric barcode molecule), wherein each of the barcode molecules comprises a nucleic acid sequence comprising, optionally in the 5' to 3' direction, an adapter region and a barcode region, and (ii) first and second barcoded oligonucleotides, wherein the first barcoded oligonucleotide comprises a barcode region annealed to the barcode region of the first barcode molecule, and wherein the second barcoded oligonucleotide comprises a barcode region annealed to the barcode region of the second barcode molecule; and (b) first and second adapter oligonucleotides, wherein the first adapter oligonucleotide comprises, optionally in the 5' to 3' direction, an adapter region capable of annealing to the adapter region of the first barcode molecule and a target region capable of annealing or ligating to a first sub-sequence of the target nucleic acid, and wherein the second adapter oligonucleotide comprises, optionally in the 5' to 3' direction, an adapter region capable of annealing to the adapter region of the second barcode molecule and a target region capable of annealing or ligating to a second sub-sequence of the target nucleic acid.

The invention further provides a kit for labelling a target nucleic acid, wherein the kit comprises: (a) a multimeric barcoding reagent comprising (i) first and second barcode molecules linked together (i.e. a multimeric barcode molecule), wherein each of the barcode molecules comprises a nucleic acid sequence comprising an adapter region and a barcode region, and (ii) first and second barcoded oligonucleotides, wherein the first barcoded oligonucleotide comprises a barcode region annealed to the barcode region of the first barcode molecule, and wherein the second barcoded oligonucleotide comprises a barcode region annealed to the barcode region of the second barcode molecule; and (b) first and second adapter oligonucleotides, wherein the first adapter oligonucleotide comprises an adapter region capable of annealing to the adapter region of the first barcode molecule and a target region capable of ligating to a first sub-sequence of the target nucleic acid, and wherein the second adapter oligonucleotide comprises an adapter region capable of annealing to the adapter region of the second barcode molecule and a target region capable of ligating to a second sub-sequence of the target nucleic acid.

The invention further provides a kit for labelling a target nucleic acid, wherein the kit comprises: (a) a multimeric barcoding reagent comprising (i) first and second barcode molecules linked together (i.e. a multimeric barcode molecule), wherein each of the barcode molecules comprises a nucleic acid sequence comprising in the 5' to 3' direction an adapter region and a barcode region, and (ii) first and second barcoded oligonucleotides, wherein the first barcoded oligonucleotide comprises a barcode region annealed to the barcode region of the first barcode molecule, and wherein the second barcoded oligonucleotide comprises a barcode region annealed to the barcode region of the second barcode molecule; and (b) first and second adapter oligonucleotides, wherein the first adapter oligonucleotide comprises in the 5' to 3' direction an adapter region capable of annealing to the adapter region of the first barcode molecule and a target region capable of annealing to a first sub-sequence of the target nucleic acid, and wherein the second adapter oligonucleotide comprises in the 5' to 3' direction an adapter region capable of annealing to the adapter region of the second barcode molecule and a target region capable of annealing to a second sub-sequence of the target nucleic acid.

The invention further provides a kit for labelling a target nucleic acid, wherein the kit comprises: (a) a multimeric barcoding reagent comprising (i) first and second barcode molecules linked together (i.e. a multimeric barcode molecule), wherein each of the barcode molecules comprises a nucleic acid sequence comprising, optionally in the 5' to 3' direction, an adapter region and a barcode region, and (ii) first and second barcoded oligonucleotides, wherein the first barcoded oligonucleotide comprises a barcode region annealed to the barcode region of the first barcode molecule, and wherein the second barcoded oligonucleotide comprises a barcode region annealed to the barcode region of the second barcode molecule; and (b) first and second adapter oligonucleotides, wherein the first adapter oligonucleotide comprises an adapter region capable of annealing to the adapter region of the first barcode molecule and capable of ligating to a first sub-sequence of the target nucleic acid, and wherein the second adapter oligonucleotide comprises an adapter region capable of annealing to the adapter region of the second barcode molecule and capable of ligating to a second sub-sequence of the target nucleic acid.

Each adapter oligonucleotide may consist essentially of or consist of an adapter region. Each adapter oligonucleotide may not comprise a target region.

Preferably, the adapter region of the first adapter oligonucleotide comprises a sequence that is complementary to and capable of annealing to the adapter region of the first barcode molecule and the adapter region of the second adapter oligonucleotide comprises a sequence that is complementary to and capable of annealing to the adapter region of the second barcode molecule. The complementary sequence of each adapter oligonucleotide may be at least 5, at least 10, at least 15, at least 20, at least 25, at least 50 or at least 100 contiguous nucleotides.

The target regions of the adapter oligonucleotides may not be capable of annealing to the multimeric barcode molecule(s)). The target regions of the adapter oligonucleotides may be non-complementary to the multimeric barcode molecule(s).

The target regions of each adapter oligonucleotide may comprise different sequences. Each target region may comprise a sequence capable of annealing to only a single sub-sequence of a target nucleic acid within a sample of nucleic acids. Each target region may comprise one or more random, or one or more degenerate, sequences to enable the target region to anneal to more than one sub-sequence of a target nucleic acid. Each target region may comprise at least 5, at least 10, at least 15, at least 20, at least 25, at least 50 or at least 100 nucleotides. Preferably, each target region comprises at least 5 nucleotides. Each target region may comprise 5 to 100 nucleotides, 5 to 10 nucleotides, 10 to 20 nucleotides, 20 to 30 nucleotides, 30 to 50 nucleotides, 50 to 100 nucleotides, 10 to 90 nucleotides, 20 to 80 nucleotides, 30 to 70 nucleotides or 50 to 60 nucleotides. Preferably, each target region comprises 30 to 70 nucleotides. Preferably each target region comprises deoxyribonucleotides, optionally all of the nucleotides in a target region are deoxyribonucleotides. One or more of the deoxyribonucleotides may be a modified deoxyribonucleotide (e.g. a deoxyribonucleotide modified with a biotin moiety or a deoxyuracil nucleotide). Each target region may comprise one or more universal bases (e.g. inosine), one or modified nucleotides and/or one or more nucleotide analogues.

The target regions may be used to anneal the adapter oligonucleotides to sub-sequences of target nucleic acids, and then may be used as primers for a primer-extension reaction or an amplification reaction e.g. a polymerase chain reaction. Alternatively, the target regions may be used to ligate the adapter oligonucleotides to sub-sequences of target nucleic acids. The target region may be at the 5' end of an adapter oligonucleotide. Such a target region may be phosphorylated. This may enable the 5' end of the target region to be ligated to the 3' end of a sub-sequence of a target nucleic acid.

The adapter oligonucleotides may comprise a linker region between the adapter region and the target region. The linker region may comprise one or more contiguous nucleotides that are not annealed to the first and second barcode molecules (i.e. the multimeric barcode molecule) and are non-complementary to the subsequences of the target nucleic acid. The linker may comprise 1 to 100, 5 to 75, 10 to 50, 15 to 30 or 20 to 25 non-complementary nucleotides. Preferably, the linker comprises 15 to 30 non-complementary nucleotides. The use of such a linker region enhances the efficiency of the barcoding reactions performed using the kits described herein.

Each of the components of the kit may take any of the forms defined herein.

The multimeric barcoding reagent(s) and adapter oligonucleotides may be provided in the kit as physically separated components.

The kit may comprise: (a) a multimeric barcoding reagent comprising at least 5, at least 10, at least 20, at least 25, at least 50, at least 75 or at least 100 barcode molecules linked together, wherein each barcode molecule is as defined herein; and (b) an adapter oligonucleotide capable of annealing to each barcode molecule, wherein each adapter oligonucleotide is as defined herein.

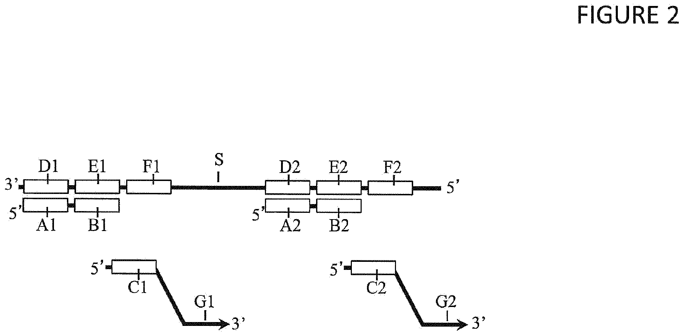

Figure 2 shows a kit comprising a multimeric barcoding reagent and adapter oligonucleotides for labelling a target nucleic acid. In more detail, the kit comprises first (D1, E1, and F1) and second (D2, E2, and F2) barcode molecules, with each incorporating a barcode region (E1 and E2) and also a 5' adapter region (F1 and F2). These first and second barcode molecules are linked together, in this embodiment by a connecting nucleic acid sequence (S).

The kit further comprises first (A1 and B1) and second (A2 and B2) barcoded oligonucleotides, which each comprise a barcode region (B1 and B2), as well as 5' regions (A1 and A2). The 5' region of each barcoded oligonucleotide is complementary to, and thus may be annealed to, the 3' regions of the barcode molecules (D1 and D2). The barcode regions (B1 and B2) are complementary to, and thus may be annealed to, the barcode regions (E1 and E2) of the barcode molecules.

The kit further comprises first (C1 and G1) and second (C2 and G2) adapter oligonucleotides, wherein each adapter oligonucleotide comprises an adapter region (C1 and C2) that is complementary to, and thus able to anneal to, the 5' adapter region of a barcode molecule (F1 and F2). These adapter oligonucleotides may be synthesised to include a 5'-terminal phosphate group. Each adapter oligonucleotide also comprises a target region (G1 and G2), which may be used to anneal the barcoded-adapter oligonucleotides (A1, B1, C1 and G1, and A2, B2, C2 and G2) to target nucleic acids, and then may be used as primers for a primer-extension reaction or a polymerase chain reaction.

The kit may comprise a library of two or more multimeric barcoding reagents, wherein each multimeric barcoding reagent is as defined herein, and adapter oligonucleotides for each of the multimeric barcoding reagents, wherein each adapter oligonucleotide is as defined herein. The barcode regions of the first and second barcoded oligonucleotides of the first multimeric barcoding reagent are different to the barcode regions of the first and second barcoded oligonucleotides of the second multimeric barcoding reagent.

The kit may comprise a library comprising at least 5, at least 10, at least 20, at least 25, at least 50, at least 75, at least 100, at least 250, at least 500, at least 10³, at least 10⁴, at least 10⁵, at least 10⁶, at least 10⁷, at least 10⁸ or at least 10⁹ multimeric barcoding reagents as defined herein. Preferably, the kit comprises a library comprising at least 10 multimeric barcoding reagents as defined herein. The kit may further comprise adapter oligonucleotides for each of the multimeric barcoding reagents, wherein each adapter oligonucleotide may take the form of any of the adapter oligonucleotides defined herein. Preferably, the barcode regions of the first and second barcoded oligonucleotides of each multimeric barcoding reagent are different to the barcode regions of the barcoded oligonucleotides of at least 9 other multimeric barcoding reagents in the library.

The barcode regions of the first and second barcoded oligonucleotides of each multimeric barcoding reagent may be different to the barcode regions of the barcoded oligonucleotides of at least 4, at least 9, at least 19, at least 24, at least 49, at least 74, at least 99, at least 249, at least 499, at least 999 (i.e. 10³-1), at least 10⁴-1, at least 10⁵-1, at least 10⁶-1, at least 10⁷-1, at least 10⁸-1 or at least 10⁹-1 other multimeric barcoding reagents in the library. The barcode regions of the first and second barcoded oligonucleotides of each multimeric barcoding reagent may be different to the barcode regions of the barcoded oligonucleotides of all of the other multimeric barcoding reagents in the library. Preferably, the barcode regions of the first and second barcoded oligonucleotides of each multimeric barcoding reagent are different to the barcode regions of the barcoded oligonucleotides of at least 9 other multimeric barcoding reagents in the library.

The barcode regions of the barcoded oligonucleotides of each multimeric barcoding reagent may be different to the barcode regions of the barcoded oligonucleotides of at least 4, at least 9, at least 19, at least 24, at least 49, at least 74, at least 99, at least 249, at least 499, at least 999 (i.e. 10³-1), at least 10⁴-1, at least 10⁵-1, at least 10⁶-1, at least 10⁷-1, at least 10⁸-1 or at least 10⁹-1 other multimeric barcoding reagents in the library. The barcode regions of the barcoded oligonucleotides of each multimeric barcoding reagent may be different to the barcode regions of the barcoded oligonucleotides of all of the other multimeric barcoding reagents in the library. Preferably, the barcode regions of the barcoded oligonucleotides of each multimeric barcoding reagent are different to the barcode regions of the barcoded oligonucleotides of at least 9 other multimeric barcoding reagents in the library

The invention provides a kit for labelling a target nucleic acid for sequencing, wherein the kit comprises: (a) a library of multimeric barcoding reagents comprising at least 10 multimeric barcoding reagents, wherein each multimeric barcoding reagent comprises: (i) first and second barcode molecules comprised within a (single) nucleic acid molecule, wherein each of the barcode molecules comprises a nucleic acid sequence comprising, optionally in the 5' to 3' direction, an adapter region and a barcode region, and (ii) first and second barcoded oligonucleotides, wherein the first barcoded oligonucleotide comprises a barcode region complementary and annealed to the barcode region of the first barcode molecule, and wherein the second barcoded oligonucleotide comprises a barcode region complementary and annealed to the barcode region of the second barcode molecule; and (b) first and second adapter oligonucleotides for each of the multimeric barcoding reagents, wherein the first adapter oligonucleotide comprises, optionally in the 5' to 3' direction, an adapter region capable of annealing to the adapter region of the first barcode molecule and a target region capable of annealing or ligating to a first sub-sequence of the target nucleic acid, and wherein the second adapter oligonucleotide comprises, optionally in the 5' to 3' direction, an adapter region capable of annealing to the adapter region of the second barcode molecule and a target region capable of annealing or ligating to a second sub-sequence of the target nucleic acid.

### 10. KITS COMPRISING MULTIMERIC BARCODING REAGENTS, ADAPTER OLIGONUCLEOTIDES AND EXTENSION PRIMERS

The invention further provides a kit for labelling a target nucleic acid for sequencing, wherein the kit comprises: (a) a multimeric barcode molecule comprising first and second barcode molecules linked together, wherein each of the barcode molecules comprises a nucleic acid sequence comprising, optionally in the 5' to 3' direction, an adapter region, a barcode region, and a priming region; (b) first and second extension primers for the multimeric barcode molecule, wherein the first extension primer comprises a sequence capable of annealing to the priming region of the first barcode molecule, and wherein the second extension primer comprises a sequence capable of annealing to the priming region of the second barcode molecule; and (c) first and second adapter oligonucleotides for the multimeric barcode molecule, wherein the first adapter oligonucleotide comprises, optionally in the 5' to 3' direction, an adapter region capable of annealing to the adapter region of the first barcode molecule and a target region capable of annealing or ligating to a first sub-sequence of the target nucleic acid, and wherein the second adapter oligonucleotide comprises, optionally in the 5' to 3' direction, an adapter region capable of annealing to the adapter region of the second barcode molecule and a target region capable of annealing or ligating to a second sub-sequence of the target nucleic acid.

The invention further provides a kit for labelling a target nucleic acid for sequencing, wherein the kit comprises: (a) a multimeric barcode molecule comprising first and second barcode molecules linked together, wherein each of the barcode molecules comprises a nucleic acid sequence comprising, optionally in the 5' to 3' direction, an adapter region, a barcode region, and a priming region; (b) first and second extension primers for the multimeric barcode molecule, wherein the first extension primer comprises a sequence capable of annealing to the priming region of the first barcode molecule, and wherein the second extension primer comprises a sequence capable of annealing to the priming region of the second barcode molecule; and (c) first and second adapter oligonucleotides for the multimeric barcode molecule, wherein the first adapter oligonucleotide comprises an adapter region capable of annealing to the adapter region of the first barcode molecule and capable of ligating to a first sub-sequence of the target nucleic acid, and wherein the second adapter oligonucleotide comprises an adapter region capable of annealing to the adapter region of the second barcode molecule and capable of ligating to a second sub-sequence of the target nucleic acid.

Each adapter oligonucleotide may consist essentially of or consist of an adapter region.

The components of the kit may take any of the forms described herein.

Preferably, the first extension primer comprises a sequence that is complementary to and capable of annealing to the priming region of the first barcode molecule and the second extension primer comprises a sequence that is complementary to and capable of annealing to the priming region of the second barcode molecule. The complementary sequence of each extension primer may be at least 5, at least 10, at least 15, at least 20, at least 25, at least 50 or at least 100 contiguous nucleotides.

The first and second extension primers may be capable of being extended using the barcode regions of the first and second barcode molecules as templates to produce first and second barcoded oligonucleotides, wherein the first barcoded oligonucleotide comprises a sequence complementary to the barcode region of the first barcode molecule and the second barcoded oligonucleotide comprises a sequence complementary to the barcode region of the second barcode molecule.

The first and second extension primers may be identical in sequence. Alternatively, the first and second extension primers may be different in sequence.

The first and/or second extension primers may further comprise one or more regions with nucleic acid sequences that are not complementary to the first barcode molecule and second barcode molecule, respectively. Optionally, such a non-complementary region may include a binding site for one or more amplification primers. Optionally, such a non-complementary region may be positioned within the 5' region of the molecule. Optionally, the first and second extension primers may comprise a terminal 5' phosphate group capable of ligating to a 3' end of a nucleic acid molecule.

The first and/or second extension primers may further comprise one or more secondary barcode regions. Optionally, a secondary barcode region may be comprised within a region of the extension primer that is non-complementary to a barcode molecule. Optionally, a secondary barcode region may be comprised within a region of the extension primer that is between a 3' region of the extension primer that is complementary to a barcode molecule and a 5' region of the extension primer that comprises a binding site for an amplification primer.

A secondary barcode region may comprise a sequence of one or more nucleotides, wherein sequences of the secondary barcode regions of the first extension primer and the second extension primer are different. Optionally, said one or more nucleotides may comprise random or degenerate nucleotides. Optionally, said one or more nucleotides may comprise different but non-random nucleotides. Any secondary barcode region may comprise at least 2, at least 3, at least 5, at least 10, at least 15, at least 20, or at least 30 nucleotides. Any secondary barcode region may comprise a contiguous sequence of barcode oligonucleotides, or may comprise two or more different segments separated by at least one non-barcode or invariant nucleotide. Optionally, any secondary barcode region may comprise a unique molecular identifier (UMI).

The kit may comprise a library of two or more multimeric barcode molecules, wherein each multimeric barcode molecule is as defined herein, and first and second extension primers, and first and second adapter oligonucleotides, for each of the multimeric barcode molecule. The extension primers and adapter oligonucleotides may take any of the forms described herein. The barcode regions of the first and second barcode molecules of the first multimeric barcode molecule are different to the barcode regions of the first and second barcode molecules of the second multimeric barcode molecule.

The kit may comprise a library comprising at least 5, at least 10, at least 20, at least 25, at least 50, at least 75, at least 100, at least 250, at least 500, at least 10³, at least 10⁴, at least 10⁵, at least 10⁶, at least 10⁷, at least 10⁸ or at least 10⁹ multimeric barcode molecules as defined herein. Preferably, the kit comprises a library comprising at least 10 multimeric barcode molecules as defined herein. The kit may further comprise extension primers and/or adapter oligonucleotides for each of the multimeric barcode molecules. The extension primers and adapter oligonucleotides may take any of the forms described herein. Preferably, the barcode regions of the first and second barcode molecules of each multimeric barcode molecule are different to the barcode regions of the barcode molecules of at least 9 other multimeric barcode molecules in the library.

The barcode regions of the first and second barcode molecules of each multimeric barcode molecule may be different to the barcode regions of the barcoded molecules of at least 4, at least 9, at least 19, at least 24, at least 49, at least 74, at least 99, at least 249, at least 499, at least 999 (i.e. 10³-1), at least 10⁴-1, at least 10⁵-1, at least 10⁶-1, at least 10⁷-1, at least 10⁸-1 or at least 10⁹-1 other multimeric barcode molecules in the library. The barcode regions of the first and second barcode molecules of each multimeric barcode molecule may be different to the barcode regions of the barcode molecules of all of the other multimeric barcode molecules in the library. Preferably, the barcode regions of the first and second barcode molecules of each multimeric barcode molecule are different to the barcode regions of the barcode molecules of at least 9 other multimeric barcode molecules in the library.

The barcode regions of the barcode molecules of each multimeric barcode molecule may be different to the barcode regions of the barcode molecules of at least 4, at least 9, at least 19, at least 24, at least 49, at least 74, at least 99, at least 249, at least 499, at least 999 (i.e. 10³-1), at least 10⁴-1, at least 10⁵-1, at least 10⁶-1, at least 10⁷-1, at least 10⁸-1 or at least 10⁹-1 other multimeric barcode molecules in the library. The barcode regions of the barcode molecules of each multimeric barcode molecules may be different to the barcode regions of the barcode molecules of all of the other multimeric barcode molecules in the library. Preferably, the barcode regions of the barcode molecules of each multimeric barcode molecule are different to the barcode regions of the barcode molecules of at least 9 other multimeric barcode molecules in the library.

The invention further provides a kit for labelling a target nucleic acid for sequencing, wherein the kit comprises: (a) a library of multimeric barcode molecules comprising at least 10 multimeric barcode molecules, each multimeric barcode molecule comprising first and second barcode molecules comprised within a (single) nucleic acid molecule, wherein each of the barcode molecules comprises a nucleic acid sequence comprising, optionally in the 5' to 3' direction, an adapter region, a barcode region, and a priming region, and wherein the barcode regions of the first and second barcode molecules of each multimeric barcode molecule are different to the barcode regions of at least 9 other multimeric barcode molecules in the library; (b) first and second extension primers for each of the multimeric barcode molecules, wherein the first extension primer comprises a sequence capable of annealing to the priming region of the first barcode molecule, and wherein the second extension primer comprises a sequence capable of annealing to the priming region of the second barcode molecule; and (c) first and second adapter oligonucleotides for each of the multimeric barcode molecules, wherein the first adapter oligonucleotide comprises, optionally in the 5' to 3' direction, an adapter region capable of annealing to the adapter region of the first barcode molecule and a target region capable of annealing or ligating to a first sub-sequence of the target nucleic acid, and wherein the second adapter oligonucleotide comprises, optionally in the 5' to 3' direction, an adapter region capable of annealing to the adapter region of the second barcode molecule and a target region capable of annealing or ligating to a second sub-sequence of the target nucleic acid.

### 11. METHODS OF PREPARING A NUCLEIC ACID SAMPLE FOR SEQUENCING

The methods of preparing a nucleic acid sample for sequencing may comprise (i) contacting the nucleic acid sample with a multimeric barcoding reagent comprising first and second barcode regions linked together, wherein each barcode region comprises a nucleic acid sequence, and (ii) appending barcode sequences to first and second sub-sequences of a target nucleic acid to produce first and second different barcoded target nucleic acid molecules, wherein the first barcoded target nucleic acid molecule comprises the nucleic acid sequence of the first barcode region and the second barcoded target nucleic acid molecule comprises the nucleic acid sequence of the second barcode region.

In methods in which the multimeric barcoding reagent comprises first and second barcoded oligonucleotides linked together, the barcode sequences may be appended to first and second sub-sequences of the target nucleic acid by any of the methods described herein.

The first and second barcoded oligonucleotides may be ligated to the first and second sub-sequences of the target nucleic acid to produce the first and second different barcoded target nucleic acid molecules. Optionally, prior to the ligation step, the method comprises appending first and second coupling sequences to the target nucleic acid, wherein the first and second coupling sequences are the first and second sub-sequences of the target nucleic acid to which the first and second barcoded oligonucleotides are ligated.

The first and second barcoded oligonucleotides may be annealed to the first and second sub-sequences of the target nucleic acid extended to produce the first and second different barcoded target nucleic acid molecules. Optionally, prior to the annealing step, the method comprises appending first and second coupling sequences to the target nucleic acid, wherein the first and second coupling sequences are the first and second sub-sequences of the target nucleic acid to which the first and second barcoded oligonucleotides are annealed.

The first and second barcoded oligonucleotides may be annealed at their 5' ends to the first and second sub-sequences of the target nucleic acid and first and second target primers may be annealed to third and fourth sub-sequences of the target nucleic acid, respectively, wherein the third subsequence is 3' of the first subsequence and wherein the fourth sub-sequence is 3' of the second subsequence. The method further comprises extending the first target primer using the target nucleic acid as template until it reaches the first sub-sequence to produce a first extended target primer, and extending the second target primer using the target nucleic acid as template until it reaches the second sub-sequence to produce a second extended target primer, and ligating the 3' end of the first extended target primer to the 5' end of the first barcoded oligonucleotide to produce a first barcoded target nucleic acid molecule, and ligating the 3' end of the second extended target primer to the 5' end of the second barcoded oligonucleotide to produce a second barcoded target nucleic acid molecule, wherein the first and second barcoded target nucleic acid molecules are different and each comprises at least one nucleotide synthesised from the target nucleic acid as a template. Optionally, prior to either or both annealing step(s), the method comprises appending first and second, and/or third and fourth, coupling sequences to the target nucleic acid, wherein the first and second coupling sequences are the first and second sub-sequences of the target nucleic acid to which the first and second barcoded oligonucleotides are annealed, and/or wherein the third and fourth coupling sequences are the third and fourth sub-sequences of the target nucleic acid to which the first and second target primers are annealed.

As described herein, prior to annealing or ligating a multimeric hybridization molecule, multimeric barcode molecule, barcoded oligonucleotide, adapter oligonucleotide or target primer to a target nucleic acid, a coupling sequence may be appended to the target nucleic acid. The multimeric hybridization molecule, multimeric barcode molecule, barcoded oligonucleotide, adapter oligonucleotide or target primer may then be annealed or ligated to the coupling sequence.

A coupling sequence may be added to the 5' end or 3' end of two or more target nucleic acids of the nucleic acid sample (e.g. a FFPE DNA sample). In this method, the target regions (of the barcoded oligonucleotides) may comprise a sequence that is complementary to the coupling sequence.

A coupling sequence may be comprised within a double-stranded coupling oligonucleotide or within a single-stranded coupling oligonucleotide. A coupling oligonucleotide may be appended to the target nucleic acid by a double-stranded ligation reaction or a single-stranded ligation reaction. A coupling oligonucleotide may comprise a single-stranded 5' or 3' region capable of ligating to a target nucleic acid and the coupling sequence may be appended to the target nucleic acid by a single-stranded ligation reaction.

A coupling oligonucleotide may comprise a blunt, recessed, or overhanging 5' or 3' region capable of ligating to a target nucleic acid and the coupling sequence may be appended to the target nucleic acid a double-stranded ligation reaction.

The end(s) of a target nucleic acid may be converted into blunt double-stranded end(s) in a blunting reaction, and the coupling oligonucleotide may comprise a blunt double-stranded end, and wherein the coupling oligonucleotide may be ligated to the target nucleic acid in a blunt-end ligation reaction.

The end(s) of a target nucleic acid may be converted into blunt double-stranded end(s) in a blunting reaction, and then converted into a form with (a) single 3' adenosine overhang(s), and wherein the coupling oligonucleotide may comprise a double-stranded end with a single 3' thymine overhang capable of annealing to the single 3' adenosine overhang of the target nucleic acid, and wherein the coupling oligonucleotide is ligated to the target nucleic acid in a double-stranded A/T ligation reaction

The target nucleic acid may be contacted with a restriction enzyme, wherein the restriction enzyme digests the target nucleic acid at restriction sites to create (a) ligation junction(s) at the restriction site(s), and wherein the coupling oligonucleotide comprises an end compatible with the ligation junction, and wherein the coupling oligonucleotide is then ligated to the target nucleic acid in a double-stranded ligation reaction.

A coupling oligonucleotide may be appended via a primer-extension or polymerase chain reaction step.

A coupling oligonucleotide may be appended via a primer-extension or polymerase chain reaction step, using one or more oligonucleotide(s) that comprise a priming segment including one or more degenerate bases.

A coupling oligonucleotide may be appended via a primer-extension or polymerase chain reaction step, using one or more oligonucleotide(s) that further comprise a priming or hybridisation segment specific for a particular target nucleic acid sequence.

A coupling sequence may be added by a polynucleotide tailing reaction. A coupling sequence may be added by a terminal transferase enzyme (e.g. a terminal deoxynucleotidyl transferase enzyme). A coupling sequence may be appended via a polynucleotide tailing reaction performed with a terminal deoxynucleotidyl transferase enzyme, and wherein the coupling sequence comprises at least two contiguous nucleotides of a homopolymeric sequence.

A coupling sequence may comprise a homopolymeric 3' tail (e.g. a poly(A) tail). Optionally, in such methods, the target regions (of the barcoded oligonucleotides) comprise a complementary homopolymeric 3' tail (e.g. a poly(T) tail).

A coupling sequence may be comprised within a synthetic transposome, and may be appended via an in vitro transposition reaction.

A coupling sequence may be appended to a target nucleic acid, and wherein a barcode oligonucleotide is appended to the target nucleic acid by at least one primer-extension step or polymerase chain reaction step, and wherein said barcode oligonucleotide comprises a region of at least one nucleotide in length that is complementary to said coupling sequence. Optionally, this region of complementarity is at the 3' end of the barcode oligonucleotide. Optionally, this region of complementarity is at least 2 nucleotides in length, at least 5 nucleotides in length, at least 10 nucleotides in length, at least 20 nucleotides in length, or at least 50 nucleotides in length.

In methods in which an adapter oligonucleotide is appended (e.g. ligated or annealed) to a target nucleic acid, the adapter region of the adapter oligonucleotide provides a coupling sequence capable of hybridizing to the adapter region of a multimeric hybridization molecule or a multimeric barcode molecule.

The invention provides a method of preparing a nucleic acid sample for sequencing comprising the steps of: (a) appending a coupling sequence to first and second sub-sequences of a target nucleic acid; (b) contacting the nucleic acid sample with a multimeric barcoding reagent comprising first and second barcode molecules linked together, wherein each of the barcode molecules comprises a nucleic acid sequence comprising (in the 5' to 3' or 3' to 5' direction), a barcode region and an adapter region; (c) annealing the coupling sequence of the first sub-sequence to the adapter region of the first barcode molecule, and annealing the coupling sequence of the second sub-sequence to the adapter region of the second barcode molecule; and (d) appending barcode sequences to each of the at least two sub-sequences of the target nucleic acid to produce first and second different barcoded target nucleic acid molecules, wherein the first barcoded target nucleic acid molecule comprises the nucleic acid sequence of the barcode region of the first barcode molecule and the second barcoded target nucleic acid molecule comprises the nucleic acid sequence of the barcode region of the second barcode molecule.

In the method, each of the barcode molecules may comprise a nucleic acid sequence comprising, in the 5' to 3' direction, a barcode region and an adapter region, and step (d) may comprise extending the coupling sequence of the first sub-sequence of the target nucleic acid using the barcode region of the first barcode molecule as a template to produce a first barcoded target nucleic acid molecule, and extending the coupling sequence of the second sub-sequence of the target nucleic acid using the barcode region of the second barcode molecule as a template to produce a second barcoded target nucleic acid molecule, wherein the first barcoded target nucleic acid molecule comprises a sequence complementary to the barcode region of the first barcode molecule and the second barcoded target nucleic acid molecule comprises a sequence complementary to the barcode region of the second barcode molecule.

In the method, each of the barcode molecules may comprise a nucleic acid sequence comprising, in the 5' to 3' direction, an adapter region and a barcode region, and step (d) may comprise (i) annealing and extending a first extension primer using the barcode region of the first barcode molecule as a template to produce a first barcoded oligonucleotide, and annealing and extending a second extension primer using the barcode region of the second barcode molecule as a template to produce a second barcoded oligonucleotide, wherein the first barcoded oligonucleotide comprises a sequence complementary to the barcode region of the first barcode molecule and the second barcoded oligonucleotide comprises a sequence complementary to the barcode region of the second barcode molecule, (ii) ligating the 3' end of the first barcoded oligonucleotide to the 5' end of the coupling sequence of the first sub-sequence of the target nucleic acid to produce a first barcoded target nucleic acid molecule and ligating the 3' end of the second barcoded oligonucleotide to the 5' end of the coupling sequence of the second sub-sequence of the target nucleic acid to produce a second barcoded target nucleic acid molecule.

In the method, each of the barcode molecules may comprise a nucleic acid sequence comprising, in the 5' to 3' direction, an adapter region, a barcode region and a priming region wherein step (d) comprises (i) annealing a first extension primer to the priming region of the first barcode molecule and extending the first extension primer using the barcode region of the first barcode molecule as a template to produce a first barcoded oligonucleotide, and annealing a second extension primer to the priming region of the second barcode molecule and extending the second extension primer using the barcode region of the second barcode molecule as a template to produce a second barcoded oligonucleotide, wherein the first barcoded oligonucleotide comprises a sequence complementary to the barcode region of the first barcode molecule and the second barcoded oligonucleotide comprises a sequence complementary to the barcode region of the second barcode molecule, (ii) ligating the 3' end of the first barcoded oligonucleotide to the 5' end of the coupling sequence of the first sub-sequence of the target nucleic acid to produce a first barcoded target nucleic acid molecule and ligating the 3' end of the second barcoded oligonucleotide to the 5' end of the coupling sequence of the second sub-sequence of the target nucleic acid to produce a second barcoded target nucleic acid molecule.

The methods for preparing a nucleic acid sample for sequencing may be used to prepare a range of different nucleic acid samples for sequencing. The target nucleic acids may be DNA molecules (e.g. genomic DNA molecules) or RNA molecules (e.g. mRNA molecules). The target nucleic acids may be from any sample. For example, an individual cell (or cells), a tissue, a bodily fluid (e.g. blood, plasma and/or serum), a biopsy or a formalin-fixed paraffin-embedded (FFPE) sample. Preferably the sample is a human sample.

Optionally, during any method of preparing a nucleic acid sample for sequencing, a target nucleic acid may comprise a sequence from a multimeric barcode molecule and/or from a multimeric barcoding reagent. Optionally, a target nucleic acid may comprise a sequence from a barcoded oligonucleotide. Optionally, a target nucleic acid may comprise a barcode sequence from a multimeric barcode molecule. Optionally, during any method of preparing a nucleic acid sample for sequencing, a barcode sequence from a first multimeric barcode molecule and/or multimeric barcoding reagent may be appended to a barcode sequence from a second multimeric barcode molecule and/or multimeric barcoding reagent.

Optionally, during any method of preparing a nucleic acid sample for sequencing, a barcoded oligonucleotide from a first multimeric barcoding reagent may be appended to a barcode sequence from a second multimeric barcode molecule and/or multimeric barcoding reagent. Optionally, during any method of preparing a nucleic acid sample for sequencing, a barcoded oligonucleotide from a first multimeric barcoding reagent may be ligated to a barcoded oligonucleotide from a multimeric barcoding reagent. Optionally, during any method of preparing a nucleic acid sample for sequencing, a barcoded oligonucleotide from a first multimeric barcoding reagent may be annealed to and extended along a barcode region from a second multimeric barcoding reagent.

Optionally, during any method of preparing a nucleic acid sample for sequencing, a barcode sequence from a first multimeric barcode molecule and/or multimeric barcoding reagent may be appended to a barcode sequence from a second multimeric barcode molecule and/or multimeric barcoding reagent, wherein said first and second multimeric barcode molecules and/or multimeric barcoding reagents are co-localised (e.g. in physical proximity) within a sample or reaction volume. Optionally, during any method of preparing a nucleic acid sample for sequencing, a barcode sequence from a first multimeric barcode molecule and/or multimeric barcoding reagent may be appended to a barcode sequence from a second multimeric barcode molecule and/or multimeric barcoding reagent, wherein said first and second multimeric barcode molecules and/or multimeric barcoding reagents are bound to the same target nucleic acid and/or to the same set of target nucleic acids (e.g. the same set of physically co-localised target nucleic acids, e.g. the same set of physically co-localised fragments of genomic DNA) within a sample. Optionally, during any method of preparing a nucleic acid sample for sequencing, a barcode sequence from a first multimeric barcode molecule and/or multimeric barcoding reagent may be appended to a barcode sequence from a second multimeric barcode molecule and/or multimeric barcoding reagent, wherein said first and second multimeric barcode molecules and/or multimeric barcoding reagents are bound and/or attached to the same cell.

Optionally, during any method of preparing a nucleic acid sample for sequencing using a library of multimeric barcode molecules and/or multimeric barcoding reagents, at least one barcode sequence from each of N multimeric barcode molecules and/or N multimeric barcoding reagents may be appended to a barcode sequence from a second multimeric barcode molecule and/or multimeric barcoding reagent, wherein N is at least 10, at least 100, or at least 10³, at least 10⁴, at least 10⁵, at least 10⁶, at least 10⁷, at least 10⁸ or at least 10⁹. Optionally, during any method of preparing a nucleic acid sample for sequencing using a library of multimeric barcode molecules and/or multimeric barcoding reagents, an average of at least 0.001, 0.01, 0.1, 1.0, 10, or 100, barcode sequences from each multimeric barcode molecule and/or multimeric barcoding reagent in the library may be appended to a barcode sequence from a second multimeric barcode molecule and/or multimeric barcoding reagent in the library. Optionally, any such method of appending sequences from first and second multimeric barcode molecules and/or multimeric barcoding reagents may be employed to determine and/or predict which multimeric barcode molecules and/or multimeric barcoding reagents within a library (or libraries) thereof are physically co-localised, and/or bound to the same target nucleic acids, sets of target nucleic acids, and/or cells within a sample and/or within a reaction volume.

The sample may comprise at least 10, at least 100, or at least 10³, at least 10⁴, at least 10⁵, at least 10⁶, at least 10⁷, at least 10⁸ or at least 10⁹ target nucleic acids

The target nucleic acid may be a (single) intact nucleic acid molecule, two or more fragments of a nucleic acid molecule (such fragments may be co-localised in the sample e.g. a FFPE sample), or two or more nucleic acid molecules (such molecules may be co-localised in the sample e.g. a FFPE sample and/or such molecules may be from/in a single cell of the sample). Therefore, sub-sequences of a target nucleic acid may be sub-sequences of the same nucleic acid molecule, sub-sequences of different fragments of the same nucleic acid molecule, or sequences or sub-sequences of different nucleic acid molecules.

As would be appreciated by the skilled person, as used herein the term target nucleic acid refers to an original target nucleic acid (molecule or fragment) present in a sample and to copies or amplicons thereof. For example, the term gDNA refers to the original gDNA present in a sample and, for example, to DNA molecules that may be prepared from the original genomic DNA fragments by a primer-extension reaction. As a further example, the term mRNA refers to the original mRNA present in a sample and, for example, to cDNA molecules that may be prepared from the original mRNA fragments by reverse transcription.

The method may comprise producing at least 2, at least 5, at least 10, at least 20, at least 25, at least 50, at least 75, at least 100, at least 250, at least 500, at least 10³, at least 10⁴, at least 10⁵, at least 10⁶, at least 10⁷, at least 10⁸ or at least 10⁹ different barcoded target nucleic acid molecules. Preferably, the method comprises producing at least 5 different barcoded target nucleic acid molecules.

Each barcoded target nucleic acid molecule may comprise at least 1, at least 5, at least 10, at least 25, at least 50, at least 100, at least 250, at least 500, at least 1000, at least 2000, at least 5000, or at least 10,000 nucleotides synthesised from the target nucleic acid as template. Preferably, each barcoded target nucleic acid molecule comprises at least 20 nucleotides synthesised from the target nucleic acid as template.

Alternatively, each barcoded target nucleic acid molecule may comprise at least 5, at least 10, at least 25, at least 50, at least 100, at least 250, at least 500, at least 1000, at least 2000, at least 5000, or at least 10,000 nucleotides of the target nucleic acid. Preferably, each barcoded target nucleic acid molecule comprises at least 5 nucleotides of the target nucleic acid.

A universal priming sequence may be added to the barcoded target nucleic acid molecules. This sequence may enable the subsequent amplification of at least 5, at least 10, at least 20, at least 25, at least 50, at least 75, at least 100, at least 250, at least 500, at least 10³, at least 10⁴, at least 10⁵, at least 10⁶, at least 10⁷, at least 10⁸, or at least 10⁹ different barcoded target nucleic acid molecules using one forward primer and one reverse primer.

The method may comprise preparing two or more independent nucleic acid samples for sequencing, wherein each nucleic acid sample is prepared using a different library of multimeric barcoding reagents (or a different library of multimeric barcode molecules), and wherein the barcode regions of each library of multimeric barcoding reagents (or multimeric barcode molecules) comprise a sequence that is different to the barcode regions of the other libraries of multimeric barcoding reagents (or multimeric barcode molecules). Following the separate preparation of each of the samples for sequencing, the barcoded target nucleic acid molecules prepared from the different samples may be pooled and sequenced together. The sequence read generated for each barcoded target nucleic acid molecule may be used to identify the library of multimeric barcoding reagents (or multimeric barcode molecules) that was used in its preparation and thereby to identify the nucleic acid sample from which it was prepared.

In any method of preparing a nucleic acid sample for sequencing, the target nucleic acid molecules may be present at particular concentrations within the nucleic acid sample, for example at concentrations of at least 100 nanomolar, at least 10 nanomolar, at least 1 nanomolar, at least 100 picomolar, at least 10 picomolar, at least 1 picomolar, at least 100 femtomolar, at least 10 femtomolar, or at least 1 femtomolar. The concentrations may be 1 picomolar to 100 nanomolar, 10 picomolar to 10 nanomolar, or 100 picomolar to 1 nanomolar. Preferably, the concentrations are 10 picomolar to 1 nanomolar.

In any method of preparing a nucleic acid sample for sequencing, the multimeric barcoding reagents may be present at particular concentrations within the nucleic acid sample, for example at concentrations of at least 100 nanomolar, at least 10 nanomolar, at least 1 nanomolar, at least 100 picomolar, at least 10 picomolar, at least 1 picomolar, at least 100 femtomolar, at least 10 femtomolar, or at least 1 femtomolar. The concentrations may be 1 picomolar to 100 nanomolar, 10 picomolar to 10 nanomolar, or 100 picomolar to 1 nanomolar. Preferably, the concentrations are 1 picomolar to 100 picomolar.

In any method of preparing a nucleic acid sample for sequencing, the multimeric barcode molecules may be present at particular concentrations within the nucleic acid sample, for example at concentrations of at least 100 nanomolar, at least 10 nanomolar, at least 1 nanomolar, at least 100 picomolar, at least 10 picomolar, at least 1 picomolar, at least 100 femtomolar, at least 10 femtomolar, or at least 1 femtomolar. The concentrations may be 1 picomolar to 100 nanomolar, 10 picomolar to 10 nanomolar, or 100 picomolar to 1 nanomolar. Preferably, the concentrations are 1 picomolar to 100 picomolar.

In any method of preparing a nucleic acid sample for sequencing, the barcoded oligonucleotides may be present at particular concentrations within the nucleic acid sample, for example at concentrations of at least 100 nanomolar, at least 10 nanomolar, at least 1 nanomolar, at least 100 picomolar, at least 10 picomolar, at least 1 picomolar, at least 100 femtomolar, at least 10 femtomolar, or at least 1 femtomolar. The concentrations may be 1 picomolar to 100 nanomolar, 10 picomolar to 10 nanomolar, or 100 picomolar to 1 nanomolar. Preferably, the concentrations are 100 picomolar to 100 nanomolar.

### 12. METHODS OF PREPARING A NUCLEIC ACID SAMPLE FOR SEQUENCING USING MULTIMERIC BARCODING REAGENTS

The invention provides a method of preparing a nucleic acid sample for sequencing, wherein the method comprises the steps of: contacting the nucleic acid sample with a multimeric barcoding reagent as defined herein; annealing the target region of the first barcoded oligonucleotide to a first sub-sequence of a target nucleic acid, and annealing the target region of the second barcoded oligonucleotide to a second sub-sequence of the target nucleic acid; and extending the first and second barcoded oligonucleotides to produce first and second different barcoded target nucleic acid molecules, wherein each of the barcoded target nucleic acid molecules comprises at least one nucleotide synthesised from the target nucleic acid as a template.

In any method of preparing a nucleic acid sample for sequencing, either the nucleic acid molecules within the nucleic acid sample, and/or the multimeric barcoding reagents, may be present at particular concentrations within the solution volume, for example at concentrations of at least 100 nanomolar, at least 10 nanomolar, at least 1 nanomolar, at least 100 picomolar, at least 10 picomolar, or at least 1 picomolar. The concentrations may be 1 picomolar to 100 nanomolar, 10 picomolar to 10 nanomolar, or 100 picomolar to 1 nanomolar. Alternative higher or lower concentrations may also be used.

The method of preparing a nucleic acid sample for sequencing may comprise contacting the nucleic acid sample with a library of multimeric barcoding reagents as defined herein, and wherein: the barcoded oligonucleotides of the first multimeric barcoding reagent anneal to sub-sequences of a first target nucleic acid and first and second different barcoded target nucleic acid molecules are produced, wherein each barcoded target nucleic acid molecule comprises at least one nucleotide synthesised from the first target nucleic acid as a template; and the barcoded oligonucleotides of the second multimeric barcoding reagent anneal to sub-sequences of a second target nucleic acid and first and second different barcoded target nucleic acid molecules are produced, wherein each barcoded target nucleic acid molecule comprises at least one nucleotide synthesised from the second target nucleic acid as a template.

In the method the barcoded oligonucleotides may be isolated from the nucleic acid sample after annealing to the sub-sequences of the target nucleic acid and before the barcoded target nucleic acid molecules are produced. Optionally, the barcoded oligonucleotides are isolated by capture on a solid support through a streptavidin-biotin interaction.

Additionally or alternatively, the barcoded target nucleic acid molecules may be isolated from the nucleic acid sample. Optionally, the barcoded target nucleic acid molecules are isolated by capture on a solid support through a streptavidin-biotin interaction.

The step of extending the barcoded oligonucleotides may be performed while the barcoded oligonucleotides are annealed to the barcode molecules.

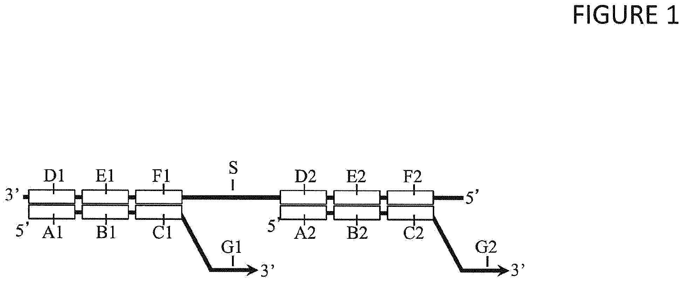

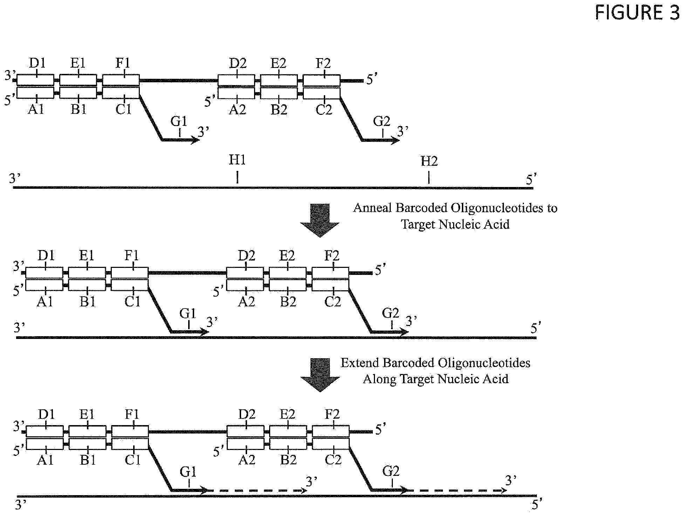

Figure 3 shows a method of preparing a nucleic acid sample for sequencing, in which a multimeric barcoding reagent defined herein (for example, as illustrated in Figure 1) is used to label and extend two or more nucleic acid sub-sequences in a nucleic acid sample. In this method, a multimeric barcoding reagent is synthesised which incorporates at least a first (A1, B1, C1, and G1) and a second (A2, B2, C2, and G2) barcoded oligonucleotide, which each comprise both a barcode region (B1 and B2) and a target region (G1 and G2 respectively).

A nucleic acid sample comprising a target nucleic acid is contacted or mixed with the multimeric barcoding reagent, and the target regions (G1 and G2) of two or more barcoded oligonucleotides are allowed to anneal to two or more corresponding sub-sequences within the target nucleic acid (H1 and H2). Following the annealing step, the first and second barcoded oligonucleotides are extended (e.g. with the target regions serving as primers for a polymerase) into the sequence of the target nucleic acid, such that at least one nucleotide of a sub-sequence is incorporated into the extended 3' end of each of the barcoded oligonucleotides. This method creates barcoded target nucleic acid molecules, wherein two or more sub-sequences from the target nucleic acid are labeled by a barcoded oligonucleotide.

Alternatively, the method may further comprise the step of dissociating the barcoded oligonucleotides from the barcode molecules before annealing the target regions of the barcoded oligonucleotides to sub-sequences of the target nucleic acid.

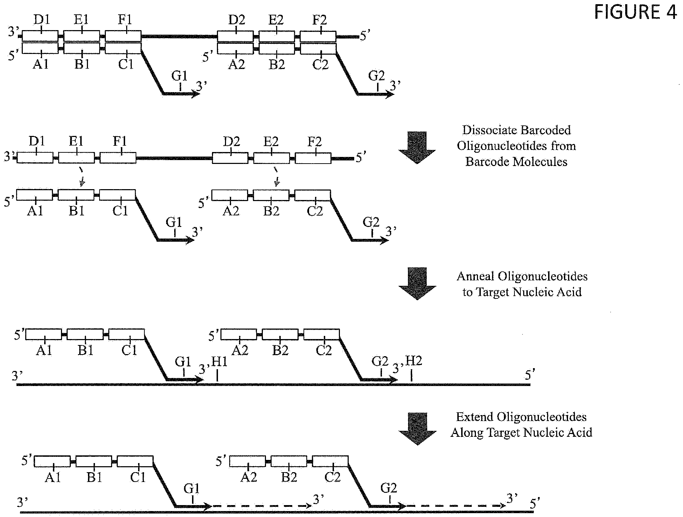

Figure 4 shows a method of preparing a nucleic acid sample for sequencing, in which a multimeric barcoding reagent described herein (for example, as illustrated in Figure 1) is used to label and extend two or more nucleic acid sub-sequences in a nucleic acid sample, but wherein the barcoded oligonucleotides from the multimeric barcoding reagent are dissociated from the barcode molecules prior to annealing to (and extension of) target nucleic acid sequences. In this method, a multimeric barcoding reagent is synthesised which incorporates at least a first (A1, B1, C1, and G1) and a second (A2, B2, C2, and G2) barcoded oligonucleotide, which each comprise a barcode region (B1 and B2) and a target region (G1 and G2).

A nucleic acid sample comprising a target nucleic acid is contacted with the multimeric barcoding reagent, and then the barcoded oligonucleotides are dissociated from the barcode molecules. This step may be achieved, for example, through exposing the reagent to an elevated temperature (e.g. a temperature of at least 35°C, at least 40°C, at least 45°C, at least 50°C, at least 55°C, at least 60°C, at least 65°C, at least 70°C, at least 75°C, at least 80°C, at least 85°C, or at least 90°C) or through a chemical denaturant, or a combination thereof. This step may also denature double-stranded nucleic acids within the sample itself. The barcoded oligonucleotides may then be allowed to for diffuse for a certain amount of time (e.g. at least 5 seconds, at least 15 seconds, at least 30 seconds, at least 60 seconds, at least 2 minutes, at least 5 minutes, at least 15 minutes, at least 30 minutes, or at least 60 minutes) (and correspondingly, to diffuse a certain physical distance within the sample).

The conditions of the reagent-sample mixture may then be changed to allow the target regions (G1 and G2) of two or more barcoded oligonucleotides to anneal to two or more corresponding sub-sequences within the target nucleic acid (H1 and H2). This could comprise, for example, lowering the temperature of the solution to allow annealing (for example, lowering the temperature to less than 90°C, less than 85°C, less than 70°C, less than 65°C, less than 60°C, less than 55°C, less than 50°C, less than 45°C, less than 40°C, less than 35°C, less than 30°C, less than 25°C, or less than 20°C). Following this annealing step (or for example, following a purification/preparation step), the first and second barcoded oligonucleotides are extended (e.g. with the target regions serving as primers for a polymerase) into the sequence of the target nucleic acid, such that at least one nucleotide of a sub-sequence is incorporated into the extended 3' end of each of the barcoded oligonucleotides.

This method creates barcoded target nucleic acid molecules wherein two or more sub-sequences from the nucleic acid sample are labeled by a barcoded oligonucleotide. In addition, the step of dissociating the barcoded oligonucleotides and allowing them to diffuse through the sample holds advantages for particular types of samples. For example, cross-linked nucleic acid samples (e.g. formalin-fixed, paraffin-embedded (FFPE) samples) may be amenable to the diffusion of relatively small, individual barcoded oligonucleotides. This method may allow labeling of nucleic acid samples with poor accessibility (e.g. FFPE samples) or other biophysical properties e.g. where target nucleic acid sub-sequences are physically far away from each other.

A universal priming sequence may be added to the barcoded target nucleic acid molecules. This sequence may enable the subsequent amplification of at least 5, at least 10, at least 20, at least 25, at least 50, at least 75, at least 100, at least 250, at least 500, at least 10³, at least 10⁴, at least 10⁵, at least 10⁶, at least 10⁷, at least 10⁸, or at least 10⁹ different barcoded target nucleic acid molecules using one forward primer and one reverse primer.

Prior to contacting the nucleic acid sample with a multimeric barcoding reagent, or library of multimeric barcoding reagents, as defined herein, a coupling sequence may be added to the 5' end or 3' end of two or more target nucleic acids of the nucleic acid sample (e.g. a FFPE DNA sample). In this method, the target regions may comprise a sequence that is complementary to the coupling sequence. The coupling sequence may comprise a homopolymeric 3' tail (e.g. a poly(A) tail). The coupling sequence may be added by a terminal transferase enzyme. In the method in which the coupling sequence comprises a poly(A) tail, the target regions may comprise a poly(T) sequence. Such coupling sequences may be added following a high-temperature incubation of the nucleic acid sample, to denature the nucleic acids contained therein prior to adding a coupling sequence.

Alternatively, a coupling sequence could be added by digestion of a target nucleic acid sample (e.g. an FFPE DNA sample) with a restriction enzyme, in which case a coupling sequence may be comprised of one or more nucleotides of a restriction enzyme recognition sequence. In this case, a coupling sequence may be at least partially double-stranded, and may comprise a blunt-ended double-stranded DNA sequence, or a sequence with a 5' overhang region of 1 or more nucleotides, or a sequence with a 3' overhang region of 1 or more nucleotides. In these cases, target regions in multimeric barcoding reagents may then comprise sequences that are either double-stranded and blunt-ended (and thus able to ligate to blunt-ended restriction digestion products), or the target regions may contain 5' or 3' overhang sequences of 1 or more nucleotides, which make them cohesive (and thus able to anneal with and ligate to) against said restriction digestion products.

The method may comprise preparing two or more independent nucleic acid samples for sequencing, wherein each nucleic acid sample is prepared using a different library of multimeric barcoding reagents (or a different library of multimeric barcode molecules), and wherein the barcode regions of each library of multimeric barcoding reagents (or multimeric barcode molecules) comprise a sequence that is different to the barcode regions of the other libraries of multimeric barcoding reagents (or multimeric barcode molecules). Following the separate preparation of each of the samples for sequencing, the barcoded target nucleic acid molecules prepared from the different samples may be pooled and sequenced together. The sequence read generated for each barcoded target nucleic acid molecule may be used to identify the library of multimeric barcoding reagents (or multimeric barcode molecules) that was used in its preparation and thereby to identify the nucleic acid sample from which it was prepared.

The invention provides a method of preparing a nucleic acid sample for sequencing, wherein the method comprises the steps of: (a) contacting the nucleic acid sample with a multimeric barcoding reagent, wherein each barcoded oligonucleotide comprises in the 5' to 3' direction a target region and a barcode region, and first and second target primers; (b) annealing the target region of the first barcoded oligonucleotide to a first sub-sequence of a target nucleic acid and annealing the target region of the second barcoded oligonucleotide to a second sub-sequence of the target nucleic acid; (c) annealing the first target primer to a third sub-sequence of the target nucleic acid, wherein the third sub-sequence is 3' of the first sub-sequence, and annealing the second target primer to a fourth sub-sequence of the target nucleic acid, wherein the fourth sub-sequence is 3' of the second sub-sequence; (d) extending the first target primer using the target nucleic acid as template until it reaches the first sub-sequence to produce a first extended target primer, and extending the second target primer using the target nucleic acid as template until it reaches the second sub-sequence to produce a second extended target primer; and (e) ligating the 3' end of the first extended target primer to the 5' end of the first barcoded oligonucleotide to produce a first barcoded target nucleic acid molecule, and ligating the 3' end of the second extended target primer to the 5' end of the second barcoded oligonucleotide to produce a second barcoded target nucleic acid molecule, wherein the first and second barcoded target nucleic acid molecules are different, and wherein each of the barcoded target nucleic acid molecules comprises at least one nucleotide synthesised from the target nucleic acid as a template.

In the method, steps (b) and (c) may be performed at the same time.

### 13. METHODS OF PREPARING A NUCLEIC ACID SAMPLE FOR SEQUENCING USING MULTIMERIC BARCODING REAGENTS AND ADAPTER OLIGONUCLEOTIDES

The methods provided below may be performed with any of the kits defined herein.

The invention further provides a method of preparing a nucleic acid sample for sequencing, wherein the method comprises the steps of: (a) contacting the nucleic acid sample with a first and second adapter oligonucleotide as defined herein; (b) annealing or ligating the first adapter oligonucleotide to a first sub-sequence of a target nucleic acid, and annealing or ligating the second adapter oligonucleotide to a second sub-sequence of the target nucleic acid; (c) contacting the nucleic acid sample with a multimeric barcoding reagent as defined herein; (d) annealing the adapter region of the first adapter oligonucleotide to the adapter region of the first barcode molecule, and annealing the adapter region of the second adapter oligonucleotide to the adapter region of the second barcode molecule; and (e) ligating the 3' end of the first barcoded oligonucleotide to the 5' end of the first adapter oligonucleotide to produce a first barcoded-adapter oligonucleotide and ligating the 3' end of the second barcoded oligonucleotide to the 5' end of the second adapter oligonucleotide to produce a second barcoded-adapter oligonucleotide.

The invention further provides a method of preparing a nucleic acid sample for sequencing, wherein the method comprises the steps of: (a) contacting the nucleic acid sample with a first and second adapter oligonucleotide as defined herein; (b) the first adapter oligonucleotide to a first sub-sequence of a target nucleic acid, and ligating the second adapter oligonucleotide to a second sub-sequence of the target nucleic acid; (c) contacting the nucleic acid sample with a multimeric barcoding reagent as defined herein; (d) annealing the adapter region of the first adapter oligonucleotide to the adapter region of the first barcode molecule, and annealing the adapter region of the second adapter oligonucleotide to the adapter region of the second barcode molecule; and (e) extending the first adapter oligonucleotide using the barcode region of the first barcode molecule as a template to produce a first barcoded target nucleic acid molecule, and extending the second adapter oligonucleotide using the barcode region of the second barcode molecule as a template to produce a second barcoded target nucleic acid molecule, wherein the first barcoded target nucleic acid molecule comprises a sequence complementary to the barcode region of the first barcode molecule and the second barcoded target nucleic acid molecule comprises a sequence complementary to the barcode region of the second barcode molecule.

The invention further provides a method of preparing a nucleic acid sample for sequencing, wherein the method comprises the steps of: (a) contacting the nucleic acid sample with a first and second adapter oligonucleotide as defined herein; (b) annealing the target region of the first adapter oligonucleotide to a first sub-sequence of a target nucleic acid, and annealing the target region of the second adapter oligonucleotide to a second sub-sequence of the target nucleic acid; (c) contacting the nucleic acid sample with a multimeric barcoding reagent as defined herein; (d) annealing the adapter region of the first adapter oligonucleotide to the adapter region of the first barcode molecule, and annealing the adapter region of the second adapter oligonucleotide to the adapter region of the second barcode molecule; and (e) ligating the 3' end of the first barcoded oligonucleotide to the 5' end of the first adapter oligonucleotide to produce a first barcoded-adapter oligonucleotide and ligating the 3' end of the second barcoded oligonucleotide to the 5' end of the second adapter oligonucleotide to produce a second barcoded-adapter oligonucleotide.

In the method the first and second barcoded-adapter oligonucleotides may be extended to produce first and second different barcoded target nucleic acid molecules each of which comprises at least one nucleotide synthesised from the target nucleic acid as a template.

Alternatively, the first and second adapter oligonucleotides may be extended to produce first and second different target nucleic acid molecules each of which comprises at least one nucleotide synthesised from the target nucleic acid as a template. In this method, step (f) produces a first barcoded target nucleic acid molecule (i.e. the first barcoded oligonucleotide ligated to the extended first adapter oligonucleotide) and a second barcoded target nucleic acid molecule (i.e. the second barcoded oligonucleotide ligated to the extended second adapter oligonucleotide).

The step of extending the adapter oligonucleotides may be performed before step (c), before step (d) and/or before step (e), and the first and second adapter oligonucleotides may remain annealed to the first and second barcode molecules until after step (e).

The method may be performed using a library of multimeric barcoding reagents as defined herein and an adapter oligonucleotide as defined herein for each of the multimeric barcoding reagents. Preferably, the barcoded-adapter oligonucleotides of the first multimeric barcoding reagent anneal to sub-sequences of a first target nucleic acid and first and second different barcoded target nucleic acid molecules are produced, wherein each barcoded target nucleic acid molecule comprises at least one nucleotide synthesised from the first target nucleic acid as a template; and the barcoded-adapter oligonucleotides of the second multimeric barcoding reagent anneal to sub-sequences of a second target nucleic acid and first and second different barcoded target nucleic acid molecules are produced, wherein each barcoded target nucleic acid molecule comprises at least one nucleotide synthesised from the second target nucleic acid as a template.

The method may be performed using a library of multimeric barcoding reagents as defined herein and an adapter oligonucleotide as defined herein for each of the multimeric barcoding reagents. Preferably, the adapter oligonucleotides of the first multimeric barcoding reagent anneal to sub-sequences of a first target nucleic acid and first and second different target nucleic acid molecules are produced, wherein each target nucleic acid molecule comprises at least one nucleotide synthesised from the first target nucleic acid as a template; and the adapter oligonucleotides of the second multimeric barcoding reagent anneal to sub-sequences of a second target nucleic acid and first and second different target nucleic acid molecules are produced, wherein each target nucleic acid molecule comprises at least one nucleotide synthesised from the second target nucleic acid as a template.

The barcoded-adapter oligonucleotides may be isolated from the nucleic acid sample after annealing to the sub-sequences of the target nucleic acid and before the barcoded target nucleic acid molecules are produced. Optionally, the barcoded-adapter oligonucleotides are isolated by capture on a solid support through a streptavidin-biotin interaction.

The barcoded target nucleic acid molecules may be isolated from the nucleic acid sample. Optionally, the barcoded target nucleic acid molecules are isolated by capture on a solid support through a streptavidin-biotin interaction.

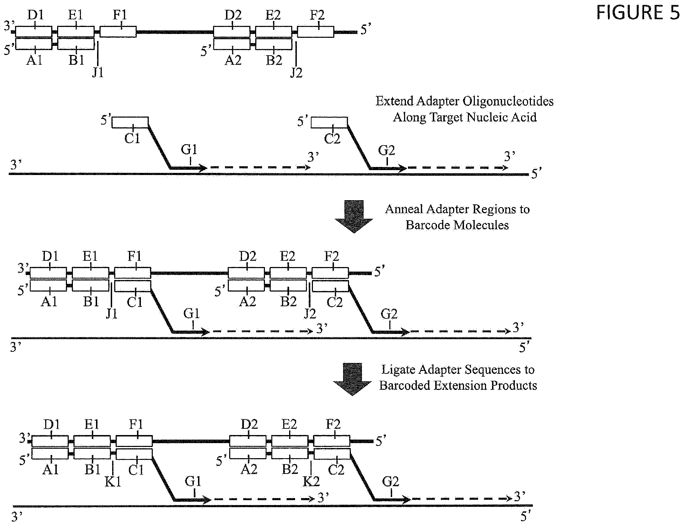

Figure 5 shows a method of preparing a nucleic acid sample for sequencing using a multimeric barcoding reagent. In the method first (C1 and G1) and second (C2 and G2) adapter oligonucleotides are annealed to a target nucleic acid in the nucleic acid sample, and then used in a primer extension reaction. Each adapter oligonucleotide is comprised of an adapter region (C1 and C2) that is complementary to, and thus able to anneal to, the 5' adapter region of a barcode molecule (F1 and F2). Each adapter oligonucleotide is also comprised of a target region (G1 and G2), which may be used to anneal the barcoded oligonucleotides to target nucleic acids, and then may be used as primers for a primer-extension reaction or a polymerase chain reaction. These adapter oligonucleotides may be synthesised to include a 5'-terminal phosphate group.

The adapter oligonucleotides, each of which has been extended to include sequence from the target nucleic acid, are then contacted with a multimeric barcoding reagent which comprises a first (D1, E1, and F1) and second (D2, E2, and F2) barcode molecule, as well as first (A1 and B1) and second (A2 and B2) barcoded oligonucleotides, which each comprise a barcode region (B1 and B2), as well as 5' regions (A1 and A2). The first and second barcode molecules each comprise a barcode region (E1 and E2), an adapter region (F1 and F2), and a 3' region (D1 and D2), and are linked together, in this embodiment by a connecting nucleic acid sequence (S).

After contacting the primer-extended nucleic acid sample with a multimeric barcoding reagent, the 5' adapter regions (C1 and C2) of each adapter oligonucleotides are able to anneal to a 'ligation junction' adjacent to the 3' end of each barcoded oligonucleotide (J1 and J2). The 5' end of the extended adapter oligonucleotides are then ligated to the 3' end of the barcoded oligonucleotides within the multimeric barcoding reagent, creating a ligated base pair (K1 and K2) where the ligation junction was formerly located. The solution may subsequently be processed further or amplified, and used in a sequencing reaction.

This method, like the methods illustrated in Figures 3 and 4, creates barcoded target nucleic acid molecules, wherein two or more sub-sequences from the nucleic acid sample are labeled by a barcoded oligonucleotide. In this method a multimeric barcoding reagent does not need to be present for the step of annealing target regions to sub-sequences of the target nucleic acid, or the step of extending the annealed target regions using a polymerase. This feature may hold advantages in certain applications, for example wherein a large number of target sequences are of interest, and the target regions are able to hybridise more rapidly to target nucleic acids when they are not constrained molecularly by a multimeric barcoding reagent.

### 14. METHODS OF PREPARING A NUCLEIC ACID SAMPLE FOR SEQUENCING USING MULTIMERIC BARCODING REAGENTS, ADAPTER OLIGONUCLEOTIDES AND EXTENSION PRIMERS

The invention further provides a method of preparing a nucleic acid sample for sequencing, wherein the method comprises the steps of: (a) contacting the nucleic acid sample with first and second adapter oligonucleotides as defined herein; (b) annealing the target region of the first adapter oligonucleotide to a first sub-sequence of a target nucleic acid, and annealing the target region of the second adapter oligonucleotide to a second sub-sequence of the target oligonucleotide; (c) contacting the nucleic acid sample with a library of multimeric barcode molecules as defined herein and first and second extension primers as defined herein; (d) annealing the adapter region of the first adapter oligonucleotide to the adapter region of the first barcode molecule, and annealing the adapter region of the second adapter oligonucleotide to the adapter region of the second barcode molecule; (e) extending the first extension primer using the barcode region of the first barcode molecule as a template to produce a first barcoded oligonucleotide, and extending the second extension primer using the barcode region of the second barcode molecule as a template to produce a second barcoded oligonucleotide, wherein the first barcoded oligonucleotide comprises a sequence complementary to the barcode region of the first barcode molecule and the second barcoded oligonucleotide comprises a sequence complementary to the barcode region of the second barcode molecule; and (f) ligating the 3' end of the first barcoded oligonucleotide to the 5' end of the first adapter oligonucleotide to produce a first barcoded-adapter oligonucleotide and ligating the 3' end of the second barcoded oligonucleotide to the 5' end of the second adapter oligonucleotide to produce a second barcoded-adapter oligonucleotide.

In the method the first and second barcoded-adapter oligonucleotides may be extended to produce first and second different barcoded target nucleic acid molecules each of which comprises at least one nucleotide synthesised from the target nucleic acid as a template.

Alternatively, the first and second adapter oligonucleotides may be extended to produce first and second different target nucleic acid molecules each of which comprises at least one nucleotide synthesised from the target nucleic acid as a template. In this method, step (f) produces a first barcoded target nucleic acid molecule (i.e. the first barcoded oligonucleotide ligated to the extended first adapter oligonucleotide) and a second barcoded target nucleic acid molecule (i.e. the second barcoded oligonucleotide ligated to the extended second adapter oligonucleotide).

The step of extending the adapter oligonucleotides may be performed before step (c), before step (d), before step (e) and/or before step (f), and the first and second adapter oligonucleotides may remain annealed to the first and second barcode molecules until after step (f).

The extension primers may be annealed to the multimeric barcode molecules prior to step (c). Alternatively, the nucleic acid sample may be contacted with a library of multimeric barcode molecules as defined herein and separate extension primers as defined herein. The extension primers may then be annealed to the multimeric barcode molecules in the nucleic acid sample. The extension primers may be annealed to the multimeric barcode molecules during step (d).

The methods may use a library of first and second extension primers e.g. the library may comprise first and second extension primers for each multimeric barcode molecule. Optionally, each extension primer in the library of extension primers may comprise a secondary barcode region, wherein said secondary barcode region is different to the secondary barcode regions within the other extension primers within the library. Optionally, such a library may comprise at least 2, at least 3, at least 4, at least 5, at least 10, at least 20, at least 50, at least 100, at least 500, at least 1000, at least 5,000, or at least 10,000 different extension primers.

### 15. METHODS OF PREPARING A NUCLEIC ACID SAMPLE FOR SEQUENCING USING MULTIMERIC BARCODING REAGENTS, ADAPTER OLIGONUCLEOTIDES AND TARGET PRIMERS

The invention further provides a method of preparing a nucleic acid sample for sequencing, wherein the method comprises the steps of: (a) contacting the nucleic acid sample with first and second adapter oligonucleotides, wherein each adapter oligonucleotide comprises in the 5' to 3' direction a target region and an adapter region, and first and second target primers; (b) annealing the target region of the first adapter oligonucleotide to a first sub-sequence of a target nucleic acid, and annealing the target region of the second adapter oligonucleotide to a second sub-sequence of the target nucleic acid; (c) annealing the first target primer to a third sub-sequence of the target nucleic acid, wherein the third sub-sequence is 3' of the first sub-sequence, and annealing the second target primer to a fourth sub-sequence of the target nucleic acid, wherein the fourth sub-sequence is 3' of the second sub-sequence; (d) extending the first target primer using the target nucleic acid as template until it reaches the first sub-sequence to produce a first extended target primer, and extending the second target primer using the target nucleic acid as template until it reaches the second sub-sequence to produce a second extended target primer; (e) ligating the 3' end of the first extended target primer to the 5' end of the first adapter oligonucleotide, and ligating the 3' end of the second extended target primer to the 5' end of the second adapter oligonucleotide; (f) contacting the nucleic acid sample with a library of multimeric barcode molecules as defined herein; (g) annealing the adapter region of the first adapter oligonucleotide to the adapter region of the first barcode molecule, and annealing the adapter region of the second adapter oligonucleotide to the adapter region of the second barcode molecule; and (h) extending the first adapter oligonucleotide using the barcode region of the first barcode molecule as a template to produce a first barcoded oligonucleotide, and extending the second adapter oligonucleotide using the barcode region of the second barcode molecule as a template to produce a second barcoded oligonucleotide, wherein the first barcoded oligonucleotide comprises a sequence complementary to the barcode region of the first barcode molecule and the second barcoded oligonucleotide comprises a sequence complementary to the barcode region of the second barcode molecule.

In the method, steps (b) and (c) may be performed at the same time.

In the method, steps (f)-(h) may be performed before steps (d) and (e). In this method, first and second different barcoded target nucleic acid molecules, each of which comprises at least one nucleotide synthesised from the target nucleic acid as a template, are produced by the completion of step (e).

In the method, steps (f)-(h) may be performed after steps (d) and (e). In this method, first and second different barcoded target nucleic acid molecules, each of which comprises at least one nucleotide synthesised from the target nucleic acid as a template, are produced by the completion of step (h).

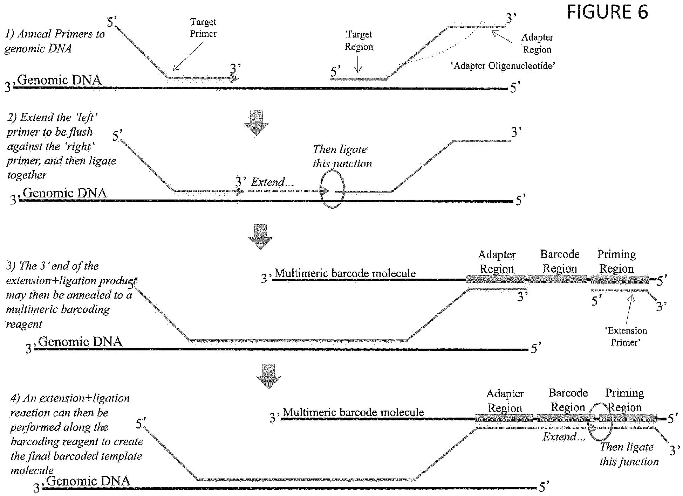

Figure 6 illustrates one way in which this method may be performed. In this method, the target nucleic acid is genomic DNA. It will be appreciated that the target nucleic acid may be another type of nucleic acid e.g. an RNA molecule such as an mRNA molecule.

### 16. METHODS OF PREPARING A NUCLEIC ACID SAMPLE FOR SEQUENCING USING MULTIMERIC BARCODING REAGENTS AND TARGET PRIMERS

The invention further provides a method of preparing a nucleic acid sample for sequencing, wherein the method comprises the steps of: (a) contacting the nucleic acid sample with first and second barcoded oligonucleotides linked together, wherein each barcoded oligonucleotide comprises in the 5' to 3' direction a target region and a barcode region, and first and second target primers; (b) annealing the target region of the first barcoded oligonucleotide to a first sub-sequence of a target nucleic acid, and annealing the target region of the second barcoded oligonucleotide to a second sub-sequence of the target nucleic acid; (c) annealing the first target primer to a third sub-sequence of the target nucleic acid, wherein the third sub-sequence is 3' of the first sub-sequence, and annealing the second target primer to a fourth sub-sequence of the target nucleic acid, wherein the fourth sub-sequence is 3' of the second sub-sequence; (d) extending the first target primer using the target nucleic acid as template until it reaches the first sub-sequence to produce a first extended target primer, and extending the second target primer using the target nucleic acid as template until it reaches the second sub-sequence to produce a second extended target primer; (e) ligating the 3' end of the first extended target primer to the 5' end of the first barcoded oligonucleotide to produce a first barcoded target nucleic acid molecule, and ligating the 3' end of the second extended target primer to the 5' end of the second barcoded oligonucleotide to produce a second barcoded target nucleic acid molecule, wherein the first and second barcoded target nucleic acid molecules are different and each comprises at least one nucleotide synthesised from the target nucleic acid as a template.

### 17. METHODS OF ASSEMBLING MULTIMERIC BARCODE MOLECULES BY ROLLING CIRCLE AMPLIFICATION

The invention further provides a method of assembling a library of multimeric barcode molecules from a library of nucleic acid barcode molecules, wherein said nucleic acid barcode molecules are amplified by one or more rolling circle amplification (RCA) processes. In this method, nucleic acid barcode molecules may each comprise, optionally in the 5' to 3' direction, a barcode region and an adapter region. Optionally, the nucleic acid barcode molecules may comprise a phosphorylated 5' end capable of ligating to a 3' end of a nucleic acid molecule.

In this method, nucleic acid barcode molecules within the library are converted into a circular form, such that the barcode region and the adapter region from a barcode molecule are comprised within a contiguous circular nucleic acid molecule. Optionally, such a step of converting nucleic acid barcode molecules into circular form may be performed by an intramolecular single-stranded ligation reaction. For example, nucleic acid barcode molecules comprising a phosphorylated 5' end may be circularised by incubation with a single-stranded nucleic acid ligase, such as T4 RNA Ligase 1, or by incubation with a thermostable single-stranded nucleic acid ligase, such as the CircLigase thermostable single-stranded nucleic acid ligase (from Epicentre Bio). Optionally, an exonuclease step may be performed to deplete or degrade uncircularised and/or unligated molecules; optionally wherein the exonuclease step is performed by E. coli exonuclease I, or by E. coli lambda exonuclease.

Optionally, a step of converting nucleic acid barcode molecules into circular form may be performed using a circularisation primer. In this embodiment, nucleic acid barcode molecules comprise a phosphorylated 5' end. Furthermore, in this embodiment, a circularisation primer comprising a 5' region complementary to the 3' region of a barcode molecule, and a 3' region complementary to the 5' region of a barcode molecule, is annealed to a barcode molecule, such that the 5' end and the 3' end of the barcode molecule are immediately adjacent to each other whilst annealed along the circularisation primer. Following the annealing step, the annealed barcode molecules are ligated with a ligase enzyme, such as T4 DNA ligase, which ligates the 3' end of the barcode molecule to the 5' end of the barcode molecule. Optionally, an exonuclease step may be performed to deplete or degrade uncircularised and/or unligated molecules; optionally wherein the exonuclease step is performed by E. coli exonuclease I, or by E. coli lambda exonuclease.

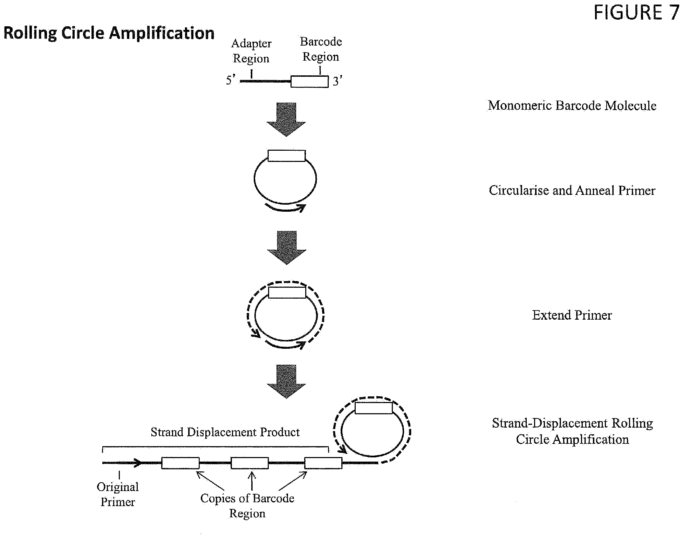

Following a circularisation step, circularised barcode molecules may be amplified with a rolling circle amplification step. In this process, a primer is annealed to a circularised nucleic acid strand comprising a barcode molecule, and the 3' end of said primer is extended with a polymerase exhibiting strand displacement behaviour. For each original circularised barcode molecule, this process may form a linear (non-circular) multimeric barcode molecule comprising copies of the original circularised barcode molecule, as illustrated in Figure 7. In one embodiment, a circularisation primer that has been annealed to a barcode molecule may serve as the primer for a rolling circle amplification step. Optionally, following circularisation, a separate amplification primer which is at least partially complementary to the circularised barcode molecule, may be annealed to the circularised barcode molecule to prime a rolling circle amplification step.

During said rolling circle amplification step, the primer may be extended by the polymerase, wherein the polymerase extends along the circularised template until it encounters the 5' end of the amplification primer and/or circularisation primer, whereupon it continues amplification along the circularised template whilst displacing the 5' end of the primer, and then displacing the previously amplified strand, in a process of rolling circle amplification. Following any such amplification step, a purification and/or cleanup step may be performed to isolate products of such rolling circle amplification. Optionally, a purification and/or cleanup step may comprise a size-selection process, such as a gel-based size selection process, or a solid-phase reversible immobilisation size-selection process, such as a magnetic bead-based solid-phase reversible immobilisation size-selection process. Optionally, amplification products at least 100 nucleotides in length, at least 500 nucleotides in length, at least 1000 nucleotides in length, at least 2000 nucleotides in length, at least 5000 nucleotides in length, at least 10,000 nucleotides in length, at least 20,000 nucleotides in length, at least 50,000 nucleotides in length, or at least 100,000 nucleotides in length may be purified. Optionally, before and/or during any rolling circle amplification step, a single-stranded DNA binding protein (such as T4 Gene 32 Protein) may be included in a reaction mixture, such as to prevent the formation of secondary structures by circularised templates and/or amplification products. During or after any such rolling circle amplification step, said single-stranded DNA binding protein may be removed and/or inactivated, such as by a heat-inactivation step.

Optionally, such a process of rolling circle amplification may be performed by phi29 DNA polymerase. Optionally, such a process of rolling circle amplification may be performed by a Bst or Bsm DNA polymerase. Optionally, such a process of rolling circle amplification may be performed such that at least one full copy of the circularised template is produced by the polymerase. Optionally, such a process of rolling circle amplification may be performed such that at least 2, at least 3, at least 5, at least 10, at least 50, at least 100, at least 200, at least 500, at least 1000, at least 2000, at least 5000, or at least 10,000 full copies of the circularised template are produced by the polymerase.

An example of this method is provided in Figure 7. In the figure, a barcode molecule comprising an adapter region and a barcode region is circularised (e.g. using a single-stranded ligation reaction). A primer is then annealed to the resulting circularised product, and said primer is then extended using a strand-displacing polymerase (such as phi29 DNA polymerase). Whilst synthesising the extension product, the polymerase then processes one circumference around the circularised product, and then displaces the original primer in a strand-displacement reaction. The rolling-circle amplification process may then proceed to create a long contiguous nucleic acid molecule comprising many tandem copies of the circularised sequence - i.e. many tandem copies of a barcode and adapter sequence (and/or sequences complementary to a barcode and adapter sequence) of a barcode molecule.

### 18. METHODS OF AMPLIFYING MULTIMERIC BARCODE MOLECULES BY ROLLING CIRCLE AMPLIFICATION

### A) Properties of Multimeric Barcode Molecules

The invention further provides a method of amplifying multimeric barcode molecules from a library of multimeric barcode molecules, wherein said multimeric barcode molecules are amplified by one or more rolling circle amplification (RCA) processes. In this method, a multimeric barcode molecule comprises at least two barcode molecules linked together within a (single) nucleic acid molecule. Optionally, each barcode region of a barcode molecule may be adjacent to one or more adapter regions; optionally, such an adapter region may be at the 5' end of the associated barcode region, or may be at the 3' end of the associated barcode region. Optionally, each barcode region is associated with both a 3' adapter region and a 5' adapter region; optionally the 3' adapter region and a 5' adapter region may comprise different adapter sequences. Optionally, one or more adapter regions may comprise a sequence complementary to or identical to an adapter region of an adapter oligonucleotide. Optionally, one or more adapter regions may comprise a sequence complementary to or identical to all or part of an extension primer. A multimeric barcode molecule may take any of the forms described herein.

Each multimeric barcode molecule may further comprise, optionally within the 5' end of the multimeric barcode molecule, a forward reagent amplification sequence, which may comprise a sequence complementary to or identical to a forward reagent amplification primer. Each multimeric barcode molecule may further comprise, optionally within the 3' end of the multimeric barcode molecule, a reverse reagent amplification sequence, which may comprise a sequence complementary to or identical to a reverse reagent amplification primer.

A multimeric barcoding molecule may comprise at least 2, at least 3, at least 4, at least 5, at least 10, at least 20, at least 25, at least 50, at least 75, at least 100, at least 200, at least 500, at least 1000, at least 5000, at least 10⁴, at least 10⁵, or at least 10⁶ different barcode molecules. Any library of multimeric barcode molecules may comprise at least 5, at least 10, at least 20, at least 25, at least 50, at least 75, at least 100, at least 250, at least 500, at least 10³, at least 10⁴, at least 10⁵, at least 10⁶, at least 10⁷, at least 10⁸, or at least 10⁹ different multimeric barcode molecules.

### B) Methods of Circularising Multimeric Barcode Molecules and/or Libraries Thereof

In a method of amplifying multimeric barcode molecules, multimeric barcode molecules (and/or a library thereof) are converted into a circular form, such that the 2 or more barcode regions (and, optionally, 2 or more adapter regions) from a multimeric barcode molecule are comprised within a contiguous circular nucleic acid molecule. Optionally, such a step of converting multimeric barcode molecules into circular form may be performed by an intramolecular single-stranded ligation reaction. For example, multimeric barcode molecules comprising a phosphorylated 5' end may be circularised by incubation with a single-stranded nucleic acid ligase, such as T4 RNA Ligase 1, or by incubation with a thermostable single-stranded nucleic acid ligase, such as the CircLigase thermostable single-stranded nucleic acid ligase (from Epicentre Bio), wherein such a said ligase enzyme ligates the 5' phosphorylated end of a multimeric barcode molecule to the 3' end of the same molecule. Optionally, an exonuclease step may be performed to deplete or degrade uncircularised and/or unligated molecules; optionally wherein the exonuclease step is performed by E. coli exonuclease I, or by E. coli lambda exonuclease.

Optionally, a step of converting multimeric barcode molecules into circular form may be performed by an intramolecular double-stranded ligation reaction. For example, multimeric barcode molecules comprising double-stranded sequences and phosphorylated 5' ends may comprise blunt ends, or optionally may have their ends converted into a blunt form with a blunting reaction. Such multimeric barcode molecules may then be converted into circular form by an intramolecular double-stranded ligation reaction with a T4 DNA Ligase enzyme, such that one end of a multimeric barcode molecule is ligated on one or both stranded to the other end of the same multimeric barcode molecule.

In an alternative embodiment, a step of converting multimeric barcode molecules into circular form may be performed by an intramolecular double-stranded ligation reaction wherein the ends of multimeric barcode molecules comprise ends generated by a restriction digestion step. In one such embodiment, multimeric barcode molecules comprising double-stranded sequences comprise recognition sites for one or more restriction endonuclease enzymes within their 5' and 3' regions. In a digestion reaction, said multimeric barcode molecules are digested with such one or more restriction endonuclease enzymes to create digested multimeric barcode molecules comprising ends with the restriction digestion products. These digested multimeric barcode molecules may optionally then be purified, for example with a gel-based or bead-based size selection step. The digested multimeric barcode molecules may then be converted into circular form by an intramolecular double-stranded ligation reaction with a T4 DNA Ligase enzyme, such that the restriction-digested site on one end of a multimeric barcode molecule is ligated to the restriction-digested site on the other end of the same multimeric barcode molecule. Optionally, the ends produced by the restriction enzyme(s) may be blunt, or may comprise a 3' overhang of 1 or more nucleotides, or may comprise a 5' overhang of 1 or more nucleotides.

Optionally, a step of converting multimeric barcode molecules into circular form may be performed using a circularisation primer. In this embodiment, multimeric barcode molecules comprise a phosphorylated 5' end. Furthermore, in this embodiment, a circularisation primer comprising a 5' region complementary to the 3' region of a multimeric barcode molecule, and a 3' region complementary to the 5' region of a multimeric barcode molecule, is annealed to a multimeric barcode molecule, such that the 5' end and the 3' end of the multimeric barcode molecule are immediately adjacent to each other whilst annealed along the circularisation primer. Optionally, the multimeric barcode molecules may comprise forward reagent amplification sequences and reverse reagent amplification sequences within their 5' and 3' ends respectively, and the circularisation primer may comprise sequences at least partially complementary to said reagent amplification sequences. Optionally, following a step of annealing circularisation primers to a multimeric barcode molecule or library thereof, excess circularisation primers, which are not annealed to multimeric barcode molecules, may be depleted from the solution by a cleanup reaction, such as a gel-based size-selection step or bead-based size selection step, such as a solid-phase reversible immobilisation step.

Following a circularisation-primer annealing step, the annealed multimeric barcode molecules are ligated with a ligase enzyme, such as T4 DNA ligase, which ligates the 3' end of the multimeric barcode molecule to the 5' end of the multimeric barcode molecule that is annealed immediately adjacent to it along the circularisation primer. Optionally, an exonuclease step may be performed to deplete or degrade uncircularised and/or unligated molecules; optionally wherein the exonuclease step is performed by E. coli exonuclease I, or by E. coli lambda exonuclease.

During any step of assembling, amplifying, ligating, and/or circularising barcode molecules and/or multimeric barcode molecules, and/or libraries or constituents thereof, the concentration of such molecules within solution may be retained within certain ranges. For example, the concentration of barcode molecules and/or multimeric barcode molecules may be less than 100 nanomolar, less than 10 nanomolar, less than 1 nanomolar, less than 100 picomolar, less than 10 picomolar, less than 1 picomolar, less than 100 femtomolar, less than 10 femtomolar, or less than 1 femtomolar. Optionally, during any step of assembling, amplifying, ligating, and/or circularising barcode molecules and/or multimeric barcode molecules, and/or libraries or constituents thereof, the concentration of such molecules within solution may allow two or more different barcode molecules and/or multimeric barcode molecules to become appended, concatenated, or ligated to each other within solution, optionally wherein such appended, concatenated, or ligated products are then further amplified during an amplification step.

### C) Methods of Amplifying Circularised Multimeric Barcode Molecules with Rolling Circle Amplification

Following a circularisation step, circularised multimeric barcode molecules are amplified with a rolling circle amplification step. In this process, a primer is annealed to a circularised nucleic acid strand comprising a multimeric barcode molecule, and the 3' end of said primer is extended with a polymerase exhibiting strand displacement behaviour. In one embodiment, a circularisation primer that has been annealed to a multimeric barcode molecule may serve as the primer for a rolling circle amplification step. Optionally, following circularisation, one or more separate amplification primer(s) which are at least partially complementary to a circularised multimeric barcode molecule, may be annealed to the circularised barcode molecule to prime a rolling circle amplification step. Optionally, oligonucleotides at least partially complementary to one or more adapter regions comprised within a multimeric barcode molecule may be employed as amplification primers. Optionally, following any step of annealing one or more amplification primers to circularised multimeric barcode molecules, a cleanup step may be performed to deplete non-annealed primers from the solution and/or to isolate primer-annealed multimeric barcode molecules. Optionally, such a cleanup step may comprise a size-selection step, such as a gel-based size-selection step or bead-based size selection step, such as a solid-phase reversible immobilisation step. Optionally, before and/or during any rolling circle amplification step, a single-stranded DNA binding protein (such as T4 Gene 32 Protein) may be included in a reaction mixture, such as to prevent the formation of secondary structures by circularised templates and/or amplification products. During or after any such rolling circle amplification step, said single-stranded DNA binding protein may be removed and/or inactivated, such as by a heat-inactivation step.

During said rolling circle amplification step, each primer may be extended by the polymerase, wherein the polymerase extends along the circularised template until it encounters the 5' end of an amplification primer and/or a circularisation primer, whereupon it continues amplification along the circularised template whilst displacing the 5' end of the primer, and then displacing the previously amplified strand, in a process of rolling circle amplification. Following any such amplification step, a purification and/or cleanup step may be performed to isolate products of such rolling circle amplification. Optionally, a purification step and/or cleanup step may comprise a size-selection process, such as a gel-based size selection process, or a solid-phase reversible immobilisation size-selection process, such as a magnetic bead-based solid-phase reversible immobilisation size-selection process. Optionally, amplification products at least 100 nucleotides in length, at least 500 nucleotides in length, at least 1000 nucleotides in length, at least 2000 nucleotides in length, at least 5000 nucleotides in length, at least 10,000 nucleotides in length, at least 20,000 nucleotides in length, at least 50,000 nucleotides in length, or at least 100,000 nucleotides in length may be purified.

Optionally, such a process of rolling circle amplification may be performed by phi29 DNA polymerase. Optionally, such a process of rolling circle amplification may be performed by a Bst or Bsm DNA polymerase. Optionally, such a process of rolling circle amplification may be performed such that at least one full copy of the circularised template is produced by the polymerase. Optionally, such a process of rolling circle amplification may be performed such that at least 2, at least 3, at least 5, at least 10, at least 50, at least 100, at least 200, at least 500, at least 1000, at least 2000, at least 5000, or at least 10,000 full copies of the circularised multimeric barcode molecule template are produced by the polymerase from each primer that has been annealed to the circularised multimeric barcode molecule..

Optionally, at least 2, at least 3, at least 5, at least 10, at least 20, at least 50, at least 100, or at least 500 primers may be annealed to a circularised multimeric barcode molecule and used to prime rolling circle amplification reactions along the multimeric barcode molecule to which they are annealed. Parts of the extension products produced from these primers may remain annealed to the circularised multimeric barcode molecule to which they were originally annealed, thus producing a macromolecular nucleic acid complex comprising a circularised multimeric barcode molecule, and two or more rolling circle amplification products at least partially annealed thereto.

The sequences within a multimeric barcode molecule may be configured such that the rolling circle amplification products comprise one or more adapter regions and/or adapter sequences, such that said adapter regions and/or adapter sequences are able to hybridise to complementary sequences, for example complementary sequences comprised within coupling oligonucleotides, adapter oligonucleotides, and/or extension primers. Part or all of any such rolling circle amplification product(s) and/or macromolecular nucleic acid complex may be used to synthesise a multimeric barcoding reagent, for example by serving as barcode molecules to synthesise barcoded oligonucleotides. Part or all of any such rolling circle amplification product(s) and/or macromolecular nucleic acid complex may serve as barcode molecules to be used to barcode nucleic acid molecules within a nucleic acid sample.

### D) Methods of Amplifying Multimeric Barcode Molecules with Secondary Rolling Circle Amplification Processes

Following any step of amplifying multimeric barcode molecules by rolling circle amplification, a process of secondary rolling circle amplification may be performed. In this process, products from the first rolling circle amplification step (or constituent parts thereof) are themselves circularised, and then used as template molecules for a second (or further) rolling circle amplification step.

For example, in one such embodiment, a library of multimeric barcode molecules are amplified in a first rolling circle amplification step. The resulting products are then converted into a double-stranded or partially double-stranded form. For example, a primer may be annealed to the said products; optionally, said primer may be complementary to or identical to all or part of one or more 'reagent amplification sequence(s)' comprised within the original multimeric barcode reagents. Optionally, following such an annealing step, a primer-extension step may be performed, wherein the 3' end of the primer is extended by at least one nucleotide by a polymerase. Optionally, such a primer extension may proceed until a full copy of the associated multimeric barcode molecule is produced, i.e. until a full double-stranded molecule is produced. Optionally, such a primer extension may be performed by a polymerase which lacks strand displacement, or 5'-3' exonuclease or flap endonuclease behaviour (such as Phusion polymerase, or T4 DNA polymerase).

The double-stranded region comprising said primer and the reagent amplification sequence to which it is annealed (along with, optionally, any primer-extension product produced by a primer extension step) may contain a recognition site for a restriction endonuclease. The resulting double-stranded or partially double-stranded products may then be digested with said restriction endonuclease, such that the molecules are linearised, and such that the ends of each molecule comprise ligation-capable restriction junctions. Optionally, the ends produced by the restriction enzyme(s) may be blunt, or may comprise a 3' overhang of 1 or more nucleotides, or may comprise a 5' overhang of 1 or more nucleotides.

The resulting digested molecules may then be converted into circular form by an intramolecular double-stranded ligation reaction with a T4 DNA Ligase enzyme, such that the restriction-digested site on one end of a molecule is ligated to the restriction-digested site on the other end of the same molecule. Optionally, before such a ligation reaction, the restriction-digested multimeric barcode molecules may be diluted in solution. Optionally, the resulting concentration of multimeric barcode molecules may be less than 100 nanomolar, less than 10 nanomolar, less than 1 nanomolar, less than 100 picomolar, less than 10 picomolar, less than 1 picomolar, less than 100 femtomolar, less than 10 femtomolar, or less than 1 femtomolar.

The resulting circularised molecules may be used for any rolling circle amplification process as described in any of the methods herein. Optionally, this overall process of performing a first rolling circle amplification process, circularising the resulting products and then performing a second rolling circle amplification process may be repeated two times, three times, four times, five times, or any larger number of times to increase the amount of products ultimately produced by the overall process. Optionally, before and/or during any rolling circle amplification step, a single-stranded DNA binding protein (such as T4 Gene 32 Protein) may be included in a reaction mixture, such as to prevent the formation of secondary structures by circularised templates and/or amplification products. During or after any such rolling circle amplification step, said single-stranded DNA binding protein may be removed and/or inactivated, such as by a heat-inactivation step.

### E) Methods of Processing Rolling-Circle-Amplified Multimeric Barcode Molecules With A Primer Extension Process

Following any process of rolling circle amplification of a multimeric barcode molecule and/or library thereof, one or more primer extension steps may be performed on the resulting products. The resulting primer-extension products may comprise single stranded nucleic acid molecules comprising all or part of multimeric barcode molecules, and or parts of two or more multimeric barcode molecules. In some embodiments, such primer-extension products may comprise a library of single stranded nucleic acid molecules, wherein each single nucleic acid strand comprises a multimeric barcode molecule. In other embodiments, such primer-extension products may be annealed or partially annealed to the template molecules from which they are synthesised. Optionally, any multimeric barcode molecules resulting from any such primer-extension process may be used to create a multimeric barcoding reagent and/or library thereof. Optionally, any multimeric barcode molecules resulting from any such primer-extension process may be used to barcode nucleic acid molecules within a nucleic acid sample; optionally the barcode sequences comprising said multimeric barcode molecules may be appended to nucleic acid molecules within a nucleic acid sample.

In one such embodiment of a primer-extension process, a primer complementary to, or identical in sequence to, all or part of a forward reagent amplification sequence and/or all or part of a reverse reagent amplification sequence may be used. In one such embodiment, a primer at least partially complementary to a reagent amplification sequence(s) comprised within the polymerase-extension products of the rolling circle amplification reaction may be used to perform one or more primer-extension reactions and/or cycles. In one embodiment of a primer-extension process, a library of random primers are used for said primer-extension process, for example random hexamer primers, random octamer primers, or random decamer primers. Optionally, any primer used in a primer-extension process may comprise one or more modifications, such as phosphorothioate bonds, and specifically such as phosphorothioate bonds within the 3' most one or two nucleotide bonds within the primer. Such 3' phosphorothioate bonds may prevent degradation of said primers by polymerases which exhibit exonuclease behaviour.

Optionally, such a primer-extension step may be performed by a polymerase that exhibits 5'-3' exonuclease behaviour (such as DNA Polymerase I from E. coli) and/or flap endonuclease behaviour (such as Taq polymerase from Thermus aquaticus), such that nucleic acid sequences annealed immediately downstream of a processing polymerase are degraded or partially degraded during the process of primer-extension by said polymerase.

Optionally, such a primer-extension step may be performed by a polymerase that exhibits strand displacement behaviour, such as phi29 DNA polymerase, Vent polymerase, Deep Vent polymerase, or exonuclease-deficient derivatives thereof (e.g. from New England Bioloabs), or Bst or Bsm DNA polymerase, such that nucleic acid sequences annealed immediately downstream of a processing polymerase are displaced during the process of primer-extension by said polymerase. Optionally, said displaced nucleic acid sequences may comprise other primer-extension products produced during the primer-extension process. Optionally, such a primer-extension step may be performed by phi29 DNA polymerase, wherein the primers used for said primer-extension step comprise random primers.

Any such primer-extension step performed by a polymerase that exhibits strand displacement behaviour may have the effect of displacing regions of multimeric barcode molecules (and/or nucleic acid strands comprising sequences from multimeric barcode molecules, e.g. those that are produced by such a primer extension process) comprising one or more adapter regions and/or adapter sequences, such that said adapter regions and/or adapter sequences are converted into a single-stranded form, such that the resulting single-stranded adapter regions are able to hybridise to complementary sequences, for example complementary sequences comprised within coupling oligonucleotides, adapter oligonucleotides, and/or extension primers. Parts of such strand-displaced molecules may remain annealed to the template molecules from which they were synthesised. Part or all of any given strand-displaced nucleic acid molecule synthesised by such a primer-extension process may be used to synthesise a multimeric barcoding reagent. Part or all of any given strand-displaced nucleic acid molecule synthesised by such a primer-extension process may be used to barcode nucleic acid molecules within a nucleic acid sample.

Optionally, such a primer-extension step may be performed by a polymerase that does not exhibit 5'-3' exonuclease, or flap endonuclease behaviour, or strand-displacement behaviour (such as Pfu and/or Phusion polymerases or derivatives thereof (New England Biolabs), or T4 DNA Polymerase), such that nucleic acid sequences annealed immediately downstream of a processing polymerase halt the extension of the polymerase when it encounters them thereat.

Optionally, any such primer-extension step may comprise at least 1, at least 5, at least 10, at least 15, at least 20, at least 30, at least 50, or at least 100 cycles of primer-extension. Optionally, such primer-extension cycles may be performed within repeating cycles of primer extension, template denaturating, and primer annealing. Optionally, any such primer-extension step may be performed in a buffer comprising one or more macromolecular crowding agents, such as poly ethylene glycol (PEG) reagents, for example PEG 8000.

Optionally, primer-extension products at least 100 nucleotides in length, at least 500 nucleotides in length, at least 1000 nucleotides in length, at least 2000 nucleotides in length, at least 5000 nucleotides in length, at least 10,000 nucleotides in length, at least 20,000 nucleotides in length, at least 50,000 nucleotides in length, or at least 100,000 nucleotides in length may be produced by any above primer extension process. Optionally, such a process of primer-extension may be performed such that at least one full copy of the circularised template is produced by the polymerase. Optionally, such a process of primer-extension may be performed such that at least 2, at least 3, at least 5, at least 10, at least 50, at least 100, at least 200, at least 500, at least 1000, at least 2000, at least 5000, or at least 10,000 copies of the multimeric barcode molecule template are produced by the polymerase during each primer extension step. Optionally, the length in time (eg seconds, or minutes) of a primer-extension reaction may be configured such that each primer-extension product is approximately the same length as a single multimeric barcode reagent within the library. For example, if a polymerase used for primer extension processes at a rate of 1000 nucleotides per minute, and the mean length of a multimeric barcode reagent within a library of multimeric barcode reagents is 1000 nucleotides, then the primer-extension cycle may be configured to be 1 minute in length.

Optionally, following one or more primer-extension steps, the resulting primer-extension products may be isolated or purified by a cleanup reaction. Optionally, such a cleanup reaction may comprise a size-selection step, such as a gel-based size-selection step or bead-based size selection step, such as a solid-phase reversible immobilisation step. Optionally, primer-extension products at least 100 nucleotides in length, at least 500 nucleotides in length, at least 1000 nucleotides in length, at least 2000 nucleotides in length, at least 5000 nucleotides in length, at least 10,000 nucleotides in length, at least 20,000 nucleotides in length, at least 50,000 nucleotides in length, or at least 100,000 nucleotides in length may be purified.

### F) Methods of Processing Rolling-Circle-Amplified And/Or Primer-Extended Multimeric Barcode Molecules With A Denaturation Process

Prior to or following any purification step and/or size selection step, and/or prior to use for synthesising multimeric barcoding reagents, and/or prior to use for barcoding nucleic acids within a sample of nucleic acids, any rolling circle amplification products or primer-extension products produced as above may be denatured with a denaturing step. Such a denaturing step may be a thermal denaturing step, wherein the products are incubated at a high temperature to melt annealed sequences and/or secondary structure. Such a denaturing step may be performed at a temperature of at least 60 degrees Celsius, at least 70 degrees Celsius, at least 80 degrees Celsius, at least 90 degrees Celsius, or at least 95 degrees Celsius. Such a denaturing step may have the effect of denaturing regions of multimeric barcode molecules comprising one or more adapter regions and/or adapter sequences into single-stranded form, such that the resulting single-stranded adapter regions are able to hybridise to complementary sequences, for example complementary sequences comprised within coupling oligonucleotides, adapter oligonucleotides, and/or extension primers.

In alternative embodiments, no such denaturing step may be performed prior to or following any purification step and/or size selection step, and/or prior to use for synthesising multimeric barcoding reagents, and/or prior to use for barcoding nucleic acids within a sample of nucleic acids. For example, nucleic acid strands comprising primer-extension products produced during a primer-extension step may remain annealed or partially annealed to the template molecules from which they were synthesised. The resulting nucleic acid macromolecules may comprise a total of at least 2 individual nucleic acid strands, at least 3 individual nucleic acid strands, at least 5 individual nucleic acid strands, at least 10 individual nucleic acid strands, at least 50 individual nucleic acid strands, at least 100 individual nucleic acid strands, at least 500 individual nucleic acid strands, at least 1000 individual nucleic acid strands, at least 5000 individual nucleic acid strands, or at least 10,000 individual nucleic acid strands. Optionally, individual nucleic acid strands may comprise all or parts of one or more multimeric barcoding molecules. Such nucleic acid macromolecules and/or libraries thereof may be used for synthesising multimeric barcoding reagents, and/or for barcoding nucleic acids within a sample of nucleic acids.

### 19. METHODS OF SYNTHESISING A MULTIMERIC BARCODING REAGENT

The invention further provides a method of synthesising a multimeric barcoding reagent for labelling a target nucleic acid comprising: (a) contacting first and second barcode molecules with first and second extension primers, wherein each of the barcode molecules comprises a single-stranded nucleic acid comprising in the 5' to 3' direction an adapter region, a barcode region and a priming region; (b) annealing the first extension primer to the priming region of the first barcode molecule and annealing the second extension primer to the priming region of the second barcode molecule; and (c) synthesising a first barcoded extension product by extending the first extension primer and synthesising a second barcoded extension product by extending the second extension primer, wherein the first barcoded extension product comprises a sequence complementary to the barcode region of the first barcode molecule and the second barcoded extension product comprises a sequence complementary to the barcode region of the second barcode molecule, and wherein the first barcoded extension product does not comprise a sequence complementary to the adapter region of the first barcode molecule and the second barcoded extension product does not comprise a sequence complementary to the adapter region of the second barcode molecule; and wherein the first and second barcode molecules are linked together.

The method may further comprise the following steps before the step of synthesising the first and second barcoded extension products: (a) contacting first and second barcode molecules with first and second blocking primers; and (b) annealing the first blocking primer to the adapter region of the first barcode molecule and annealing the second blocking primer to the adapter region of the second barcode molecule; and wherein the method further comprises the step of dissociating the blocking primers from the barcode molecules after the step of synthesising the barcoded extension products.

In the method, the extension step, or a second extension step performed after the synthesis of an extension product, may be performed, in which one or more of the four canonical deoxyribonucleotides is excluded from the extension reaction, such that the second extension step terminates at a position before the adapter region sequence, wherein the position comprises a nucleotide complementary to the excluded deoxyribonucleotide. This extension step may be performed with a polymerase lacking 3' to 5' exonuclease activity.

In any method employing one or more extension steps, any one or more ligation steps may be employed following the extension steps, wherein one or more oligonucleotides are ligated to either or both end(s) of any extension product. Optionally, two or more such ligation steps may be employed. Optionally, two or more such ligation steps may be employed in series, wherein a first ligation step is employed in which a first ligated extension product is formed, and then a second ligation step is employed in which a second oligonucleotide is ligated to the first ligated extension product, to form a second ligated extension product. Any such one or more ligation steps may optionally be performed on any adapter oligonucleotide, extension primer, barcoded oligonucleotide, and/or coupling sequence.

The barcode molecules may be provided by a single-stranded multimeric barcode molecule as defined herein.

The barcode molecules may be synthesised by any of the methods defined herein. The barcode regions may uniquely identify each of the barcode molecules. The barcode molecules may be linked on a nucleic acid molecule. The barcode molecules may be linked together in a ligation reaction. The barcode molecules may be linked together by a further step comprising attaching the barcode molecules to a solid support.

The first and second barcode molecules may be assembled as a double-stranded multimeric barcode molecule by any of the methods defined herein prior to step (a) defined above (i.e. contacting first and second barcode molecules with first and second extension primers). The double-stranded multimeric barcode molecule may be dissociated to produce single-stranded multimeric barcode molecules for use in step (a) defined above (i.e. contacting first and second barcode molecules with first and second extension primers).

The method may further comprise the steps of: (a) annealing an adapter region of a first adapter oligonucleotide to the adapter region of the first barcode molecule and annealing an adapter region of a second adapter oligonucleotide to the adapter region of the second barcode molecule, wherein the first adapter oligonucleotide further comprises a target region capable of annealing to a first sub-sequence of the target nucleic acid and the second adapter oligonucleotide further comprises a target region capable of annealing to a second sub-sequence of the target nucleic acid; and (b) ligating the 3' end of the first barcoded extension product to the 5' end of the first adapter oligonucleotide to produce a first barcoded oligonucleotide and ligating the 3' end of the second barcoded extension product to the 5' end of the second adapter oligonucleotide to produce a second barcoded oligonucleotide. Optionally, the annealing step (a) may be performed before the step of synthesising the first and second barcoded extension products and wherein the step of synthesising the first and second barcoded extension products is conducted in the presence of a ligase enzyme that performs the ligation step (b). The ligase may be a thermostable ligase. The extension and ligation reaction may proceed at over 37 degrees Celsius, over 45 degrees Celsius, or over 50 degrees Celsius.

The target regions may comprise different sequences. Each target region may comprise a sequence capable of annealing to only a single sub-sequence of a target nucleic acid within a sample of nucleic acids. Each target region may comprise one or more random, or one or more degenerate, sequences to enable the target region to anneal to more than one sub-sequence of a target nucleic acid. Each target region may comprise at least 5, at least 10, at least 15, at least 20, at least 25, at least 50 or at least 100 nucleotides. Preferably, each target region comprises at least 5 nucleotides. Each target region may comprise 5 to 100 nucleotides, 5 to 10 nucleotides, 10 to 20 nucleotides, 20 to 30 nucleotides, 30 to 50 nucleotides, 50 to 100 nucleotides, 10 to 90 nucleotides, 20 to 80 nucleotides, 30 to 70 nucleotides or 50 to 60 nucleotides. Preferably, each target region comprises 30 to 70 nucleotides. Preferably each target region comprises deoxyribonucleotides, optionally all of the nucleotides in a target region are deoxyribonucleotides. One or more of the deoxyribonucleotides may be a modified deoxyribonucleotide (e.g. a deoxyribonucleotide modified with a biotin moiety or a deoxyuracil nucleotide). Each target region may comprise one or more universal bases (e.g. inosine), one or modified nucleotides and/or one or more nucleotide analogues.

The adapter region of each adapter oligonucleotide may comprise a constant region. Optionally, all adapter regions of adapter oligonucleotides that anneal to a single multimeric barcoding reagent are substantially identical. The adapter region may comprise at least 4, at least 5, at least 6, at least 8, at least 10, at least 15, at least 20, at least 25, at least 50, at least 100, or at least 250 nucleotides. Preferably, the adapter region comprises at least 4 nucleotides. Preferably each adapter region comprises deoxyribonucleotides, optionally all of the nucleotides in an adapter region are deoxyribonucleotides. One or more of the deoxyribonucleotides may be a modified deoxyribonucleotide (e.g. a deoxyribonucleotide modified with a biotin moiety or a deoxyuracil nucleotide). Each adapter region may comprise one or more universal bases (e.g. inosine), one or modified nucleotides and/or one or more nucleotide analogues.

For any of the methods involving adapter oligonucleotides, the 3' end of the adapter oligonucleotide may include a reversible terminator moiety or a reversible terminator nucleotide (for example, a 3'-O-blocked nucleotide), for example at the 3' terminal nucleotide of the target region. When used in an extension and/or extension and ligation reaction, the 3' ends of these adapter oligonucleotides may be prevented from priming any extension events. This may minimize mis-priming or other spurious extension events during the production of barcoded oligonucleotides. Prior to using the assembled multimeric barcoding reagents, the terminator moiety of the reversible terminator may be removed by chemical or other means, thus allowing the target region to be extended along a target nucleic acid template to which it is annealed.

Similarly, for any of the methods involving adapter oligonucleotides, one or more blocking oligonucleotides complementary to one or more sequences within the target region(s) may be employed during extension and/or extension and ligation reactions. The blocking oligonucleotides may comprise a terminator and/or other moiety on their 3' and/or 5' ends such that they are not able to be extended by polymerases. The blocking oligonucleotides may be designed such that they anneal to sequences fully or partially complementary to one or more target regions, and are annealed to said target regions prior to an extension and/or extension and ligation reaction. The use of blocking primers may prevent target regions from annealing to, and potentially mis-priming along, sequences within the solution for which such annealing is not desired (for example, sequence features within barcode molecules themselves). The blocking oligonucleotides may be designed to achieve particular annealing and/or melting temperatures. Prior to using the assembled multimeric barcoding reagents, the blocking oligonucleotide(s) may then be removed by, for example, heat-denaturation and then size-selective cleanup, or other means. The removal of the blocking oligonucleotide(s) may allow the target region to be extended along a target nucleic acid template to which it is annealed.

The method may comprise synthesising a multimeric barcoding reagent comprising at least 5, at least 10, at least 20, at least 25, at least 50, at least 75 or at least 100 barcode molecules, and wherein: (a) each barcode molecule is as defined herein; and (b) a barcoded extension product is synthesised from each barcode molecule according to any method defined herein; and, optionally, (c) an adapter oligonucleotide is ligated to each of the barcoded extension products to produce barcoded oligonucleotides according to any of the methods defined herein.

The invention further provides a method of synthesising a library of multimeric barcoding reagents, wherein the method comprises repeating the steps of any of the methods defined herein to synthesise two or more multimeric barcoding reagents. Optionally, the method comprises synthesising a library of at least 5, at least 10, at least 20, at least 25, at least 50, at least 75, at least 100, at least 250, at least 500, at least 10³, at least 10⁴, at least 10⁵, at least 10⁶, at least 10⁷, at least 10⁸, at least 10⁹ or at least 10¹⁰ multimeric barcoding reagents as defined herein. Preferably, the library comprises at least 5 multimeric barcoding reagents as defined herein. Preferably, the barcode regions of each of the multimeric barcoding reagents may be different to the barcode regions of the other multimeric barcoding reagents.

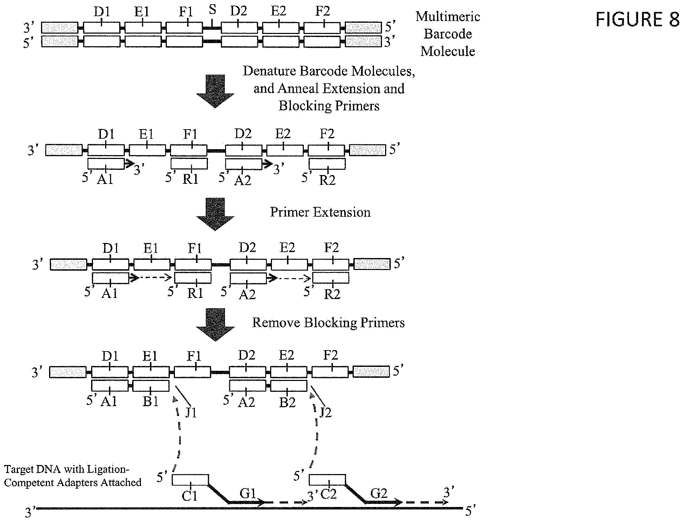

Figure 8 illustrates a method of synthesizing a multimeric barcoding reagent for labeling a target nucleic acid. In this method, first (D1, E1, and F1) and second (D2, E2, and F2) barcode molecules, which each include a nucleic acid sequence comprising a barcode region (E1 and E2), and which are linked by a connecting nucleic acid sequence (S), are denatured into single-stranded form. To these single-stranded barcode molecules, a first and second extension primer (A1 and A2) is annealed to the 3' region of the first and second barcode molecules (D1 and D2), and a first and second blocking primer (R1 and R2) is annealed to the 5' adapter region (F1 and F2) of the first and second barcode molecules. These blocking primers (R1 and R2) may be modified on the 3' end such that they cannot serve as a priming site for a polymerase.

A polymerase is then used to perform a primer extension reaction, in which the extension primers are extended to make a copy (B1 and B2) of the barcode region of the barcode molecules (E1 and E2). This primer extension reaction is performed such that the extension product terminates immediately adjacent to the blocking primer sequence, for example through use of a polymerase which lacks strand displacement or 5'-3' exonuclease activity. The blocking primers (R1 and R2) are then removed, for example through high-temperature denaturation.

This method thus creates a multimeric barcoding reagent containing a first and second ligation junction (J1 and J2) adjacent to a single-stranded adapter region (F1 and F2). This multimeric barcoding reagent may be used in the method illustrated in Figure 5.

The method may further comprise the step of ligating the 3' end of the first and second barcoded oligonucleotides created by the primer-extension step (the 3' end of B1 and B2) to first (C1 and G1) and second (C2 and G2) adapter oligonucleotides, wherein each adapter oligonucleotide comprises an adapter region (C1 and C2) which is complementary to, and thus able to anneal to, the adapter region of a barcode molecule (F1 and F2). The adapter oligonucleotides may be synthesised to include a 5'-terminal phosphate group.

Each adapter oligonucleotide may also comprise a target region (G1 and G2), which may be used to anneal the barcoded oligonucleotides to target nucleic acids, and may separately or subsequently be used as primers for a primer-extension reaction or a polymerase chain reaction. The step of ligating the first and second barcoded oligonucleotides to the adapter oligonucleotides produces a multimeric barcoding reagent as illustrated in Figure 1 that may be used in the methods illustrated in Figure 3 and/or Figure 4.

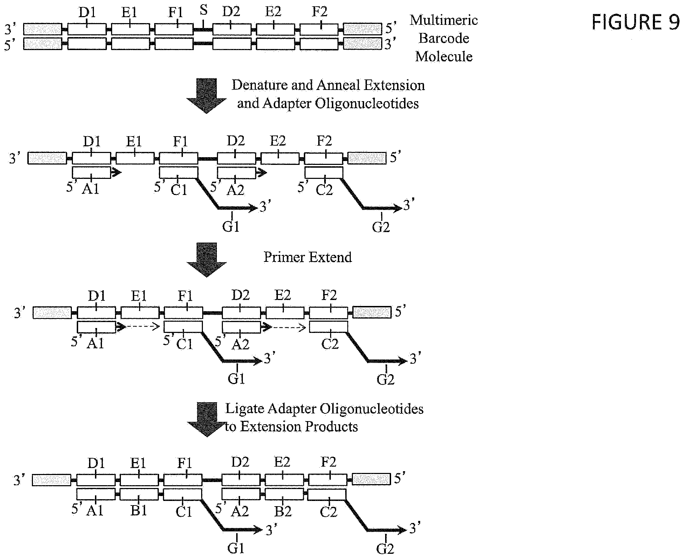

Figure 9 shows a method of synthesizing multimeric barcoding reagents (as illustrated in Figure 1) for labeling a target nucleic acid. In this method, first (D1, E1, and F1) and second (D2, E2, and F2) barcode molecules, which each include a nucleic acid sequence comprising a barcode region (E1 and E2), and which are linked by a connecting nucleic acid sequence (S), are denatured into single-stranded form. To these single-stranded barcode molecules, a first and second extension primer (A1 and A2) is annealed to the 3' region of the first and second barcode molecules (D1 and D2), and the adapter regions (C1 and C2) of first (C1 and G1) and second (C2 and G2) adapter oligonucleotides are annealed to the 5' adapter regions (F1 and F2) of the first and second barcode molecules. These adapter oligonucleotides may be synthesised to include a 5'-terminal phosphate group.

A polymerase is then used to perform a primer extension reaction, in which the extension primers are extended to make a copy (B1 and B2) of the barcode region of the barcode molecules (E1 and E2). This primer extension reaction is performed such that the extension product terminates immediately adjacent to the adapter region (C1 and C2) sequence, for example through use of a polymerase which lacks strand displacement or 5'-3' exonuclease activity.

A ligase enzyme is then used to ligate the 5' end of the adapter oligonucleotides to the adjacent 3' end of the corresponding extension product. In an alternative embodiment, a ligase enzyme may be included with the polymerase enzyme in one reaction which simultaneously effects both primer-extension and ligation of the resulting product to the adapter oligonucleotide. Through this method, the resulting barcoded oligonucleotides may subsequently be used as primers for a primer-extension reaction or a polymerase chain reaction, for example as in the method shown in Figure 3 and/or Figure 4.

The invention further provides a method of synthesising a multimeric barcoding reagent comprising appending one or more (donor) multimeric barcoding reagents to a support. Multimeric hybridization molecules (e.g. multimeric barcode molecules) may be appended to a support. Additionally or alternatively, barcoded oligonucleotides, which may have been synthesised from a multimeric barcode molecule, may be appended to a support. The support may be any support described herein e.g. a macromolecule, solid support or semi-solid support.

The support may be selected based on the desired structural and/or functional properties of the multimeric barcoding reagent. For example: barcoded oligonucleotides may be appended to magnetic beads. This may allow a laboratory scientist to easily manipulate the barcoded oligonucleotides, for example to perform washing steps, or purification steps. Furthermore, the functional properties of the bead may enable a scientist to isolate or purify nucleic acids from a nucleic acid sample that may be hybridised to and/or barcoded with the barcoded oligonucleotides. Furthermore, appending barcoded oligonucleotides to a support may change the overall structural nature of the barcoded oligonucleotides. For example, appending barcoded oligonucleotides to a streptavidin tetramer may change the three-dimensional structure of the barcoded oligonucleotides such that cross-hybridisation between the target regions of different barcoded oligonucleotides is reduced, thereby reducing the amount of potential mis-priming between barcoded oligonucleotides, and/or enhancing the accessibility of the target regions to potential target nucleic acids within a sample.

The methods are further defined in the following numbered clauses:
1) A method of synthesising a multimeric barcoding reagent comprises:
   (a) contacting a donor multimeric barcoding reagent with a support, wherein the multimeric barcoding reagent comprises: first and second hybridization molecules linked together (i.e. a multimeric hybridization molecule), wherein each of the hybridization molecules comprises a nucleic acid sequence comprising a hybridization region; and first and second barcoded oligonucleotides, wherein the first barcoded oligonucleotide is annealed to the hybridization region of the first hybridization molecule and wherein the second barcoded oligonucleotide is annealed to the hybridization region of the second hybridization molecule, and wherein the first and second barcoded oligonucleotides each comprise a barcode region, optionally wherein the first hybridization molecule and the second hybridization molecule are comprised within a (single) nucleic acid molecule; and
   (b) appending the first and second barcoded oligonucleotides to the support to form a multimeric barcoding reagent.
2) The method of clause 1, wherein the method comprises:
   (a) contacting a donor multimeric barcoding reagent with a support, wherein the multimeric barcoding reagent comprises: first and second barcode molecules linked together (i.e. a multimeric barcode molecule), wherein each of the barcode molecules comprises a nucleic acid sequence comprising a barcode region; and first and second barcoded oligonucleotides, wherein the first barcoded oligonucleotide comprises a barcode region annealed to the barcode region of the first barcode molecule and wherein the second barcoded oligonucleotide comprises a barcode region annealed to the barcode region of the second barcode molecule, optionally wherein the first barcode molecule and the second barcode molecule are comprised within a (single) nucleic acid molecule; and
   (b) appending the first and second barcoded oligonucleotides to the support to form a multimeric barcoding reagent.
3) The method of clause 1, wherein the method comprises:
   (a) synthesising the first barcoded oligonucleotide and the second barcoded oligonucleotide from a multimeric barcode molecule as template to form the donor multimeric barcoding reagent, wherein the multimeric barcode molecule comprises first and second barcode molecules linked together, wherein each of the barcode molecules comprises a nucleic acid sequence comprising a barcode region, and wherein the first barcoded oligonucleotide comprises a sequence complementary to (all or part of) the first barcode region and the second barcoded oligonucleotide comprises a sequence complementary to (all of part of) the second barcode region;
   (b) contacting the donor multimeric barcoding reagent with a support; and
   (c) appending the first and second barcoded oligonucleotides to the support to form a multimeric barcoding reagent.
4) The method of clause 3, wherein step (a) is performed by a primer-extension reaction. Optionally, the primers used for said primer-extension reaction may comprise one or more degenerate nucleotides. Optionally, the primers used for said primer-extension reaction may comprise random primers, such as random hexamers, random octamers, or random decamers. Optionally, the primers used for said primer-extension reaction may comprise one or more universal bases, such as an inosine base.
5) The method of clause 4, wherein a priming region is located at the 3' end of each barcode region, wherein step (a) is performed by a primer-extension reaction, and wherein an extension primer at least partially complementary to said priming region is annealed to each priming region, and then extended in a primer-extension reaction by a polymerase.
6) The method of clause 4 or clause 5, wherein an appending moiety is comprised within or attached to an extension primer. Optionally, said appending moiety may be comprised within or attached to the extension primer prior to a primer-extension step. Optionally, said appending moiety may be attached to the extension primer during or following a primer-extension step. Optionally, said appending moiety may be attached to the extension primer following a primer-extension step, wherein said appending moiety is comprised within or attached to an oligonucleotide, and said oligonucleotide is ligated to the 5' end of said extension primer after said primer-extension step.
7) The method of any preceding clause, wherein an appending moiety is comprised within or attached to the barcoded oligonucleotides. Optionally, said appending moiety may be attached to the barcoded oligonucleotides during a process of primer-extension which synthesises said barcoded oligonucleotides. Optionally, said appending moiety may be attached to the barcoded oligonucleotides during a process of primer-extension which synthesises said barcoded oligonucleotides, wherein an appending moiety is comprised within a modified deoxynucleotide triphosphate, which is incorporated into the barcoded oligonucleotide during its synthesis by a primer-extension process. Optionally, the appending moiety comprised within the said modified deoxynucleotide triphosphate is a biotin moiety.
8) The method of any preceding clause, wherein the first barcoded oligonucleotide and the second barcoded oligonucleotide are at least partially annealed to the multimeric hybridization molecule or multimeric barcode molecule until they are appended to the support.
9) The method of any preceding clause, wherein the first and second barcode molecules of the multimeric barcode molecule each further comprise an adapter region, and wherein the first barcoded oligonucleotide further comprises an adapter region complementary and annealed to the adapter region of the first barcode molecule and the second barcoded oligonucleotide further comprises an adapter region complementary and annealed to the adapter region of the second barcode molecule.
10) The method of any preceding clause, wherein the donor multimeric barcoding reagent comprises: first and second barcode molecules linked together (i.e. a multimeric barcode molecule), wherein each of the barcode molecules comprises a nucleic acid sequence comprising a barcode region; and first and second adapter oligonucleotides, wherein the first adapter oligonucleotide comprises an adapter region annealed to an adapter region of the first barcode molecule, and wherein the second adapter oligonucleotide comprises a adapter region annealed to the adapter region of the second barcode molecule.
11) The method of any preceding clause, wherein an appending moiety is comprised within or attached to a multimeric barcode molecule, and/or an appending moiety is comprised within or attached to an adapter oligonucleotide, and/or an appending moiety is comprised within or attached to a barcoded oligonucleotide.
12) The method of any preceding clause, wherein an appending moiety comprises a hapten molecule such as a biotin moiety.
13) The method of any preceding clause, wherein an appending moiety comprises a reactive chemical group, such as a primary amine, an azide group, or an alkyne group.
14) The method of any preceding clauses, wherein an appending moiety comprises a nucleic acid sequence, such as an adapter sequence or a hybridisation sequence.
15) The method of any preceding clause, wherein an appending moiety comprises a ligation junction capable of being ligated to a nucleic acid molecule. Optionally, said ligation junction may comprise a single-stranded 3' end capable of being ligated to a 5' end of a nucleic acid molecule. Optionally, said ligation junction may comprise a single-stranded 5' end capable of being ligated to a 3' end of a nucleic acid molecule. Optionally, said ligation junction may comprise a blunt end of a double-stranded region of a DNA molecule. Optionally, said ligation junction may comprise a 5' overhanging end of a double-stranded region of a DNA molecule. Optionally, said ligation junction may comprise a 3' overhanging end of a double-stranded region of a DNA molecule. Optionally, any such overhanging end may comprise 1 nucleotide, 2 nucleotides, 3 nucleotides, at least 4 nucleotides, at least 10 nucleotides, at least 20 nucleotides, at least 50 nucleotides, or at least 100 nucleotides. Optionally, any 5' end of a nucleic acid strand within a ligation junction may comprise a phosphate group. Any ligation junction may be able to participate in a single-stranded ligation reaction. Any ligation junction may be able to participate in a double-stranded ligation reaction.
16) The method of any preceding clauses, wherein an appending moiety further comprises a linker region. Optionally, said linker region may comprise a multi-carbon spacer such as a 3-carbon spacer, a 6-carbon spacer, a 12-carbon spacer, or an 18-carbon spacer.
17) The method of any preceding clause, wherein the support comprises one or more appending sites capable of binding to the appending moiety. Optionally, such an appending site may comprise a streptavidin molecule. Optionally, such an appending site may comprise an avidin molecule. Optionally, such an appending site may comprise a reactive chemical group, such as a carboxyl group, an azide group, or an alkyne group. Optionally, such an appending site may comprise a nucleic acid sequence, such as an adapter sequence or a hybridisation sequence.
18) The method of any preceding clause, wherein the support comprises one or more appending sites capable of binding to the appending moiety, wherein such an appending site comprises a ligation junction capable of being ligated to a nucleic acid molecule. Optionally, said ligation junction may comprise a single-stranded 3' end capable of being ligated to a 5' end of a nucleic acid molecule. Optionally, said ligation junction may comprise a single-stranded 5' end capable of being ligated to a 3' end of a nucleic acid molecule. Optionally, said ligation junction may comprise a blunt end of a double-stranded region of a DNA molecule. Optionally, said ligation junction may comprise a 5' overhanging end of a double-stranded region of a DNA molecule. Optionally, said ligation junction may comprise a 3' overhanging end of a double-stranded region of a DNA molecule. Optionally, any such overhanging end may comprise 1 nucleotide, 2 nucleotides, 3 nucleotides, at least 4 nucleotides, at least 10 nucleotides, at least 20 nucleotides, at least 50 nucleotides, or at least 100 nucleotides. Optionally, any 5' end of a nucleic acid strand within a ligation junction may comprise a phosphate group. Any ligation junction may be able to participate in a single-stranded ligation reaction. Any ligation junction may be able to participate in a double-stranded ligation reaction.
19) The method of any preceding clause, wherein the support is of any of the forms described herein. The support may comprise a planar surface. The support may be a slide e.g. a glass slide. The slide may be a flow cell for sequencing. If the support is a slide, the first and second barcoded oligonucleotides may be appended to a discrete region on the slide. Optionally, the barcoded oligonucleotides of each donor multimeric barcoding reagent in a library are appended to a different discrete region on the slide to the barcoded oligonucleotides of the other donor multimeric barcoding reagents in the library (each discrete region provides a different support). The support may be a plate comprising wells, optionally wherein the first and second barcoded oligonucleotides are appended to the same well. Optionally, the barcoded oligonucleotides of each donor multimeric barcoding reagent in library are appended to a different well of the plate to the barcoded oligonucleotides of the other donor multimeric barcoding reagents in the library (each well provides a different support). The support may be a bead (e.g. a gel bead). The bead may be an agarose bead, a silica bead, a styrofoam bead, a gel bead (such as those available from 10x Genomics^{®}), an antibody conjugated bead, an oligo-dT conjugated bead, a streptavidin bead or a magnetic bead (e.g. a superparamagnetic bead). Optionally, the barcoded oligonucleotides of each donor multimeric barcoding reagent in a library are linked together on a different bead to the barcoded oligonucleotides of the other donor multimeric barcoding reagents in the library (each bead provides a different support).
20) The method of any preceding clause, wherein the support comprises a macromolecule. The macromolecule may be a synthetic polymer (e.g. a dendrimer) or a biopolymer such as a nucleic acid (e.g. DNA), a peptide, a polypeptide or a protein (e.g. a multimeric protein). The dendrimer may comprise at least 2, at least 3, at least 5, or at least 10 generations. The nucleic acid may comprise a single-stranded nucleic acid molecule (e.g. single-stranded DNA). The protein be a homomeric protein or a heteromeric protein e.g. the protein may comprise streptavidin e.g. tetrameric streptavidin.
21) The method of any preceding clause, wherein a step of appending comprises a process of binding an appending moiety to an appending site. Optionally, said binding may comprise a covalent binding event. Optionally, said binding may comprise a non-covalent binding event. Optionally, said binding step(s) may comprise a process of covalent or non-covalent chemical complexation. Optionally, said binding step(s) may comprise a process of protein-protein interaction. Optionally, said binding step(s) may comprise a process of protein-hapten interaction. Optionally, said binding step(s) may comprise a process of nucleic acid hybridisation.
22) The method of any preceding clause, wherein an appending moiety is attached to a multimeric barcode molecule, and/or a barcoded oligonucleotide, and/or an adapter oligonucleotide with one or more attachment steps. Optionally, said attachment step(s) may be performed during synthesis of said multimeric barcode molecule, and/or barcoded oligonucleotide, and/or adapter oligonucleotide. Optionally, said attachment step(s) may be performed after synthesis of said multimeric barcode molecule, and/or barcoded oligonucleotide, and/or adapter oligonucleotide. Optionally, said attachment step(s) may comprise a process of covalent or non-covalent chemical complexation. Optionally, said attachment step(s) may comprise a process of protein-protein interaction. Optionally, said attachment step(s) may comprise a process of protein-hapten interaction. Optionally, said attachment step(s) may comprise a process of nucleic acid hybridisation.
23) Any method as in any clause above wherein an appending moiety is attached to a multimeric barcode molecule, and/or a barcoded oligonucleotide, and/or an adapter oligonucleotide with one or more attachment steps, wherein said attachment steps comprise attachment of one or more intermediary molecules to said multimeric barcode molecule, and/or barcoded oligonucleotide, and/or adapter oligonucleotide, and attachment of an appending moiety to said one or more intermediary molecules. Optionally, any said attachment step(s) may comprise a process of covalent or non-covalent chemical complexation. Optionally, any said attachment step(s) may comprise a process of protein-protein interaction. Optionally, any said attachment step(s) may comprise a process of protein-hapten interaction. Optionally, any said attachment step(s) may comprise a process of nucleic acid hybridisation.
24) The method of any preceding clause, wherein method further comprises separating the multimeric hybridization molecule or multimeric barcode molecule from the first and second barcoded oligonucleotides. Optionally, this separation step is performed after the first and second barcoded oligonucleotides have been appended to the support. Optionally, this separation step is performed with a thermal denaturation step, wherein the regions of hybridisation between the multimeric hybridization molecule or multimeric barcode molecule and the first and second barcoded oligonucleotides are denatured. Optionally, this thermal denaturation step may take place at a temperature of at least 40 degrees Celsius, at least 50 degrees Celsius, at least 60 degrees Celsius, at least 70 degrees Celsius, at least 80 degrees Celsius, at least 90 degrees Celsius, or at least 95 degrees Celsius. Optionally, this thermal denaturation step may take place within a solution comprising a nucleic acid denaturant, such as dimethyl sulfoxide, or betaine.
25) The method of any preceding clause, wherein each barcode region is adjacent in sequence to one or more adapter regions. Optionally, each barcode region is adjacent in sequence to an adapter region at its 5' end. Optionally, each barcode region is adjacent in sequence to an adapter region at its 3' end. Optionally, each barcode region is adjacent in sequence to an adapter region at both its 3' and 5' ends.
26) The method of any preceding clause, wherein a multimeric hybridization molecule or multimeric barcode molecule consists of, or consists essentially of, a single-stranded sequence, a double-stranded sequence, or a partially single-stranded and partially double-stranded sequence. Optionally, a multimeric hybridization molecule or multimeric barcode molecule may comprise a phosphorylated 5' end capable of ligating to the 3' end of nucleic acid molecules.
27) A method of synthesising a library of multimeric barcoding reagents, wherein the method comprises:
   (a) contacting two or more donor multimeric barcoding reagents with two or more supports; and
   (b) appending the first and second barcoded oligonucleotides of the first donor multimeric barcoding reagent to a first support to form a first multimeric barcoding reagent and appending the first and second barcoded oligonucleotides of the second donor multimeric barcoding reagent to a second support to form a second multimeric barcoding reagent.
28) The method of clause 27, wherein the method comprising synthesising a library of at least 5, at least 10, at least 20, at least 25, at least 50, at least 75, at least 100, at least 250, at least 500, at least 10³, at least 10⁴, at least 10⁵, at least 10⁶, at least 10⁷, at least 10⁸ or at least 10⁹ different multimeric barcoding reagents.

### 20. METHODS OF SPLITTING A MULTIMERIC BARCODING REAGENT

The invention provides a method of splitting a (parent) multimeric barcoding reagent as described herein (e.g. a multimeric barcode molecule) into two or more nucleic acid fragments. The fragments may be separated from one another e.g. free to diffuse away from each other in solution. Alternatively, the fragments may remain linked together in any of the forms described herein but are not comprised within a single nucleic acid molecule. For example, a parent multimeric barcoding reagent may be nicked and the two resulting fragments of the multimeric barcoding reagente remain linked together. For example, by being held together by a nucleic acid strand hybridized to both fragments, with the hybridised sequences flanking the nick site.

The fragments produced by the methods of splitting described herein may be particularly useful for use in preparing nucleic acid samples for sequencing where the nucleic acid sample is a single-cell sample or a FFPE sample.

The methods may include splitting large 'parent' multimeric barcoding reagents (e.g. multimeric barcode molecules) into smaller individual multimeric barcoding reagents that are more optimally sized for particular barcoding tasks, samples, or applications. For example, a library of fragments of multimeric barcoding reagents may be prepared in which the fragments are more optimally sized to diffuse into a solution of target nucleic acids within a sample e.g. fragments approximately 1,000 nucleotides in length may be prepared for use in preparing an FFPE sample for sequencing.

Importantly, these methods allow a single, parent library of multimeric barcoding reagents to be used for a variety of different barcoding applications, including applications that most optimally use a library of large multimeric barcoding reagents (in which case the unmodified, parent library may be employed), and applications that most optimally use a library of smaller multimeric barcoding reagents (in which case fragments derived from splitting the parent library, may be employed).

The methods of splitting a (parent) multimeric barcoding reagent may be further defined by the following numbered clauses:
1) A method of splitting a multimeric barcoding reagent (e.g. a multimeric barcode molecule) as described herein (or library of multimeric barcoding reagents as described herein) comprising dividing the multimeric barcoding reagent(s), wherein first and second fragments are produced for each (parent) multimeric barcoding reagent, and wherein each first fragment comprises a first barcode region and each second fragment comprises a second barcode region.
2) The method of clause 1, wherein the first and second barcode regions of each (parent) multimeric barcoding reagent are comprised within a nucleic acid molecule, and wherein a recognition sequence for a restriction endonuclease is comprised within the nucleic acid molecule, and wherein the multimeric barcoding reagent is divided by a process comprising cleavage by the restriction endonuclease
3) The method of clause 2, wherein the recognition sequence for the restriction endonuclease is comprised within a cleavage adapter region comprised within the multimeric barcoding reagent, and wherein a cleavage adapter oligonucleotide at least partially complementary to said cleavage adapter region is annealed to said cleavage adapter region to generate a double-stranded cleavage site which is cleaved by the restriction endonuclease.
4) The method of clause 2, wherein the recognition sequence for the restriction endonuclease is comprised within an adapter region comprised within a multimeric barcode molecule (of the multimeric barcoding reagent), and wherein the adapter region of an adapter oligonucleotide is annealed to the adapter region of the multimeric barcode molecule to generate a double-stranded cleavage site which is cleaved by the restriction endonuclease.
5) The method of clause 2, wherein the recognition sequence for the restriction endonuclease is comprised within an adapter region comprised within a multimeric barcode molecule (of the multimeric barcoding reagent), and wherein the adapter region of a barcoded oligonucleotide is annealed to the adapter region of the multimeric barcode molecule to generate a double-stranded cleavage site which is cleaved by the restriction endonuclease.
6) The method of any one of clauses 2-5, wherein said cleavage adapter oligonucleotide(s), and/or adapter oligonucleotide(s), and/or barcoded oligonucleotide(s) comprise one or more phosphorothioate bond(s). Optionally, one such phosphorothioate bond may be located at the bond cleaved by the restriction endonuclease. Optionally, one or more such phosphorothioate bonds may be located at one of the bonds that are adjacent to the bond cleaved by the restriction endonuclease. Optionally, any such one or more phosphorothioate bonds may prevent the restriction endonuclease from cleaving said cleavage adapter oligonucleotide(s), and/or said adapter oligonucleotide(s), and/or said barcoded oligonucleotide(s); optionally, any such one or more phosphorothioate bonds may not prevent the restriction endonuclease from cleaving the multimeric barcoding reagent. Optionally, 2'-O-methyl nucleotide bond(s), and/or any other modified nucleotide bond, may be used in any method as above in place of phosphorothioate bond(s).
7) The method of any one of clauses 2-6, wherein the restriction endonuclease is EcoRV, Pstl, Spel, Sphl, Xbal or Xhol.
8) The method of any one of clauses 2-7, wherein the restriction endonuclease is a nicking endonuclease.
9) The method of any one of clauses 2-8, wherein the cleavage adapter oligonucleotide(s), and/or adapter oligonucleotide(s), and/or barcoded oligonucleotide(s) comprise a region of at least 5, at least 10, at least 15, at least 20, at least 25, at least 30, or at least 50 nucleotides that are complementary to a multimeric barcoding reagent. Optionally, the said oligonucleotide(s) comprise a region of at least 5, at least 10, at least 15, at least 20, at least 25, or at least 30 nucleotides that are complementary to a multimeric barcoding reagent, wherein said region is located within the 5' region of the cleavage adapter oligonucleotide relative to the bond within the cleavage adapter oligonucleotide that would be cleaved by the restriction endonuclease. Optionally, the said oligonucleotide(s) comprise a region of at least 5, at least 10, at least 15, at least 20, at least 25, or at least 30 nucleotides that are complementary to a multimeric barcoding reagent, wherein said region is located within the 3' region of said oligonucleotide(s) relative to the bond within said oligonucleotide(s) that would be cleaved by the restriction endonuclease.
10) The method of any one of clauses 2-9, wherein the first and second fragments remain annealed to the cleavage adapter oligonucleotide(s), and/or adapter oligonucleotide(s), and/or barcoded oligonucleotide(s) following digestion with the restriction endonuclease.
11) The method of any one of clauses 2-10, wherein the first and second fragments remain annealed to the cleavage adapter oligonucleotide(s), and/or adapter oligonucleotide(s), and/or barcoded oligonucleotide(s) following digestion with a restriction endonuclease, wherein the step of digestion with a restriction endonuclease cleaves the multimeric barcoding reagent but does not cleave the cleavage adapter oligonucleotide(s) or adapter oligonucleotide(s) or barcoded oligonucleotide(s).
12) The method of any preceding clause, wherein the multimeric barcoding reagent is comprised of at least a first barcode region and a second barcode region comprised within a nucleic acid molecule, and wherein said nucleic acid molecule comprises at least one uracil nucleotide, and wherein said dividing step comprises excising at least one uracil base by a uracil DNA glycosylase enzyme, and wherein said dividing step produces the first and second fragments.
13) The method of clause 12, wherein the uracil DNA glycosylase enzyme is uracil DNA glycosylase enzyme from E. coli.
14) The method of any preceding clause, wherein the dividing step comprises a step of acoustic shearing or a step of sonication. Optionally, such a shearing or sonication step may be performed with a sonication instrument comprising adaptive focused acoustics technology, such as a Covaris AFA sonication instrument. Optionally, such a shearing or sonication step may be used to generate fragments of approximately 200 nucleotides in length, approximately 500 nucleotides in length, approximately 1000 nucleotides in length, approximately 2000 nucleotides in length, or approximately 5000 nucleotides in length.
15) The method of any preceding clause, wherein the dividing step comprises digestion with a deoxyribonuclease enzyme. Optionally, said deoxyribonuclease enzyme may exhibit single-stranded deoxyribonuclease behaviour. Optionally, said deoxyribonuclease enzyme may exhibit double-stranded deoxyribonuclease behaviour. Optionally, said deoxyribonuclease enzyme may be recombinant bovine pancreas DNAse I.
16) The method of any preceding clause, wherein prior to or after a dividing step, the multimeric barcoding reagents or fragments thereof (e.g. multimeric barcode molecules, barcode regions within multimeric barcode molecules, and/or oligonucleotides (such as adapter oligonucleotides and/or barcoded oligonucleotides)) are appended to a support. Optionally, such a support may comprise a solid support, such as a paramagnetic bead or styrofoam bead. Optionally, such multimeric barcoding reagents or fragments thereof may comprise one or more biotin moieties, and said supports may each comprise one or more streptavidin moieties, and the step of appending to a support comprises binding said biotin moieties to said streptavidin moieties.
17) The method of any preceding clause, wherein each barcode region is adjacent in sequence to one or more adapter regions. Optionally, each barcode region is adjacent in sequence to an adapter region at its 5' end. Optionally, each barcode region is adjacent in sequence to an adapter region at its 3' end. Optionally, each barcode region is adjacent in sequence to an adapter region at both its 3' and 5' ends.
18) The method of any preceding clause, wherein the multimeric barcoding reagent comprises an entirely single-stranded sequence, or an entirely double-stranded sequence, or a partially single-stranded and partially double-stranded sequence. Optionally, the multimeric barcoding reagent may comprise a phosphorylated 5' end capable of ligating to the 3' end of nucleic acid molecules.
19) The method of any preceding clause, wherein the multimeric barcoding reagent comprise at least 2, at least 3, at least 4, at least 5, at least 10, at least 20, at least 25, at least 50, at least 75, at least 100, at least 250, at least 500, at least 10³, at least 10⁴, at least 10⁵ or at least 10⁶ barcode regions or barcode molecules.
20) The method of any preceding clause, wherein the library of multimeric barcoding reagents comprises at least 5, at least 10, at least 20, at least 25, at least 50, at least 75, at least 100, at least 250, at least 500, at least 10³, at least 10⁴, at least 10⁵, at least 10⁶, at least 10⁷, at least 10⁸ or at least 10⁹ different multimeric barcoding reagents.
21) The method of any preceding clause, wherein one of more of the fragments comprise at least 2, at least 3, at least 4, at least 5, at least 10, at least 20, at least 25, at least 50, at least 75, at least 100, at least 250, at least 500, at least 10³, at least 10⁴, at least 10⁵ or at least 10⁶ barcode regions or barcode molecules.
22) The method of any preceding clause, a multimeric barcoding reagent is divided into at least 2, at least 3, at least 4, at least 5, at least 10, at least 50, at least 100, at least 1000, at least 10,000, at least 50,000, or at least 100,000 fragments.

The invention further provides a library of multimeric barcoding reagent (e.g. multimeric barcode molecule) fragments as described herein and a kit comprising a library of multimeric barcoding reagent fragments as described herein. The library or kit may be obtained by any of the methods described herein. Optionally, said library or kit may comprise at least 2, at least 3, at least 4, at least 5, at least 10, at least 20, at least 25, at least 50, at least 75, at least 100, at least 250, at least 500, at least 10³, at least 10⁴, at least 10⁵ or at least 10⁶ multimeric barcoding reagent fragments.

### 21. METHODS OF SEQUENCING AND/OR PROCESSING SEQUENCING DATA

The invention further provides a method of sequencing a sample, wherein the sample has been prepared by any one of the methods of preparing a nucleic acid sample for sequencing as defined herein. The method of sequencing the sample comprises the steps of: isolating the barcoded target nucleic acid molecules, and producing a sequence read from each barcoded target nucleic acid molecule that comprises the barcode region, the target region and at least one additional nucleotide from the target nucleic acid. Each sequence read may comprise at least 5, at least 10, at least 25, at least 50, at least 100, at least 250, at least 500, at least 1000, at least 2000, at least 5000, or at least 10,000 nucleotides from the target nucleic acid. Preferably, each sequence read comprises at least 5 nucleotides from the target nucleic acid.

The methods may produce a sequence read from one or more barcoded target nucleic acid molecule produced from at least at least 10, at least 100, or at least 10³, at least 10⁴, at least 10⁵, at least 10⁶, at least 10⁷, at least 10⁸ or at least 10⁹ different target nucleic acids.

Sequencing may be performed by any method known in the art. For example, by chain-termination or Sanger sequencing. Preferably, sequencing is performed by a next-generation sequencing method such as sequencing by synthesis, sequencing by synthesis using reversible terminators (e.g. Illumina sequencing), pyrosequencing (e.g. 454 sequencing), sequencing by ligation (e.g. SOLiD sequencing), single-molecule sequencing (e.g. Single Molecule, Real-Time (SMRT) sequencing, Pacific Biosciences), or by nanopore sequencing (e.g. on the Minion or Promethion platforms, Oxford Nanopore Technologies).

The invention further provides a method for processing sequencing data obtained by any of the methods defined herein. The method for processing sequence data comprises the steps of: (a) identifying for each sequence read the sequence of the barcode region and the sequence from the target nucleic acid; and (b) using the information from step (a) to determine a group of sequences from the target nucleic acid that were labelled with barcode regions from the same multimeric barcoding reagent.

The method may further comprise the step of determining a sequence of a target nucleic acid by analysing the group of sequences to identify contiguous sequences, wherein the sequence of the target nucleic acid comprises nucleotides from at least two sequence reads.

The target nucleic acid may be an intact nucleic acid molecule, co-localised fragments of a nucleic acid molecule, or nucleic acid molecules from a single cell. Preferably, the target nucleic acid is a single intact nucleic acid molecule, two or more co-localised fragments of a single nucleic acid molecule, or two or more nucleic acid molecules from a single cell.

The invention further provides an algorithm for processing (or analysing) sequencing data obtained by any of the methods defined herein. The algorithm may be configured to perform any of the methods for processing sequencing data defined herein. The algorithm may be used to detect the sequence of a barcode region within each sequence read, and also to detect the sequence within a sequence read that is derived from a target nucleic acid, and to separate these into two associated data sets.

The invention further provides a method of generating a synthetic long read from a target nucleic acid comprising the steps of: (a) preparing a nucleic acid sample for sequencing according to any of the methods defined herein; (b) sequencing the sample, optionally wherein the sample is sequenced by any of the methods defined herein; and (c) processing the sequence data obtained by step (b), optionally wherein the sequence data is processed according to any of the methods defined herein; wherein step (c) generates a synthetic long read comprising at least one nucleotide from each of the at least two sequence reads.

The method may enable the phasing of a target sequence of a target nucleic acid molecule i.e. it may enable the determination of which copy of a chromosome (i.e. paternal or maternal) the sequence is located. The target sequence may comprise a specific target mutation, translocation, deletion or amplification and the method may be used to assign the mutation, translocation, deletion or amplification to a specific chromosome. The phasing two or more target sequences may also enable the detection of aneuploidy.

The synthetic long read may comprise at least 50, at least 100, at least 250, at least 500, at least 750, at least 1000, at least 2000, at least 10⁴, at least 10⁵, at least 10⁶, at least 10⁷ or at least 10⁸ nucleotides. Preferably, the synthetic long read comprises at least 50 nucleotides.

The invention further provides a method of sequencing two or more co-localised target nucleic acids comprising the steps of: (a) preparing a nucleic acid sample for sequencing according to any of the methods defined herein; (b) sequencing the sample, optionally wherein the sample is sequenced by any of the methods defined herein; and (c) processing the sequence data obtained by step (b), optionally wherein the sequence data is processed according to any of the methods defined herein; wherein step (c) identifies at least two sequence reads comprising nucleotides from at least two target nucleic acids co-localised in the sample.

The invention further provides a method of sequencing target nucleic acids from an individual cell comprising the steps of: (a) preparing a nucleic acid sample for sequencing according any of the methods defined herein, wherein the multimeric barcoding reagent(s), or multimeric barcode molecule(s), and/or adapter oligonucleotides are introduced into the cell; (b) sequencing the sample, optionally wherein the sample is sequenced by any of the methods defined herein; and (c) processing the sequence data obtained by step (b), optionally wherein the sequence data is processed according to any of the methods defined herein; wherein step (c) identifies at least two sequence reads comprising nucleotides from at least two target nucleic acids from the cell.

The multimeric barcoding reagent(s) and/or adapter oligonucleotides may be introduced into the cell by chemical complexation with a lipid transfection reagent and then transfection into the cell.

The multimeric barcoding reagent(s) and/or adapter oligonucleotides may be introduced into the cell through the steps of: (a) permeabilising the cell membrane by contacting it with a chemical surfactant; and then (b) contacting the cell with the multimeric barcoding reagent(s) and/or adapter oligonucleotides. The chemical surfactant may be a non-ionic surfactant. The chemical surfactant may be Triton X-100 (C₁₄H₂₂O(C₂H₄O)*ₙ*(n=9-10)). The chemical surfactant may be in solution at a concentration of less than 200 micromolar, or less than 500 micromolar, or less than 1 milimolar.

In the method, following the step of introducing the multimeric barcoding reagent(s) and/or adapter oligonucleotides into the cell, the cell may be incubated for a period of time to allow the target regions of the multimeric barcoding reagent(s) or adapter oligonucleotide(s) to anneal to sub-sequences of the target nucleic acids within the cell. The incubation period may be at least 1 minute, or at least 5 minutes, or at least 15 minutes, or at least 30 minutes, or at least 60 minutes. Preferably, the incubation period is at least 1 minute. The incubation may take place within a solution containing a nucleic acid denaturant e.g. dimethyl sulfoxide (DMSO) or betaine. The incubation may take place at a temperature of at least 20 degrees Celsius, at least 37 degrees Celsius, at least 45 degrees Celsius, or at least 50 degrees Celsius. Preferably, the incubation takes place at a temperature of at least 20 degrees Celsius.

In methods involving the use of multimeric barcoding reagents, the incubation step may substantially dissociate the barcoded oligonucleotides from the barcode molecules (or multimeric barcode molecule). This may enable the barcoded oligonucleotides to diffuse more readily throughout the cell improving the efficiency with which the target regions of the barcoded oligonucleotides are able to anneal to sub-sequences of the target nucleic acids.

In the method, following introduction of the multimeric barcoding reagent(s) and/or adapter oligonucleotides into the cell, and optionally following the incubation step, the cell may be contacted by a solution of oligonucleotides complementary to the target regions of the multimeric barcoding reagents.

In the method, following introduction of the multimeric barcoding reagent(s) and/or adapter oligonucleotides into the cell, and optionally following the incubation step, the cell may be isolated from a reaction mixture e.g. by centrifugation.

In the method, following introduction of the multimeric barcoding reagent(s) and/or adapter oligonucleotides into the cell, and optionally following the incubation step, the barcoded oligonucleotides and/or barcoded target nucleic acid molecules and/or multimeric barcoding reagent(s) may be isolated from the cell.

The multimeric barcoding reagents, barcoded oligonucleotides and/or adapter oligonucleotides may comprise one or more biotin moieties.

In the method, following introduction of the multimeric barcoding reagent(s) and/or adapter oligonucleotides into the cell, and optionally following the incubation step, the barcoded oligonucleotides and/or barcoded target nucleic acid molecules and/or multimeric barcoding reagent(s) may be isolated by a process of: (a) optionally dissolving the cell membranes e.g. using a chemical surfactant or by incubation at high temperature; (b) contacting the resulting mixture with a solid support, optionally wherein the solid support comprises streptavidin moieties; and (c) capturing the barcoded oligonucleotides and/or barcoded target nucleic acid molecules and/or multimeric barcoding reagent(s) on the solid support, optionally through streptavidin-biotin interaction. The solid support may be one or more magnetic beads, optionally wherein the one or more magnetic beads comprise streptavidin molecules on their surface. The magnetic bead(s) may be isolated from a reaction mixture with a magnet.

The target nucleic acids may be DNA molecules (e.g. genomic DNA molecules) or RNA molecules (e.g. mRNA molecules).

Preferably, each barcoded target nucleic acid molecule is produced after isolation of the barcoded oligonucleotide annealed to a target mRNA molecule by extending the barcoded oligonucleotide using a reverse transcriptase and the target mRNA molecule as the template.

The mRNA molecules may be mRNA molecules corresponding to alpha and/or beta chains of a T-cell receptor sequence, optionally wherein the sequences of alpha and beta chains paired within an individual cell are determined.

The mRNA molecules may be mRNA molecules corresponding to light and/or heavy chains of an immunoglobulin sequence, optionally wherein the sequences of light and heavy chains paired within an individual cell are determined.

The method may be used to sequence target nucleic acids in at least 10, at least 100, or at least 10³, at least 10⁴, at least 10⁵, at least 10⁶, at least 10⁷, at least 10⁸ or at least 10⁹ cells. Preferably, the method may be used to sequence target nucleic acids in at least 10 cells. Preferably the cells are T-cells and/or B-cells.

Any method of analysing barcoded nucleic acid molecules by sequencing (e.g. to generate synthetic long reads, or to analyse nucleic acid sequences from single cells) may comprise a redundant sequencing reaction, wherein target nucleic acid molecules that have been barcoded in a barcoding reaction are sequenced two or more times within a sequencing reaction. Optionally, each such barcoded molecule from a sample may be sequenced, on average, at least twice, at least 3 times, at least 5 times, at least 10 times, at least 20 times, at least 50 times, or at least 100 times.

In any method of analysing barcoded nucleic acid molecules by sequencing (e.g. to generate synthetic long reads, or to analyse nucleic acid sequences from single cells), an error correction process may be employed. This process may comprise the steps of: (i) determining two or more sequence reads from a sequencing dataset comprising the same barcode sequence, and (ii) aligning the sequences from said two or more sequence reads to each other. Optionally, this error correction process may further comprise a step of (iii) determining a majority and/or most common and/or most likely nucleotide at each position within the sequence read and/or at each position within the sequence of the target nucleic acid molecule. This step may optionally comprise establishing a consensus sequence of each target nucleic acid sequence by any process of error correction, error removal, error detection, error counting, or statistical error removal. This step may further comprise the step of collapsing multiple sequence reads comprising the same barcode sequence into a representation comprising a single, error-corrected read. Optionally, any step of determining two or more sequence reads from a sequencing dataset comprising the same barcode sequence, may comprise determining sequence reads comprising barcode sequences with at least a certain extent of identical nucleotides and/or sequence similarity, for example at least 70%, at least 80%, at least 90%, or at least 95% sequence similarity (for example, allowing for mismatches and/or insertions or deletions at any point between to barcode sequences).

In any method of analysing barcoded nucleic acid molecules by sequencing (e.g. to generate synthetic long reads, or to analyse nucleic acid sequences from single cells), an alternative error correction process may be employed, comprising the steps of: (i) determining two or more sequence reads from a sequencing dataset that comprise the same target nucleic acid sequence, wherein said two or more sequence reads further comprise two or more different barcode sequences, wherein the barcode sequences are from the same multimeric barcode molecule and/or multimeric barcoding reagent, and (ii) aligning the sequences from said two or more sequence reads to each other. Optionally, this error correction process may further comprise a step of (iii) determining a majority and/or most common and/or most likely nucleotide at each position within the sequence of the target nucleic acid molecule. This step may optionally comprise establishing a consensus sequence of the target nucleic acid molecule by any process of error correction, error removal, error detection, error counting, or statistical error removal. This step may further comprise the step of collapsing multiple sequence reads comprising the same target nucleic acid molecule into a representation comprising a single, error-corrected read. The target nucleic acid molecule may comprise, for example, a genomic DNA sequence; alternatively, the target nucleic acid molecule may comprise all or part of a messenger RNA sequence such as an expressed gene or an expressed adaptive immune receptor chain. Optionally, any step of comparing two barcode sequences, and/or comparing a sequenced barcode sequence and a reference barcode sequence, may comprise determining sequences comprising at least a certain extent of identical nucleotides and/or sequence similarity, for example at least 70%, at least 80%, at least 90%, or at least 95% sequence similarity (for example, allowing for mismatches and/or insertions or deletions at any point between to barcode sequences).

### 22. USES OF A MULTIMERIC BARCODING REAGENT, LIBRARY OR KIT

The invention further provides the use of a multimeric barcoding reagent as defined herein, a library of multimeric barcoding reagents as defined herein, or a kit as defined herein, to produce two or more sequence reads from a target nucleic acid, wherein two or more sequence reads can be identified as derived from the same target nucleic acid and combined to produce a synthetic long read.

The invention further provides the use of a multimeric barcoding reagent as defined herein, a library of multimeric barcoding reagents as defined herein, or a kit as defined herein, to label a formalin-fixed paraffin-embedded (FFPE) nucleic acid sample, wherein the multimeric barcoding reagent or the components of the kit is/are introduced into the sample and used to label a set of two or more co-localised target nucleic acids for sequencing.

The multimeric barcoding reagents for use in labelling a FFPE nucleic acid sample may be less than 10kb, less than 5kb, less than 2kb, less than 1kb in length or less than 500bp. Preferably, the multimeric barcoding reagents are less than 1kb in length.

The invention further provides the use of a multimeric barcoding reagent as defined herein, a library of multimeric barcoding reagents as defined herein, or a kit as defined herein, to label target nucleic acids in an individual cell, wherein the multimeric barcoding reagent or the components of the kit is/are introduced into a cell and used to label a set of two or more target nucleic acids in the cell for sequencing.

The invention further provides the use of a multimeric barcoding reagent as defined herein, a library of multimeric barcoding reagents as defined herein, or a kit as defined herein, to label target nucleic acids in a sample of human plasma or serum, wherein the multimeric barcoding reagent or the components of the kit is/are used to label a set of two or more target nucleic acids in the plasma or serum for sequencing.

### 23. METHODS FOR PROFILING A MULTIMERIC BARCODING REAGENT OR A LIBRARY OF MULTIMERIC BARCODING REAGENTS

The invention further provides a method for profiling a multimeric barcoding reagent comprising the steps of: (a) preparing a nucleic acid sample for sequencing, optionally wherein the nucleic acid sample is prepared for sequencing according to one of the methods defined herein, wherein the sample comprises a target nucleic acid of known sequence; (b) sequencing the sample, optionally wherein the sample is sequenced by any of the methods defined herein; (c) processing the sequence data obtained by step (b), wherein the processing comprises identifying sequence reads comprising a sequence from the target nucleic acid of known sequence, identifying within these sequence reads the sequence of a barcode region, and determining the sequences of two or more barcode regions of the multimeric barcoding reagent.

The invention further provides a method for profiling a library of two or more multimeric barcoding reagents comprising the steps of: (a) preparing a nucleic acid sample for sequencing, optionally wherein the nucleic acid sample is prepared for sequencing by any one of the methods defined herein, wherein the sample comprises a first target nucleic acid of known sequence and a second target nucleic acid of known sequence; (b) sequencing the sample, optionally wherein the sample is sequenced by any of the methods defined herein; (c) processing the sequence data obtained by step (b), wherein the processing comprises (i) identifying sequence reads comprising a sequence from the first target nucleic acid of known sequence, identifying within these sequence reads the sequence of a barcode region, and determining the sequences of two or more barcode regions of the first multimeric barcoding reagent; and (ii) identifying sequence reads comprising a sequence from the second target nucleic acid of known sequence, identifying within these sequence reads the sequence of a barcode region, and determining the sequences of two or more barcode regions of the second multimeric barcoding reagent.

The methods of profiling may be used to determine which barcodes are present within each individual multimeric barcoding reagent within a solution of many such reagents. This would be useful where it is not known a priori which barcodes have been included in each barcoding reagent.

Target nucleic acids of known sequence may be prepared by synthesising a library of short (for example approximately 40-100 nucleotide) oligonucleotides; each oligonucleotide may comprise two constant (unvariable) regions at each end, flanking a central variable region comprising a stretch of randomised nucleotides (for example, 10 nucleotides in a sequence which could each be any one of the four canonical nucleotides).

Preferably, the number of distinct variable sequences present in this library may be configured to be substantially larger than the number of distinct barcodes present in the coding strand library to be profiled.

The oligonucleotides may be circularised, for example using a single-stranded ligase, or by first creating a double-stranded molecule using a primer-extension reaction from the 3' end of the molecule and then employing a double-stranded ligase for intramolecular circularisation. Following circularisation, double-stranded molecules may be denatured to convert them into single-stranded form.

A single primer may then bound to one of the constant regions of each of these circularised molecules and used to prime a strand-displacement amplification reaction using a processive polymerase with a strand-displacing activity (e.g. phi29 polymerase). This process may create a large number of tandem, linear copies of each individual circularised molecule, each comprised as a long single-stranded DNA sequence. These tandemly-repeated synthetic molecules then serve as the target nucleic acids of known sequence in the methods of the invention.

The invention further provides a method for profiling a multimeric barcode molecule comprising the steps of: (a) preparing a nucleic acid sample for sequencing, optionally wherein the nucleic acid sample is prepared for sequencing according to one of the methods defined herein, wherein the nucleic acid sample is comprised of one or more multimeric barcode molecules as defined herein (or a library of multimeric barcode molecules as defined herein); (b) sequencing the sample, optionally wherein the sample is sequenced by any of the methods defined herein; (c) processing the sequence data obtained by step (b), wherein the processing comprises identifying sequence reads and/or parts of sequence reads comprising all or part of a sequence from barcode regions within a multimeric barcode molecule; and d) determining the sequences of two or more barcode regions that are linked and/or comprised within a multimeric barcode molecule. Optionally, step (d) may determine at least 3, at least 5, at least 10, at least 20, at least 50, at least 100, or at least 500 barcode regions that are linked and/or comprised within a multimeric barcode molecule (or within each multimeric barcode molecule in a library of multimeric barcode molecules). Optionally, any one or more multimeric barcode molecule may be prepared for sequencing such that any part of any one or more multimeric barcode molecule are sequenced two or more times. On average, any part of any one or more multimeric barcode molecules may be sequenced at least 2 times, at least 3 times, at least 5 times, at least 10 times, at least 20 times, at least 30 times, at least 50 times, or at least 100 times. Optionally, an error-correction algorithm may be applied to said sequencing data. Optionally, only barcode sequences that are detected to exist in the data at least 2 times, at least 3 times, at least 5 times, or at least 10 times may be analysed in subsequent steps.

Optionally, step (d) may comprise analysis with a genome assembly and/or genome phasing algorithm. Optionally, step (d) may comprise analysis with a network analysis algorithm, wherein each barcode sequence comprises or potentially comprises a node within a network, and wherein each link or potential link between barcode sequences within a multimeric barcode molecule comprises an edge between nodes within a network. Optionally, analysis with said network analysis algorithm may comprise determining edges with at least a certain minimal and/or threshold level of strength and/or significance. Optionally, such strength and/or significance level may be determined by evaluating the number of times that a first barcode sequence (corresponding to a first node) and a second barcode sequence (corresponding to a second node) are found to be sequenced within the same sequenced molecule. Optionally, analysis with said network analysis algorithm may comprise a step of isolating networks or sub-networks with particular characteristics or within one or more ranges of characteristics, such as number of edges, number of nodes, average strength of edges, median strength of edges, networks or sub-networks wherein each edge has at least a particular minimal or threshold strength, and/or any combination of such characteristics. Optionally, nodes comprised within such networks or sub-networks may correspond to determined barcode regions within multimeric barcode molecules.

The invention is further defined in the following set of numbered clauses:
1. A method of preparing a nucleic acid sample for sequencing, wherein the nucleic acid sample comprises a formalin-fixed paraffin-embedded (FFPE) sample, and wherein the method comprises the steps of:
   (a) contacting the nucleic acid sample with a multimeric barcoding reagent comprising first and second barcode regions linked together, wherein each barcode region comprises a nucleic acid sequence, and
   (b) appending barcode sequences to each of the nucleic acid sequences of first and second fragments of a target nucleic acid molecule to produce first and second different barcoded target nucleic acid molecules, wherein the first barcoded target nucleic acid molecule comprises the nucleic acid sequence of the first barcode region and the second barcoded target nucleic acid molecule comprises the nucleic acid sequence of the second barcode region.
2. The method of clause 1, wherein the multimeric barcoding reagent comprises first and second barcode molecules linked together, and wherein each of the barcode molecules comprises a nucleic acid sequence comprising a barcode region.
3. The method of clause 1 or clause 2, wherein the multimeric barcoding reagent comprises first and second barcoded oligonucleotides, wherein the first barcoded oligonucleotide comprises, optionally in the 5' to 3' direction, a barcode region, and a target region capable of annealing or ligating to the first fragment of the target nucleic acid, and wherein the second barcoded oligonucleotide comprises, optionally in the 5' to 3' direction, a barcode region, and a target region capable of annealing or ligating to the second fragment of the target nucleic acid.
4. The method of any one of clauses 1-3, wherein the multimeric barcoding reagent comprises:
   (i) first and second barcode molecules linked together, wherein each of the barcode molecules comprises a nucleic acid sequence comprising a barcode region; and
   (ii) first and second barcoded oligonucleotides, wherein the first barcoded oligonucleotide comprises, optionally in the 5' to 3' direction, a barcode region, and a target region capable of annealing or ligating to the first fragment of the target nucleic acid, and wherein the second barcoded oligonucleotide comprises, optionally in the 5' to 3' direction, a barcode region, and a target region capable of annealing or ligating to the second fragment of the target nucleic acid.
5. The method of clause 3 or clause 4, wherein the method comprises
   (a) contacting the nucleic acid sample with a multimeric barcoding reagent as defined in clause 3 or clause 4;
   (b) ligating the target region of the first barcoded oligonucleotide to the first fragment of the target nucleic acid molecule to produce a first barcoded target nucleic acid molecule, and ligating the target region of the second barcoded oligonucleotide to the second fragment of the target nucleic acid molecule to produce a second barcoded target nucleic acid molecule, wherein each of the barcoded target nucleic acid molecules comprises at least one nucleotide synthesised from the target nucleic acid as a template.
6. The method of clause 3 or clause 4, wherein the method comprises:
   (a) contacting the nucleic acid sample with a multimeric barcoding reagent as defined in clause 3 or clause 4;
   (b) annealing the target region of the first barcoded oligonucleotide to the first fragment of the target nucleic acid, and annealing the target region of the second barcoded oligonucleotide to the second fragment of the target nucleic acid; and
   (c) extending the first and second barcoded oligonucleotides to produce first and second different barcoded target nucleic acid molecules, wherein each of the barcoded target nucleic acid molecules comprises at least one nucleotide synthesised from the target nucleic acid as a template.
7. The method of clause 1 or clause 2, wherein the method comprises:
   (a) contacting the sample with a multimeric barcoding reagent comprising first and second barcode molecules linked together, wherein each of the barcode molecules comprises a nucleic acid sequence comprising a barcode region and an adapter region;
   (b) appending a coupling sequence to each of first and second fragments of the target nucleic acid molecule;
   (c) annealing the coupling sequence of the first fragment to the adapter region of the first barcode molecule, and annealing the coupling sequence of the second fragment to the adapter region of the second barcode molecule; and
   (d) appending barcode sequences to the first and second fragments of the target nucleic acid to produce first and second different barcoded target nucleic acid molecules, wherein the first barcoded target nucleic acid molecule comprises the nucleic acid sequence of the barcode region of the first barcode molecule and the second barcoded target nucleic acid molecule comprises the nucleic acid sequence of the barcode region of the second barcode molecule.
8. The method of clause 1 or clause 7, wherein the multimeric barcoding reagent comprises:
   (i) first and second barcode molecules linked together, wherein each of the barcode molecules comprises a nucleic acid sequence comprising, optionally in the 5' to 3' direction, an adapter region and a barcode region, and
   (ii) first and second barcoded oligonucleotides, wherein the first barcoded oligonucleotide comprises a barcode region annealed to the barcode region of the first barcode molecule, and wherein the second barcoded oligonucleotide comprises a barcode region annealed to the barcode region of the second barcode molecule.
9. The method of clause 7, wherein the method comprises:
   (a) contacting the nucleic acid sample with a first and second adapter oligonucleotide, wherein the first and second adapter oligonucleotides each comprise, optionally in the 5' to 3' direction, an adaptor region and a target region;
   (b) annealing the target region of the first adapter oligonucleotide to the first fragment of the target nucleic acid molecule, and annealing the target region of the second adapter oligonucleotide to a second fragment of the target nucleic acid molecule;
   (c) contacting the nucleic acid sample with a multimeric barcoding reagent as defined in clause 8;
   (d) annealing the adapter region of the first adapter oligonucleotide to the adapter region of the first barcode molecule, and annealing the adapter region of the second adapter oligonucleotide to the adapter region of the second barcode molecule; and
   (e) ligating the 3' end of the first barcoded oligonucleotide to the 5' end of the first adapter oligonucleotide to produce a first barcoded-adapter oligonucleotide and ligating the 3' end of the second barcoded oligonucleotide to the 5' end of the second adapter oligonucleotide to produce a second barcoded-adapter oligonucleotide;
   and wherein either: (i) the first and second barcoded-adapter oligonucleotides are extended to produce first and second different barcoded target nucleic acid molecules each of which comprises at least one nucleotide synthesised from the target nucleic acid molecule as a template, or (ii) the first and second adapter oligonucleotides are extended after step (b) to produce first and second different target nucleic acid molecules each of which comprises at least one nucleotide synthesised from the target nucleic acid molecule as a template, and wherein the first and second barcoded-adapter oligonucleotides produced in step (e) are first and second different barcoded target nucleic acid molecules.
10. The method of any one of clauses 1-9, wherein the method further comprises the step of removing paraffin from the nucleic acid sample.
11. The method of any one of clauses 1-10, wherein the method further comprises the step of removing crosslinks from the nucleic acid sample.
12. The method of any one of clauses 1-11, wherein the method further comprises the step of proteinase digestion of the nucleic acid sample.
13. The method of any one of clauses 1-12, wherein the method comprises the steps of:
   (i) contacting the nucleic acid sample with a library of multimeric barcoding reagents comprising a multimeric barcoding reagent for each of two or more target nucleic acid molecules, wherein each multimeric barcoding reagent is as defined in any one of clauses 1-12, and
   (ii) producing at least two barcoded target nucleic acid molecules from each of the at least two target nucleic acid molecules, and wherein the at least two barcoded target nucleic acid molecules produced from a single target nucleic acid molecule each comprise the nucleic acid sequence of a barcode region from the same multimeric barcoding reagent.
14. A method of preparing a nucleic acid sample for sequencing, wherein the method comprises the steps of:
   (a) contacting the nucleic acid sample with a transposome complex comprising a transposon and a transposase;
   (b) fragmenting a target nucleic acid molecule in the nucleic acid sample and inserting first and second transferred strands at different positions in the target nucleic acid molecule while maintaining the contiguity of the target nucleic acid molecule, wherein the first and second transferred strands are from the same transposon or from different transposons;
   (c) contacting the nucleic acid sample with a multimeric barcoding reagent comprising first and second barcode regions linked together, wherein each barcode region comprises a nucleic acid sequence, and
   (d) appending a barcode sequence to each of first and second nucleic acid sequences to produce first and second different barcoded target nucleic acid molecules, wherein the first barcoded target nucleic acid molecule comprises the nucleic acid sequence of the first barcode region, the nucleic acid sequence of all or part of the first transferred strand and a nucleic acid sequence of the region that is adjacent to the first transferred strand in the target nucleic molecule, and wherein the second barcoded target nucleic acid molecule comprises the nucleic acid sequence of the second barcode region, the nucleic acid sequence of all or part of the second transferred strand and a nucleic acid sequence of the region that is adjacent to the second transferred strand in the target nucleic molecule.
15. The method of clause 14, wherein steps (b)-(d) are performed sequentially or simultaneously.
16. The method of clause 14 or clause 15, wherein the multimeric barcoding reagent comprises first and second barcode molecules linked together, and wherein each of the barcode molecules comprises a nucleic acid sequence comprising a barcode region.
17. The method of any one of clauses 14-16, wherein the multimeric barcoding reagent comprises first and second barcoded oligonucleotides, wherein the first barcoded oligonucleotide comprises in the 5' to 3' direction a barcode region and a target region capable of annealing to the first transferred strand in the target nucleic acid molecule, and wherein the second barcoded oligonucleotide comprises in the 5' to 3' direction a barcode region and a target region capable of annealing to the second transferred strand in the target nucleic acid molecule.
18. The method of any one of clauses 14-17, wherein the multimeric barcoding reagent comprises:
   (i) first and second barcode molecules linked together, wherein each of the barcode molecules comprises a nucleic acid sequence comprising a barcode region; and
   (ii) first and second barcoded oligonucleotides, wherein the first barcoded oligonucleotide comprises in the 5' to 3' direction a barcode region annealed to the barcode region of the first barcode molecule and a target region capable of annealing to the first transferred strand in the target nucleic acid molecule, and wherein the second barcoded oligonucleotide comprises in the 5' to 3' direction a barcode region annealed to the barcode region of the second barcode molecule and a target region capable of annealing to the second transferred strand in the target nucleic acid molecule.
19. The method of any one of clauses 14-16, wherein the method comprises:
   (i) contacting the sample with a multimeric barcoding reagent comprising first and second barcode molecules linked together, wherein each of the barcode molecules comprises a nucleic acid sequence comprising a barcode region and an adapter region;
   (ii) appending a coupling sequence to first and second transferred strands in the target nucleic acid molecule;
   (iii) annealing the coupling sequence of the first transferred strand to the adapter region of the first barcode molecule, and annealing the coupling sequence of the second transferred strand to the adapter region of the second barcode molecule; and
   (iv) appending a barcode sequence to each of first and second nucleic acid sequences to produce first and second different barcoded target nucleic acid molecules, wherein the first barcoded target nucleic acid molecule comprises the nucleic acid sequences of the first barcode region, the coupling sequence of the first transferred strand, all or part of the first transferred strand and a region that is adjacent to the first transferred strand in the target nucleic molecule, and wherein the second barcoded target nucleic acid molecule comprises the nucleic acid sequences of the second barcode region, the coupling sequence of the second transferred strand, all or part of the second transferred strand and a region that is adjacent to the second transferred strand in the target nucleic molecule.
20. The method of clause 19, wherein each of the barcode molecules comprises a nucleic acid sequence comprising, in the 5' to 3' direction, an adapter region and a barcode region, and step (iv) comprises: annealing and extending a first extension primer using the barcode region of the first barcode molecule as a template to produce a first barcoded oligonucleotide, and annealing and extending a second extension primer using the barcode region of the second barcode molecule as a template to produce a second barcoded oligonucleotide, wherein the first barcoded oligonucleotide comprises a sequence complementary to the barcode region of the first barcode molecule and the second barcoded oligonucleotide comprises a sequence complementary to the barcode region of the second barcode molecule, (ii) ligating the 3' end of the first barcoded oligonucleotide to the 5' end of the coupling sequence of the first transferred strand to produce a first barcoded target nucleic acid molecule and ligating the 3' end of the second barcoded oligonucleotide to the 5' end of the coupling sequence of the second transferred strand to produce a second barcoded target nucleic acid molecule.
21. The method of clause 19, wherein the multimeric barcoding reagent comprises:
   (i) first and second barcode molecules linked together, wherein each of the barcode molecules comprises a nucleic acid sequence comprising, optionally in the 5' to 3' direction, an adapter region and a barcode region, and
   (ii) first and second barcoded oligonucleotides, wherein the first barcoded oligonucleotide comprises a barcode region annealed to the barcode region of the first barcode molecule, and wherein the second barcoded oligonucleotide comprises a barcode region annealed to the barcode region of the second barcode molecule;
   and wherein step (iv) comprises ligating the first barcoded oligonucleotide to the coupling sequence of the first transferred strand to produce a first barcoded target nucleic acid molecule and ligating the second barcoded oligonucleotide to the coupling sequence of the second transferred strand to produce a second barcoded target nucleic acid molecule.
22. The method of any one of clauses 14-16, wherein the method comprises:
   (i) contacting the sample with a multimeric barcoding reagent comprising first and second barcode molecules linked together, wherein each of the barcode molecules comprises a nucleic acid sequence comprising a barcode region and an adapter region, and wherein the first transferred strand comprises an adapter region capable of annealing to the adapter region of the first barcode molecule and the second transferred strand comprises an adapter region capable of annealing to the adapter region of the second barcode molecule;
   (ii) annealing the adapter region of the first transferred strand to the adapter region of the first barcode molecule, and annealing the adapter region of the second transferred strand to the adapter region of the second barcode molecule; and
   (iv) appending a barcode sequence to each of first and second nucleic acid sequences to produce first and second different barcoded target nucleic acid molecules, wherein the first barcoded target nucleic acid molecule comprises the nucleic acid sequences of the first barcode region, the adapter region of the first transferred strand, all or part of the first transferred strand and a region that is adjacent to the first transferred strand in the target nucleic molecule, and wherein the second barcoded target nucleic acid molecule comprises the nucleic acid sequences of the second barcode region, the adapter region of the second transferred strand, all or part of the second transferred strand and a region that is adjacent to the second transferred strand in the target nucleic molecule.
23. The method of clause 22, wherein each of the barcode molecules comprises a nucleic acid sequence comprising, in the 5' to 3' direction, an adapter region and a barcode region, and step (iv) comprises: annealing and extending a first extension primer using the barcode region of the first barcode molecule as a template to produce a first barcoded oligonucleotide, and annealing and extending a second extension primer using the barcode region of the second barcode molecule as a template to produce a second barcoded oligonucleotide, wherein the first barcoded oligonucleotide comprises a sequence complementary to the barcode region of the first barcode molecule and the second barcoded oligonucleotide comprises a sequence complementary to the barcode region of the second barcode molecule, (ii) ligating the 3' end of the first barcoded oligonucleotide to the 5' end of the adapter region of the first transferred strand to produce a first barcoded target nucleic acid molecule and ligating the 3' end of the second barcoded oligonucleotide to the 5' end of the adapter region of the second transferred strand to produce a second barcoded target nucleic acid molecule.
24. The method of clause 22, wherein the multimeric barcoding reagent comprises:
   (i) first and second barcode molecules linked together, wherein each of the barcode molecules comprises a nucleic acid sequence comprising, optionally in the 5' to 3' direction, an adapter region and a barcode region, and
   (ii) first and second barcoded oligonucleotides, wherein the first barcoded oligonucleotide comprises a barcode region annealed to the barcode region of the first barcode molecule, and wherein the second barcoded oligonucleotide comprises a barcode region annealed to the barcode region of the second barcode molecule;
   and wherein step (iv) comprises ligating the first barcoded oligonucleotide to the adapter region of the first transferred strand to produce a first barcoded target nucleic acid molecule and ligating the second barcoded oligonucleotide to the adapter region of the second transferred strand to produce a second barcoded target nucleic acid molecule.
25. The method of any one of clauses 14-18, wherein the multimeric barcoding reagent comprises:
   (i) first and second barcode molecules linked together, wherein each of the barcode molecules comprises a nucleic acid sequence comprising, optionally in the 5' to 3' direction, an adapter region and a barcode region, and
   (ii) first and second barcoded oligonucleotides, wherein the first barcoded oligonucleotide comprises a barcode region annealed to the barcode region of the first barcode molecule, and wherein the second barcoded oligonucleotide comprises a barcode region annealed to the barcode region of the second barcode molecule.
26. The method of any one of clauses 14-16, wherein the first and second transferred strands each comprise an adapter region capable of hybridizing to the multimeric barcoding reagent.
27. The method of any one of clauses 14-16 and 26, wherein the 5' end of the first and second transferred strands each comprise a terminal 5' phosphate group capable of being ligated to a 3' end of a nucleic acid strand.
28. The method of any one of clauses 14-27, wherein the method comprises the steps of:
   (i) performing steps (a) and (b) for each of at least two target nucleic acid molecules;
   (ii) contacting the nucleic acid sample with a library of multimeric barcoding reagents comprising a multimeric barcoding reagent for each of two or more target nucleic acid molecules, wherein each multimeric barcoding reagent is as defined in any one of clauses 14-27, and
   (iii) performing step (d) wherein at least two barcoded target nucleic acid molecules are produced from each of the at least two target nucleic acid molecules, and wherein the at least two barcoded target nucleic acid molecules produced from a single target nucleic acid molecule each comprise the nucleic acid sequence of a barcode region from the same multimeric barcoding reagent.
29. A method of preparing a nucleic acid sample for sequencing, wherein the method comprises the steps of:
   (a) appending a nucleic acid sequence consisting of a first coupling sequence to a first sub-sequence of a target nucleic acid and appending a nucleic acid sequence consisting of a second coupling sequence to a second subsequence of the target nucleic acid, wherein the first coupling sequence consists of a nucleic acid sequence capable of annealing to the adapter region of a first hybridization molecule, and wherein the second coupling sequence consists of a nucleic acid sequence capable of annealing to the adapter region of a second hybridization molecule;
   (b) contacting the nucleic acid sample with a multimeric barcoding reagent comprising first and second hybridization molecules linked together, wherein each of the hybridization molecules comprises a nucleic acid sequence comprising a hybridization region and an adapter region, wherein the multimeric barcoding reagent further comprises first and second barcoded oligonucleotides, and wherein the first barcoded oligonucleotide is annealed to the hybridization region of the first hybridization molecule and wherein the second barcoded oligonucleotide is annealed to the hybridization region of the second hybridization molecule;
   (c) annealing the first coupling sequence to the adapter region of the first hybridization molecule, and annealing the second coupling sequence to the adapter region of the second hybridization molecule; and
   (d) appending the first barcoded oligonucleotide to the first coupling sequence to produce a first barcoded target nucleic acid molecule and appending the second barcoded oligonucleotide to the second coupling sequence to produce a second barcoded target nucleic acid molecule, wherein the first and second barcoded target nucleic acid molecules are different.
30. The method of clause 29, wherein step (a) comprises ligating a first adapter oligonucleotide to the first sub-sequence of a target nucleic acid, wherein the first adapter oligonucleotide consists of the first coupling sequence, and ligating a second adapter oligonucleotide to the second subsequence of the target nucleic acid, wherein the second adaptor oligonucleotide consists of the second adapter sequence.
31. A method of preparing a nucleic acid sample for sequencing, wherein the method comprises the steps of:
   (a) appending a first coupling sequence to a first sub-sequence of a target nucleic acid and appending a second coupling sequence to a second subsequence of the target nucleic acid, wherein the coupling sequences are appended to the sub-sequences of the target nucleic acid by a terminal transferase enzyme;
   (b) contacting the nucleic acid sample with a multimeric barcoding reagent comprising first and second hybridization molecules linked together, wherein each of the hybridization molecules comprises a nucleic acid sequence comprising a hybridization region and an adapter region, and wherein the multimeric barcoding reagent further comprises first and second barcoded oligonucleotides, wherein the first barcoded oligonucleotide is annealed to the hybridization region of the first hybridization molecule and wherein the second barcoded oligonucleotide is annealed to the hybridization region of the second hybridization molecule;
   (c) annealing the first coupling sequence to the adapter region of the first hybridization molecule, and annealing the second coupling sequence to the adapter region of the second hybridization molecule; and
   (d) appending the first barcoded oligonucleotide to the first coupling sequence to produce a first barcoded target nucleic acid molecule and appending the second barcoded oligonucleotide to the second coupling sequence to produce a second barcoded target nucleic acid molecule, wherein the first and second barcoded target nucleic acid molecules are different.
32. The method of any one of clauses 29-31, wherein the multimeric barcoding reagent comprises first and second barcode molecules linked together, wherein each of the barcode molecules comprises a nucleic acid sequence comprising a barcode region and an adapter region, wherein the multimeric barcoding reagent further comprises first and second barcoded oligonucleotides, and wherein the first barcoded oligonucleotide is annealed to the barcode region of the first barcode molecule and wherein the second barcoded oligonucleotide is annealed to the barcode region of the second barcode molecule.
33. The method of any one of clauses 29-32, wherein step (d) comprises ligating the first barcoded oligonucleotide to the first coupling sequence to produce a first barcoded target nucleic acid molecule and ligating the second barcoded oligonucleotide to the second coupling sequence to produce a second barcoded target nucleic acid molecule.
34. The method of clause 33, wherein step (d) comprises ligating the 3' end of the first barcoded oligonucleotide to the 5' end of the first coupling sequence to produce a first barcoded target nucleic acid molecule and ligating the 3' end of the second barcoded oligonucleotide to the 5' end of the second coupling sequence to produce a second barcoded target nucleic acid molecule.
35. A method of preparing a nucleic acid sample for sequencing, wherein the method comprises the steps of:
   (a) appending a first coupling sequence to a first sub-sequence of a target nucleic acid and appending a second coupling sequence to a second subsequence of the target nucleic acid, wherein the coupling sequences are appended to the sub-sequences of the target nucleic acid by a terminal transferase enzyme;
   (b) contacting the nucleic acid sample with a multimeric barcoding reagent comprising first and second barcode molecules linked together, wherein each of the barcode molecules comprises a nucleic acid sequence comprising, a barcode region and an adapter region;
   (c) annealing the first coupling sequence to the adapter region of the first barcode molecule, and annealing the second coupling sequence to the adapter region of the second barcode molecule; and
   (d) appending barcode sequences to each of the at least two sub-sequences of the target nucleic acid to produce first and second different barcoded target nucleic acid molecules, wherein the first barcoded target nucleic acid molecule comprises the nucleic acid sequence of the barcode region of the first barcode molecule and the second barcoded target nucleic acid molecule comprises the nucleic acid sequence of the barcode region of the second barcode molecule.
36. The method of clause 35, wherein each of the barcode molecules comprises a nucleic acid sequence comprising, in the 5' to 3' direction, a barcode region and an adapter region, and step (d) comprises extending the first coupling sequence using the barcode region of the first barcode molecule as a template to produce a first barcoded target nucleic acid molecule, and extending the second coupling sequence using the barcode region of the second barcode molecule as a template to produce a second barcoded target nucleic acid molecule, wherein the first barcoded target nucleic acid molecule comprises a sequence complementary to the barcode region of the first barcode molecule and the second barcoded target nucleic acid molecule comprises a sequence complementary to the barcode region of the second barcode molecule.
37. The method of clause 35, wherein each of the barcode molecules comprises a nucleic acid sequence comprising, in the 5' to 3' direction, an adapter region and a barcode region, and step (d) comprises (i) annealing and extending a first extension primer using the barcode region of the first barcode molecule as a template to produce a first barcoded oligonucleotide, and annealing and extending a second extension primer using the barcode region of the second barcode molecule as a template to produce a second barcoded oligonucleotide, wherein the first barcoded oligonucleotide comprises a sequence complementary to the barcode region of the first barcode molecule and the second barcoded oligonucleotide comprises a sequence complementary to the barcode region of the second barcode molecule, (ii) ligating the 3' end of the first barcoded oligonucleotide to the 5' end of the first coupling sequence to produce a first barcoded target nucleic acid molecule and ligating the 3' end of the second barcoded oligonucleotide to the 5' end of the second coupling sequence to produce a second barcoded target nucleic acid molecule.
38. The method of any one of clauses 29-37, wherein the first coupling sequence comprises at least one nucleotide complementary to the adapter region of the first hybridization molecule or the first barcode molecule, and wherein the second coupling sequence comprises at least one nucleotide complementary to the adapter region of the second hybridization molecule or the second barcode molecule.
39. The method of clause 38, wherein the first coupling sequence consists of a nucleic acid sequence that is complementary to all or portion of the adapter region of the first hybridization molecule or the first barcode molecule, and wherein the second coupling sequence consists of a nucleic acid sequence that is complementary to all or a portion of the adapter region of the second hybridization molecule or the second barcode molecule.
40. The method of any one of clauses 29-39, wherein the target nucleic acid is genomic DNA or mRNA.
41. The method of any one of clauses 29-30, wherein the method comprises:
   (i) appending first and second coupling sequences to two or more target nucleic acids in the nucleic acid sample, wherein the coupling sequences are appended according to any one of clauses 29-40;
   (ii) contacting the nucleic acid sample with a library of multimeric barcoding reagents comprising a multimeric barcoding reagent for each of two or more target nucleic acid molecules, wherein each multimeric barcoding reagent is as defined in any one of clauses 29-40; and
   (iii) performing step (d) wherein at least two barcoded target nucleic acid molecules are produced from each of the at least two target nucleic acids, and wherein the at least two barcoded target nucleic acid molecules produced from a single target nucleic acid each comprise the nucleic acid sequence of a barcode region from the same multimeric barcoding reagent.
42. A method of preparing a nucleic acid sample for sequencing, wherein the method comprises the steps of:
   (a) contacting the nucleic acid sample with first and second primers, wherein each primer comprises a target region;
   (b) annealing the target region of the first primer to a first sub-sequence of a target nucleic acid molecule, and annealing the target region of the second primer to a second sub-sequence of the same target nucleic acid molecule;
   (c) extending the first primer and the second primer to produce a first primer-extension product and a second primer-extension product, wherein the first and second primer-extension products each comprise at least one nucleotide synthesised from the target nucleic acid molecule as a template;
   (d) contacting the nucleic acid sample with a multimeric barcoding reagent comprising first and second barcode regions linked together, wherein each barcode region comprises a nucleic acid sequence, and
   (e) appending a barcode sequence to each of the first and second primers to produce first and second different barcoded primers, wherein the first barcoded primer comprises the barcode sequence of the first barcode region and the second barcoded primer comprise the barcode sequence of the second barcode region, wherein either (i) the first and second barcoded primers are extended to produce first and second barcoded target nucleic acid molecules, or (ii) the first and second extension primers are extended after step (b) to produce first and second primer-extension products, and wherein the first and second barcoded primers produced in step (e) are first and second different barcoded target nucleic acid molecules;
   and wherein the first and second primers remain hybridized to the target nucleic acid molecule until the first and second different barcoded target nucleic acid molecules have been produced.
43. The method of clause 42, wherein the first and second primers each comprise an adapter region capable of annealing to the multimeric barcoding reagent and step (d) further comprises annealing the first and second primers to the multimeric barcoding reagent.
44. The method of clause 42 or clause 43, wherein the multimeric barcoding reagent comprises:
   (i) first and second barcode molecules linked together, wherein each of the barcode molecules comprises a nucleic acid sequence comprising, optionally in the 5' to 3' direction, an adapter region and a barcode region; and
   (ii) first and second barcoded oligonucleotides, wherein the first barcoded oligonucleotide comprises a barcode region annealed to the barcode region of the first barcode molecule, and wherein the second barcoded oligonucleotide comprises a barcode region annealed to the barcode region of the second barcode molecule.
45. The method of clause 44, wherein the method comprises the steps of:
   (a) contacting the nucleic acid sample with first and second adapter oligonucleotides, wherein the first adapter oligonucleotide comprises in the 5' to 3' direction an adapter region capable of annealing to the adapter region of the first barcode molecule and a target region capable of annealing to the first sub-sequence of the target nucleic acid molecule, and wherein the second adapter oligonucleotide comprises in the 5' to 3' direction an adapter region capable of annealing to the adapter region of the second barcode molecule and a target region capable of annealing to the second sub-sequence of the target nucleic acid molecule;
   (b) annealing the target region of the first adapter oligonucleotide to the first sub-sequence of the target nucleic acid molecule, and annealing the target region of the second adapter oligonucleotide to the second sub-sequence of the same target nucleic acid nucleic acid molecule;
   (c) extending the first adapter oligonucleotide and the second adapter oligonucleotide to produce a first primer-extension product and a second primer-extension product;
   (d) contacting the nucleic acid sample with a multimeric barcoding reagent as defined in clause 44 and annealing the adapter region of the first adapter oligonucleotide to the adapter region of the first barcode molecule, and annealing the adapter region of the second adapter oligonucleotide to the adapter region of the second barcode molecule, and
   (e) ligating the 3' end of the first barcoded oligonucleotide to the 5' end of the first adapter oligonucleotide to produce a first barcoded-adapter oligonucleotide and ligating the 3' end of the second barcoded oligonucleotide to the 5' end of the second adapter oligonucleotide to produce a second barcoded-adapter oligonucleotide; and wherein either: the first and second barcoded-adapter oligonucleotides are extended to produce first and second different barcoded target nucleic acid molecules each of which comprises at least one nucleotide synthesised from the target nucleic acid molecule as a template, or the first and second adapter oligonucleotides are extended after step (b) to produce first and second different target nucleic acid molecules each of which comprises at least one nucleotide synthesised from the target nucleic acid molecule as a template, and wherein the first and second barcoded-adapter oligonucleotides produced in step (e) are first and second different barcoded target nucleic acid molecules.
46. The method of any one of clauses 42-45, wherein the target regions of the first and second primers are capable of random priming.
47. The method of any one of clauses 42-46, wherein the target regions of the first and second primers comprise one or more degenerate nucleotides.
48. The method of any one of clauses 42-47, wherein step (c) is performed using a strand displacing polymerase.
49. The method of any one of clauses 42-48, wherein the method comprises the steps of:
   (i) contacting the nucleic acid sample with first and second primers for at least two target nucleic acid molecules, wherein each primer comprises a target region;
   (ii) performing steps (b) and (c) for each target nucleic acid molecule;
   (iii) contacting the nucleic acid sample with a library of multimeric barcoding reagents comprising a multimeric barcoding reagent for each target nucleic acid molecule, wherein each multimeric barcoding reagent is as defined in any one of clauses 42-48; and
   (iv) performing step (e) wherein at least two barcoded target nucleic acid molecules are produced from each of the at least two target nucleic acid molecules, and wherein the barcoded target nucleic acid molecules produced from a single target nucleic acid molecule each comprise the nucleic acid sequence of a barcode region from the same multimeric barcoding reagent.

The invention is further defined in the following set of numbered clauses:
1. A method of preparing a nucleic acid sample for sequencing, wherein the nucleic acid sample comprises a formalin-fixed paraffin-embedded (FFPE) sample, and wherein the method comprises the steps of:
   (a) contacting the nucleic acid sample with a library of multimeric barcoding reagents, wherein the library comprises first and second multimeric barcoding reagents, wherein each multimeric barcoding reagent comprises first and second different barcode regions linked together, wherein each barcode region comprises a nucleic acid sequence, and wherein the first and second barcode regions of the first multimeric barcoding reagent are different to the first and second barcode regions of the second multimeric barcoding reagent, and
   (b) appending barcode sequences from the first multimeric barcoding reagent to each of the nucleic acid sequences of first and second fragments of a first target nucleic acid molecule to produce first and second different barcoded target nucleic acid molecules, wherein the first barcoded target nucleic acid molecule comprises the nucleic acid sequence of the first barcode region of the first multimeric barcoding reagent and the second barcoded target nucleic acid molecule comprises the nucleic acid sequence of the second barcode region of the first multimeric barcoding reagent, and appending barcode sequences from the second multimeric barcoding reagent to each of the nucleic acid sequences of first and second fragments of a second target nucleic acid molecule to produce third and fourth different barcoded target nucleic acid molecules, wherein the third barcoded target nucleic acid molecule comprises the nucleic acid sequence of the first barcode region of the second multimeric barcoding reagent and the fourth barcoded target nucleic acid molecule comprises the nucleic acid sequence of the second barcode region of the second multimeric barcoding reagent,
   wherein prior to and/or during step (a) and/or prior to and/or during step (b), the method further comprises the step of removing or depleting all or a fraction of the paraffin from the nucleic acid sample.
2. The method of clause 1, wherein the first and second target nucleic acid molecules are genomic DNA.
3. The method of clause 1, wherein the first and second target nucleic acid molecules are mRNA.
4. The method of any one of clauses 1-3, wherein the first and second multimeric barcoding reagents each comprise first and second barcode molecules linked together, and wherein each of the barcode molecules comprises a nucleic acid sequence comprising a barcode region.
5. The method of any one of clauses 1-4, wherein the first and second multimeric barcoding reagents each comprise first and second barcoded oligonucleotides, wherein the first barcoded oligonucleotide comprises, optionally in the 5' to 3' direction, a barcode region, and a target region capable of annealing or ligating to the first fragment of the target nucleic acid, and wherein the second barcoded oligonucleotide comprises, optionally in the 5' to 3' direction, a barcode region, and a target region capable of annealing or ligating to the second fragment of the target nucleic acid.
6. The method of any one of clauses 1-5, wherein the first and secon4 multimeric barcoding reagents each comprise:
   (i) first and second barcode molecules linked together, wherein each of the barcode molecules comprises a nucleic acid sequence comprising a barcode region; and
   (ii) first and second barcoded oligonucleotides, wherein the first barcoded oligonucleotide comprises, optionally in the 5' to 3' direction, a barcode region, and a target region capable of annealing or ligating to the first fragment of the target nucleic acid, and wherein the second barcoded oligonucleotide comprises, optionally in the 5' to 3' direction, a barcode region, and a target region capable of annealing or ligating to the second fragment of the target nucleic acid.
7. The method of clause 5 or clause 6, wherein the method comprises
   (a) contacting the nucleic acid sample with a library of multimeric barcoding reagents as defined in clause 5 or clause 6;
   (b) for each of the first and second multimeric barcoding reagents in the library, ligating the target region of the first barcoded oligonucleotide to the first fragment of a target nucleic acid molecule to produce a first barcoded target nucleic acid molecule, and ligating the target region of the second barcoded oligonucleotide to the second fragment of the target nucleic acid molecule to produce a second barcoded target nucleic acid molecule, wherein each of the barcoded target nucleic acid molecules comprises at least one nucleotide synthesised from the target nucleic acid as a template.
8. The method of clause 5 or clause 6, wherein the method comprises:
   (a) contacting the nucleic acid sample with a library of multimeric barcoding reagents as defined in clause 5 or clause 6;
   (b) for each of the first and second multimeric barcoding reagents in the library, annealing the target region of the first barcoded oligonucleotide to the first fragment of a target nucleic acid, and annealing the target region of the second barcoded oligonucleotide to the second fragment of the target nucleic acid; and
   (c) for each of the first and second multimeric barcoding reagents in the library, extending the first and second barcoded oligonucleotides to produce first and second different barcoded target nucleic acid molecules, wherein each of the barcoded target nucleic acid molecules comprises at least one nucleotide synthesised from the target nucleic acid as a template.
9. The method of clause 1 or clause 2, wherein the method comprises:
   (a) appending a coupling sequence to each of first and second fragments of at least first and second target nucleic acid molecules;
   (b) contacting the sample with a library of multimeric barcoding reagents, wherein the library comprises first and second multimeric barcoding reagents, wherein each multimeric barcoding reagent comprises first and second barcode molecules linked together, wherein each of the barcode molecules comprises a nucleic acid sequence comprising a barcode region and an adapter region, wherein each multimeric barcoding reagent comprises first and second different barcode regions linked together, and wherein the first and second barcode regions of the first multimeric barcoding reagent are different to the first and second barcode regions of the second multimeric barcoding reagent;
   (c) (i) for the first target nucleic acid molecule, annealing the coupling sequence of the first fragment to the adapter region of the first barcode molecule of the first multimeric barcoding reagent, and annealing the coupling sequence of the second fragment to the adapter region of the second barcode molecule of the first multimeric barcoding reagent, and (ii) for the second target nucleic acid molecule, annealing the coupling sequence of the first fragment to the adapter region of the first barcode molecule of the second multimeric barcoding reagent, and annealing the coupling sequence of the second fragment to the adapter region of the second barcode molecule of the second multimeric barcoding reagent; and
   (d) (i) appending barcode sequences to the first and second fragments of the first target nucleic acid molecule to produce first and second different barcoded target nucleic acid
   molecules, wherein the first barcoded target nucleic acid molecule comprises the nucleic acid sequence of the barcode region of the first barcode molecule of the first multimeric barcoding reagent and the second barcoded target nucleic acid molecule comprises the nucleic acid sequence of the barcode region of the second barcode molecule of the first multimeric barcoding reagent, and (ii) appending barcode sequences to the first and second fragments of the second target nucleic acid molecule to produce third and fourth different barcoded target nucleic acid molecules, wherein the third barcoded target nucleic acid molecule comprises the nucleic acid sequence of the barcode region of the first barcode molecule of the second multimeric barcoding reagent and the fourth barcoded target nucleic acid molecule comprises the nucleic acid sequence of the barcode region of the second barcode molecule of the second multimeric barcoding reagent.
10. The method of clause 1 or clause 9, wherein the first and second multimeric barcoding reagents each comprise:
   (i) first and second barcode molecules linked together, wherein each of the barcode molecules comprises a nucleic acid sequence comprising, optionally in the 5' to 3' direction, an adapter region and a barcode region, and
   (ii) first and second barcoded oligonucleotides, wherein the first barcoded oligonucleotide comprises a barcode region annealed to the barcode region of the first barcode molecule, and wherein the second barcoded oligonucleotide comprises a barcode region annealed to the barcode region of the second barcode molecule.
11. The method of any one of clauses 1-10, wherein the method further comprises the step of partially or fully removing crosslinks from the nucleic acid sample.
12. The method of any one of clauses 1-11, wherein the method further comprises the step of proteinase digestion of the nucleic acid sample.

### BRIEF DESCRIPTION OF THE DRAWINGS

The invention, together with further objects and advantages thereof, may best be understood by making reference to the description taken together with the accompanying drawings, in which:
Figure 1 illustrates a multimeric barcoding reagent that may be used in the method illustrated in Figure 3 or Figure 4.
Figure 2 illustrates a kit comprising a multimeric barcoding reagent and adapter oligonucleotides for labelling a target nucleic acid.
Figure 3 illustrates a first method of preparing a nucleic acid sample for sequencing using a multimeric barcoding reagent.
Figure 4 illustrates a second method of preparing a nucleic acid sample for sequencing using a multimeric barcoding reagent.
Figure 5 illustrates a method of preparing a nucleic acid sample for sequencing using a multimeric barcoding reagent and adapter oligonucleotides.
Figure 6 illustrates a method of preparing a nucleic acid sample for sequencing using a multimeric barcoding reagent, adapter oligonucleotides and target oligonucleotides.
Figure 7 illustrates a method of assembling a multimeric barcode molecule using a rolling circle amplification process.
Figure 8 illustrates a method of synthesizing multimeric barcoding reagents for labeling a target nucleic acid that may be used in the methods illustrated in Figure 3, Figure 4 and/or Figure 5.
Figure 9 illustrates an alternative method of synthesizing multimeric barcoding reagents (as illustrated in Figure 1) for labeling a target nucleic acid that may be used in the method illustrated in Figure 3 and/or Figure 4.

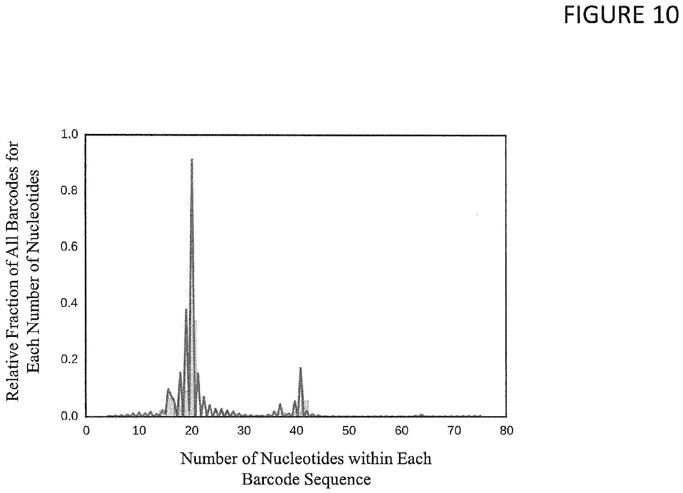

Figure 10 is a graph showing the total number of nucleotides within each barcode sequence.

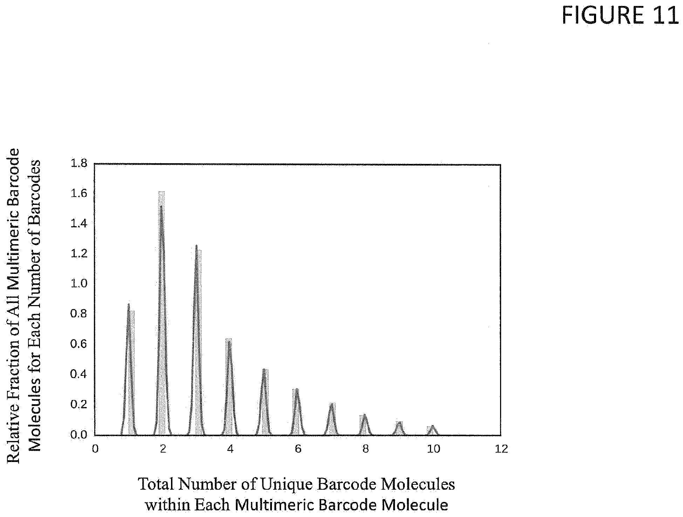

Figure 11 is a graph showing the total number of unique barcode molecules in each sequenced multimeric barcode molecule.

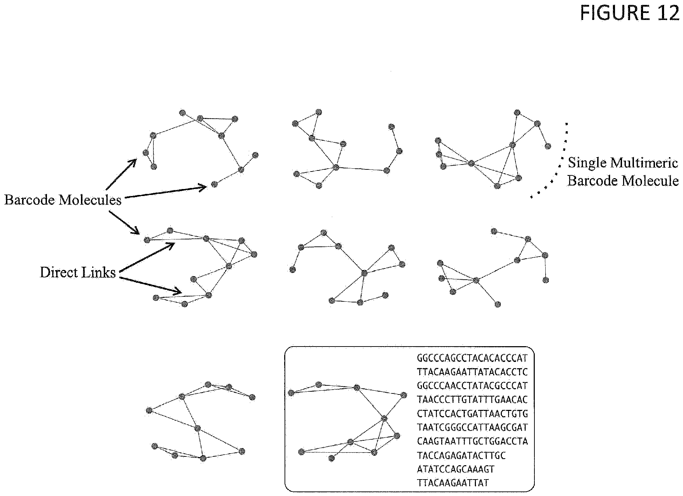

Figure 12 shows representative multimeric barcode molecules that were detected by the analysis script.

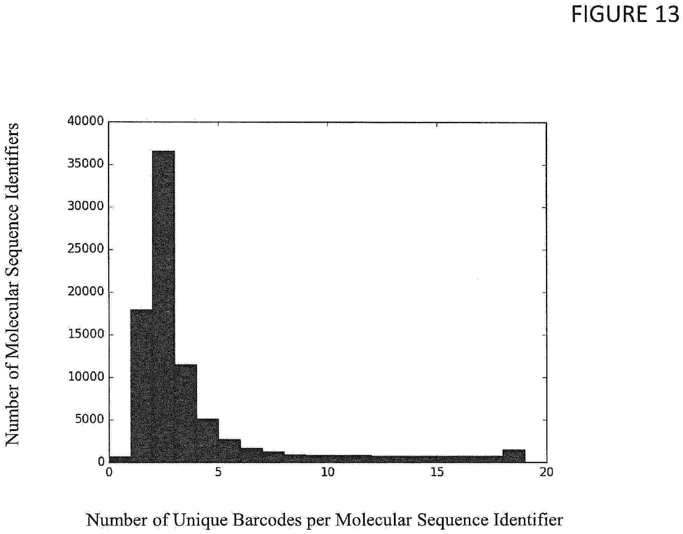

Figure 13 is a graph showing the number of unique barcodes per molecular sequence identifier against the number of molecular sequence identifiers following the barcoding of synthetic DNA templates of known sequence with multimeric barcoding reagents containing barcoded oligonucleotides.

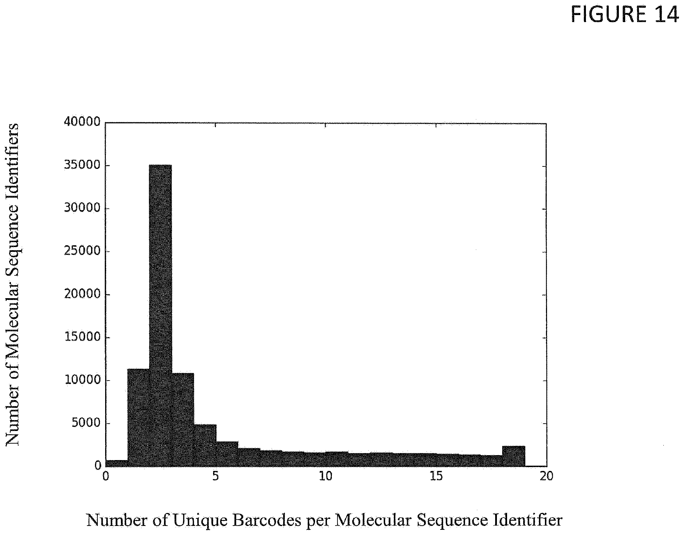

Figure 14 is a graph showing the number of unique barcodes per molecular sequence identifier against the number of molecular sequence identifiers following the barcoding of synthetic DNA templates of known sequence with multimeric barcoding reagents and separate adapter oligonucleotides.

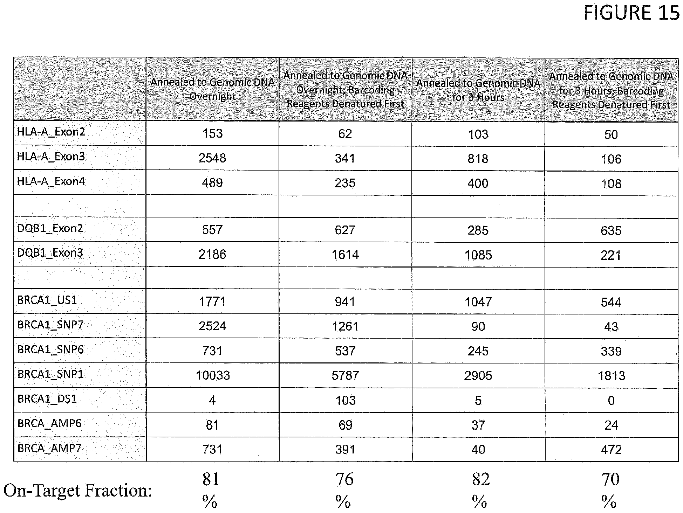

Figure 15 is a table showing the results of barcoding genomic DNA loci of three human genes (BRCA1, HLA-A and DQB1) with multimeric barcoding reagents containing barcoded oligonucleotides.

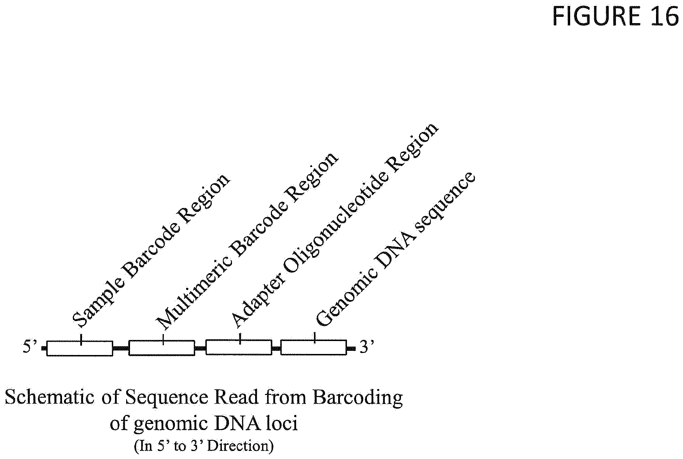

Figure 16 is a schematic illustration of a sequence read obtained from barcoding genomic DNA loci with multimeric barcoding reagents containing barcoded oligonucleotides.

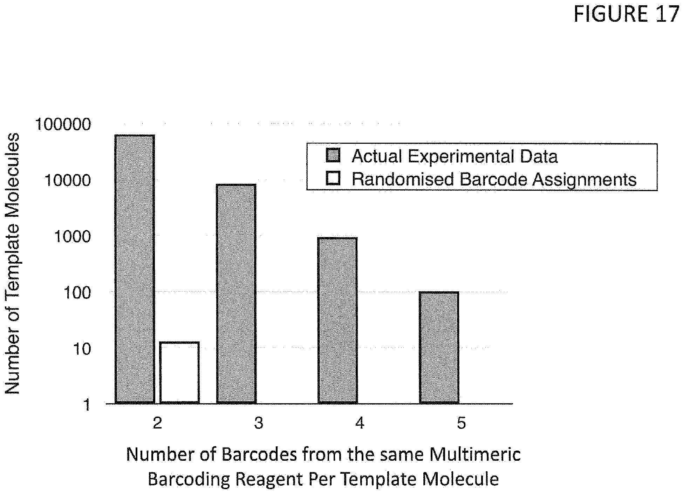

Figure 17 is a graph showing the number of barcodes from the same multimeric barcoding reagent that labelled sequences on the same synthetic template molecule against the number of synthetic template molecules.
Figure 18 illustrates a method of appending barcode sequences to fragments of a target nucleic acid molecule within an FFPE sample using coupling sequences.
Figure 19 illustrates a method of appending barcode sequences using a contiguity-preserving in vitro transposition process.
Figure 20 illustrates a method of appending barcode sequences using coupling sequences.
Figure 21 illustrates a method of preparing a nucleic acid sample for sequencing to enable synthetic long read sequencing. The method is a primer-extension method that uses a strand-displacing polymerase.

### EXAMPLES

### MATERIALS AND METHODS

### Method 1 - Synthesis of a Library of Nucleic Acid Barcode Molecules

### Synthesis of Double-Stranded Sub-Barcode Molecule Library

In a PCR tube, 10 microliters of 10 micromolar BC_MX3 (an equimolar mixture of all sequences in SEQ ID NO: 18 to 269) were added to 10 microliters of 10 micromolar BC_ADD_TP1 (SEQ ID NO: 1), plus 10 microliters of 10X CutSmart Buffer (New England Biolabs) plus 1.0 microliter of 10 millimolar deoxynucleotide triphosphate nucleotide mix (Invitrogen) plus 68 microliters H₂O, to final volume of 99 microliters. The PCR tube was placed on a thermal cycler and incubated at 75°C for 5 minutes, then slowly annealed to 4°C, then held 4°C, then placed on ice. 1.0 microliter of Klenow polymerase fragment (New England Biolabs; at 5 U/uL) was added to the solution and mixed. The PCR tube was again placed on a thermal cycler and incubated at 25°C for 15 minutes, then held at 4°C. The solution was then purified with a purification column (Nucleotide Removal Kit; Qiagen), eluted in 50 microliters H₂O, and quantitated spectrophotometrically.

### Synthesis of Double-Stranded Downstream Adapter Molecule

In a PCR tube, 0.5 microliters of 100 micromolar BC_ANC_TP1 (SEQ ID NO: 2) were added to 0.5 microliters of 100 micromolar BC_ANC_BT1 (SEQ ID NO: 3), plus 20 microliters of 10X CutSmart Buffer (New England Biolabs) plus 178 microliters H₂O, to final volume of 200 microliters. The PCR tube was placed on a thermal cycler and incubated at 95°C for 5 minutes, then slowly annealed to 4°C, then held 4°C, then placed on ice, then stored at -20°C.

### Ligation of Double-Stranded Sub-Barcode Molecule Library to Double-Stranded Downstream Adapter Molecule

In a 1.5 milliliter Eppendorf tube, 1.0 microliter of Double-Stranded Downstream Adapter Molecule solution was added to 2.5 microliters of Double-Stranded Sub-Barcode Molecule Library, plus 2.0 microliters of 10X T4 DNA Ligase buffer, and 13.5 microliters H₂O to final volume of 19 microliters. 1.0 microliter of T4 DNA Ligase (New England Biolabs; high concentration) was added to the solution and mixed. The tube was incubated at room temperature for 60 minutes, then purified with 1.8X volume (34 microliters) Ampure XP Beads (Agencourt; as per manufacturer's instructions), and eluted in 40 microliters H₂O.

### PCR Amplification of Ligated Library

In a PCR tube, 2.0 microliters of Ligated Library were added to 2.0 microliters of 50 micromolar BC_FWD_PR1 (SEQ ID NO: 4), plus 2.0 microliters of 50 micromolar BC_REV_PR1 (SEQ ID NO: 5), plus 10 microliters of 10X Taq PCR Buffer (Qiagen) plus 2.0 microliter of 10 millimolar deoxynucleotide triphosphate nucleotide mix (Invitrogen) plus 81.5 microliters H₂O, plus 0.5 microliters Qiagen Taq Polymerase (at 5U/uL) to final volume of 100 microliters. The PCR tube was placed on a thermal cycler and amplified for 15 cycles of: 95°C for 30 seconds, then 59°C for 30 seconds, then 72°C for 30 seconds; then held at 4°C. The solution was then purified with 1.8X volume (180 microliters) Ampure XP Beads (Agencourt; as per manufacturer's instructions), and eluted in 50 microliters H₂O.

### Uracil Glycosylase Enzyme Digestion

To an eppendorf tube 15 microliters of the eluted PCR amplification, 1.0 microliters H2O, plus 2.0 microliters 10X CutSmart Buffer (New England Biolabs), plus 2.0 microliter of USER enzyme solution (New England Biolabs) was added and mixed. The tube was incubated at 37°C for 60 minutes, then the solution was purified with 1.8X volume (34 microliters) Ampure XP Beads (Agencourt; as per manufacturer's instructions), and eluted in 34 microliters H₂O.

### Mlyl Restriction Enzyme Cleavage

To the eluate from the previous (glycosylase digestion) step, 4.0 microliters 10X CutSmart Buffer (New England Biolabs), plus 2.0 microliter of Mlyl enzyme (New England Biolabs, at 5U/uL) was added and mixed. The tube was incubated at 37°C for 60 minutes, then the solution was purified with 1.8X volume (72 microliters) Ampure XP Beads (Agencourt; as per manufacturer's instructions), and eluted in 40 microliters H₂O.

### Ligation of Sub-Barcode Library to Mlyl-Cleaved Solution

In a 1.5 milliliter Eppendorf tube, 10 microliter of Mlyl-Cleaved Solution solution was added to 2.5 microliters of Double-Stranded Sub-Barcode Molecule Library, plus 2.0 microliters of 10X T4 DNA Ligase buffer, and 4.5 microliters H₂O to final volume of 19 microliters. 1.0 microliter of T4 DNA Ligase (New England Biolabs; high concentration) was added to the solution and mixed. The tube was incubated at room temperature for 60 minutes, then purified with 1.8X volume (34 microliters) Ampure XP Beads (Agencourt; as per manufacturer's instructions), and eluted in 40 microliters H₂O.

### Repeating Cycles of Sub-Barcode Addition

The experimental steps of: 1) Ligation of Sub-Barcode Library to Mlyl-Cleaved Solution, 2) PCR Amplification of Ligated Library, 3) Uracil Glycosylase Enzyme Digestion, and 4) Mlyl Restriction Enzyme Cleavage were repeated, in sequence, for a total of five cycles.

### Synthesis of Double-Stranded Upstream Adapter Molecule

In a PCR tube, 1.0 microliters of 100 micromolar BC_USO_TP1 (SEQ ID NO: 6) were added to 1.0 microliters of 100 micromolar BC_USO_BT1 (SEQ ID NO: 7), plus 20 microliters of 10X CutSmart Buffer (New England Biolabs) plus 178 microliters H₂O, to final volume of 200 microliters. The PCR tube was placed on a thermal cycler and incubated at 95°C for 60 seconds, then slowly annealed to 4°C, then held 4°C, then placed on ice, then stored at -20°C.

### Ligation of Double-Stranded Upstream Adapter Molecule

In a 1.5 milliliter Eppendorf tube, 3.0 microliters of Upstream Adapter solution were added to 10.0 microliters of final (after the fifth cycle) Mlyl-Cleaved solution, plus 2.0 microliters of 10X T4 DNA Ligase buffer, and 5.0 microliters H₂O to final volume of 19 microliters. 1.0 microliter of T4 DNA Ligase (New England Biolabs; high concentration) was added to the solution and mixed. The tube was incubated at room temperature for 60 minutes, then purified with 1.8X volume (34 microliters) Ampure XP Beads (Agencourt; as per manufacturer's instructions), and eluted in 40 microliters H₂O.

### PCR Amplification of Upstream Adapter-Ligated Library

In a PCR tube, 6.0 microliters of Upstream Adapter-Ligated Library were added to 1.0 microliters of 100 micromolar BC_CS_PCR_FWD1 (SEQ ID NO: 8), plus 1.0 microliters of 100 micromolar BC_CS_PCR_REV1 (SEQ ID NO: 9), plus 10 microliters of 10X Taq PCR Buffer (Qiagen) plus 2.0 microliter of 10 millimolar deoxynucleotide triphosphate nucleotide mix (Invitrogen) plus 73.5 microliters H₂O, plus 0.5 microliters Qiagen Taq Polymerase (at 5U/uL) to final volume of 100 microliters. The PCR tube was placed on a thermal cycler and amplified for 15 cycles of: 95°C for 30 seconds, then 61°C for 30 seconds, then 72°C for 30 seconds; then held at 4°C. The solution, containing a library of amplified nucleic acid barcode molecules, was then purified with 1.8X volume (180 microliters) Ampure XP Beads (Agencourt; as per manufacturer's instructions). The library of amplified nucleic acid barcode molecules was then eluted in 40 microliters H₂O.

The library of amplified nucleic acid barcode molecules sythesised by the method described above was then used to assemble a library of multimeric barcode molecules as described below.

### Method 2 - Assembly of a Library of Multimeric Barcode Molecules

A library of multimeric barcode molecules was assembled using the library of nucleic acid barcode molecules synthesised according to the methods of Method 1.

### Primer-Extension with Forward Termination Primer and Forward Splinting Primer

In a PCR tube, 5.0 microliters of the library of amplified nucleic acid barcode molecules were added to 1.0 microliters of 100 micromolar CS_SPLT_FWD1 (SEQ ID NO: 10), plus 1.0 microliters of 5 micromolar CS_TERM_FWD1 (SEQ ID NO: 11), plus 10 microliters of 10X Thermopol Buffer (NEB) plus 2.0 microliter of 10 millimolar deoxynucleotide triphosphate nucleotide mix (Invitrogen) plus 80.0 microliters H₂O, plus 1.0 microliters Vent Exo-Minus Polymerase (New England Biolabs, at 2U/uL) to final volume of 100 microliters. The PCR tube was placed on a thermal cycler and amplified for 1 cycle of: 95°C for 30 seconds, then 53°C for 30 seconds, then 72°C for 60 seconds, then 1 cycle of: 95°C for 30 seconds, then 50°C for 30 seconds, then 72°C for 60 seconds, then held at 4°C. The solution was then purified a PCR purification column (Qiagen), and eluted in 85.0 microliters H₂O.

### Primer-Extension with Reverse Termination Primer and Reverse Splinting Primer

In a PCR tube, the 85.0 microliters of forward-extension primer-extension products were added to 1.0 microliters of 100 micromolar CS_SPLT_REV1 (SEQ ID NO: 12), plus 1.0 microliters of 5 micromolar CS_TERM_REV1 (SEQ ID NO: 13), plus 10 microliters of 10X Thermopol Buffer (NEB) plus 2.0 microliter of 10 millimolar deoxynucleotide triphosphate nucleotide mix (Invitrogen), plus 1.0 microliters Vent Exo-Minus Polymerase (New England Biolabs, at 2U/uL) to final volume of 100 microliters. The PCR tube was placed on a thermal cycler and amplified for 1 cycle of: 95°C for 30 seconds, then 53°C for 30 seconds, then 72°C for 60 seconds, then 1 cycle of: 95°C for 30 seconds, then 50°C for 30 seconds, then 72°C for 60 seconds, then held at 4°C. The solution was then purified a PCR purification column (Qiagen), and eluted in 43.0 microliters H₂O.

### Linking Primer-Extension Products with Overlap-Extension PCR

In a PCR tube were added the 43.0 microliters of reverse-extension primer-extension products, plus 5.0 microliters of 10X Thermopol Buffer (NEB) plus 1.0 microliter of 10 millimolar deoxynucleotide triphosphate nucleotide mix (Invitrogen), plus 1.0 microliters Vent Exo-Minus Polymerase (New England Biolabs, at 2U/uL) to final volume of 50 microliters. The PCR tube was placed on a thermal cycler and amplified for 5 cycles of: 95°C for 30 seconds, then 60°C for 60 seconds, then 72°C for 2 minutes; then 5 cycles of: 95°C for 30 seconds, then 60°C for 60 seconds, then 72°C for 5 minutes; then 5 cycles of: 95°C for 30 seconds, then 60°C for 60 seconds, then 72°C for 10 minutes; then held at 4°C. The solution was then purified with 0.8X volume (80 microliters) Ampure XP Beads (Agencourt; as per manufacturer's instructions), and eluted in 40 microliters H₂O.

### Amplification of Overlap-Extension Products

In a PCR tube were added 2.0 microliters of Overlap-Extension PCR solution, plus 1.0 microliters of 100 micromolar CS_PCR_FWD1 (SEQ ID NO: 14), plus 1.0 microliters of 100 micromolar CS_PCR_REV1 (SEQ ID NO: 15), plus 10 microliters of 10X Thermopol Buffer (NEB) plus 2.0 microliter of 10 millimolar deoxynucleotide triphosphate nucleotide mix (Invitrogen), plus 1.0 microliters Vent Exo-Minus Polymerase (New England Biolabs, at 2U/uL), plus 83.0 microliters H₂O to final volume of 100 microliters. The PCR tube was placed on a thermal cycler and amplified for 15 cycles of: 95°C for 30 seconds, then 58°C for 30 seconds, then 72°C for 10 minutes; then held at 4°C. The solution was then purified with 0.8X volume (80 microliters) Ampure XP Beads (Agencourt; as per manufacturer's instructions), and eluted in 50 microliters H₂O, and quantitated spectrophotometrically.

### Gel-Based Size Selection of Amplified Overlap-Extension Products

Approximately 250 nanograms of Amplified Overlap-Extension Products were loaded and run on a 0.9% agarose gel, and then stained and visualised with ethidium bromide. A band corresponding to 1000 nucleotide size (plus and minus 100 nucleotides) was excised and purified with a gel extraction column (Gel Extraction Kit, Qiagen) and eluted in 50 microliters H₂O.

### Amplification of Overlap-Extension Products

In a PCR tube were added 10.0 microliters of Gel-Size-Selected solution, plus 1.0 microliters of 100 micromolar CS_PCR_FWD1 (SEQ ID NO: 14), plus 1.0 microliters of 100 micromolar CS_PCR_REV1 (SEQ ID NO: 15), plus 10 microliters of 10X Thermopol Buffer (NEB) plus 2.0 microliter of 10 millimolar deoxynucleotide triphosphate nucleotide mix (Invitrogen), plus 1.0 microliters Vent Exo-Minus Polymerase (New England Biolabs, at 2U/uL) plus 75.0 microliters H₂O to final volume of 100 microliters. The PCR tube was placed on a thermal cycler and amplified for 15 cycles of: 95°C for 30 seconds, then 58°C for 30 seconds, then 72°C for 4 minutes; then held at 4°C. The solution was then purified with 0.8X volume (80 microliters) Ampure XP Beads (Agencourt; as per manufacturer's instructions), and eluted in 50 microliters H₂O, and quantitated spectrophotometrically.

### Selection and Amplification of Quantitatively Known Number of Multimeric Barcode Molecules

Amplified gel-extracted solution was diluted to a concentration of 1 picogram per microliter, and then to a PCR tube was added 2.0 microliters of this diluted solution (approximately 2 million individual molecules), plus 0.1 microliters of 100 micromolar CS_PCR_FWD1 (SEQ ID NO: 14), plus 0.1 microliters of 100 micromolar CS_PCR_REV1 (SEQ ID NO: 15), plus 1.0 microliter 10X Thermopol Buffer (NEB) plus 0.2 microliter of 10 millimolar deoxynucleotide triphosphate nucleotide mix (Invitrogen), plus 0.1 microliters Vent Exo-Minus Polymerase (New England Biolabs, at 2U/uL) plus 6.5 microliters H₂O to final volume of 10 microliters. The PCR tube was placed on a thermal cycler and amplified for 11 cycles of: 95°C for 30 seconds, then 57°C for 30 seconds, then 72°C for 4 minutes; then held at 4°C.

To the PCR tube was added 1.0 microliters of 100 micromolar CS_PCR_FWD1 (SEQ ID NO: 14), plus 1.0 microliters of 100 micromolar CS_PCR_REV1 (SEQ ID NO: 15), plus 9.0 microliters of 10X Thermopol Buffer (NEB) plus 2.0 microliter of 10 millimolar deoxynucleotide triphosphate nucleotide mix (Invitrogen), plus 1.0 microliters Vent Exo-Minus Polymerase (New England Biolabs, at 2U/uL) plus 76.0 microliters H₂O to final volume of 100 microliters. The PCR tube was placed on a thermal cycler and amplified for 10 cycles of: 95°C for 30 seconds, then 57°C for 30 seconds, then 72°C for 4 minutes; then held at 4°C. The solution was then purified with 0.8X volume (80 microliters) Ampure XP Beads (Agencourt; as per manufacturer's instructions), and eluted in 50 microliters H₂O, and quantitated spectrophotometrically.

### Method 3: Production of Single-Stranded Multimeric Barcode Molecules by In Vitro Transcription and cDNA Synthesis

This method describes a series of steps to produce single-stranded DNA strands, to which oligonucleotides may be annealed and then barcoded along. This method begins with four identical reactions performed in parallel, in which a promoter site for the T7 RNA Polymerase is appended to the 5' end of a library of multimeric barcode molecules using an overlap-extension PCR amplification reaction. Four identical reactions are performed in parallel and then merged to increase the quantitative amount and concentration of this product available. In each of four identical PCR tubes, approximately 500 picograms of size-selected and PCR-amplified multimeric barcode molecules (as produced in the 'Selection and Amplification of Quantitatively Known Number of Multimeric Barcode Molecules' step of Method 2) were mixed with 2.0 microliters of 100 micromolar CS_PCR_FWD1_T7 (SEQ ID NO. 270) and 2.0 microliters of 100 micromolar CS_PCR_REV4 (SEQ ID NO. 271), plus 20.0 microliters of 10X Thermopol PCR buffer, plus 4.0 microliters of 10 millimolar deoxynucleotide triphosphate nucleotide mix, and 2.0 microliters Vent Exo Minus polymerse (at 5 units per microliter) plus water to a total volume of 200 microliters. The PCR tube was placed on a thermal cycler and amplified for 22 cycles of: 95°C for 60 seconds, then 60°C for 30 seconds, then 72°C for 3 minutes; then held at 4°C. The solution from all four reactions was then purified with a gel extraction column (Gel Extraction Kit, Qiagen) and eluted in 52 microliters H₂O.

Fifty (50) microliters of the eluate was mixed with 10 microliters 10X NEBuffer 2 (NEB), plus 0.5 microliters of 10 millimolar deoxynucleotide triphosphate nucleotide mix, and 1.0 microliters Vent Exo Minus polymerse (at 5 units per microliter) plus water to a total volume of 100 microliters. The reaction was incubated for 15 minutes at room temperature, then purified with 0.8X volume (80 microliters) Ampure XP Beads (Agencourt; as per manufacturer's instructions), and eluted in 40 microliters H₂O, and quantitated spectrophotometrically.

A transcription step is then performed, in which the library of PCR-amplified templates containing T7 RNA Polymerase promoter site (as produced in the preceding step) is used as a template for T7 RNA polymerase. This comprises an amplification step to produce a large amount of RNA-based nucleic acid corresponding to the library of multimeric barcode molecules (since each input PCR molecule can serve as a template to produce a large number of cognate RNA molecules). In the subsequent step, these RNA molecules are then reverse transcribed to create the desired, single-stranded multimeric barcode molecules. Ten (10) microliters of the eluate was mixed with 20 microliters 5X Transcription Buffer (Promega), plus 2.0 microliters of 10 millimolar deoxynucleotide triphosphate nucleotide mix, plus 10 microliters of 0.1 milimolar DTT, plus 4.0 microliters SuperAseln (Ambion), and 4.0 microliters Promega T7 RNA Polymerase (at 20 units per microliter) plus water to a total volume of 100 microliters. The reaction was incubated 4 hours at 37°C, then purified with an RNEasy Mini Kit (Qiagen), and eluted in 50 micoliters H₂O, and added to 6.0 microliters SuperAseln (Ambion).

The RNA solution produced in the preceding in vitro transcription step is then reverse transcribed (using a primer specific to the 3' ends of the RNA molecules) and then digested with RNAse H to create single-stranded DNA molecules corresponding to multimeric barcode molecules, to which oligonucleotides maybe be annealed and then barcoded along. In two identical replicate tubes, 23.5 microliters of the eluate was mixed with 5.0 microliters of 10 millimolar deoxynucleotide triphosphate nucleotide mix, plus 3.0 microliters SuperAseln (Ambion), and 10.0 microliters of 2.0 micromolar CS_PCR_REV1 (SEQ ID NO. 272) plus water to final volume of 73.5 microliters. The reaction was incubated on a thermal cycler at 65°C for 5 minutes, then 50°C for 60 seconds; then held at 4°C. To the tube was added 20 microliters 5X Reverse Transcription buffer (Invitrogen), plus 5.0 microliters of 0.1 milimolar DTT, and 1.75 microliters Superscript III Reverse Transcriptase (Invitrogen). The reaction was incubated at 55°C for 45 minutes, then 60°C for 5 minutes; then 70°C for 15 minutes, then held at 4°C., then purified with a PCR Cleanup column (Qiagen) and eluted in 40 microliters H₂O.

Sixty (60) microliters of the eluate was mixed with 7.0 microliters 10X RNAse H Buffer (Promega), plus 4.0 microliters RNAse H (Promega. The reaction was incubated 12 hours at 37°C, then 95°C for 10 minutes, then held at 4°C, then purified with 0.7X volume (49 microliters) Ampure XP Beads (Agencourt; as per manufacturer's instructions), and eluted in 30 microliters H₂O, and quantitated spectrophotometrically.

### Method 4: Production of Multimeric Barcoding Reagents Containing Barcoded Oligonucleotides

This method describes steps to produce multimeric barcoding reagents from single-stranded multimeric barcode molecules (as produced in Method 3) and appropriate extension primers and adapter oligonucleotides.

In a PCR tube, approximately 45 nanograms of single-stranded RNAse H-digested multimeric barcode molecules (as produced in the last step of Method 3) were mixed with 0.25 microliters of 10 micromolar DS_ST_05 (SEQ ID NO. 273, an adapter oligonucleotide) and 0.25 microliters of 10 micromolar US_PCR_Prm Only_03 (SEQ ID NO. 274, an extension primer), plus 5.0 microliters of 5X Isothermal extension/ligation buffer, plus water to final volume of 19.7 microliters. In order to anneal the adapter oligonucleotides and extension primers to the multimeric barcode molecules, in a thermal cycler, the tube was incubated at 98°C for 60 seconds, then slowly annealed to 55°C, then held at 55°C for 60 seconds, then slowly annealed to 50°C then held at 50°C for 60 seconds, then slowly annealed to 20°C at 0.1°C/sec, then held at 4°C. To the tube was added 0.3 microliters (0.625U) Phusion Polymerase (NEB; 2 U/uL) 2.5 microliters (100 U) Taq DNA Ligase (NEB; 40 U/uL); and 2.5 microliters 100 milimolar DTT. In order to extend the extension primer(s) across the adjacent barcode region(s) of each multimeric barcode molecule, and then to ligate this extension product to the phosphorylated 5' end of the adapter oligonucleotide annealed to the downstream thereof, the tube was then incubated at 50°C for 3 minutes, then held at 4°C. The reaction was then purified with a PCR Cleanup column (Qiagen) and eluted in 30 microliters H₂O, and quantitated spectrophotometrically.

### Method 5: Production of Synthetic DNA Templates of Known Sequence

This method describes a technique to produce synthetic DNA templates with a large number of tandemly-repeated, co-linear molecular sequence identifiers, by circularizing and then tandemly amplifying (with a processive, strand-displacing polymerase) oligonucleotides containing said molecular sequence identifiers. This reagent may then be used to evaluate and measure the multimeric barcoding reagents described herein.

In a PCR was added 0.4 microliters of 1.0 micromolar Syn_Temp_01 (SEQ ID NO. 275) and 0.4 microliters of 1.0 micromolar ST_Splint_02 (SEQ ID NO. 276) and 10.0 microliters of 10X NEB CutSmart buffer. On a thermal cycler, the tube was incubated at 95°C for 60 seconds, then held at 75°C for 5 minutes, then slowly annealed to 20°C then held at 20°C for 60 seconds, then held at 4°C. To circularize the molecules through an intramolecular ligation reaction, the tube was then added 10.0 microliters ribo-ATP and 5.0 microliters T4 DNA Ligase (NEB; High Concentration). The tube was then incubated at room temperature for 30 minutes, then at 65°C for 10 minutes, then slowly annealed to 20°C then held at 20°C for 60 seconds, then held at 4°C. To each tube was then added 10X NEB CutSmart buffer, 4.0 microliters of 10 millimolar deoxynucleotide triphosphate nucleotide mix, and 1.5 microliters of diluted phi29 DNA Polymerase (NEB; Diluted 1:20 in 1X CutSmart buffer) plus water to a total volume of 200 microliters. The reaction was incubated at 30°C for 5 minutes, then held at 4°C, then purified with 0.7X volume (140 microliters) Ampure XP Beads (Agencourt; as per manufacturer's instructions), and eluted in 30 microliters H₂O, and quantitated spectrophotometrically.

### Method 6: Barcoding Synthetic DNA Templates of Known Sequence with Multimeric Barcoding Reagents Containing Barcoded Oligonucleotides

In a PCR tube were added 10.0 microliters 5X Phusion HF buffer (NEB), plus 1.0 microliters 10 millimolar deoxynucleotide triphosphate nucleotide mix, plus 2.0 microliters (10 nanograms) 5.0 nanogram/ microliters Synthetic DNA Templates of Known Sequence (as produced by Method 5), plus water to final volume of 42.5 microliters. The tube was then incubated at 98°C for 60 seconds, then held at 20°C. To the tube was added 5.0 microliters of 5.0 picogram/microliter Multimeric Barcoding Reagents Containing Barcoded Oligonucleotides (as produced by Method 4). The reaction was then incubated at 70°C for 60 seconds, then slowly annealed to 60°C, then 60°C for five minutes, then slowly annealed to 55°C, then 55°C for five minutes, then slowly annealed to 50°C, then 50°C for five minutes, then held at 4°C. To the reaction was added 0.5 microliters of Phusion Polymerase (NEB), plus 2.0 microliters 10 uM SynTemp_PE2_B1_Short1 (SEQ ID NO. 277, a primer that is complementary to part of the extension products produced by annealing and extending the multimeric barcoding reagents created by Method 4 along the synthetic DNA templates created by Method 5, serves as a primer for the primer-extension and then PCR reactions described in this method). Of this reaction, a volume of 5.0 microliters was added to a new PCR tube, which was then incubated for 30 seconds at 55°C, 30 seconds 60°C, and 30 seconds 72°C, then followed by 10 cycles of: 98°C then 65°C then 72°C for 30 seconds each, then held at 4°C. To each tube was then added 9.0 microliters 5X Phusion buffer, plus 1.0 microliters 10 millimolar deoxynucleotide triphosphate nucleotide mix, plus 1.75 microliters 10 uM SynTemp_PE2_B1_Short1 (SEQ ID NO. 277), plus 1.75 microliters 10 uM US_PCR_Prm Only_02 (SEQ ID NO. 278, a primer partially complementary to the extension primer employed to generate the multimeric barcoding reagents as per Method 4, and serving as the 'forward' primer in this PCR amplification reaction), plus 0.5 microliters Phusion Polymerase (NEB), plus water to final volume of 50 microliters. The PCR tube was placed on a thermal cycler and amplified for 24 cycles of: 98°C for 30 seconds, then 72°C for 30 seconds; then held at 4°C, then purified with 1.2X volume (60 microliters) Ampure XP Beads (Agencourt; as per manufacturer's instructions), and eluted in 30 microliters H₂O, and quantitated spectrophotometrically.

The resulting library was then barcoded for sample identification by a PCR-based method, amplified, and sequenced by standard methods using a 150-cycle, mid-output NextSeq flowcell (Illumina), and demultiplexed informatically for further analysis.

### Method 7: Barcoding Synthetic DNA Templates of Known Sequence with Multimeric Barcoding Reagents and Separate Adapter Oligonucleotides

To anneal and extend adapter oligonucleotides along the synthetic DNA templates, in a PCR tube were added 10.0 microliters 5X Phusion HF buffer (NEB), plus 1.0 microliters 10 millimolar deoxynucleotide triphosphate nucleotide mix, plus 5.0 microliters (25 nanograms) 5.0 nanogram/ microliters Synthetic DNA Templates of Known Sequence (as produced by Method 5), plus 0.25 microliters of 10 micromolar DS_ST_05 (SEQ ID NO. 273, an adapter oligonucleotide), plus water to final volume of 49.7 microliters. On a thermal cycler, the tube was incubated at 98°C for 2 minutes, then 63°C for 1 minute, then slowly annealed to 60°C then held at 60°C for 1 minute, then slowly annealed to 57°C then held at 57°C for 1 minute, then slowly annealed to 54°C then held at 54°C for 1 minute, then slowly annealed to 50°C then held at 50°C for 1 minute, then slowly annealed to 45°C then held at 45°C for 1 minute, then slowly annealed to 40°C then held at 40°C for 1 minute, then held at 4°C. To the tube was added 0.3 microliters Phusion Polymerase (NEB), and the reaction was incubated at 45°C for 20 seconds, then 50°C for 20 seconds, then 55°C for 20 seconds, 60°C for 20 seconds, then 72°C for 20 seconds, then held at 4°C; the reaction was then purified with 0.8X volume (40 microliters) Ampure XP Beads (Agencourt; as per manufacturer's instructions), and eluted in 30 microliters H₂O, and quantitated spectro photometrically.

In order to anneal adapter oligonucleotides (annealed and extended along the synthetic DNA templates as in the previous step) to multimeric barcode molecules, and then to anneal and then extend extension primer(s) across the adjacent barcode region(s) of each multimeric barcode molecule, and then to ligate this extension product to the phosphorylated 5' end of the adapter oligonucleotide annealed to the downstream thereof, to a PCR tube was added 10 microliters of the eluate from the previous step (containing the synthetic DNA templates along which the adapter oligonucleotides have been annealed and extended), plus 3.0 microliters of a 50.0 nanomolar solution of RNAse H-digested multimeric barcode molecules (as produced in the last step of Method 3), plus 6.0 microliters of 5X Isothermal extension/ligation buffer, plus water to final volume of 26.6 microliters. On a thermal cycler, the tube was incubated at 70°C for 60 seconds, then slowly annealed to 60°C, then held at 60°C for 5 minutes, then slowly annealed to 55°C then held at 55°C for 5 minutes, then slowly annealed to 50°C at 0.1°C/sec then held at 50°C for 30 minutes, then held at 4°C. To the tube was added 0.6 microliters 10 uM US_PCR_Prm Only_02 (SEQ ID NO: 278, an extension primer), and the reaction was incubated at 50°C for 10 minutes, then held at 4°C. To the tube was added 0.3 microliters (0.625U) Phusion Polymerase (NEB; 2 U/uL) 2.5 microliters (100 U) Taq DNA Ligase (NEB; 40 U/uL); and 2.5 microliters 100 milimolar DTT. The tube was then incubated at 50°C for 5 minutes, then held at 4°C. The reaction was then purified with 0.7X volume (21 microliters) Ampure XP Beads (Agencourt; as per manufacturer's instructions), and eluted in 30 microliters H₂O, and quantitated spectro photometrically.

To a new PCR tube was add 25.0 microliters of the eluate, plus 10.0 microliters 5X Phusion HF buffer (NEB), plus 1.0 microliters 10 millimolar deoxynucleotide triphosphate nucleotide mix, plus 2.0 microliters 10 uM SynTemp_PE2_B1_Short1 (SEQ ID NO: 277; a primer that is complementary to part of the extension products produced by the above steps; serves as a primer for the primer-extension and then PCR reactions described here), plus 0.5 uL Phusion Polymerase (NEB), plus water to final volume of 49.7 microliters. Of this reaction, a volume of 5.0 microliters was added to a new PCR tube, which was then incubated for 30 seconds at 55°C, 30 seconds 60°C, and 30 seconds 72°C, then followed by 10 cycles of: 98°C then 65°C then 72°C for 30 seconds each, then held at 4°C. To each tube was then added 9.0 microliters 5X Phusion buffer, plus 1.0 microliters 10 millimolar deoxynucleotide triphosphate nucleotide mix, plus 1.75 microliters 10 uM SynTemp_PE2_B1_Short1 (SEQ ID NO: 277), plus 1.75 microliters 10 uM US_PCR_Prm Only_02 (SEQ ID NO: 278), plus 0.5 microliters Phusion Polymerase (NEB), plus water to final volume of 50 microliters. The PCR tube was placed on a thermal cycler and amplified for 24 cycles of: 98°C for 30 seconds, then 72°C for 30 seconds; then held at 4°C, then purified with 1.2X volume (60 microliters) Ampure XP Beads (Agencourt; as per manufacturer's instructions), and eluted in 30 microliters H₂O, and quantitated spectrophotometrically.

The resulting library was then barcoded for sample identification by a PCR-based method, amplified, and sequenced by standard methods using a 150-cycle, mid-output NextSeq flowcell (Illumina), and demultiplexed informatically for further analysis.

### Method 9: Barcoding Genomic DNA Loci with Multimeric Barcoding Reagents Containing Barcoded Oligonucleotides

This method describes a framework for barcoding targets within specific genomic loci (e.g. barcoding a number of exons within a specific gene) using multimeric barcoding reagents that contain barcoded oligonucleotides. First, a solution of Multimeric Barcode Molecules was produced by In Vitro Transcription and cDNA Synthesis (as described in Method 3). Then, solutions of multimeric barcoding reagents containing barcoded oligonucleotides was produced as described in Method 4, with a modification made such that instead of using an adapter oligonucleotide targeting a synthetic DNA template (i.e. DS_ST_05, SEQ ID NO: 273, as used in Method 4), adapter oligonucleotides targeting the specific genomic loci were included at that step. Specifically, a solution of multimeric barcoding reagents containing appropriate barcoded oligonucleotides was produced individually for each of three different human genes: BRCA1 (containing 7 adapter oligonucleotides, SEQ ID NOs 279-285), HLA-A (containing 3 adapter oligonucleotides, SEQ ID NOs 286-288), and DQB1 (containing 2 adapter oligonucleotides, SEQ ID NOs 289-290). The process of Method 4 was conducted for each of these three solutions as described above. These three solutions were then merged together, in equal volume, and diluted to a final, total concentration all barcoded oligonucleotides of approximately 50 nanomolar.

In a PCR tube were plus 2.0 microliters 5X Phusion HF buffer (NEB), plus 1.0 microliter of 100 nanogram/microliter human genomic DNA (NA12878 from Coriell Institute) to final volume of 9.0 microliters. In certain variant versions of this protocol, the multimeric barcoding reagents (containing barcoded oligonucleotides) were also added at this step, prior to the high-temperature 98°C incubation. The reaction was incubated at 98°C for 120 seconds, then held at 4°C. To the tube was added 1.0 microliters of the above 50 nanomolar solution of multimeric barcode reagents, and then the reaction was incubated for 1 hour at 55°C, then 1 hour at 50°C, then 1 hour at 45°C, then held at 4°C. (Note that for certain samples, this last annealing process was extended to occur overnight, for a total of approximately 4 hours per temperature step).

In order to add a reverse universal priming sequence to each amplicon sequence (and thus to enable subsequent amplification of the entire library at once, using just one forward and one reverse amplification primer), the reaction was diluted 1:100, and 1.0 microliter of the resulting solution was added in a new PCR tube to 20.0 microliters 5X Phusion HF buffer (NEB), plus 2.0 microliters 10 millimolar deoxynucleotide triphosphate nucleotide mix, plus 1.0 microliters a reverse-primer mixture (equimolar concentration of SEQ ID Nos 291-303, each primer at 5 micromolar concentration), plus 1.0 uL Phusion Polymerase (NEB), plus water to final volume of 100 microliters. The reaction was incubated at 53°C for 30 seconds, 72°C for 45 seconds, 98°C for 90 seconds, then 68°C for 30 seconds, then 64°C for 30 seconds, then 72°C for 30 seconds; then held at 4°C. The reaction was then purified with 0.8X volume (80 microliters) Ampure XP Beads (Agencourt; as per manufacturer's instructions), and eluted in 30 microliters H₂O, and quantitated spectrophotometrically.

The resulting library was then barcoded for sample identification by a PCR-based method, amplified, and sequenced by standard methods using a 150-cycle, mid-output NextSeq flowcell (Illumina), and demultiplexed informatically for further analysis.

### Method 10 - Sequencing the Library of Multimeric Barcode Molecules

### Preparing Amplified Selected Molecules for Assessment with High-Throughput Sequencing

To a PCR tube was added 1.0 microliters of the amplified selected molecule solution, plus 1.0 microliters of 100 micromolar CS_SQ_AMP_REV1 (SEQ ID NO: 16), plus 1.0 microliters of 100 micromolar US_PCR_Prm Only_02 (SEQ ID NO: 17), plus 10 microliters of 10X Thermopol Buffer (NEB) plus 2.0 microliter of 10 millimolar deoxynucleotide triphosphate nucleotide mix (Invitrogen), plus 1.0 microliters Vent Exo-Minus Polymerase (New England Biolabs, at 2U/uL) plus 84.0 microliters H₂O to final volume of 100 microliters. The PCR tube was placed on a thermal cycler and amplified for 3 cycles of: 95°C for 30 seconds, then 56°C for 30 seconds, then 72°C for 3 minutes; then held at 4°C. The solution was then purified with 0.8X volume (80 microliters) Ampure XP Beads (Agencourt; as per manufacturer's instructions), and eluted in 85 microliters H₂O.

This solution was then added to a new PCR tube, plus 1.0 microliters of 100 micromolar Illumina_PE1, plus 1.0 microliters of 100 micromolar Illumina_PE2, plus 10 microliters of 10X Thermopol Buffer (NEB) plus 2.0 microliter of 10 millimolar deoxynucleotide triphosphate nucleotide mix (Invitrogen), plus 1.0 microliters Vent Exo-Minus Polymerase (New England Biolabs, at 2U/uL) to final volume of 100 microliters. The PCR tube was placed on a thermal cycler and amplified for 4 cycles of: 95°C for 30 seconds, then 64°C for 30 seconds, then 72°C for 3 minutes; then 18 cycles of: 95°C for 30 seconds, then 67°C for 30 seconds, then 72°C for 3 minutes; then held at 4°C. The solution was then purified with 0.8X volume (80 microliters) Ampure XP Beads (Agencourt; as per manufacturer's instructions), and eluted in 40 microliters H₂O.

High-throughput Illumina sequencing was then performed on this sample using a MiSeq sequencer with paired-end, 250-cycle V2 sequencing chemistry.

### Method 11 - Assessment of Multimeric Nature of Barcodes Annealed and Extended Along Single Synthetic Template DNA Molecules

A library of barcoded synthetic DNA templates was created using a solution of multimeric barcoding reagents produced according to a protocol as described generally in Method 3 and Method 4, and using a solution of synthetic DNA templates as described in Method 5, and using a laboratory protocol as described in Method 6; the resulting library was then barcoded for sample identification by a PCR-based method, amplified, and sequenced by standard methods using a 150-cycle, mid-output NextSeq flowcell (Illumina), and demultiplexed informatically for further analysis. The DNA sequencing results from this method were then compared informatically with data produced from Method 10 to assess the degree of overlap between the multimeric barcoding of synthetic DNA templates and the arrangement of said barcodes on individual multimeric barcoding reagents (the results are shown in Figure 17).

### RESULTS

### Structure and Expected Sequence Content of Each Sequence Multimeric Barcoding Reagent Molecule

The library of multimeric barcode molecules synthesised as described in Methods 1 to 3 was prepared for high-throughput sequencing, wherein each molecule sequenced includes a contiguous span of a specific multimeric barcode molecule (including one or more barcode sequences, and one or more associate upstream adapter sequences and/or downstream adapter sequences), all co-linear within the sequenced molecule. This library was then sequenced with paired-end 250 nucleotide reads on a MiSeq sequencer (Illumina) as described. This yielded approximately 13.5 million total molecules sequenced from the library, sequenced once from each end, for a total of approximately 27 million sequence reads.

Each forward read is expected to start with a six nucleotide sequence, corresponding to the 3' end of the upstream adapter: TGACCT

This forward read is followed by the first barcode sequence within the molecule (expected to be 20 nt long).

This barcode is then followed by an 'intra-barcode sequence' (in this case being sequenced in the 'forward' direction (which is 82 nucleotides including both the downstream adapter sequence and upstream adapter sequence in series):

ATACCTGACTGCTCGTCAGTTGAGCGAATTCCGTATGGTGGTACACACCTACACTACTCGGA
CGCTCTTCCGATCTTGACCT

Within the 250 nucleotide forward read, this will then be followed by a second barcode, another intra-barcode sequence, and then a third barcode, and then a fraction of another intra-barcode sequence.

Each reverse read is expected to start with a sequence corresponding to the downstream adapter sequence: GCTCAACTGACGAGCAGTCAGGTAT

This reverse read is then followed by the first barcode coming in from the opposite end of the molecule (also 20 nucleotides long, but sequenced from the opposite strand of the molecule and thus of the inverse orientation to those sequenced by the forward read)

This barcode is then followed by the 'intra-barcode sequence' but in the inverse orientation (as it is on the opposite strand):

AGGTCAAGATCGGAAGAGCGTCCGAGTAGTGTAGGTGTGTACCACCATACGGAATTCGCTC
AACTGACGAGCAGTCAGGTAT

Likewise this 250 nucleotide reverse read will then be followed by a second barcode, another intra-barcode sequence, and then a third barcode, and then a fraction of another intra-barcode sequence.

### Sequence Extraction and Analysis

With scripting in Python, each associated pair of barcode and flanking upstream-adapter and downstream-adapter sequence were isolated, with each individual barcode sequence of each barcode molecule then isolated, and each barcode sequence that was sequenced within the same molecule being annotated as belonging to the same multimeric barcode molecule in the library of multimeric barcode molecules. A simple analysis script (Networkx; Python) was employed to determine overall multimeric barcode molecule barcode groups, by examining overlap of barcode-barcode pairs across different sequenced molecules. Several metrics of this data were made, including barcode length, sequence content, and the size and complexity of the multimeric barcode molecules across the library of multimeric barcode molecules.

### Number of Nucleotides within Each Barcode Sequence

Each individual barcode sequence from each barcode molecule, contained within each Illumina-sequenced molecule was isolated, and the total length of each such barcode was determined by counting the number of nucleotides between the upstream adapter molecule sequence, and the downstream adapter molecule sequence. The results are shown in Figure 10.

The overwhelming majority of barcodes are 20 nucleotides long, which corresponds to five additions of our four-nucleotide-long sub-barcode molecules from our double-stranded sub-barcode library. This is thus the expected and desired result, and indicates that each 'cycle' of: Ligation of Sub-Barcode Library to Mlyl-Cleaved Solution, PCR Amplification of the Ligated Library, Uracil Glycosylase Enzyme Digestion, and Mlyl Restriction Enzyme Cleavage, was successful and able to efficiently add new four-nucleotide sub-barcode molecules at each cycle, and then was successfully able to amplify and carry these molecules forward through the protocol for continued further processing, including through the five total cycles of sub-barcode addition, to make the final, upstream-adapter-ligated libraries.

We also used this sequence analysis method to quantitate the total number of unique barcodes in total, across all sequenced multimeric barcode molecules: this amounted to 19,953,626 total unique barcodes, which is essentially identical to the 20 million barcodes that would be expected, given that we synthesised 2 million multimeric barcode molecules, each with approximately 10 individual barcode molecules.

Together, this data and analysis thus shows that the methods of creating complex, combinatoric barcodes from sub-barcode sequences is effective and useful for the purpose of synthesising multimeric barcode molecules.

### Total Number of Unique Barcode Molecules in Each Multimeric Barcode Molecule

Figure 11 shows the results of the quantification of the total number of unique barcode molecules (as determined by their respective barcode sequences) in each sequenced multimeric barcode molecule. As described above, to do this we examined, in the first case, barcode sequences which were present and detected within the same individual molecules sequenced on the sequencer. We then employed an additional step of clustering barcode sequences further, wherein we employed a simple network analysis script (Networkx) which can determine links between individual barcode sequences based both upon explicit knowledge of links (wherein the barcodes are found within the same, contiguous sequenced molecule), and can also determine `implicit' links, wherein two or more barcodes, which are not sequenced within the same sequenced molecule, instead both share a direct link to a common, third barcode sequence (this shared, common link thus dictating that the two first barcode sequences are in fact located on the same multimeric barcode molecule).

This figure shows that the majority of multimeric barcode molecules sequenced within our reaction have two or more unique barcodes contained therein, thus showing that, through our Overlap-Extension PCR linking process, we are able to link together multiple barcode molecules into multimeric barcode molecules. Whilst we would expect to see more multimeric barcode molecules exhibiting closer to the expected number of barcode molecules (10), we expect that this observed effect is due to insufficiently high sequencing depth, and that with a greater number of sequenced molecules, we would be able to observe a greater fraction of the true links between individual barcode molecules. This data nonetheless suggest that the fundamental synthesis procedure we describe here is efficacious for the intended purpose.

### Representative Multimeric Barcode Molecules

Figure 12 shows representative multimeric barcode molecules that have been detected by our analysis script. In this figure, each 'node' is a single barcode molecule (from its associated barcode sequence), each line is a 'direct link' between two barcode molecules that have been sequenced at least once in the same sequenced molecule, and each cluster of nodes is an individual multimeric barcode molecule, containing both barcodes with direct links and those within implicit, indirect links as determined by our analysis script. The inset figure includes a single multimeric barcode molecule, and the sequences of its constituent barcode molecules contained therein.

This figure illustrates the our multimeric barcode molecule synthesis procedure: that we are able to construct barcode molecules from sub-barcode molecule libraries, that we are able to link multiple barcode molecules with an overlap-extension PCR reaction, that we are able to isolate a quantitatively known number of individual multimeric barcode molecules, and that we are able to amplify these and subject them to downstream analysis and use.

### Barcoding Synthetic DNA Templates of Known Sequence with (i) Multimeric Barcoding Reagents Containing Barcoded Oligonucleotides, and (ii) Multimeric Barcoding Reagents and Separate Adapter Oligonucleotides

### Sequence Extraction and Analysis

With scripting in Python and implemented in an Amazon Web Services (AWS) framework, for each sequence read following sample-demultiplexing, each barcode region from the given multimeric barcode reagent was isolated from its flanking upstream-adapter and downstream-adapter sequence. Likewise, each molecular sequence identifier region from the given synthetic DNA template molecule was isolated from its flanking upstream and downstream sequences. This process was repeated for each molecule in the sample library; a single filtering step was performed in which individual barcodes and molecular sequence identifiers that were present in only a single read (thus likely to represent either sequencing error or error from the enzymatic sample-preparation process) were censored from the data. For each molecular sequence identifier, the total number of unique (ie with different sequences) barcode regions found associated therewith within single sequence reads was quantitated. A histogram plot was then created to visualize the distribution of this number across all molecular sequence identifiers found in the library.

### Discussion

Figure 13 shows the results of this analysis for Method 6 (Barcoding Synthetic DNA Templates of Known Sequence with Multimeric Barcoding Reagents Containing Barcoded Oligonucleotides). This figure makes clear that the majority of multimeric barcoding reagents are able to successfully label two or more of the tandemly-repeated copies of each molecular sequence identifier with which they are associated. A distribution from 1 to approximately 5 or 6 `labelling events' is observed, indicating that there may be a degree of stochastic interactions that occur with this system, perhaps due to incomplete enzymatic reactions, or steric hindrance at barcode reagent/synthetic template interface, or other factors.

Figure 14 shows the results of this same analysis conducted using Method 7 (Barcoding Oligonucleoitdes Synthetic DNA Templates of Known Sequence with Multimeric Barcode Molecules and Separate Adapter Oligonucleotides). This figure also clearly shows that the majority of multimeric barcoding reagents are able to successfully label two or more of the tandemly-repeated copies of each molecular sequence identifier with which they are associated, with a similar distribution to that observed for the previous analysis.

Together, these two figures show that this framework for multimeric molecular barcoding is an effective one, and furthermore that the framework can be configured in different methodologic ways. Figure 13 shows results based on a method in which the framework is configured such that the multimeric barcode reagents already contain barcoded oligonucleotides, prior to their being contacted with a target (synthetic) DNA template. In contrast, Figure 14 shows results based on an alternative method in which the adapter oligonucleotides first contact the synthetic DNA template, and then in a subsequent step the adapter oligonucleotides are barcoded through contact with a multimeric barcode reagent. Together these figures demonstrate both the multimeric barcoding ability of these reagents, and their versatility in different key laboratory protocols.

To analyse whether, and the extent to which, individual multimeric barcoding reagents successfully label two or more sub-sequences of the same synthetic DNA template, the groups of different barcodes on each individual multimeric barcoding reagent in the library (as predicted from the Networkx analysis described in the preceding paragraph and as illustrated in Figure 12) was compared with the barcodes annealed and extended along single synthetic DNA templates (as described in Method 11). Each group of barcodes found on individual multimeric barcoding reagents was given a numeric 'reagent identifier label'. For each synthetic DNA template molecular sequence identifier (i.e., for each individual synthetic DNA template molecule) that was represented in the sequencing data of Method 11 by two or more barcodes (i.e., wherein two or more sub-sequences of the synthetic template molecule were annealed and extended by a barcoded oligonucleotide), the corresponding 'reagent identifier label' was determined. For each such synthetic template molecule, the total number of multimeric barcodes coming from the same, single multimeric barcoding reagent was then calculated (i.e., the number of different sub-sequences in the synthetic template molecule that were labeled by a *different* barcoded oligonucleotide but from the *same,* single multimeric barcoding reagent was calculated). This analysis was then repeated and compared with a 'negative control' condition, in which the barcodes assigned to each 'reagent identifier label' were randomized (i.e. the same barcode sequences remain present in the data, but they no longer correspond to the actual molecular linkage of different barcode sequences across the library of multimeric barcoding reagents).

The data from this analysis is shown in Figure 17, for both the actual experimental data and for the control data with randomized barcode assignments (note the logarithmic scale of the vertical axis). As this figure shows, though the number of unique barcoding events per target synthetic DNA template molecule is small, they overlap almost perfectly with the known barcode content of individual multimeric barcoding reagents. That is, when compared with the randomized barcode data (which contains essentially no template molecules that appear to be `multivalently barcoded'), the overwhelming majority (over 99.9%) of template molecules in the actual experiment that appear to be labeled by multiple barcoded oligonucleotides from the same, individual multimeric barcoding reagent, are in fact labeled multiply by the same, single reagents in solution. By contrast, if there were no non-random association between the different barcodes that labelled individual synthetic DNA templates (that is, if Figure 17 showed no difference between the actual experimental data and the randomized data), then this would have indicated that the barcoding had not occurred in a spatially-constrained manner as directed by the multimeric barcoding reagents. However, as explained above, the data indicates convincingly that the desired barcoding reactions did occur, in which sub-sequences found on single synthetic DNA templates interacted with (and were then barcoded by) only single, individual multimeric barcoding reagents.

### Barcoding Genomic DNA Loci with Multimeric Barcoding Reagents Containing Barcoded Oligonucleotides

### Sequence Extraction and Analysis

As with other analysis, scripting was composed in Python and implemented in an Amazon Web Services (AWS) framework. For each sequence read following sample-demultiplexing, each barcode region from the given multimeric barcode reagent was isolated from its flanking upstream-adapter and downstream-adapter sequence and recorded independently for further analysis. Likewise, each sequence to the 3' end of the downstream region (representing sequence containing the barcoded oligonucleotide, and any sequences that the oligonucleotide had primed along during the experimental protocol) was isolated for further analysis. Each downstream sequence of each read was analysed for the presence of expected adapter oligonucleotide sequences (i.e. from the primers corresponding to one of the three genes to which the oligonucleotides were directed) and relevant additional downstream sequences. Each read was then recorded as being either 'on-target' (with sequence corresponding to one of the expected, targeted sequence) or 'off-target'. Furthermore, for each of the targeted regions, the total number of unique multimeric barcodes (i.e. with identical but duplicate barcodes merged into a single-copy representation) was calculated. A schematic of each expected sequence read, and the constituent components thereof, is shown in Figure 16.

### Discussion

Figure 15 shows the results of this analysis for this method, for four different independent samples. These four samples represent a method wherein the process of annealing the multimeric barcode reagents took place for either 3 hours, or overnight (approximately 12 hours). Further, for each of these two conditions, the method was performed either with the multimeric barcode reagents retained intact as originally synthesized, or with a modified protocol in which the barcoded oligonucleotides are first denatured away from the barcode molecules themselves (through a high-temperature melting step). Each row represents a different amplicon target as indicated, and each cell represents the total number of unique barcode found associated with each amplicon in each of the four samples. Also listed is the total proportion of on-target reads, across all targets summed together, for each sample.

As seen in the figure, the majority of reads across all samples are on-target; however there is seen a large range in the number of unique barcode molecules observed for each amplicon target. These trends across different amplicons seem to be consistent across the different experimental conditions, and could be due to different priming (or mis-priming) efficiencies of the different oligonucleotides, or different amplification efficiencies, or different mapping efficiencies, plus potential other factors acting independently or in combination. Furthermore, it is clear that the samples that were annealed for longer have a larger number of barcodes observed, likely due to more complete overall annealing of the multimeric reagents to their cognate genomic targets. And furthermore, the samples where the barcoded oligonucleotides were first denatured from the barcode molecules show lower overall numbers of unique barcodes, perhaps owing to an avidity effect wherein fully assembled barcode molecules can more effectively anneal clusters of primers to nearby genomic targets at the same locus. In any case, taken together, this figure illustrates the capacity of multimeric reagents to label genomic DNA molecules, across a large number of molecules simultaneously, and to do so whether the barcoded oligonucleotides remain bound on the multimeric barcoding reagents or whether they have been denatured therefrom and thus potentially able to diffuse more readily in solution.

### Experimental Method for Barcoding Fragments of Genomic DNA from FFPE Samples with Multimeric Barcoding Reagents Containing Barcoded Oligonucleotides

An FFPE specimen (e.g. an FFPE specimen from a tissue biopsy of a cancer patient) is cut into 5 sections 5 microns to 10 microns thick, and then the paraffin is depleted from the sample with a xylene-dissolution step as described in the manufacturer's instructions of the QIAamp DNA FFPE Tissue Kit (Qiagen), then centrifuged to pellet the de-paraffinised sample, and then washed with 100% ethanol to remove residual xylene, and then centrifuged again, the ethanol is aspirated, and any remaining ethanol is allowed to evaporate as per manufacturer's instructions. The sample is then resuspended in 180 microliters Buffer ATL and digested with 5.0 microliter Proteinase K at 50 degrees Celsius for 15 minutes. The sample is then pelleted at top speed on a desktop microcentrifuge for 5 minutes, and the solution is aspirated. The pellet is then resuspended in NEBNext Ultra II End Prep Reaction Buffer (New England Biolabs; e.g. 20.0 microliters of 1.1X Reaction Buffer) and fragments of genomic DNA from the sample are blunted and A-Tailed with the NEBNext Ultra II End Prep Enzyme Mix (New England Biolabs) at 20 degrees Celsius for 30 minutes, followed by heat-inactivation of the enzyme mix at 65 degrees Celsius for 30 minutes (as per the manufacturer's instructions).

A library of at least 10 million different multimeric barcoding reagents is then added to and mixed with the sample solution, wherein each multimeric barcoding reagent is a contiguous multimeric barcode molecule made of 10-30 individual barcode molecules, with each barcode molecule comprising a barcode region with a different sequence from the other barcode molecules on that multimeric barcoding reagent, and with a barcoded oligonucleotide annealed to each barcode molecule, wherein each barcoded oligonucleotide comprises the full forward (read 1) Illumina sequencing primer and adapter sequence on its 5' end, and a double-stranded target region with a single 3' thymine overhang nucleotide (i.e a T-overhang, able to ligate to a 3'-A overhang DNA molecule from an A-Tailing reaction) at its 3' end.

An appropriate volume of 'NEBNext Ultra II Ligation Master Mix', and 'NEBNext Ligation Enhancer' (New England Biolabs) (as per manufacturer's instructions) are then added to the solution, and then the samples are incubated at 20 degrees Celsius for 120 on a thermal cycler with the heated lid turned off. An appropriate volume of 'NEBNext Adapter' (New England Biolabs) is then added to the sample, and the solutions are mixed gently; the solutions were then incubated at 20 degrees Celsius for 60 minutes on a thermal cycler with the heated lid turned off. To each tube is then added 'NEBNext USER Enzyme', and the solutions are mixed gently; the solutions are then incubated at 20 degrees Celsius for 20 minutes at 37 degrees Celsius for 30 minutes on a thermal cycler with a heated lid set to 50 degrees Celsius, and then held at 4 degrees Celsius. To the sample is then added 180 microliters Buffer ATL and 20.0 microliter Proteinase K, and proteinase-digested at 56 degrees Celsius for 120 minutes using manufacturer's instructions from the QIAamp DNA FFPE Tissue Kit (Qiagen). The sample is then incubated at 90 degrees Celsius for 60 minutes, and added to 200 microliters Buffer AL (Qiagen), and bound to and washed with QIAamp MinElute columns and eluted in 100 microliters Buffer ATE (all from QIAamp DNA FFPE Tissue Kit; Qiagen). The entire resulting eluted sample is then amplified with PCR using the standard Illumina PCR primers to yield 100-1000 ng total DNA following cleanup with 1.2X-volume Ampure XP SPRI beads (Agencourt; as per manufacturer's instructions), and then sequenced to an appropriate depth (e.g. at least 100 million total reads) on a standard Illumina Sequencer. Raw sequences may then be quality-trimmed and length-trimmed, constant adapter/primer sequences are trimmed away, and the genomic DNA sequences and barcode sequences from each retained sequence read are isolated informatically. Linked sequences are determined by detecting genomic DNA sequences that are appended different barcode sequences from the same set of barcode sequences (i.e. from the same multimeric barcoding reagent).

## Claims

1. A method of preparing two or more independent nucleic acid samples for sequencing, wherein each of the nucleic acid samples is prepared using a different library of multimeric barcoding reagents, and wherein each nucleic acid sample comprises a formalin-fixed paraffin-embedded (FFPE) sample, and wherein each nucleic acid sample is prepared for sequencing with a method comprising the steps of:
(a) contacting the nucleic acid sample with a library of multimeric barcoding reagents, wherein the library comprises first and second multimeric barcoding reagents, wherein each multimeric barcoding reagent comprises first and second different barcode regions linked together, wherein each barcode region comprises a nucleic acid sequence, and wherein the first and second barcode regions of the first multimeric barcoding reagent are different to the first and second barcode regions of the second multimeric barcoding reagent, and
(b) appending barcode sequences from the first multimeric barcoding reagent to each of the nucleic acid sequences of first and second fragments of a first target nucleic acid molecule to produce first and second different barcoded target nucleic acid molecules, wherein the first barcoded target nucleic acid molecule comprises the nucleic acid sequence of the first barcode region of the first multimeric barcoding reagent and the second barcoded target nucleic acid molecule comprises the nucleic acid sequence of the second barcode region of the first multimeric barcoding reagent, and appending barcode sequences from the second multimeric barcoding reagent to each of the nucleic acid sequences of first and second fragments of a second target nucleic acid molecule to produce third and fourth different barcoded target nucleic acid molecules, wherein the third barcoded target nucleic acid molecule comprises the nucleic acid sequence of the first barcode region of the second multimeric barcoding reagent and the fourth barcoded target nucleic acid molecule comprises the nucleic acid sequence of the second barcode region of the second multimeric barcoding reagent,
wherein prior to and/or during step (a) and/or prior to and/or during step (b), the method further comprises the step of removing or depleting all or a fraction of the paraffin from the nucleic acid sample,
and wherein the barcode regions of each library of multimeric barcoding reagents comprise a sequence that is different to the barcode regions of the other libraries of multimeric barcoding reagents.

2. The method of claim 1, wherein the first and second target nucleic acid molecules are genomic DNA.

3. The method of claim 1, wherein the first and second target nucleic acid molecules are mRNA.

4. The method of any one of claims 1-3, wherein the first and second multimeric barcoding reagents each comprise first and second barcode molecules linked together, and wherein each of the barcode molecules comprises a nucleic acid sequence comprising a barcode region.

5. The method of any one of claims 1-4, wherein the first and second multimeric barcoding reagents each comprise first and second barcoded oligonucleotides, wherein the first barcoded oligonucleotide comprises, optionally in the 5' to 3' direction, a barcode region, and a target region capable of annealing or ligating to the first fragment of the target nucleic acid, and wherein the second barcoded oligonucleotide comprises, optionally in the 5' to 3' direction, a barcode region, and a target region capable of annealing or ligating to the second fragment of the target nucleic acid.

6. The method of any one of claims 1-5, wherein the first and second multimeric barcoding reagents each comprise:
(i) first and second barcode molecules linked together, wherein each of the barcode molecules comprises a nucleic acid sequence comprising a barcode region; and
(ii) first and second barcoded oligonucleotides, wherein the first barcoded oligonucleotide comprises, optionally in the 5' to 3' direction, a barcode region, and a target region capable of annealing or ligating to the first fragment of the target nucleic acid, and wherein the second barcoded oligonucleotide comprises, optionally in the 5' to 3' direction, a barcode region, and a target region capable of annealing or ligating to the second fragment of the target nucleic acid.

7. The method of claim 5 or claim 6, wherein each nucleic acid sample is prepared for sequencing with the method comprising:
(a) contacting the nucleic acid sample with a library of multimeric barcoding reagents as defined in claim 5 or claim 6;
(b) for each of the first and second multimeric barcoding reagents in the library, ligating the target region of the first barcoded oligonucleotide to the first fragment of a target nucleic acid molecule to produce a first barcoded target nucleic acid molecule, and ligating the target region of the second barcoded oligonucleotide to the second fragment of the target nucleic acid molecule to produce a second barcoded target nucleic acid molecule, wherein each of the barcoded target nucleic acid molecules comprises at least one nucleotide synthesised from the target nucleic acid as a template.

8. The method of claim 5 or claim 6, wherein each nucleic acid sample is prepared for sequencing with the method comprising:
(a) contacting the nucleic acid sample with a library of multimeric barcoding reagents as defined in claim 5 or claim 6;
(b) for each of the first and second multimeric barcoding reagents in the library, annealing the target region of the first barcoded oligonucleotide to the first fragment of a target nucleic acid, and annealing the target region of the second barcoded oligonucleotide to the second fragment of the target nucleic acid; and
(c) for each of the first and second multimeric barcoding reagents in the library, extending the first and second barcoded oligonucleotides to produce first and second different barcoded target nucleic acid molecules, wherein each of the barcoded target nucleic acid molecules comprises at least one nucleotide synthesised from the target nucleic acid as a template.

9. The method of claim 1 or claim 2, wherein each nucleic acid sample is prepared for sequencing with the method comprising:
(a) appending a coupling sequence to each of first and second fragments of at least first and second target nucleic acid molecules;
(b) contacting the sample with a library of multimeric barcoding reagents, wherein the library comprises first and second multimeric barcoding reagents, wherein each multimeric barcoding reagent comprises first and second barcode molecules linked together, wherein each of the barcode molecules comprises a nucleic acid sequence comprising a barcode region and an adapter region, wherein each multimeric barcoding reagent comprises first and second different barcode regions linked together, and wherein the first and second barcode regions of the first multimeric barcoding reagent are different to the first and second barcode regions of the second multimeric barcoding reagent;
(c) (i) for the first target nucleic acid molecule, annealing the coupling sequence of the first fragment to the adapter region of the first barcode molecule of the first multimeric barcoding reagent, and annealing the coupling sequence of the second fragment to the adapter region of the second barcode molecule of the first multimeric barcoding reagent, and (ii) for the second target nucleic acid molecule, annealing the coupling sequence of the first fragment to the adapter region of the first barcode molecule of the second multimeric barcoding reagent, and annealing the coupling sequence of the second fragment to the adapter region of the second barcode molecule of the second multimeric barcoding reagent; and
(d) (i) appending barcode sequences to the first and second fragments of the first target nucleic acid molecule to produce first and second different barcoded target nucleic acid molecules, wherein the first barcoded target nucleic acid molecule comprises the nucleic acid sequence of the barcode region of the first barcode molecule of the first multimeric barcoding reagent and the second barcoded target nucleic acid molecule comprises the nucleic acid sequence of the barcode region of the second barcode molecule of the first multimeric barcoding reagent, and (ii) appending barcode sequences to the first and second fragments of the second target nucleic acid molecule to produce third and fourth different barcoded target nucleic acid molecules, wherein the third barcoded target nucleic acid molecule comprises the nucleic acid sequence of the barcode region of the first barcode molecule of the second multimeric barcoding reagent and the fourth barcoded target nucleic acid molecule comprises the nucleic acid sequence of the barcode region of the second barcode molecule of the second multimeric barcoding reagent.

10. The method of claim 1 or claim 9, wherein the first and second multimeric barcoding reagents each comprise:
(i) first and second barcode molecules linked together, wherein each of the barcode molecules comprises a nucleic acid sequence comprising, optionally in the 5' to 3' direction, an adapter region and a barcode region, and
(ii) first and second barcoded oligonucleotides, wherein the first barcoded oligonucleotide comprises a barcode region annealed to the barcode region of the first barcode molecule, and wherein the second barcoded oligonucleotide comprises a barcode region annealed to the barcode region of the second barcode molecule.

11. The method of any one of claims1-10, wherein each nucleic acid sample is prepared for sequencing with the method which further comprises the step of partially or fully removing crosslinks from the nucleic acid sample.

12. The method of any one of claims 1-11, wherein each nucleic acid sample is prepared for sequencing with the method which further comprises the step of proteinase digestion of the nucleic acid sample.

13. The method of any one of claims 1-12, wherein the barcoded target nucleic acid molecules prepared from the two or more independent nucleic acid samples are pooled and sequenced together.
